# EUROPEAN PATENT SPECIFICATION

(11) **EP 2 361 260 B1**
(45) Date of publication and mention of the grant of the patent: **17.08.2016**
(21) Application number: 09801170.3
(22) Date of filing: 22.12.2009
(51) Int. Cl.: C07K 14/16, A61K 47/48

(54) **NOVEL TRANSPORTER CONSTRUCTS AND TRANSPORTER CARGO CONJUGATE MOLECULES**
NEUE TRANSPORTPRODUKTE UND TRANSPORTER-LADUNG KONJUGATE MOLEKÜLEN
NOUVEAUX COMPOSÉS TRANSPORTEURS ET CONJUGUÉS DE CES PEPTIDES TRANSPORTEURS AVEC UNE CARGAISON

(30) Priority: 22.12.2008 WO PCT/EP2008/011003; 02.06.2009 WO PCT/EP2009/003927
(43) Date of publication of application: 31.08.2011
(73) Proprietor: Xigen Inflammation Ltd., 3030 Limassol (CY)
(72) Inventor: BONNY, Christophe, CH-1006 Lausanne (CH)
(74) Representative: Graf von Stosch, Andreas
(86) International application number: PCT/EP2009/009229
(87) International publication number: WO 2010/072406

(56) References cited:
- WO-A2-02/065986
- US-B1- 6 740 524
- Y HAYASHI ET AL.: "Development of oligoarginine-drug conjugates linked to new peptidic self-cleavable spacers toward effective intestinal absorption" BIOORGANIC & MEDICINAL CHEMISTRY LETTERS, vol. 17, no. 18, 15 September 2007 (2007-09-15), pages 5129-5132, XP22206837 Pergamon, Elsevier Science ISSN: 0960-894X
- I NEUNDORF ET AL.: "Detailed analysis concerning the biodistribution and metabolism of human calcitonin-derived cell-pnetrating peptides" BIOCONJUGATE CHEMISTRY., vol. 19, no. 8, August 2008 (2008-08), pages 1596-1603, XP002575961 ACS, WASHINGTON, DC. ISSN: 1043-1802

### SEQUENCE LISTING

<110> xi-gen S.A.
<120> Novel Transporter Constructs and Transporter Cargo Conjugate Molecules
<130> CX01P027WO1
<150> PCT/EP2008/011003
   <151> 2008-12-22
<160> 262
<170> Patentin version 3.5
<210> 1
   <211> 9
   <212> PRT
   <213> Artificial sequence
<220>
   <223> Description of artificial sequence: generic formula (I) DlLLLXDm(LLLyDn)a
<220>
   <221> VARIANT
   <222> (1) .. (9)
   <223> /replace="Any amino acid"
<220>
   <221> REPEAT
   <222> (1)..(1)
   <223> number of repeats: 1 or 2
<220>
   <221> REPEAT
   <222> (1)..(1)
   <223> number of repeats: 1 or 2
<220>
   <221> VARIANT
   <222> (1)..(1)
   <223> /replace="D-amino acid"
<220>
   <221> REPEAT
   <222> (4)..(4)
   <223> number of repeats: 0, 1 or 2
<220>
   <221> REPEAT
   <222> (5)..(5)
   <223> number of repeats: 1 or 2
<220>
   <221> VARIANT
   <222> (5)..(5)
   <223> /replace="D-amino acid"
<220>
   <221> REPEAT
   <222> (6)..(9)
   <223> number of repeats: 0, 1, 2 or 3
<220>
   <221> REPEAT
   <222> (8)..(8)
   <223> number of repeats: 0, 1 or 2
<220>
   <221> REPEAT
   <222> (9)..(9)
   <223> number of repeats: 1 or 2
<220>
   <221> VARIANT
   <222> (9)..(9)
   <223> /replace="D-amino acid"
<400> 1
   Xaa Xaa Xaa Xaa Xaa Xaa Xaa Xaa Xaa
   1 5
<210> 2
   <211> 5
   <212> PRT
   <213> Artificial Sequence
<220>
   <223> Description of artificial sequence: generic subformula (Ia) DILLLxDm
<220>
   <221> VARIANT
   <222> (1)..(5)
   <223> /replace="any amino acid"
<220>
   <221> VARIANT
   <222> (1)..(1)
   <223> /replace="amino acid is D-amino acid"
<220>
   <221> REPEAT
   <222> (1)..(1)
   <223> number of repeats is 1 or 2
<220>
   <221> REPEAT
   <222> (4)..(4)
   <223> number of repeats is 0, 1 or 2
<220>
   <221> VARIANT
   <222> (5)..(5)
   <223> /replace="amino acid is D-amino acid"
<220>
   <221> REPEAT
   <222> (5)..(5)
   <223> number of repeats is 1 or 2
<400> 2
   Xaa Xaa Xaa Xaa Xaa
   1 5
<210> 3
   <211> 9
   <212> PRT
   <213> Artificial sequences
<220>
   <223> Description of artificial sequence: generic subformula (Ib) DILLLxDmLLLyDn
<220>
   <221> VARIANT
   <222> (1)..(9)
   <223> /replace="any amino acid"
<220>
   <221> VARIANT
   <222> (1)..(1)
   <223> /replace="D-amino acid""
<220>
   <221> REPEAT
   <222> (1)..(1)
   <223> number of repeats is 1 or 2
<220>
   <221> REPEAT
   <222> (4)..(4)
   <223> number of repeats is 0, 1 or 2
<220>
   <221> VARIANT
   <222> **(**5**)..(**5**)**
   <223> /replace="D-amino acid""
<220>
   <221> REPEAT
   <222> (5)..(5)
   <223> number of repeats is 1 or 2
<220>
   <221> REPEAT
   <222> (8)..(8)
   <223> number of repeats is 0, 1 or 2
<220>
   <221> VARIANT
   <222> (9)..(9)
   <223> /replace="D-amino acid""
<220>
   <221> REPEAT
   <222> (9)..(9)
   <223> number of repeats is 1 or 2
<400> 3
   Xaa Xaa Xaa Xaa Xaa Xaa Xaa Xaa Xaa
   1               5
<210> 4
   <211> 13
   <212> PRT
   <213> Artificial Sequence
<220>
   <223> Description of artificial sequence: generic subformula (IC) DlLLLxDmLLLyDnLLLyDn
<220>
   <221> VARIANT
   <222> (1)..(13)
   <223> /replace="any amino acid"
<220>
   <221> VARIANT
   <222> (1) .. (1)
   <223> /replace="D-amino acid"
<220>
   <221> REPEAT
   <222> (1) .. (1)
   <223> number of repeats is 1 or 2
<220>
   <221> REPEAT
   <222> (4) .. (4)
   <223> number of repeats is 0, 1 or 2
<220>
   <221> VARIANT
   <222> (5)..(5)
   <223> /replace="D-amino acid"
<220>
   <221> REPEAT
   <222> (5) .. (5)
   <223> number of repeats is 1 or 2
<220>
   <221> REPEAT
   <222> (8)..(8)
   <223> number of repeats is 0, 1 or 2
<220>
   <221> VARIANT
   <222> (9)..(9)
   <223> /replace="D-amino acid"
<220>
   <221> REPEAT
   <222> (9)..(9)
   <223> number of repeats is 1 or 2
<220>
   <221> REPEAT
   <222> (12)..(12)
   <223> number of repeats is 0, 1 or 2
<220>
   <221> VARIANT
   <222> (13)..(13)
   <223> /replace="D-amino acid"
<220>
   <221> REPEAT
   <222> (13)..(13)
   <223> number of repeats is 1 or 2
<400> 4
   Xaa Xaa Xaa Xaa Xaa Xaa Xaa Xaa Xaa Xaa Xaa Xaa Xaa
   1 5 10
<210> 5
   <211> 17
   <212> PRT
   <213> Artificial sequence
<220>
   <223> Description of artificial sequence: generic subformulas (Id) DlLLLxDmLLLyDnLLLyDnLLLyDn
<220>
   <221> VARIANT
   <222> (1)..(17)
   <223> /replace="any amino acid"
<220>
   <221> VARIANT
   <222> (1)..(1)
   <223> /replace="D-amino acid"
<220>
   <221> REPEAT
   <222> (1)..(1)
   <223> number of repeats is 1 or 2
<220>
   <221> REPEAT
   <222> (4)..(4)
   <223> number of repeats is 0, 1 or 2
<220>
   <221> VARIANT
   <222> (5)..(5)
   <223> /replace="D-amino acid"
<220>
   <221> REPEAT
   <222> (5)..(5)
   <223> number of repeats is 1 or 2
<220>
   <221> REPEAT
   <222> (8)..(8)
   <223> number of repeats is 0, 1 or 2
<220>
   <221> VARIANT
   <222> (9)..(9)
   <223> /replace="D-amino acid"
<220>
   <221> REPEAT
   <222> (9)...(9)
   <223> number of repeats is 1 or 2
<220>
   <221> REPEAT
   <222> (12)..(12)
   <223> number of repeats is 0, 1 or 2
<220>
   <221> VARIANT
   <222> (13)..(13)
   <223> /replace="D-amino acid"
<220>
   <221> REPEAT
   <222> (13)...(13)
   <223> number of repeats is 1 or 2
<220>
   <221> REPEAT
   <222> (16)..(16)
   <223> number of repeats is 0, 1 or 2
<220>
   <221> VARIANT
   <222> (17)..(17)
   <223> /replace="D-amino acid"
<400> 5 **xaa**
<210> 6
   <211> 9
   <212> PRT
   <213> Artificial Sequence
<220>
   <223> Description of artificial sequence: generic subformula (Ie) DLLLD(LLLD)a
<220>
   <221> VARIANT
   <222> (1)..(9)
   <223> /replace="any amino acid"
<220>
   <221> VARIANT
   <222> (1)..(1)
   <223> /replace="D-amino acid".
<220>
   <221> VARIANT
   <222> (5)..(5)
   <223> /replace="D-amino acid",
<220>
   <221> REPEAT
   <222> (6)..(9)
   <223> number of repeats is 0, 1, 2 or 3
<220>
   <221> VARIANT
   <222> (9)..(9)
   <223> /replace="D-amino acid"
<400> 6
   Xaa Xaa Xaa Xaa Xaa Xaa Xaa Xaa Xaa
   1               5
<210> 7
   <211> 9
   <212> PRT
   <213> Artificial Sequence
<220>
   <223> Description of artificial sequence: generic subformula (If) DLLLDLLLD
<220>
   <221> VARIANT
   <222> (1)..(9)
   <223> /replace="any amino acid"
<220>
   <221> VARIANT
   <222> (1)..(1)
   <223> /replace="D-amino acid"
<220>
   <221> VARIANT
   <222> (5)..(5)
   <223> /replace="D-amino acid"
<220>
   <221> VARIANT
   <222> (9)..(9)
   <223> /replace="D-amino acid"
<400> 7
   Xaa Xaa Xaa Xaa Xaa Xaa Xaa Xaa Xaa
   1               5
<210> 8
   <211> 86
   <212> PRT
   <213> Human immunodeficiency virus type 1
<220>
   <221> misc_feature
   <223> Description of sequence: HIV-1 TAT sequence (aa 1-86)
<400> 8
   Met Glu Pro Val Asp Pro Arg Leu Glu Pro Trp Lys His Pro Gly Ser
   1               5                   10                  15
   Gln Pro Lys Thr Ala Cys Thr Asn Cys Tyr Cys Lys Lys Cys Cys Phe
               20                  25                  30
   His Cys Gln Val Cys Phe Ile Thr Lys Ala Leu Gly Ile Ser Tyr Gly
           35                  40                  45
   Arg Lys Lys Arg Arg Gln Arg Arg Arg Pro Pro Gln Gly Ser Gln Thr
       50                  55                  60
   His Gln Val Ser Leu Ser Lys Gln Pro Thr Ser Gln Ser Arg Gly Asp
   65                  70                  75                  80
   Pro Thr Gly Pro Lys Glu
                   85
<210> 9
   <211> 36
   <212> PRT
   <213> Human immunodeficiency virus type 1
<220>
   <221> misc_feature
   <223> Description of sequence: HIV-1 TAT sequence (aa 37-72)
<400> 9
   Cys Phe Ile Thr Lys Ala Leu Gly Ile Ser Tyr Gly Arg Lys Lys Arg
   1               5                   10                  15
   Arg Gln Arg Arg Arg Pro Pro Gln Gly Ser Gln Thr His Gln Val Ser
               20                  25                  30
   Leu Ser Lys Gln
           35
<210> 10
   <211> 22
   <212> PRT
   <213> Human immunodeficiency virus type 1
<220>
   <221> misc_feature
   <223> Description of sequence: HIV-1 TAT sequence (aa 37-58)
<400> 10
   Cys Phe Ile Thr Lys Ala Leu Gly Ile Ser Tyr Gly Arg Lys Lys Arg
   1               5                   10                  15
   Arg Gln Arg Arg Arg Pro
               20
<210> 11
   <211> 24
   <212> PRT
   <213> Human immunodeficiency virus type 1
<220>
   <221> misc_feature
   <223> Description of sequence: HIV-1 TAT sequence (aa 38-58) including an additional N-terminal GCC
<400> 11
   Phe Ile Thr Lys Ala Leu Gly Ile Ser Tyr Gly Arg Lys Lys Arg Arg
   1               5                   10                  15
   Gln Arg Arg Arg Pro Gly Gly Cys
               20
<210> 12
   <211> 15
   <212> PRT
   <213> Human immunodeficiency virus type 1
<220>
   <221> misc_feature
   <223> Description of sequence: HIV-1 TAT sequence (aa -47-58) including an additional C-terminal GCC
<400> 12
   Cys Gly Gly Tyr Gly Arg Lys Lys Arg Arg Gln Arg Arg Arg Pro
   1               5                   10                  15
<210> 13
   <211> 15
   <212> PRT
   <213> Human immunodeficiency virus type 1
<220>
   <221> misc_feature
   <223> Description of sequence: HIV-1 TAT sequence (aa 47-58) including an additional N-terminal GCC
<400> 13
   Tyr Gly Arg Lys Lys Arg Arg Gln Arg Arg Arg Pro Gly Gly Cys
   1               5                   10                  15
<210> 14
   <211> 56
   <212> PRT
   <213> Human immunodeficiency virus type 1
<220>
   <221> misc_feature
   <223> Description of sequence: HIV-1 TAT sequence (aa 1-72) including a mutated Cys to Ala residue at position 37
<400> 14
   Met Glu Pro Val Asp Pro Arg Leu Glu Pro Trp Lys His Pro Gly Ser
   1 5 10 15
   Gln Pro Lys Thr Ala Phe Ile Thr Lys Ala Leu Gly Ile Ser Tyr Gly
   20 25 30
   Arg Lys Lys Arg Arg Gln Arg Arg Arg Pro Pro Gln Gly Ser Gln Thr
   35 40 45
   His Gln Val Ser Leu Ser Lys Gln
   50 55
<210> 15
   <211> 17
   <212> PRT
   <213> Artificial
<220>
   <223> Description of sequence: trafficking sequence L-generic-TAT (see Table 1)
<220>
   <221> VARIANT
   <222> (1)..(17)
   <223> xaa is selected from any amino acid residue
<400> 15 xaa
<210> 16
   <211> 11
   <212> PRT
   <213> Artificial
<220>
   <223> Description of sequence: trafficking sequence L-generic-TAT (s) (see Table 1)
<220>
   <221> misc_feature
   <223> General formula: NH2-xnb-RKKRRQRRR-Xnb-cOOH (see Table 1)
<220>
   <221> VARIANT
   <222> (1)..(1)
   <223> xaa is xnb as defined in the general formula, wherein xaa represents an amino acid residue, preferably selected from any (native) amino acid residue;
<220>
   <221> REPEAT
   <222> (1)..(1)
   <223> xaa is xnb as defined in the general formula, wherein n is 0-5, 5-10, 10-15, 15-20, 20-30 or more for Xnb
<220>
   <221> VARIANT
   <222> (11)..(11)
   <223> xaa is xnb as defined in the general formula, wherein xaa represents an amino acid residue, preferably selected from any (native) amino acid residue;
<220>
   <221> REPEAT
   <222> (11)..(11)
   <223> xaa is xnb as defined in the general formula, wherein n is 0-5, 5-10, 10-15, 15-20, 20-30 or more for xnb
<400> 16
   Xaa Arg Lys Lys Arg Arg Gln Arg Arg Arg Xaa
   1 5 10
<210> 17
   <211> 10
   <212> PRT
   <213> Artificial
<220>
   <223> Description of sequence: trafficking sequence L-TAT (s1a) (see Table 1)
<400> 17
   Gly Arg Lys Lys Arg Arg Gln Arg Arg Arg
   1 5 10
<210> 18
   <211> 9
   <212> PRT
   <213> Artificial Sequence
<220>
   <223> Description of sequence: trafficking sequence L-TAT (s1b) (see Table 1)
<400> 18
   Arg Lys Lys Arg Arg Gln Arg Arg Arg
   1 5
<210> 19
   <211> 11
   <212> PRT
   <213> Artificial sequence
<220>
   <223> Description of sequence: trafficking sequence L-TAT (s1c) (see Table 1)
<400> 19
   Tyr Asp Arg Lys Lys Arg Arg Gln Arg Arg Arg
   1 5 10
<210> 20
   <211> 9
   <212> PRT
   <213> Artificial sequence
<220>
   <223> Description of sequence: trafficking sequence r3-L-TAT (see Table 1)
<400> 20
   Arg Lys Lys Arg Arg Gln Arg Arg Arg
   1 5
<210> 21
   <211> 9
   <212> PRT
   <213> Artificial Sequence
<220>
   <223> Description of sequence: trafficking sequence r3-L-TATi (see Table 1)
<400> 21
   Arg Arg Arg Gln Arg Arg Lys Lys Arg
   1 5
<210> 22
   <211> 9
   <212> PRT
   <213> Artificial sequence
<220>
   <223> Description of sequence: trafficking sequence betaA-r3-L-TAT (see Table 1)
<400> 22
   Arg Lys Lys Arg Arg Gln Arg Arg Arg
   1 5
<210> 23
   <211> 9
   <212> PRT
   <213> Artificial Sequence
<220>
   <223> Description of sequence: trafficking sequence betaA-r3-L-TAT (see Table 1)
<400> 23
   Arg Arg Arg Gln Arg Arg Lys Lys Arg
   1 5
<210> 24
   <211> 9
   <212> PRT
   <213> Artificial sequence
<220>
   <223> Description of sequence: trafficking sequence FITC-betaA-r3-L-TAT (see Table 1)
<400> 24
   Arg Lys Lys Arg Arg Gln Arg Arg Arg
   1 5
<210> 25
   <211> 9
   <212> PRT
   <213> Artificial Sequence
<220>
   <223> Description of sequence: trafficking sequence FITC-betaA-r3-L-TAT (see Table 1)
<400> 25
   Arg Arg Arg Gln Arg Arg Lys Lys Arg
   1 5
<210> 26
   <211> 9
   <212> PRT
   <213> Artificial sequence
<220>
   <223> Description of sequence: trafficking sequence TAT(s2-1) (see Table 1)
<400> 26
   Arg Ala Lys Arg Arg Gln Arg Arg Arg
   1 5
<210> 27
   <211> 9
   <212> PRT
   <213> Artificial sequence
<220>
   <223> Description of sequence: trafficking sequence TAT(s2-2) (see Table 1)
<400> 27
   Arg Lys Ala Arg Arg Gln Arg Arg Arg
   1 5
<210> 28
   <211> 9
   <212> PRT
   <213> Artificial Sequence
<220>
   <223> Description of sequence: trafficking sequence TAT(s2-3) (see Table 1)
<400> 28
   Arg Lys Lys Ala Arg Gln Arg Arg Arg
   1 5
<210> 29
   <211> 9
   <212> PRT
   <213> Artificial Sequence
<220>
   <223> Description of sequence: trafficking sequence TAT(s2-4) (see Table 1)
<400> 29
   Arg Lys Lys Arg Arg Ala Arg Arg Arg
   1               5
<210> 30
   <211> 9
   <212> PRT
   <213> Artificial Sequence
<220>
   <223> Description of sequence: trafficking sequence TAT(s2-5) (see Table 1)
<400> 30
   Arg Lys Lys Arg Arg Gln Ala Arg Arg
   1               5
<210> 31
   <211> 9
   <212> PRT
   <213> Artificial sequence
<220>
   <223> Description of sequence: trafficking sequence TAT(s2-6) (see Table 1)
<400> 31
   Arg Lys Lys Arg Arg Gln Arg Ala Arg
   1               5
<210> 32
   <211> 9
   <212> PRT
   <213> Artificial Sequence
<220>
   <223> Description of sequence: trafficking sequence TAT(s2-7) (see Table 1)
<400> 32
   Arg Asp Lys Arg Arg Gln Arg Arg Arg
   1               5
<210> 33
   <211> 9
   <212> PRT
   <213> Artificial sequence
<220>
   <223> Description of sequence: trafficking sequence TAT(s2-8) (see Table 1)
<400> 33
   Arg Lys Asp Arg Arg Gln Arg Arg Arg
   1               5
<210> 34
   <211> 9
   <212> PRT
   <213> Artificial Sequence
<220>
   <223> Description of sequence: trafficking sequence TAT(s2-9) (see Table 1)
<400> 34
   Arg Lys Lys Asp Arg Gln Arg Arg Arg
   1               5
<210> 35
   <211> 9
   <212> PRT
   <213> Artificial Sequence
<220>
   <223> Description of sequence: trafficking sequence TAT(s2-10) (see Table 1)
<400> 35
   Arg Lys Lys Arg Arg Asp Arg Arg Arg
   1               5
<210> 36
   <211> 9
   <212> PRT
   <213> Artificial sequence
<220>
   <223> Description of sequence: trafficking sequence TAT(s2-11) (see Table 1)
<400> 36
   Arg Lys Lys Arg Arg Gln Asp Arg Arg
   1               5
<210> 37
   <211> 9
   <212> PRT
   <213> Artificial Sequence
<220>
   <223> Description of sequence: trafficking sequence TAT(s2-12) (see Table 1)
<400> 37
   Arg Lys Lys Arg Arg Gln Arg Asp Arg
   1               5
<210> 38
   <211> 9
   <212> PRT
   <213> Artificial Sequence
<220>
   <223> Description of sequence: trafficking sequence TAT(s2-13) (see Table 1)
<400> 38
   Arg Glu Lys Arg Arg Gln Arg Arg Arg
   1               5
<210> 39
   <211> 9
   <212> PRT
   <213> Artificial sequence
<220>
   <223> Description of sequence: trafficking sequence TAT(s2-14) (see Table 1)
<400> 39
   Arg Lys Glu Arg Arg Gln Arg Arg Arg
   1               5
<210> 40
   <211> 9
   <212> PRT
   <213> Artificial sequence
<220>
   <223> Description of sequence: trafficking sequence TAT(s2-15) (see Table 1)
<400> 40
   Arg Lys Lys Glu Arg Gln Arg Arg Arg
   1               5
<210> 41
   <211> 9
   <212> PRT
   <213> Artificial sequence
<220>
   <223> Description of sequence: trafficking sequence TAT(s2-16) (see Table 1)
<400> 41
   Arg Lys Lys Arg Arg Glu Arg Arg Arg
   1               5
<210> 42
   <211> 9
   <212> PRT
   <213> Artificial Sequence
<220>
   <223> Description of sequence: trafficking sequence TAT(s2-17) (see Table 1)
<400> 42
   Arg Lys Lys Arg Arg Gln Glu Arg Arg
   1 5
<210> 43
   <211> 9
   <212> PRT
   <213> Artificial Sequence
<220>
   <223> Description of sequence: trafficking sequence TAT(s2-18) (see Table 1)
<400> 43
   Arg Lys Lys Arg Arg Gln Arg Glu Arg
   1 5
<210> 44
   <211> 9
   <212> PRT
   <213> Artificial Sequence
<220>
   <223> Description of sequence: trafficking sequence TAT(s2-19) (see Table 1)
<400> 44
   Arg Phe Lys Arg Arg Gln Arg Arg Arg
   1 5
<210> 45
   <211> 9
   <212> PRT
   <213> Artificial sequence
<220>
   <223> Description of sequence: trafficking sequence TAT(s2-20) (see Table 1)
<400> 45
   Arg Lys Phe Arg Arg Gln Arg Arg Arg
   1 5
<210> 46
   <211> 9
   <212> PRT
   <213> Artificial sequence
<220>
   <223> Description of sequence: trafficking sequence TAT(s2-21) (see Table 1)
<400> 46
   Arg Lys Lys Phe Arg Gln Arg Arg Arg
   1               5
<210> 47
   <211> 9
   <212> PRT
   <213> Artificial Sequence
<220>
   <223> Description of sequence: trafficking sequence TAT(s2-22) (see Table 1)
<400> 47
   Arg Lys Lys Arg Arg Phe Arg Arg Arg
   1               5
<210> 48
   <211> 9
   <212> PRT
   <213> Artificial sequence
<220>
   <223> Description of sequence: trafficking sequence TAT(s2-23) (see Table 1)
<400> 48
   Arg Lys Lys Arg Arg Gln Phe Arg Arg
   1               5
<210> 49
   <211> 9
   <212> PRT
   <213> Artificial sequence
<220>
   <223> Description of sequence: trafficking sequence TAT(s2-24) (see Table 1)
<400> 49
   Arg Lys Lys Arg Arg Gln Arg Phe Arg
   1               5
<210> 50
   <211> 9
   <212> PRT
   <213> Artificial sequence
<220>
   <223> Description of sequence: trafficking sequence TAT(s2-25) (see Table 1)
<400> 50
   Arg Arg Lys Arg Arg Gln Arg Arg Arg
   1 5
<210> 51
   <211> 9
   <212> PRT
   <213> Artificial Sequence
<220>
   <223> Description of sequence: trafficking sequence TAT(s2-26) (see Table 1)
<400> 51
   Arg Lys Arg Arg Arg Gln Arg Arg Arg
   1 5
<210> 52
   <211> 9
   <212> PRT
   <213> Artificial sequence
<220>
   <223> Description of sequence: trafficking sequence TAT(s2-27) (see Table 1)
<400> 52
   Arg Lys Lys Lys Arg Gln Arg Arg Arg
   1 5
<210> 53
   <211> 9
   <212> PRT
   <213> Artificial sequence
<220>
   <223> Description of sequence: trafficking sequence TAT(s2-28) (see Table 1)
<400> 53
   Arg Lys Lys Arg Arg Arg Arg Arg Arg
   1 5
<210> 54
   <211> 9
   <212> PRT
   <213> Artificial Sequence
<220>
   <223> Description of sequence: trafficking sequence TAT(s2-29) (see Table 1)
<400> 54
   Arg Lys Lys Arg Arg Gln Lys Arg Arg
   1 5
<210> 55
   <211> 9
   <212> PRT
   <213> Artificial Sequence
<220>
   <223> Description of sequence: trafficking sequence TAT(s2-30) (see Table 1)
<400> 55
   Arg Lys Lys Arg Arg Gln Arg Lys Arg
   1               5
<210> 56
   <211> 9
   <212> PRT
   <213> Artificial Sequence
<220>
   <223> Description of sequence: trafficking sequence TAT(s2-31) (see Table 1)
<400> 56
   Arg His Lys Arg Arg Gln Arg Arg Arg
   1               5
<210> 57
   <211> 9
   <212> PRT
   <213> Artificial sequence
<220>
   <223> Description of sequence: trafficking sequence TAT(s2-32) (see Table 1)
<400> 57
   Arg Lys His Arg Arg Gln Arg Arg Arg
   1               5
<210> 58
   <211> 9
   <212> PRT
   <213> Artificial Sequence
<220>
   <223> Description of sequence: trafficking sequence TAT(s2-33) (see Table 1)
<400> 58
   Arg Lys Lys His Arg Gln Arg Arg Arg
   1               5
<210> 59
   <211> 9
   <212> PRT
   <213> Artificial sequence
<220>
   <223> Description of sequence: trafficking sequence TAT(s2-34) (see Table 1)
<400> 59
   Arg Lys Lys Arg Arg His Arg Arg Arg
   1 5
<210> 60
   <211> 9
   <212> PRT
   <213> Artificial Sequence
<220>
   <223> Description of sequence: trafficking sequence TAT(s2-35) (see Table 1)
<400> 60
   Arg Lys Lys Arg Arg Gln His Arg Arg
   1 5
<210> 61
   <211> 9
   <212> PRT
   <213> Artificial Sequence
<220>
   <223> Description of sequence: trafficking sequence TAT(s2-36) (see Table 1)
<400> 61
   Arg Lys Lys Arg Arg Gln Arg His Arg
   1 5
<210> 62
   <211> 9
   <212> PRT
   <213> Artificial Sequence
<220>
   <223> Description of sequence: trafficking sequence TAT(s2-37) (see Table 1)
<400> 62
   Arg Ile Lys Arg Arg Gln Arg Arg Arg
   1 5
<210>
   <211> 9
   <212> PRT
   <213> Artificial Sequence
<220>
   <223> Description of sequence: trafficking sequence TAT(s2-38) (see Table 1)
<400> 63
<210> 64
   <211> 9
   <212> PRT
   <213> Artificial Sequence
<220>
   <223> Description of sequence: trafficking sequence TAT(s2-39) (see Table 1)
<400> 64
   Arg Lys Lys Ile Arg Gln Arg Arg Arg
   1               5
<210> 65
   <211> 9
   <212> PRT
   <213> Artificial Sequence
<220>
   <223> Description of sequence: trafficking sequence TAT(s2-40) (see Table 1)
<400> 65
   Arg Lys Lys Arg Arg Ile Arg Arg Arg
   1               5
<210> 66
   <211> 9
   <212> PRT
   <213> Artificial Sequence
<220>
   <223> Description of sequence: trafficking sequence TAT(s2-41) (see Table 1)
<400> 66
   Arg Lys Lys Arg Arg Gln Ile Arg Arg
   1               5
<210> 67
   <211> 9
   <212> PRT
   <213> Artificial sequence
<220>
   <223> Description of sequence: trafficking sequence TAT(s2-42) (see Table 1)
<400> 67
   Arg Lys Lys Arg Arg Gln Arg Ile Arg
   1               5
<210> 68
   <211> 9
   <212> PRT
   <213> Artificial Sequence
<220>
   <223> Description of sequence: trafficking sequence TAT(s2-43) (see Table 1)
<400> 68
   Arg Leu Lys Arg Arg Gln Arg Arg Arg
   1 5
<210> 69
   <211> 9
   <212> PRT
   <213> Artificial sequence
<220>
   <223> Description of sequence: trafficking sequence TAT(s2-44) (see Table 1)
<400> 69
   Arg Lys Leu Arg Arg Gln Arg Arg Arg
   1 5
<210> 70
   <211> 9
   <212> PRT
   <213> Artificial sequence
<220>
   <223> Description of sequence: trafficking sequence TAT(s2-45) (see Table 1)
<400> 70
   Arg Lys Lys Leu Arg Gln Arg Arg Arg
   1 5
<210> 71
   <211> 9
   <212> PRT
   <213> Artificial Sequence
<220>
   <223> Description of sequence: trafficking sequence TAT(s2-46) (see Table 1)
<400> 71
   Arg Lys Lys Arg Arg Leu Arg Arg Arg
   1 5
<210> 72
   <211> 9
   <212> PRT
   <213> Artificial Sequence
<220>
   <223> Description of sequence: trafficking sequence TAT(s2-47) (see Table 1)
<400> 72
   Arg Lys Lys Arg Arg Gln Leu Arg Arg
   1               5
<210> 73
   <211> 9
   <212> PRT
   <213> Artificial sequence
<220>
   <223> Description of sequence: trafficking sequence TAT(s2-48) (see Table 1)
<400> 73
   Arg Lys Lys Arg Arg Gln Arg Leu Arg
   1               5
<210> 74
   <211> 9
   <212> PRT
   <213> Artificial Sequence
<220>
   <223> Description of sequence: trafficking sequence TAT(s2-49) (see Table 1)
<400> 74
   Arg Met Lys Arg Arg Gln Arg Arg Arg
   1               5
<210> 75
   <211> 9
   <212> PRT
   <213> Artificial sequence
<220>
   <223> Description of sequence: trafficking sequence TAT(s2-50) (see Table 1)
<400> 75
   Arg Lys Met Arg Arg Gln Arg Arg Arg
   1               5
<210> 76
   <211> 9
   <212> PRT
   <213> Artificial Sequence
<220>
   <223> Description of sequence: trafficking sequence TAT(s2-51) (see Table 1)
<400> 76
   Arg Lys Lys Met Arg Gln Arg Arg Arg
   1 5
<210> 77
   <211> 9
   <212> PRT
   <213> Artificial sequence
<220>
   <223> Description of sequence: trafficking sequence TAT(s2-52) (see Table 1)
<400> 77
   Arg Lys Lys Arg Arg Met Arg Arg Arg
   1 5
<210> 78
   <211> 9
   <212> PRT
   <213> Artificial Sequence
<220>
   <223> Description of sequence: trafficking sequence TAT(s2-53) (see Table 1)
<400> 78
   Arg Lys Lys Arg Arg Gln Met Arg Arg
   1 5
<210> 79
   <211> 9
   <212> PRT
   <213> Artificial sequence
<220>
   <223> Description of sequence: trafficking sequence TAT(s2-54) (see Table 1)
<400> 79
   Arg Lys Lys Arg Arg Gln Arg Met Arg
   1 5
<210> 80
   <211> 9
   <212> PRT
   <213> Artificial sequence
<220>
   <223> Description of sequence: trafficking sequence TAT(s2-55) (see Table 1)
<400> 80
   Arg Asn Lys Arg Arg Gln Arg Arg Arg
   1 5
<210> 81
   <211> 9
   <212> PRT
   <213> Artificial sequence
<220>
   <223> Description of sequence: trafficking sequence TAT(s2-56) (see Table 1)
<400> 81
   Arg Lys Asn Arg Arg Gln Arg Arg Arg
   1 5
<210> 82
   <211> 9
   <212> PRT
   <213> Artificial sequence
<220>
   <223> Description of sequence: trafficking sequence TAT(s2-57) (see Table 1)
<400> 82
   Arg Lys Lys Asn Arg Gln Arg Arg Arg
   1 5
<210> 83
   <211> 9
   <212> PRT
   <213> Artificial Sequence
<220>
   <223> Description of sequence: trafficking sequence TAT(s2-58) (see Table 1)
<400> 83
   Arg Lys Lys Arg Arg Asn Arg Arg Arg
   1 5
<210> 84
   <211> 9
   <212> PRT
   <213> Artificial Sequence
<220>
   <223> Description of sequence: trafficking sequence TAT(s2-59) (see Table 1)
<400> 84
   Arg Lys Lys Arg Arg Gln Asn Arg Arg
   1 5
<210> 85
   <211> 9
   <212> PRT
   <213> Artificial Sequence
<220>
   <223> Description of sequence: trafficking sequence TAT(s2-60) (see Table 1)
<400> 85
   Arg Lys Lys Arg Arg Gln Arg Asn Arg
   1               5
<210> 86
   <211> 9
   <212> PRT
   <213> Artificial sequence
<220>
   <223> Description of sequence: trafficking sequence TAT(s2-61) (see Table 1)
<400> 86
   Arg Gln Lys Arg Arg Gln Arg Arg Arg
   1               5
<210> 87
   <211> 9
   <212> PRT
   <213> Artificial Sequence
<220>
   <223> Description of sequence: trafficking sequence TAT(s2-62) (see Table 1)
<400> 87
   Arg Lys Gln Arg Arg Gln Arg Arg Arg
   1               5
<210> 88
   <211> 9
   <212> PRT
   <213> Artificial Sequence
<220>
   <223> Description of sequence: trafficking sequence TAT(s2-63) (see Table 1)
<400> 88
   Arg Lys Lys Gln Arg Gln Arg Arg Arg
   1               5
<210> 89
   <211> 9
   <212> PRT
   <213> Artificial Sequence
<220>
   <223> Description of sequence: trafficking sequence TAT(s2-64) (see Table 1)
<400> 89
   Arg Lys Lys Arg Arg Lys Arg Arg Arg
   1               5
<210> 90
   <211> 9
   <212> PRT
   <213> Artificial sequence
<220>
   <223> Description of sequence: trafficking sequence TAT(s2-65) (see Table 1)
<400> 90
   Arg Lys Lys Arg Arg Gln Gln Arg Arg
   1 5
<210> 91
   <211> 9
   <212> PRT
   <213> Artificial sequence
<220>
   <223> Description of sequence: trafficking sequence TAT(s2-66) (see Table 1)
<400> 91
   Arg Lys Lys Arg Arg Gln Arg Gln Arg
   1 5
<210> 92
   <211> 9
   <212> PRT
   <213> Artificial sequence
<220>
   <223> Description of sequence: trafficking sequence TAT(s2-67) (see Table 1)
<400> 92
   Arg Ser Lys Arg Arg Gln Arg Arg Arg
   1 5
<210> 93
   <211> 9
   <212> PRT
   <213> Artificial sequence
<220>
   <223> Description of sequence: trafficking sequence TAT(s2-68) (see Table 1)
<400> 93
   Arg Lys Ser Arg Arg Gln Arg Arg Arg
   1 5
<210> 94
   <211> 9
   <212> PRT
   <213> Artificial Sequence
<220>
   <223> Description of sequence: trafficking sequence TAT(s2-69) (see Table 1)
<400> 94
   Arg Lys Lys Ser Arg Gln Arg Arg Arg
   1 5
<210> 95
   <211> 9
   <212> PRT
   <213> Artificial sequence
<220>
   <223> Description of sequence: trafficking sequence TAT(s2-70) (see Table 1)
<400> 95
   Arg Lys Lys Arg Arg Ser Arg Arg Arg
   1 5
<210> 96
   <211> 9
   <212> PRT
   <213> Artificial Sequence
<220>
   <223> Description of sequence: trafficking sequence TAT(s2-71) (see Table 1)
<400> 96
   Arg Lys Lys Arg Arg Gln Ser Arg Arg
   1 5
<210> 97
   <211> 9
   <212> PRT
   <213> Artificial Sequence
<220>
   <223> Description of sequence: trafficking sequence TAT(s2-72) (see Table 1)
<400> 97
   Arg Lys Lys Arg Arg Gln Arg Ser Arg
   1 5
<210> 98
   <211> 9
   <212> PRT
   <213> Artificial Sequence
<220>
   <223> Description of sequence: trafficking sequence TAT(s2-73) (see Table 1)
<400> 98
   Arg Thr Lys Arg Arg Gln Arg Arg Arg
   1 5
<210> 99
   <211> 9
   <212> PRT
   <213> Artificial Sequence
<220>
   <223> Description of sequence: trafficking sequence TAT(s2-74) (see Table 1)
<400> 99
   Arg Lys Thr Arg Arg Gln Arg Arg Arg
   1 5
<210> 100
   <211> 9
   <212> PRT
   <213> Artificial Sequence
<220>
   <223> Description of sequence: trafficking sequence TAT(s2-75) (see Table 1)
<400> 100
   Arg Lys Lys Thr Arg Gln Arg Arg Arg
   1 5
<210> 101
   <211> 9
   <212> PRT
   <213> Artificial sequence
<220>
   <223> Description of sequence: trafficking sequence TAT(s2-76) (see Table 1)
<400> 101
   Arg Lys Lys Arg Arg Thr Arg Arg Arg
   1 5
<210> 102
   <211> 9
   <212> PRT
   <213> Artificial Sequence
<220>
   <223> Description of sequence: trafficking sequence TAT(s2-77) (see Table 1)
<400> 102
   Arg Lys Lys Arg Arg Gln Thr Arg Arg
   1 5
<210> 103
   <211> 9
   <212> PRT
   <213> Artificial sequence
<220>
   <223> Description of sequence: trafficking sequence TAT(s2-78) (see Table 1)
<400> 103
   Arg Lys Lys Arg Arg Gln Arg Thr Arg
   1 5
<210> 104
   <211> 9
   <212> PRT
   <213> Artificial sequence
<220>
   <223> Description of sequence: trafficking sequence TAT(s2-79) (see Table 1)
<400> 104
   Arg Val Lys Arg Arg Gln Arg Arg Arg
   1 5
<210> 105
   <211> 9
   <212> PRT
   <213> Artificial sequence
<220>
   <223> Description of sequence: trafficking sequence TAT(s2-80) (see Table 1)
<400> 105
   Arg Lys Val Arg Arg Gln Arg Arg Arg
   1 5
<210> 106
   <211> 9
   <212> PRT
   <213> Artificial Sequence
<220>
   <223> Description of sequence: trafficking sequence TAT(s2-81) (see Table 1)
<400> 106
   Arg Lys Lys Val Arg Gln Arg Arg Arg
   1 5
<210> 107
   <211> 9
   <212> PRT
   <213> Artificial Sequence
<220>
   <223> Description of sequence: trafficking sequence TAT(s2-82) (see Table 1)
<400> 107
   Arg Lys Lys Arg Arg Val Arg Arg Arg
   1               5
<210> 108
   <211> 9
   <212> PRT
   <213> Artificial sequence
<220>
   <223> Description of sequence: trafficking sequence TAT(s2-83) (see Table 1)
<400> 108
   Arg Lys Lys Arg Arg Gln Val Arg Arg
   1               5
<210> 109
   <211> 9
   <212> PRT
   <213> Artificial sequence
<220>
   <223> Description of sequence: trafficking sequence TAT(s2-84) (see Table 1)
<400> 109
   Arg Lys Lys Arg Arg Gln Arg Val Arg
   1               5
<210> 110
   <211> 9
   <212> PRT
   <213> Artificial sequence
<220>
   <223> Description of sequence: trafficking sequence TAT(s2-85) (see Table 1)
<400> 110
   Arg Trp Lys Arg Arg Gln Arg Arg Arg
   1               5
<210> 111
   <211> 9
   <212> PRT
   <213> Artificial Sequence
<220>
   <223> Description of sequence: trafficking sequence TAT(s2-86) (see Table 1)
<400> 111
   Arg Lys Trp Arg Arg Gln Arg Arg Arg
   1               5
<210> 112
   <211> 9
   <212> PRT
   <213> Artificial Sequence
<220>
   <223> Description of sequence: trafficking sequence TAT(s2-87) (see Table 1)
<400> 112
   Arg Lys Lys Trp Arg Gln Arg Arg Arg
   1               5
<210> 113
   <211> 9
   <212> PRT
   <213> Artificial Sequence
<220>
   <223> Description of sequence: trafficking sequence TAT(s2-88) (see Table 1)
<400> 113
   Arg Lys Lys Arg Arg Trp Arg Arg Arg
   1               5
<210> 114
   <211> 9
   <212> PRT
   <213> Artificial Sequence
<220>
   <223> Description of sequence: trafficking sequence TAT(s2-89) (see Table 1)
<400> 114
   Arg Lys Lys Arg Arg Gln Trp Arg Arg
   1               5
<210> 115
   <211> 9
   <212> PRT
   <213> Artificial Sequence
<220>
   <223> Description of sequence: trafficking sequence TAT(s2-90) (see Table 1)
<400> 115
   Arg Lys Lys Arg Arg Gln Arg Trp Arg
   1               5
<210> 116
   <211> 9
   <212> PRT
   <213> Artificial Sequence
<220>
   <223> Description of sequence: trafficking sequence TAT(s2-91) (see Table 1)
<400> 116
   Arg Tyr Lys Arg Arg Gln Arg Arg Arg
   1 5
<210> 117
   <211> 9
   <212> PRT
   <213> Artificial Sequence
<220>
   <223> Description of sequence: trafficking sequence TAT(s2-92) (see Table 1)
<400> 117
   Arg Lys Tyr Arg Arg Gln Arg Arg Arg
   1 5
<210> 118
   <211> 9
   <212> PRT
   <213> Artificial sequence
<220>
   <223> Description of sequence: trafficking sequence TAT(s2-93) (see Table 1)
<400> 118
   Arg Lys Lys Tyr Arg Gln Arg Arg Arg
   1 5
<210> 119
   <211> 9
   <212> PRT
   <213> Artificial Sequence
<220>
   <223> Description of sequence: trafficking sequence TAT(s2-94) (see Table 1)
<400> 119
   Arg Lys Lys Arg Arg Tyr Arg Arg Arg
   1 5
<210> 120
   <211> 9
   <212> PRT
   <213> Artificial sequence
<220>
   <223> Description of sequence: trafficking sequence TAT(s2-95) (see Table 1)
<400> 120
   Arg Lys Lys Arg Arg Gln Tyr Arg Arg
   1 5
<210> 121
   <211> 9
   <212> PRT
   <213> Artificial sequence
<220>
   <223> Description of sequence: trafficking sequence TAT(s2-96) (see Table 1)
<400> 121
   Arg Lys Lys Arg Arg Gln Arg Tyr Arg
   1 5
<210> 122
   <211> 9
   <212> PRT
   <213> Artificial Sequence
<220>
   <223> Description of sequence: trafficking sequence r3R6 (see Table 1)
<220>
   <221> VARIANT
   <222> (1)..(1)
   <223> /replace="D-enatiomeric amino acid arginine"
<220>
   <221> VARIANT
   <222> (4)..(4)
   <223> /replace="D-enatiomeric amino acid arginine"
<220>
   <221> VARIANT
   <222> (9)..(9)
   <223> /replace="D-enatiomeric amino acid arginine"
<400> 122
   Arg Arg Arg Arg Arg Arg Arg Arg Arg
   1 5
<210> 123
   <211> 9
   <212> PRT
   <213> Artificial Sequence
<220>
   <223> Description of sequence: trafficking sequence L-R9 (see Table 1)
<400> 123
   Arg Arg Arg Arg Arg Arg Arg Arg Arg
   1 5
<210> 124
   <211> 8
   <212> PRT
   <213> Artificial sequence
<220>
   <223> Description of sequence: trafficking sequence L-R8 (see Table 1)
<400> 124
   Arg Arg Arg Arg Arg Arg Arg Arg
   1 5
<210> 125
   <211> 7
   <212> PRT
   <213> Artificial sequence
<220>
   <223> Description of sequence: trafficking sequence L-R7 (see Table 1)
<400> 125
   Arg Arg Arg Arg Arg Arg Arg
   1 5
<210> 126
   <211> 6
   <212> PRT
   <213> Artificial Sequence
<220>
   <223> Description of sequence: trafficking sequence L-R6 (see Table 1)
<400> 126
   Arg Arg Arg Arg Arg Arg
   1 5
<210> 127
   <211> 5
   <212> PRT
   <213> Artificial Sequence
<220>
   <223> Description of sequence: trafficking sequence L-R5 (see Table 1)
<400> 127
   Arg Arg Arg Arg Arg
   1 5
<210> 128
   <211> 11
   <212> PRT
   <213> Artificial sequence
<220>
   <223> Description of sequence: trafficking sequence PTD-4 (see Table 1)
<400> 128
   Tyr Ala Arg Ala Ala Ala Arg Gln Ala Arg Ala
   1               5                   10
<210> 129
   <211> 11
   <212> PRT
   <213> Artificial sequence
<220>
   <223> Description of sequence: trafficking sequence PTD-4 (variant 1) (see Table 1)
<400> 129
   Trp Ala Arg Ala Ala Ala Arg Gln Ala Arg Ala
   1               5                   10
<210> 130
   <211> 11
   <212> PRT
   <213> Artificial Sequence
<220>
   <223> Description of sequence: trafficking sequence PTD-4 (variant 2) (see Table 1)
<400> 130
   Trp Ala Arg Ala Gln Arg Ala Ala Ala Arg Ala
   1               5                   10
<210> 131
   <211> 16
   <212> PRT
   <213> Artificial sequence
<220>
   <223> Description of sequence: trafficking sequence L-P1 (Penetratin) (see Table 1)
<400> 131
   Arg Gln Val Lys Val Trp Phe Gln Asn Arg Arg Met Lys Trp Lys Lys
   1               5                   10                  15
<210> 132
   <211> 16
   <212> PRT
   <213> Artificial Sequence
<220>
   <223> Description of sequence: trafficking sequence D-P1 (Penetratin) (see Table 1)
<400> 132
   Lys Lys Trp Lys Met Arg Arg Asn Gln Phe Trp Val Lys Val Gln Arg
   1               5                   10                  15
<210> 133
   <211> 17
   <212> PRT
   <213> Artificial Sequence
<220>
   <223> Description of sequence: trafficking sequence JNK1, bestfit (see Table 1)
<400> 133
   Trp Lys Arg Ala Ala Ala Arg Lys Ala Arg Ala Met Ser Leu Asn Leu
   1               5                   10                  15
   Phe
<210> 134
   <211> 17
   <212> PRT
   <213> Artificial Sequence
<220>
   <223> Description of sequence: trafficking sequence JNK1. bestfit (variant 1) (see Table 1)
<400> 134
   Trp Lys Arg Ala Ala Ala Arg Ala Ala Arg Ala Met Ser Leu Asn Leu
   1               5                   10                  15
   Phe
<210> 135
   <211> 9
   <212> PRT
   <213> Artificial sequence
<220>
   <223> Description of sequence: trafficking sequence MDCK transcytose sequence (see Table 1)
<400> 135
   Arg Tyr Arg Gly Asp Leu Gly Arg Arg
   1               5
<210> 136
   <211> 4
   <212> PRT
   <213> Artificial Sequence
<220>
   <223> Description of sequence: trafficking sequence YKGL (see Table 1)
<400> 136
   Tyr Lys Gly Leu
   1
<210> 137
   <211> 2953
   <212> DNA
   <213> Rattus norvegicus
<220>
   <221> misc_feature
   <223> description of sequence: rat IB1 CDNA sequence
<400> 137
   ccgccccagc tcagtccgaa ccccgcggcg gcggcggcct cctccacacg cctccacctc 60
   cgccgccgcc gccgccgccg ccgcctcccg cgccgctctc cgcccggatg gccaggctga 120
   gcccgggaat ggcggagcga gagagcggcc tgagcggggg tgccgcgtcc ccaccggccg 180
   cttccccatt cctgggactg cacatcgcgt cgcctcccaa tttcaggctc acccatgata 240
   tcagcctgga ggagtttgag gatgaagacc tttcggagat cactgatgag tgtggcatca 300
   gcctgcagtg caaagacacc ttgtctctcc ggcccccgcg cgccgggcta ctgtctgcgg 360
   gtagcagcgg tagcgcgggg agccggctgc aggcggagat gctgcagatg gacctgatcg 420
   acgcggcaag tgacactccg ggcgccgagg acgacgaaga ggacgacgac gagctcgctg 480
   cccaacggcc aggagtgggg ccttccaaag ccgagtctgg ccaggagccg gcgtctcgca 540
   gccagggtca gggccagggc cccggcacag gctgcggaga cacctaccgg cccaagaggc 600
   ctaccacgct caaccttttc ccgcaggtgc cgcggtctca ggacacgctg aataataact 660
   ctttaggcaa aaagcacagt tggcaggacc gtgtgtctcg atcatcctcc cctctgaaga 720
   caggggagca gacgcctcca catgaacata tctgcctgag tgatgagctg ccgccccagg 780
   gcagtcctgt tcccacccag gatcgtggca cttccaccga cagcccttgt cgccgtactg 840
   cagccaccca gatggcacct ccaagtggtc cccctgccac tgcacctggt ggccggggcc 900
   actcccatcg agatcggtcc atatcagcag atgtgcggct cgaggcgact gaggagatct 960
   acctgacccc agtgcagagg cccccagacc ctgcagaacc cacctccacc ttcttgccac 1020
   ccactgagag ccggatgtct gtcagctcgg atcctgaccc tgccgcttac tctgtaactg 1080
   cagggcgacc gcacccttcc atcagtgaag aggatgaggg cttcgactgt ctgtcatccc 1140
   cagagcaagc tgagccacca ggtggagggt ggcggggaag cctcggggag ccaccaccgc 1200
   ctccacgggc ctcactgagc tcggacacca gcgcactgtc ctacgactct gtcaagtaca 1260
   cactggtggt ggatgagcat gcccagcttg agttggtgag cctgcggcca tgttttggag 1320
   attacagtga cgaaagcgac tctgccactg tctatgacaa ctgtgcctct gcctcctcgc 1380
   cctacgagtc agccattggt gaggaatatg aggaggcccc tcaaccccgg cctcccacct 1440
   gcctgtcaga ggactccaca ccggatgagc ctgacgtcca cttctctaag aagtttctga 1500
   atgtcttcat gagtggccgc tctcgttcct ccagtgccga gtcctttggg ctgttctcct 1560
   gtgtcatcaa tggggaggag catgagcaaa cccatcgggc tatattcagg tttgtgcctc 1620
   ggcatgaaga tgaacttgag ctggaagtgg acgaccctct gctggtggag ctgcaggcag 1680
   aagactattg gtatgaggcc tataacatgc gcactggagc ccgtggtgtc tttcctgcct 1740
   actatgccat tgaggtcacc aaggagcctg agcacatggc agcccttgcc aaaaacagcg 1800
   actggattga ccagttccgg gtgaagttcc tgggctctgt ccaggttcct tatcacaagg 1860
   gcaatgatgt cctctgtgct gctatgcaaa agatcgccac cacccgccgg ctcaccgtgc 1920
   actttaaccc gccctccagc tgtgtccttg aaatcagcgt taggggtgtc aagataggtg 1980
   tcaaagctga tgaagctcag gaggccaagg gaaataaatg tagccacttt ttccagctaa 2040
   aaaacatctc tttctgtggg taccatccaa agaacaacaa gtactttggg tttatcacta 2100
   agcaccctgc tgaccaccgg tttgcctgcc atgtctttgt gtctgaagat tccaccaaag 2160
   ccctggcaga gtctgtgggg cgtgcatttc agcagttcta caagcaattt gtggaatata 2220
   cctgtcctac agaagatatc tacttggagt agcagcaacc cccctctctg cagcccctca 2280
   gccccaggcc agtactagga cagctgactg ctgacaggat gttgtactgc cacgagagaa 2340
   tgggggagtg agggctgttg gggtcggggg gcaggggttt ggggagaggc agatgcagtt 2400
   tattgtaata tatggggtta gattaatcta tggaggacag tacaggctct ctcggggctg 2460
   gggaagggca gggctggggt gggggtcagg catctggcca caaaggggtc ccctagggac 2520
   agaggcgctg caccatcctg ggcttgtttc atactagagg ccctggcttt ctggctcttg      2580
   ggtcctgcct tgacaaagcc cagccacctg gaagtgtcac cttcccttgt ccacctcacc      2640
   cagtgccctg agctcatgct gagcccaagc acctccgaag gactttccag taaggaaatg      2700
   gcaacatgtg acagtgagac cctgttctca tctgtggggc tccggcagct ccgaccccca     2760
   gcctggccag cacgctgacc ctggcaagct tgtgtgttca aagaaggaga gggccacagc      2820
   aagccctgcc tgccagggaa ggttccctct cagctggccc cagccaactg gtcactgtct      2880
   tgtcacctgg ctactactat taaagtgcca tttcttgtct gaaaaaaaaa aaaaaaaaaa      2940
   aaaaaaactc gag                                                         2953
<210> 138
   <211> 714
   <212> PRT
   <213> Rattus norvegicus
<220>
   <221> misc_feature
   <223> description of sequence: amino acid sequence encoded by of rat IB1 CDNA sequence
<400> 138
   Met Ala Arg Leu Ser Pro Gly Met Ala Glu Arg Glu Ser Gly Leu Ser
   1               5                   10                  15
   Gly Gly Ala Ala Ser Pro Pro Ala Ala Ser Pro Phe Leu Gly Leu His
               20                  25                  30
   Ile Ala Ser Pro Pro Asn Phe Arg Leu Thr His Asp Ile Ser Leu Glu
           35                  40                  45
   Glu Phe Glu Asp Glu Asp Leu Ser Glu Ile Thr Asp Glu Cys Gly Ile
       50                  55                  60
   Ser Leu Gln Cys Lys Asp Thr Leu Ser Leu Arg Pro Pro Arg Ala Gly
   65                  70                  75                  80
   Leu Leu Ser Ala Gly Ser Ser Gly Ser Ala Gly Ser Arg Leu Gln Ala
                   85                  90                  95
   Glu Met Leu Gln Met Asp Leu Ile Asp Ala Ala Ser Asp Thr Pro Gly
               100                 105                 110
   Ala Glu Asp Asp Glu Glu Asp Asp Asp Glu Leu Ala Ala Gln Arg Pro
           115                 120                 125
   Gly Val Gly Pro Ser Lys Ala Glu Ser Gly Gln Glu Pro Ala Ser Arg
       130                 135                 140
   Ser Gln Gly Gln Gly Gln Gly Pro Gly Thr Gly Cys Gly Asp Thr Tyr
   145                 150                 155                 160
   Arg Pro Lys Arg Pro Thr Thr Leu Asn Leu Phe Pro Gln Val Pro Arg
                   165                 170                 175
   Ser Gln Asp Thr Leu Asn Asn Asn Ser Leu Gly Lys Lys His Ser Trp
               180                 185                 190
   Gln Asp Arg Val Ser Arg Ser Ser Ser Pro Leu Lys Thr Gly Glu Gln
           195                 200                 205
   Thr Pro Pro His Glu His Ile Cys Leu Ser Asp Glu Leu Pro Pro Gln
       210                 215                 220
   Gly Ser Pro Val Pro Thr Gln Asp Arg Gly Thr Ser Thr Asp Ser Pro
   225                 230                 235                 240
   Cys Arg Arg Thr Ala Ala Thr Gln Met Ala Pro Pro Ser Gly Pro Pro
                   245                 250                 255
   Ala Thr Ala Pro Gly Gly Arg Gly His Ser His Arg Asp Arg Ser Ile
               260                 265                 270
   Ser Ala Asp Val Arg Leu Glu Ala Thr Glu Glu Ile Tyr Leu Thr Pro
           275                 280                 285
   Val Gln Arg Pro Pro Asp Pro Ala Glu Pro Thr Ser Thr Phe Leu Pro
       290                 295                 300
   Pro Thr Glu Ser Arg Met Ser Val Ser Ser Asp Pro Asp Pro Ala Ala
   305                 310                 315                 320
   Tyr Ser Val Thr Ala Gly Arg Pro His Pro Ser Ile Ser Glu Glu Asp
                   325                 330                 335
   Glu Gly Phe Asp Cys Leu Ser Ser Pro Glu Gln Ala Glu Pro Pro Gly
               340                 345                 350
   Gly Gly Trp Arg Gly Ser Leu Gly Glu Pro Pro Pro Pro Pro Arg Ala
           355                 360                 365
   Ser Leu Ser Ser Asp Thr Ser Ala Leu Ser Tyr Asp Ser Val Lys Tyr
       370                 375                 380
   Thr Leu Val Val Asp Glu His Ala Gln Leu Glu Leu Val Ser Leu Arg
   385                 390                 395                 400
   Pro Cys Phe Gly Asp Tyr Ser Asp Glu Ser Asp Ser Ala Thr Val Tyr
                   405                 410                 415
   Asp Asn Cys Ala Ser Ala Ser Ser Pro Tyr Glu Ser Ala Ile Gly Glu
               420                 425                 430
   Glu Tyr Glu Glu Ala Pro Gln Pro Arg Pro Pro Thr Cys Leu Ser Glu
           435                 440                 445
   Asp Ser Thr Pro Asp Glu Pro Asp Val His Phe Ser Lys Lys Phe Leu
       450                 455                 460
   Asn Val Phe Met Ser Gly Arg Ser Arg Ser Ser Ser Ala Glu Ser Phe
   465                 470                 475                 480
   Gly Leu Phe Ser Cys Val Ile Asn Gly Glu Glu His Glu Gln Thr His
                   485                 490                 495
   Arg Ala Ile Phe Arg Phe Val Pro Arg His Glu Asp Glu Leu Glu Leu
               500                 505                 510
   Glu Val Asp Asp Pro Leu Leu Val Glu Leu Gln Ala Glu Asp Tyr Trp
           515                 520                 525
   Tyr Glu Ala Tyr Asn Met Arg Thr Gly Ala Arg Gly Val Phe Pro Ala
       530                 535                 540
   Tyr Tyr Ala Ile Glu Val Thr Lys Glu Pro Glu His Met Ala Ala Leu
   545                 550                 555                 560
   Ala Lys Asn Ser Asp Trp Ile Asp Gln Phe Arg Val Lys Phe Leu Gly
                   565                 570                 575
   Ser Val Gln Val Pro Tyr His Lys Gly Asn Asp Val Leu Cys Ala Ala
               580                 585                 590
   Met Gln Lys Ile Ala Thr Thr Arg Arg Leu Thr Val His Phe Asn Pro
           595                 600                 605
   Pro Ser Ser Cys Val Leu Glu Ile Ser Val Arg Gly Val Lys Ile Gly
       610                 615                 620
   Val Lys Ala Asp Glu Ala Gln Glu Ala Lys Gly Asn Lys Cys Ser His
   625                 630                 635                 640
   Phe Phe Gln Leu Lys Asn Ile Ser Phe Cys Gly Tyr His Pro Lys Asn
                   645                 650                 655
   Asn Lys Tyr Phe Gly Phe Ile Thr Lys His Pro Ala Asp His Arg Phe
               660                 665                 670
   Ala Cys His Val Phe Val Ser Glu Asp Ser Thr Lys Ala Leu Ala Glu
           675                 680                 685
   Ser Val Gly Arg Ala Phe Gln Gln Phe Tyr Lys Gln Phe Val Glu Tyr
       690                 695                 700
   Thr Cys Pro Thr Glu Asp Ile Tyr Leu Glu
   705 710
<210> 139
   <211> 711
   <212> PRT
   <213> Homo sapiens
<220>
   <221> misc_feature
   <223> description of sequence: human IB1 protein sequence
<220>
   <221> misc_feature
   <223> description of sequence: human IB1 protein sequence
<400> 139
   Met Ala Glu Arg Glu Ser Gly Gly Leu Gly Gly Gly Ala Ala Ser Pro
   1 5 10 15
   Pro Ala Ala Ser Pro Phe Leu Gly Leu His Ile Ala Ser Pro Pro Asn
   20 25 30
   Phe Arg Leu Thr His Asp Ile Ser Leu Glu Glu Phe Glu Asp Glu Asp
   35 40 45
   Leu Ser Glu Ile Thr Asp Glu Cys Gly Ile Ser Leu Gln Cys Lys Asp
   50 55 60
   Thr Leu Ser Leu Arg Pro Pro Arg Ala Gly Leu Leu Ser Ala Gly Gly
   65 70 75 80
   Gly Gly Ala Gly Ser Arg Leu Gln Ala Glu Met Leu Gln Met Asp Leu
   85 90 95
   Ile Asp Ala Thr Gly Asp Thr Pro Gly Ala Glu Asp Asp Glu Glu Asp
   100 105 110
   Asp Asp Glu Glu Arg Ala Ala Arg Arg Pro Gly Ala Gly Pro Pro Lys
   115 120 125
   Ala Glu Ser Gly Gln Glu Pro Ala Ser Arg Gly Gln Gly Gln Ser Gln
   130 135 140
   Gly Gln Ser Gln Gly Pro Gly Ser Gly Asp Thr Tyr Arg Pro Lys Arg
   145 150 155 160
   Pro Thr Thr Leu Asn Leu Phe Pro Gln Val Pro Arg Ser Gln Asp Thr
   165 170 175
   Leu Asn Asn Asn Ser Leu Gly Lys Lys His Ser Trp Gln Asp Arg Val
   180 185 190
   Ser Arg Ser Ser Ser Pro Leu Lys Thr Gly Glu Gln Thr Pro Pro His
           195                 200                 205
   Glu His Ile Cys Leu Ser Asp Glu Leu Pro Pro Gln Ser Gly Pro Ala
       210                 215                 220
   Pro Thr Thr Asp Arg Gly Thr Ser Thr Asp Ser Pro Cys Arg Arg Ser
   225                 230                 235                 240
   Thr Ala Thr Gln Met Ala Pro Pro Gly Gly Pro Pro Ala Ala Pro Pro
                   245                 250                 255
   Gly Gly Arg Gly His Ser His Arg Asp Arg Ile His Tyr Gln Ala Asp
               260                 265                 270
   Val Arg Leu Glu Ala Thr Glu Glu Ile Tyr Leu Thr Pro Val Gln Arg
           275                 280                 285
   Pro Pro Asp Ala Ala Glu Pro Thr Ser Ala Phe Leu Pro Pro Thr Glu
       290                 295                 300
   Ser Arg Met Ser Val Ser Ser Asp Pro Asp Pro Ala Ala Tyr Pro Ser
   305                 310                 315                 320
   Thr Ala Gly Arg Pro His Pro Ser Ile Ser Glu Glu Glu Glu Gly Phe
                   325                 330                 335
   Asp Cys Leu Ser Ser Pro Glu Arg Ala Glu Pro Pro Gly Gly Gly Trp
               340                 345                 350
   Arg Gly Ser Leu Gly Glu Pro Pro Pro Pro Pro Arg Ala Ser Leu Ser
           355                 360                 365
   Ser Asp Thr Ser Ala Leu Ser Tyr Asp Ser Val Lys Tyr Thr Leu Val
       370                 375                 380
   Val Asp Glu His Ala Gln Leu Glu Leu Val Ser Leu Arg Pro Cys Phe
   385                 390                 395                 400
   Gly Asp Tyr Ser Asp Glu Ser Asp Ser Ala Thr Val Tyr Asp Asn Cys
                   405                 410                 415
   Ala Ser Val Ser Ser Pro Tyr Glu Ser Ala Ile Gly Glu Glu Tyr Glu
               420                 425                 430
   Glu Ala Pro Arg Pro Gln Pro Pro Ala Cys Leu Ser Glu Asp Ser Thr
           435                 440                 445
   Pro Asp Glu Pro Asp Val His Phe Ser Lys Lys Phe Leu Asn Val Phe
       450                 455                 460
   Met Ser Gly Arg Ser Arg Ser Ser Ser Ala Glu Ser Phe Gly Leu Phe
   465 470 475 480
   Ser Cys Ile Ile Asn Gly Glu Glu Gln Glu Gln Thr His Arg Ala Ile
   485 490 495
   Phe Arg Phe Val Pro Arg His Glu Asp Glu Leu Glu Leu Glu Val Asp
   500 505 510
   Asp Pro Leu Leu Val Glu Leu Gln Ala Glu Asp Tyr Trp Tyr Glu Ala
   515 520 525
   Tyr Asn Met Arg Thr Gly Ala Arg Gly Val Phe Pro Ala Tyr Tyr Ala
   530 535 540
   Ile Glu Val Thr Lys Glu Pro Glu His Met Ala Ala Leu Ala Lys Asn
   545 550 555 560
   Ser Asp Trp Val Asp Gln Phe Arg Val Lys Phe Leu Gly Ser Val Gln
   565 570 575
   Val Pro Tyr His Lys Gly Asn Asp Val Leu Cys Ala Ala Met Gln Lys
   580 585 590
   Ile Ala Thr Thr Arg Arg Leu Thr Val His Phe Asn Pro Pro Ser Ser
   595 600 605
   Cys Val Leu Glu Ile Ser Val Arg Gly Val Lys Ile Gly Val Lys Ala
   610 615 620
   Asp Asp Ser Gln Glu Ala Lys Gly Asn Lys Cys Ser His Phe Phe Gln
   625 630 635 640
   Leu Lys Asn Ile Ser Phe Cys Gly Tyr His Pro Lys Asn Asn Lys Tyr
   645 650 655
   Phe Gly Phe Ile Thr Lys His Pro Ala Asp His Arg Phe Ala Cys His
   660 665 670
   Val Phe Val Ser Glu Asp Ser Thr Lys Ala Leu Ala Glu Ser Val Gly
   675 680 685
   Arg Ala Phe Gln Gln Phe Tyr Lys Gln Phe Val Glu Tyr Thr Cys Pro
   690 695 700
   Thr Glu Asp Ile Tyr Leu Glu
   705 710
<210> 140
   <211> 2136
   <212> DNA
   <213> Homo sapiens
<220>
   <221> misc_feature
   <223> description of sequence: nucleic acid sequence encoding human IB1 protein
<400> 140
   atggcggagc gagaaagcgg cggcctggga gggggggccg cgtccccgcc cgccgcctcc 60
   ccgttcctgg ggctgcacat cgcttcgcct cccaatttca ggctcaccca tgacatcagc 120
   ctggaggagt ttgaggatga agacctctcg gagatcactg atgagtgtgg catcagctta 180
   cagtgcaaag acaccctgtc cttacggccc ccgcgcgccg ggctgctctc tgcgggcggc 240
   ggcggcgcgg ggagccggtt gcaggccgag atgctgcaga tggacctgat cgacgcgacg 300
   ggggacactc ccggggccga ggacgacgag gaggacgacg acgaggagcg cgcggcccgg 360
   cggccgggag cggggccgcc caaggccgag tccggccagg agccggcgtc ccgcggccag 420
   ggccagagcc aaggccagag ccagggcccg ggcagcgggg acacgtaccg gcccaagcgg 480
   cccaccacgc tcaacctctt tccgcaggtg ccgcggtctc aggacacact gaataataat 540
   tctctgggca aaaagcacag ttggcaggat cgggtgtctc gatcatcctc acccctgaag 600
   acaggggagc agacaccacc gcatgaacac atctgcctga gcgatgagct gcccccccag 660
   agcggccccg cccccaccac agatcgaggc acctccaccg acagcccttg ccgccgcagc 720
   acagccaccc agatggcacc tccgggtggt ccccctgctg ccccgcctgg gggtcggggc 780
   cactcgcatc gagaccgaat ccactaccag gccgatgtgc gactagaggc cactgaggag 840
   atctacctga ccccagtgca gaggccccca gacgctgcag agcccacctc cgccttcctg 900
   ccgcccactg agagccggat gtcagtcagc tccgatccag accctgccgc ctacccctcc 960
   acggcagggc ggccgcaccc ctccatcagt gaagaggaag agggcttcga ctgcctgtcg 1020
   tccccagagc gggctgagcc cccaggcgga gggtggcggg ggagcctggg ggagccgccg 1080
   ccacctccac gggcctctct gagctcggac accagcgccc tgtcctatga ctctgtcaag 1140
   tacacgctgg tggtagatga gcatgcacag ctggagctgg tgagcctgcg gccgtgcttc 1200
   ggagactaca gtgacgagag tgactctgcc accgtctatg acaactgtgc ctccgtctcc 1260
   tcgccctatg agtcggccat cggagaggaa tatgaggagg ccccgcggcc ccagccccct 1320
   gcctgcctct ccgaggactc cacgcctgat gaacccgacg tccatttctc caagaaattc 1380
   ctgaacgtct tcatgagtgg ccgctcccgc tcctccagtg ctgagtcctt cgggctgttc 1440
   tcctgcatca tcaacgggga ggagcaggag cagacccacc gggccatatt caggtttgtg 1500
   cctcgacacg aagacgaact tgagctggaa gtggatgacc ctctgctagt ggagctccag 1560
   gctgaagact actggtacga ggcctacaac atgcgcactg gtgcccgggg tgtctttcct 1620
   gcctattacg ccatcgaggt caccaaggag cccgagcaca tggcagccct ggccaaaaac 1680
   agtgactggg tggaccagtt ccgggtgaag ttcctgggct cagtccaggt tccctatcac 1740
   aagggcaatg acgtcctctg tgctgctatg caaaagattg ccaccacccg ccggctcacc 1800
   gtgcacttta acccgccctc cagctgtgtc ctggagatca gcgtgcgggg tgtgaagata 1860
   ggcgtcaagg ccgatgactc ccaggaggcc aaggggaata aatgtagcca ctttttccag 1920
   ttaaaaaaca tctctttctg cggatatcat ccaaagaaca acaagtactt tgggttcatc 1980
   accaagcacc ccgccgacca ccggtttgcc tgccacgtct ttgtgtctga agactccacc 2040
   aaagccctgg cagagtccgt ggggagagca ttccagcagt tctacaagca gtttgtggag 2100
   tacacctgcc ccacagaaga tatctacctg gagtag 2136
<210> 141
   <211> 19
   <212> PRT
   <213> Artificial
<220>
   <223> Description of sequence: peptide L-IB1(s) (see Table 3)
<400> 141
   Arg Pro Lys Arg Pro Thr Thr Leu Asn Leu Phe Pro Gln Val Pro Arg
   1               5                   10                  15
   Ser Gln Asp
<210> 142
   <211> 19
   <212> PRT
   <213> Artificial
<220>
   <223> Description of sequence: Peptide D-IB1(s) (see Table 3)
<220>
   <221> MUTAGEN
   <222> (1) .. (19)
   <223> all amino acids are D-amino acids
<400> 142
   Asp Gln Ser Arg Pro Val Gln Pro Phe Leu Asn Leu Thr Thr Pro Arg
   1               5                   10                  15
   Lys Pro Arg
<210> 143
   <211> 19
   <212> PRT
   <213> Artificial
<220>
   <223> Description of sequence: Peptide L-IB (generic) (s) (see Table 3)
<220>
   <221> misc_feature
   <223> Description of sequence: Description of sequence: general formula: NH2-Xnb-Xna-RPTTLXLXXXXXXXQD-Xnb-COOH (see Table 1)
<220>
   <221> VARIANT
   <222> (1) .. (1)
   <223> Xaa is Xnb as defined in the general formula, wherein Xaa represents an amino acid residue, preferably selected from any (native) amino acid residue;
<220>
   <221> REPEAT
   <222> (1) .. (1)
   <223> Xaa is Xnb as defined in the general formula, wherein n is 0-5, 5-10, 10-15, 15-20, 20-30 or more for Xnb
<220>
   <221> VARIANT
   <222> (2)..(2)
   <223> Xaa is Xna as defined in the general formula, wherein Xaa represents an amino acid residue, preferably selected from any (native) amino acid residue residue except serine and threonine
<220>
   <221> REPEAT
   <222> (2)..(2)
   <223> Xaa is Xna as defined in the general formula, wherein n is 0 or 1
<220>
   <221> VARIANT
   <222> (8)..(8)
   <223> Xaa represents an amino acid residue, preferably selected from any (native) amino acid residue;
<220>
   <221> VARIANT
   <222> (10)..(16)
   <223> Xaa represents an amino acid residue, preferably selected from any (native) amino acid residue;
<220>
   <221> REPEAT
   <222> (19)..(19)
   <223> Xaa is Xnb as defined in the general formula, wherein n is 0-5, 5-10, 10-15, 15-20, 20-30 or more for Xnb
<220>
   <221> VARIANT
   <222> (19) .. (19)
   <223> Xaa is Xnb as defined in the general formula, wherein Xaa represents an amino acid residue, preferably selected from any (native) amino acid residue;
<400> 143
   Xaa Xaa Arg Pro Thr Thr Leu Xaa Leu Xaa Xaa Xaa Xaa Xaa Xaa Xaa
   1               5                   10                  15
   Gln Asp Xaa
<210> 144
   <211> 19
   <212> PRT
   <213> Artificial
<220>
   <223> Description of sequence: Peptide D-IB (generic) (s) (see Table 3)
<220>
   <221> misc_feature
   <223> Description of sequence: general formula: NH2-Xnb-DQXXXXXXXLXLTTPR-Xna-Xnb-COOH,
<220>
   <221> MUTAGEN
   <222> (1)..(19)
   <223> all amino acids are D-amino acids
<220>
   <221> VARIANT
   <222> (1) .. (11)
   <223> Xaa is xnb as defined in the general formula, wherein Xaa represents an amino acid residue, preferably selected from any (native) amino acid residue;
<220>
   <221> REPEAT
   <222> (1) .. (1)
   <223> Xaa is xnb as defined in the general formula, wherein n is 0-5, 5-10, 10-15, 15-20, 20-30 or more for Xnb
<220>
   <221> VARIANT
   <222> (4) .. (10)
   <223> Xaa represents an amino acid residue, preferably selected from any (native) amino acid residue;
<220>
   <221> VARIANT
   <222> (12)..(12)
   <223> xaa represents an amino acid residue, preferably selected from any (native) amino acid residue;
<220>
   <221> REPEAT
   <222> (18)..(18)
   <223> Xaa is Xna as defined in the general formula, wherein n is 0 or 1
<220>
   <221> VARIANT
   <222> (18)..(18)
   <223> Xaa is Xna as defined in the general formula, wherein Xaa represents an amino acid residue, preferably selected from any (native) amino acid residue residue except serine and Threonine
<220>
   <221> VARIANT
   <222> (18)..(18)
   <223> Xaa is Xna as defined in the general formula, wherein Xaa represents an amino acid residue, preferably selected from any (native) amino acid residue residue except serine and threonine
<220>
   <221> REPEAT
   <222> (19)..(19)
   <223> Xaa is Xnb as defined in the general formula, wherein n is 0-5, 5-10, 10-15, 15-20, 20-30 or more for Xnb
<220>
   <221> VARIANT
   <222> (19)..(19)
   <223> Xaa is Xnb as defined in the general formula, wherein Xaa represents an amino acid residue, preferably selected from any (native) amino acid residue;
<400> 144
   Xaa Asp Gln Xaa Xaa Xaa Xaa Xaa Xaa Xaa Leu Xaa Leu Thr Thr Pro
   1               5                   10                  15
   Arg Xaa Xaa
<210> 145
   <211> 29
   <212> PRT
   <213> Artificial
<220>
   <223> Description of sequence: peptide IB1-long (see Table 3)
<400> 145
   Pro Gly Thr Gly Cys Gly Asp Thr Tyr Arg Pro Lys Arg Pro Thr Thr
   1               5                   10                  15
   Leu Asn Leu Phe Pro Gln Val Pro Arg Ser Gln Asp Thr
               20                  25
<210> 146
   <211> 27
   <212> PRT
   <213> Artificial
<220>
   <223> Description of sequence: Peptide IB2-long (see Table 3)
<400> 146
   Ile Pro Ser Pro Ser Val Glu Glu Pro His Lys His Arg Pro Thr Thr
   1               5                   10                  15
   Leu Arg Leu Thr Thr Leu Gly Ala Gln Asp Ser
               20                  25
<210> 147
   <211> 29
   <212> PRT
   <213> Artificial
<220>
   <223> Description of sequence: Peptide derived from C-Jun (see Table 3)
<400> 147
   Gly Ala Tyr Gly Tyr Ser Asn Pro Lys Ile Leu Lys Gln Ser Met Thr
   1               5                   10                  15
   Leu Asn Leu Ala Asp Pro Val Gly Asn Leu Lys Pro His
               20                  25
<210> 148
   <211> 29
   <212> PRT
   <213> Artificial
<220>
   <223> Description of sequence: Peptide derived from ATF2 (see Table 3)
<400> 148
   Thr Asn Glu Asp His Leu Ala Val His Lys His Lys His Glu Met Thr
   1               5                   10                  15
   Leu Lys Phe Gly Pro Ala Arg Asn Asp Ser Val Ile Val
               20                  25
<210> 149
   <211> 23
   <212> PRT
   <213> Artificial
<220>
   <223> Description of sequence: Peptide L-IB1 (see Table 3)
<400> 149
   Asp Thr Tyr Arg Pro Lys Arg Pro Thr Thr Leu Asn Leu Phe Pro Gln
   1               5                   10                  15
   Val Pro Arg Ser Gln Asp Thr
               20
<210> 150
   <211> 23
   <212> PRT
   <213> Artificial
<220>
   <223> Description of sequence: Peptide D-IB1 (see Table 3)
<220>
   <221> MUTAGEN
   <222> (1)..(23)
   <223> all amino acids are D-amino acids
<400> 150
   Thr Asp Gln Ser Arg Pro Val Gln Pro Phe Leu Asn Leu Thr Thr Pro
   1 5 10 15
   Arg Lys Pro Arg Tyr Thr Asp
   20
<210> 151
   <211> 19
   <212> PRT
   <213> Artificial
<220>
   <223> Description of sequence: Peptide L-IB (generic) (see Table 3)
<220>
   <221> VARIANT
   <222> (1) .. (1)
   <223> Xaa is selected from any amino acid residue,
<220>
   <221> VARIANT
   <222> (7)..(7)
   <223> Xaa is selected from any amino acid residue,
<220>
   <221> VARIANT
   <222> (9)..(15)
   <223> Xaa is selected from any amino acid residue,
<220>
   <221> VARIANT
   <222> (18)..(18)
   <223> Xaa is selected from serine or threonine,
<220>
   <221> VARIANT
   <222> (19)..(19)
   <223> Xaa is selected from any amino acid residue,
<400> 151
   Xaa Arg Pro Thr Thr Leu Xaa Leu Xaa Xaa Xaa Xaa Xaa Xaa Xaa Gln
   1 5 10 15
   Asp Xaa Xaa
<210> 152
   <211> 19
   <212> PRT
   <213> Artificial
<220>
   <223> Description of sequence: Peptide D-IB (generic) (see Table 3)
<220>
   <221> MUTAGEN
   <222> (1)..(19)
   <223> all amino acids are D-amino acids
<220>
   <221> VARIANT
   <222> (1)..(1)
   <223> Xaa is selected from any amino acid residue
<220>
   <221> VARIANT
   <222> (2)..(2)
   <223> Xaa is selected from serine or threonine
<220>
   <221> VARIANT
   <222> (5)..(11)
   <223> Xaa is selected from any amino acid residue
<220>
   <221> VARIANT
   <222> (13)..(13)
   <223> Xaa is selected from any amino acid residue
<220>
   <221> VARIANT
   <222> (19) .. (19)
   <223> Xaa is selected from any amino acid residue
<400> 152
   Xaa Xaa Asp Gln Xaa Xaa Xaa Xaa Xaa Xaa Xaa Leu Xaa Leu Thr Thr
   1               5                   10                  15
   Pro Arg Xaa
<210> 153
   <211> 13
   <212> PRT
   <213> Artificial
<220>
   <223> Description of sequence: L-IB1(s1) (see Table 3)
<400> 153
   Thr Leu Asn Leu Phe Pro Gln Val Pro Arg Ser Gln Asp
   1               5                   10
<210> 154
   <211> 13
   <212> PRT
   <213> Artificial
<220>
   <223> Description of sequence: L-IB1(s2) (see Table 3)
<400> 154
   Thr Thr Leu Asn Leu Phe Pro Gln Val Pro Arg Ser Gln
   1               5                   10
<210> 155
   <211> 13
   <212> PRT
   <213> Artificial
<220>
   <223> Description of sequence: L-IB1(s3) (see Table 3)
<400> 155
   Pro Thr Thr Leu Asn Leu Phe Pro Gln Val Pro Arg Ser
   1               5                   10
<210> 156
   <211> 13
   <212> PRT
   <213> Artificial
<220>
   <223> Description of sequence: L-IB1(s4) (see Table 3)
<400> 156
   Arg Pro Thr Thr Leu Asn Leu Phe Pro Gln Val Pro Arg
   1               5                   10
<210> 157
   <211> 13
   <212> PRT
   <213> Artificial
<220>
   <223> Description of sequence: L-IB1(s5) (see Table 3)
<400> 157
   Lys Arg Pro Thr Thr Leu Asn Leu Phe Pro Gln Val Pro
   1               5                   10
<210> 158
   <211> 13
   <212> PRT
   <213> Artificial
<220>
   <223> Description of sequence: L-IB1(s6) (see Table 3)
<400> 158
   Pro Lys Arg Pro Thr Thr Leu Asn Leu Phe Pro Gln Val
   1               5                   10
<210> 159
   <211> 13
   <212> PRT
   <213> Artificial
<220>
   <223> Description of sequence: L-IB1(s7) (see Table 3)
<400> 159
   Arg Pro Lys Arg Pro Thr Thr Leu Asn Leu Phe Pro Gln
   1 5 10
<210> 160
   <211> 12
   <212> PRT
   <213> Artificial
<220>
   <223> Description of sequence: L-IB1(s8) (see Table 3)
<400> 160
   Leu Asn Leu Phe Pro Gln Val Pro Arg Ser Gln Asp
   1 5 10
<210> 161
   <211> 12
   <212> PRT
   <213> Artificial
<220>
   <223> Description of sequence: L-IB1(s9) (see Table 3)
<400> 161
   Thr Leu Asn Leu Phe Pro Gln Val Pro Arg Ser Gln
   1 5 10
<210> 162
   <211> 12
   <212> PRT
   <213> Artificial
<220>
   <223> Description of sequence: L-IB1(s10) (see Table 3)
<400> 162
   Thr Thr Leu Asn Leu Phe Pro Gln Val Pro Arg Ser
   1 5 10
<210> 163
   <211> 12
   <212> PRT
   <213> Artificial
<220>
   <223> Description of sequence: L-IB1(s11) (see Table 3)
<400> 163
   Pro Thr Thr Leu Asn Leu Phe Pro Gln Val Pro Arg
   1 5 10
<210> 164
   <211> 12
   <212> PRT
   <213> Artificial
<220>
   <223> Description of sequence: L-IB1(s12) (see Table 3)
<400> 164
   Arg Pro Thr Thr Leu Asn Leu Phe Pro Gln Val Pro
   1 5 10
<210> 165
   <211> 12
   <212> PRT
   <213> Artificial
<220>
   <223> Description of sequence: L-IB1(s13) (see Table 3)
<400> 165
   Lys Arg Pro Thr Thr Leu Asn Leu Phe Pro Gln Val
   1 5 10
<210> 166
   <211> 12
   <212> PRT
   <213> Artificial
<220>
   <223> Description of sequence: L-IB1(s14) (see Table 3)
<400> 166
   Pro Lys Arg Pro Thr Thr Leu Asn Leu Phe Pro Gln
   1 5 10
<210> 167
   <211> 12
   <212> PRT
   <213> Artificial
<220>
   <223> Description of sequence: L-IB1(s15) (see Table 3)
<400> 167
   Arg Pro Lys Arg Pro Thr Thr Leu Asn Leu Phe Pro
   1 5 10
<210> 168
   <211> 11
   <212> PRT
   <213> Artificial
<220>
   <223> Description of sequence: L-IB1(s16) (see Table 3)
<400> 168
   Asn Leu Phe Pro Gln Val Pro Arg Ser Gln Asp
   1 5 10
<210> 169
   <211> 11
   <212> PRT
   <213> Artificial
<220>
   <223> Description of sequence: L-IB1(s17) (see Table 3)
<400> 169
   Leu Asn Leu Phe Pro Gln Val Pro Arg Ser Gln
   1 5 10
<210> 170
   <211> 11
   <212> PRT
   <213> Artificial
<220>
   <223> Description of sequence: L-IB1(s18) (see Table 3)
<400> 170
   Thr Leu Asn Leu Phe Pro Gln Val Pro Arg Ser
   1 5 10
<210> 171
   <211> 11
   <212> PRT
   <213> Artificial
<220>
   <223> Description of sequence: L-IB1(s19) (see Table 3)
<400> 171
   Thr Thr Leu Asn Leu Phe Pro Gln Val Pro Arg
   1 5 10
<210> 172
   <211> 11
   <212> PRT
   <213> Artificial
<220>
   <223> Description of sequence: L-IB1(s20) (see Table 3)
<400> 172
   Pro Thr Thr Leu Asn Leu Phe Pro Gln Val Pro
   1 5 10
<210> 173
   <211> 11
   <212> PRT
   <213> Artificial
<220>
   <223> Description of sequence: L-IB1(s21) (see Table 3)
<400> 173
   Arg Pro Thr Thr Leu Asn Leu Phe Pro Gln Val
   1 5 10
<210> 174
   <211> 11
   <212> PRT
   <213> Artificial
<220>
   <223> Description of sequence: L-IB1(s22) (see Table 3)
<400> 174
   Lys Arg Pro Thr Thr Leu Asn Leu Phe Pro Gln
   1 5 10
<210> 175
   <211> 11
   <212> PRT
   <213> Artificial
<220>
   <223> Description of sequence: L-IB1(s23) (see Table 3)
<400> 175
   Pro Lys Arg Pro Thr Thr Leu Asn Leu Phe Pro
   1 5 10
<210> 176
   <211> 11
   <212> PRT
   <213> Artificial
<220>
   <223> Description of sequence: L-IB1(s24) (see Table 3)
<400> 176
   Arg Pro Lys Arg Pro Thr Thr Leu Asn Leu Phe
   1 5 10
<210> 177
   <211> 10
   <212> PRT
   <213> Artificial
<220>
   <223> Description of sequence: L-IB1(s25) (see Table 3)
<400> 177
   Leu Phe Pro Gln Val Pro Arg Ser Gln Asp
   1 5 10
<210> 178
   <211> 10
   <212> PRT
   <213> Artificial
<220>
   <223> Description of sequence: L-IB1(s26) (see Table 3)
<400> 178
   Asn Leu Phe Pro Gln Val Pro Arg Ser Gln
   1 5 10
<210> 179
   <211> 10
   <212> PRT
   <213> Artificial
<220>
   <223> Description of sequence: L-IB1(s27) (see Table 3)
<400> 179
   Leu Asn Leu Phe Pro Gln Val Pro Arg Ser
   1 5 10
<210> 180
   <211> 10
   <212> PRT
   <213> Artificial
<220>
   <223> Description of sequence: L-IB1(s28) (see Table 3)
<400> 180
   Thr Leu Asn Leu Phe Pro Gln Val Pro Arg
   1 5 10
<210> 181
   <211> 10
   <212> PRT
   <213> Artificial
<220>
   <223> Description of sequence: L-IB1(s29) (see Table 3)
<400> 181
   Thr Thr Leu Asn Leu Phe Pro Gln Val Pro
   1 5 10
<210> 182
   <211> 10
   <212> PRT
   <213> Artificial
<220>
   <223> Description of sequence: L-IB1(s30) (see Table 3)
<400> 182
   Pro Thr Thr Leu Asn Leu Phe Pro Gln Val
   1 5 10
<210> 183
   <211> 10
   <212> PRT
   <213> Artificial
<220>
   <223> Description of sequence: L-IB1(s31) (see Table 3)
<400> 183
   Arg Pro Thr Thr Leu Asn Leu Phe Pro Gln
   1 5 10
<210> 184
   <211> 10
   <212> PRT
   <213> Artificial
<220>
   <223> Description of sequence: L-IB1(s32) (see Table 3)
<400> 184
   Lys Arg Pro Thr Thr Leu Asn Leu Phe Pro
   1 5 10
<210> 185
   <211> 10
   <212> PRT
   <213> Artificial
<220>
   <223> Description of sequence: L-IB1(s33) (see Table 3)
<400> 185
   Pro Lys Arg Pro Thr Thr Leu Asn Leu Phe
   1 5 10
<210> 186
   <211> 10
   <212> PRT.
   <213> Artificial
<220>
   <223> Description of sequence: L-IB1(s34) (see Table 3)
<400> 186
   Arg Pro Lys Arg Pro Thr Thr Leu Asn Leu
   1 5 10
<210> 187
   <211> 13
   <212> PRT
   <213> Artificial
<220>
   <223> Description of sequence: D-IB1(s1) (see Table 3)
<220>
   <221> MUTAGEN
   <222> (1)..(13)
   <223> all amino acids are D-amino acids
<400> 187
   Gln Pro Phe Leu Asn Leu Thr Thr Pro Arg Lys Pro Arg
   1 5 10
<210> 188
   <211> 13
   <212> PRT
   <213> Artificial
<220>
   <223> Description of sequence: D-IB1(s2) (see Table 3)
<220>
   <221> MUTAGEN
   <222> (1)..(13)
   <223> all amino acids are D-amino acids
<400> 188
   Val Gln Pro Phe Leu Asn Leu Thr Thr Pro Arg Lys Pro
   1               5                   10
<210> 189
   <211> 13
   <212> PRT
   <213> Artificial
<220>
   <223> Description of sequence: D-IB1(s3) (see Table 3)
<220>
   <221> MUTAGEN
   <222> (1)..(13)
   <223> all amino acids are D-amino acids
<400> 189
   Pro Val Gln Pro Phe Leu Asn Leu Thr Thr Pro Arg Lys
   1               5                   10
<210> 190
   <211> 13
   <212> PRT
   <213> Artificial
<220>
   <223> Description of sequence: D-IB1(s4) (see Table 3)
<220>
   <221> MUTAGEN
   <222> (1)..(13)
   <223> all amino acids are D-amino acids
<400> 190
   Arg Pro Val Gln Pro Phe Leu Asn Leu Thr Thr Pro Arg
   1               5                   10
<210> 191
   <211> 13
   <212> PRT
   <213> Artificial
<220>
   <223> Description of sequence: D-IB1(s5) (see Table 3)
<220>
   <221> MUTAGEN
   <222> (1)..(13)
   <223> all amino acids are D-amino acids
<400> 191
   Ser Arg Pro Val Gln Pro Phe Leu Asn Leu Thr Thr Pro
   1 5 10
<210> 192
   <211> 13
   <212> PRT
   <213> Artificial
<220>
   <223> Description of sequence: D-IB1(s6) (see Table 3)
<220>
   <221> MUTAGEN
   <222> (1)..(13)
   <223> all amino acids are D-amino acids
<400> 192
   Gln Ser Arg Pro Val Gln Pro Phe Leu Asn Leu Thr Thr
   1 5 10
<210> 193
   <211> 13
   <212> PRT
   <213> Artificial
<220>
   <223> Description of sequence: D-IB1(s7) (see Table 3)
<220>
   <221> MUTAGEN
   <222> (1)..(13)
   <223> all amino acids are D-amino acids
<400> 193
   Asp Gln Ser Arg Pro Val Gln Pro Phe Leu Asn Leu Thr
   1 5 10
<210> 194
   <211> 12
   <212> PRT
   <213> Artificial
<220>
   <223> Description of sequence: D-IB1(sB) (see Table 3)
<220>
   <221> MUTAGEN
   <222> (1)..(12)
   <223> all amino acids are D-amino acids
<400> 194
   Pro Phe Leu Asn Leu Thr Thr Pro Arg Lys Pro Arg
   1 5 10
<210> 195
   <211> 12
   <212> PRT
   <213> Artificial
<220>
   <223> Description of sequence: D-IB1(s9) (see Table 3)
<220>
   <221> MUTAGEN
   <222> (1)..(12)
   <223> all amino acids are D-amino acids
<400> 195
   Gln Pro Phe Leu Asn Leu Thr Thr Pro Arg Lys Pro
   1 5 10
<210> 196
   <211> 12
   <212> PRT
   <213> Artificial
<220>
   <223> Description of sequence: D-IB1(s10) (see Table 3)
<220>
   <221> MUTAGEN
   <222> (1)..(12)
   <223> all amino acids are D-amino acids
<400> 196
   Val Gln Pro Phe Leu Asn Leu Thr Thr Pro Arg Lys
   1 5 10
<210> 197
   <211> 12
   <212> PRT
   <213> Artificial
<220>
   <223> Description of sequence: D-IB1(s11) (see Table 3)
<220>
   <221> MUTAGEN
   <222> (1)..(12)
   <223> all amino acids are D-amino acids
<400> 197
   Pro Val Gln Pro Phe Leu Asn Leu Thr Thr Pro Arg
   1 5 10
<210> 198
   <211> 12
   <212> PRT
   <213> Artificial
<220>
   <223> Description of sequence: D-IB1(s12) (see Table 3)
<220>
   <221> MUTAGEN
   <222> (1)..(12)
   <223> all amino acids are D-amino acids
<400> 198
   Arg Pro Val Gln Pro Phe Leu Asn Leu Thr Thr Pro
   1 5 10
<210> 199
   <211> 12
   <212> PRT
   <213> Artificial
<220>
   <223> Description of sequence: D-IB1(s13) (see Table 3)
<220>
   <221> MUTAGEN
   <222> (1)..(12)
   <223> all amino acids are D-amino acids
<400> 199
   Ser Arg Pro Val Gln Pro Phe Leu Asn Leu Thr Thr
   1 5 10
<210> 200
   <211> 12
   <212> PRT
   <213> Artificial
<220>
   <223> Description of sequence: D=IB1(s14) (see Table 3)
<220>
   <221> MUTAGEN
   <222> (1)..(12)
   <223> all amino acids are D-amino acids
<400> 200
   Gln Ser Arg Pro Val Gln Pro Phe Leu Asn Leu Thr
   1 5 10
<210> 201
   <211> 12
   <212> PRT
   <213> Artificial
<220>
   <223> Description of sequence: D-IB1(s15) (see Table 3)
<220>
   <221> MUTAGEN
   <222> (1)..(12)
   <223> all amino acids are D-amino acids
<400> 201
   Asp Gln Ser Arg Pro Val Gln Pro Phe Leu Asn Leu
   1 5 10
<210> 202
   <211> 11
   <212> PRT
   <213> Artificial
<220>
   <223> Description of sequence: D-IB1(s16) (see Table 3)
<220>
   <221> MUTAGEN
   <222> (1)..(11)
   <223> all amino acids are D-amino acids
<400> 202
   Phe Leu Asn Leu Thr Thr Pro Arg Lys Pro Arg
   1 5 10
<210> 203
   <211> 11
   <212> PRT
   <213> Artificial
<220>
   <223> Description of sequence: D-IB1(s17) (see Table 3)
<220>
   <221> MUTAGEN
   <222> (1)..(11)
   <223> all amino acids are D-amino acids
<400> 203
   Pro Phe Leu Asn Leu Thr Thr Pro Arg Lys Pro
   1 5 10
<210> 204
   <211> 11
   <212> PRT
   <213> Artificial
<220>
   <223> Description of sequence: D-IB1(s18) (see Table 3)
<220>
   <221> MUTAGEN
   <222> (1)..(11)
   <223> all amino acids are D-amino acids
<400> 204
   Gln Pro Phe Leu Asn Leu Thr Thr Pro Arg Lys
   1 5 10
<210> 205
   <211> 11
   <212> PRT
   <213> Artificial
<220>
   <223> Description of sequence: D-IB1(s19) (see Table 3)
<220>
   <221> MUTAGEN
   <222> (1)..(11)
   <223> all amino acids are D-amino acids
<400> 205
   Val Gln Pro Phe Leu Asn Leu Thr Thr Pro Arg
   1 5 10
<210> 206
   <211> 11
   <212> PRT
   <213> Artificial
<220>
   <223> Description of sequence: D-IB1(s20) (see Table 3)
<220>
   <221> MUTAGEN
   <222> (1)..(11)
   <223> all amino acids are D-amino acids
<400> 206
   Pro Val Gln Pro Phe Leu Asn Leu Thr Thr Pro
   1 5 10
<210> 207
   <211> 11
   <212> PRT
   <213> Artificial
<220>
   <223> Description of sequence: D-IB1(s21) (see Table 3)
<220>
   <221> MUTAGEN
   <222> (1)..(11)
   <223> all amino acids are D-amino acids
<400> 207
   Arg Pro Val Gln Pro Phe Leu Asn Leu Thr Thr
   1               5                   10
<210> 208
   <211> 11
   <212> PRT
   <213> Artificial
<220>
   <223> Description of sequence: D-IB1(s22) (see Table 3)
<220>
   <221> MUTAGEN
   <222> (1)..(11)
   <223> all amino acids are D-amino acids
<400> 208
   Ser Arg Pro Val Gln Pro Phe Leu Asn Leu Thr
   1               5                   10
<210> 209
   <211> 11
   <212> PRT
   <213> Artificial
<220>
   <223> Description of sequence: D-IB1(s23) (see Table 3)
<220>
   <221> MUTAGEN
   <222> (1)..(11)
   <223> all amino acids are D-amino acids
<400> 209
   Gln Ser Arg Pro Val Gln Pro Phe Leu Asn Leu
   1               5                   10
<210> 210
   <211> 11
   <212> PRT
   <213> Artificial
<220>
   <223> Description of sequence: D-IB1(s24) (see Table 3)
<220>
   <221> MUTAGEN
   <222> (1)..(11)
   <223> all amino acids are D-amino acids
<400> 210
   Asp Gln Ser Arg Pro Val Gln Pro Phe Leu Asn
   1               5                   10
<210> 211
   <211> 10
   <212> PRT
   <213> Artificial
<220>
   <223> Description of sequence: D-IB1(s25) (see Table 3)
<220>
   <221> MUTAGEN
   <222> (1)..(10)
   <223> all amino acids are D-amino acids
<400> 211
   Asp Gln Ser Arg Pro Val Gln Pro Phe Leu
   1               5                   10
<210> 212
   <211> 10
   <212> PRT
   <213> Artificial
<220>
   <223> Description of sequence: D-IB1(s26) (see Table 1)
<220>
   <221> MUTAGEN
   <222> (1)..(10)
   <223> all amino acids are D-amino acids
<400> 212
   Gln Ser Arg Pro Val Gln Pro Phe Leu Asn
   1               5                   10
<210> 213
   <211> 10
   <212> PRT
   <213> Artificial
<220>
   <223> Description of sequence: D-IB1(s27) (see Table 3)
<220>
   <221> MUTAGEN
   <222> (1)..(10)
   <223> all amino acids are D-amino acids
<400> 213
   Ser Arg Pro Val Gln Pro Phe Leu Asn Leu
   1               5                   10
<210> 214
   <211> 10
   <212> PRT
   <213> Artificial
<220>
   <223> Description of sequence: D-IB1(s28) (see Table 3)
<220>
   <221> MUTAGEN
   <222> (1)..(10)
   <223> all amino acids are D-amino acids
<400> 214
   Arg Pro Val Gln Pro Phe Leu Asn Leu Thr
   1               5                   10
<210> 215
   <211> 10
   <212> PRT
   <213> Artificial
<220>
   <223> Description of sequence: D-IB1(s29) (see Table 3)
<220>
   <221> MUTAGEN
   <222> (1)..(10)
   <223> all amino acids are D-amino acids
<400> 215
   Pro Val Gln Pro Phe Leu Asn Leu Thr Thr
   1               5                   10
<210> 216
   <211> 10
   <212> PRT
   <213> Artificial
<220>
   <223> Description of sequence: D-IB1(s30) (see Table 3)
<220>
   <221> MUTAGEN
   <222> (1)..(10)
   <223> all amino acids are D-amino acids
<400> 216
   Val Gln Pro Phe Leu Asn Leu Thr Thr Pro
   1               5                   10
<210> 217
   <211> 10
   <212> PRT
   <213> Artificial
<220>
   <223> Description of sequence: D-IB1(s31) (see Table 3)
<220>
   <221> MUTAGEN
   <222> (1)..(10)
   <223> all amino acids are D-amino acids
<400> 217
   Gln Pro Phe Leu Asn Leu Thr Thr Pro Arg
   1               5                   10
<210> 218
   <211> 10
   <212> PRT
   <213> Artificial
<220>
   <223> Description of sequence: D-IB1(s32) (see Table 3)
<220>
   <221> MUTAGEN
   <222> (1)..(10)
   <223> all amino acids are D-amino acids
<400> 218
   Pro Phe Leu Asn Leu Thr Thr Pro Arg Lys
   1               5                   10
<210> 219
   <211> 10
   <212> PRT
   <213> Artificial
<220>
   <223> Description of sequence: D-IB1(s33) (see Table 3)
<220>
   <221> MUTAGEN
   <222> (1)..(10)
   <223> all amino acids are D-amino acids
<400> 219
   Phe Leu Asn Leu Thr Thr Pro Arg Lys Pro
   1               5                   10
<210> 220
   <211> 10
   <212> PRT
   <213> Artificial
<220>
   <223> Description of sequence: D-IB1(s34) (see Table 3)
<220>
   <221> MUTAGEN
   <222> (1)..(10)
   <223> all amino acids are D-amino acids
<400> 220
   Leu Asn Leu Thr Thr Pro Arg Lys Pro Arg
   1 5 10
<210> 221
   <211> 241
   <212> PRT
   <213> Homo sapiens
<220>
   <221> MISC_FEATURE
   <223> Description of sequence: amino acid sequence of Bid (human) (transcript variant 1)
<400> 221
   Met Cys Ser Gly Ala Gly Val Met Met Ala Arg Trp Ala Ala Arg Gly
   1               5                   10                  15
   Arg Ala Gly Trp Arg Ser Thr Val Arg Ile Leu Ser Pro Leu Gly His
               20                  25                  30
   Cys Glu Pro Gly Val Ser Arg Ser Cys Arg Ala Ala Gln Ala Met Asp
           35                  40                  45
   Cys Glu Val Asn Asn Gly Ser Ser Leu Arg Asp Glu Cys Ile Thr Asn
       50                  55                  60
   Leu Leu Val Phe Gly Phe Leu Gln Ser Cys Ser Asp Asn Ser Phe Arg
   65                  70                  75                  80
   Arg Glu Leu Asp Ala Leu Gly His Glu Leu Pro Val Leu Ala Pro Gln
                   85                  90                  95
   Trp Glu Gly Tyr Asp Glu Leu Gln Thr Asp Gly Asn Arg Ser Ser His
               100                 105                 110
   Ser Arg Leu Gly Arg Ile Glu Ala Asp Ser Glu Ser Gln Glu Asp Ile
           115                 120                 125
   Ile Arg Asn Ile Ala Arg His Leu Ala Gln Val Gly Asp Ser Met Asp
       130                 135                 140
   Arg Ser Ile Pro Pro Gly Leu Val Asn Gly Leu Ala Leu Gln Leu Arg
   145                 150                 155                 160
   Asn Thr Ser Arg Ser Glu Glu Asp Arg Asn Arg Asp Leu Ala Thr Ala
                   165                 170                 175
   Leu Glu Gln Leu Leu Gln Ala Tyr Pro Arg Asp Met Glu Lys Glu Lys
               180                 185                 190
   Thr Met Leu Val Leu Ala Leu Leu Leu Ala Lys Lys Val Ala Ser His
           195                 200                 205
   Thr Pro Ser Leu Leu Arg Asp Val Phe His Thr Thr Val Asn Phe Ile
       210                 215                 220
   Asn Gln Asn Leu Arg Thr Tyr Val Arg Ser Leu Ala Arg Asn Gly Met
   225                 230                 235                 240
   Asp
<210> 222
   <211> 168
   <212> PRT
   <213> Homo sapiens
<220>
   <221> MISC_FEATURE
   <223> Description of sequence: amino acid sequence of Bad (human)
<400> 222
   Met Phe Gln Ile Pro Glu Phe Glu Pro Ser Glu Gln Glu Asp Ser Ser
   1 5 10 15
   Ser Ala Glu Arg Gly Leu Gly Pro Ser Pro Ala Gly Asp Gly Pro Ser
   20 25 30
   Gly Ser Gly Lys His His Arg Gln Ala Pro Gly Leu Leu Trp Asp Ala
   35 40 45
   Ser His Gln Gln Glu Gln Pro Thr Ser Ser Ser His His Gly Gly Ala
   50 55 60
   Gly Ala Val Glu Ile Arg Ser Arg His Ser Ser Tyr Pro Ala Gly Thr
   65 70 75 80
   Glu Asp Asp Glu Gly Met Gly Glu Glu Pro Ser Pro Phe Arg Gly Arg
   85 90 95
   Ser Arg Ser Ala Pro Pro Asn Leu Trp Ala Ala Gln Arg Tyr Gly Arg
   100 105 110
   Glu Leu Arg Arg Met Ser Asp Glu Phe Val Asp Ser Phe Lys Lys Gly
   115 120 125
   Leu Pro Arg Pro Lys Ser Ala Gly Thr Ala Thr Gln Met Arg Gln Ser
   130 135 140
   Ser Ser Trp Thr Arg Val Phe Gln Ser Trp Trp Asp Arg Asn Leu Gly
   145 150 155 160
   Arg Gly Ser Ser Ala Pro Ser Gln
   165
<210> 223
   <211> 483
   <212> PRT
   <213> Homo sapiens
<220>
   <221> misc_feature
   <223> Description of sequence: amino acid sequence of Noxa1 (human)
<400> 223
   Met Ala Ser Leu Gly Asp Leu Val Arg Ala Trp His Leu Gly Ala Gln
   1               5                   10                  15
   Ala Val Asp Arg Gly Asp Trp Ala Arg Ala Leu His Leu Phe Ser Gly
               20                  25                  30
   Val Pro Ala Pro Pro Ala Arg Leu Cys Phe Asn Ala Gly Cys Val His
           35                  40                  45
   Leu Leu Ala Gly Asp Pro Glu Ala Ala Leu Arg Ala Phe Asp Gln Ala
       50                  55                  60
   Val Thr Lys Asp Thr Cys Met Ala Val Gly Phe Phe Gln Arg Gly Val
   65                  70                  75                  80
   Ala Asn Phe Gln Leu Ala Arg Phe Gln Glu Ala Leu Ser Asp Phe Trp
                   85                  90                  95
   Leu Ala Leu Glu Gln Leu Arg Gly His Ala Ala Ile Asp Tyr Thr Gln
               100                 105                 110
   Leu Gly Leu Arg Phe Lys Leu Gln Ala Trp Glu Val Leu His Asn Val
           115                 120                 125
   Ala Ser Ala Gln Cys Gln Leu Gly Leu Trp Thr Glu Ala Ala Ser Ser
       130                 135                 140
   Leu Arg Glu Ala Met Ser Lys Trp Pro Glu Gly Ser Leu Asn Gly Leu
   145                 150                 155                 160
   Asp Ser Ala Leu Asp Gln Val Gln Arg Arg Gly Ser Leu Pro Pro Arg
                   165                 170                 175
   Gln Val Pro Arg Gly Glu Val Phe Arg Pro His Arg Trp His Leu Lys
   180 185 190
   His Leu Glu Pro Val Asp Phe Leu Gly Lys Ala Lys Val Val Ala Ser
   195 200 205
   Ala Ile Pro Asp Asp Gln Gly Trp Gly Val Arg Pro Gln Gln Pro Gln
   210 215 220
   Gly Pro Gly Ala Asn His Asp Ala Arg Ser Leu Ile Met Asp Ser Pro
   225 230 235 240
   Arg Ala Gly Thr His Gln Gly Pro Leu Asp Ala Glu Thr Glu Val Gly
   245 250 255
   Ala Asp Arg Cys Thr Ser Thr Ala Tyr Gln Glu Gln Arg Pro Gln Val
   260 265 270
   Glu Gln Val Gly Lys Gln Ala Pro Leu Ser Pro Gly Leu Pro Ala Met
   275 280 285
   Gly Gly Pro Gly Pro Gly Pro Cys Glu Asp Pro Ala Gly Ala Gly Gly
   290 295 300
   Ala Gly Ala Gly Gly Ser Glu Pro Leu Val Thr Val Thr Val Gln Cys
   305 310 315 320
   Ala Phe Thr Val Ala Leu Arg Ala Arg Arg Gly Ala Asp Leu Ser Ser
   325 330 335
   Leu Arg Ala Leu Leu Gly Gln Ala Leu Pro His Gln Ala Gln Leu Gly
   340 345 350
   Gln Leu Ser Tyr Leu Ala Pro Gly Glu Asp Gly His Trp Val Pro Ile
   355 360 365
   Pro Glu Glu Glu Ser Leu Gln Arg Ala Trp Gln Asp Ala Ala Ala Cys
   370 375 380
   Pro Arg Gly Leu Gln Leu Gln Cys Arg Gly Ala Gly Gly Arg Pro Val
   385 390 395 400
   Leu Tyr Gln Val Val Ala Gln His Ser Tyr Ser Ala Gln Gly Pro Glu
   405 410 415
   Asp Leu Gly Phe Arg Gln Gly Asp Thr Val Asp Val Leu Cys Glu Glu
   420 425 430
   Pro Asp Val Pro Leu Ala Val Asp Gln Ala Trp Leu Glu Gly His Cys
   435 440 445
   Asp Gly Arg Ile Gly Ile Phe Pro Lys Cys Phe Val Val Pro Ala Gly
   450 455 460
   Pro Arg Met Ser Gly Ala Pro Gly Arg Leu Pro Arg Ser Gln Gln Gly
   465 470 475 480
   Asp Gln Pro
<210> 224
   <211> 193
   <212> PRT
   <213> Homo sapiens
<220>
   <221> MISC_FEATURE
   <223> Description of sequence: amino acid sequence of Puma (human)
<400> 224
   Met Ala Arg Ala Arg Gln Glu Gly Ser Ser Pro Glu Pro Val Glu Gly
   1 5 10 15
   Leu Ala Arg Asp Gly Pro Arg Pro Phe Pro Leu Gly Arg Leu Val Pro
   20 25 30
   Ser Ala Val Ser Cys Gly Leu Cys Glu Pro Gly Leu Ala Ala Ala Pro
   35 40 45
   Ala Ala Pro Thr Leu Leu Pro Ala Ala Tyr Leu Cys Ala Pro Thr Ala
   50 55 60
   Pro Pro Ala Val Thr Ala Ala Leu Gly Gly Ser Arg Trp Pro Gly Gly
   65 70 75 80
   Pro Arg Ser Arg Pro Arg Gly Pro Arg Pro Asp Gly Pro Gln Pro Ser
   85 90 95
   Leu Ser Leu Ala Glu Gln His Leu Glu Ser Pro Val Pro Ser Ala Pro
   100 105 110
   Gly Ala Leu Ala Gly Gly Pro Thr Gln Ala Ala Pro Gly Val Arg Gly
   115 120 125
   Glu Glu Glu Gln Trp Ala Arg Glu Ile Gly Ala Gln Leu Arg Arg Met
   130 135 140
   Ala Asp Asp Leu Asn Ala Gln Tyr Glu Arg Arg Arg Gln Glu Glu Gln
   145 150 155 160
   Gln Arg His Arg Pro Ser Pro Trp Arg Val Leu Tyr Asn Leu Ile Met
   165 170 175
   Gly Leu Leu Pro Leu Pro Arg Gly His Arg Ala Pro Glu Met Glu Pro
   180 185 190
   Asn
<210> 225
   <211> 198
   <212> PRT
   <213> Homo sapiens
<220>
   <221> MISC_FEATURE
   <223> Description of sequence: amino acid sequence of Bim (human) (transcript variant 1)
<400> 225
<210> 226
   <211> 160
   <212> PRT
   <213> Homo sapiens
<220>
   <221> MISC_FEATURE
   <223> Description of sequence: amino acid sequence of Bik (human)
<400> 226
   Met Ser Glu Val Arg Pro Leu Ser Arg Asp Ile Leu Met Glu Thr Leu
   1               5                   10                  15
   Leu Tyr Glu Gln Leu Leu Glu Pro Pro Thr Met Glu Val Leu Gly Met
               20                  25                  30
   Thr Asp Ser Glu Glu Asp Leu Asp Pro Met Glu Asp Phe Asp Ser Leu
           35                  40                  45
   Glu Cys Met Glu Gly Ser Asp Ala Leu Ala Leu Arg Leu Ala Cys Ile
       50                  55                  60
   Gly Asp Glu Met Asp Val Ser Leu Arg Ala Pro Arg Leu Ala Gln Leu
   65                  70                  75                  80
   Ser Glu Val Ala Met His Ser Leu Gly Leu Ala Phe Ile Tyr Asp Gln
                   85                  90                  95
   Thr Glu Asp Ile Arg Asp Val Leu Arg Ser Phe Met Asp Gly Phe Thr
               100                 105                 110
   Thr Leu Lys Glu Asn Ile Met Arg Phe Trp Arg Ser Pro Asn Pro Gly
           115                 120                 125
   Ser Trp Val Ser Cys Glu Gln Val Leu Leu Ala Leu Leu Leu Leu Leu
       130                 135                 140
   Ala Leu Leu Leu Pro Leu Leu Ser Gly Gly Leu His Leu Leu Leu Lys
   145                 150                 155                 160
<210> 227
   <211> 18
   <212> PRT
   <213> Homo sapiens
<220>
   <221> misc_feature
   <223> Description of sequence: native L-amino acid sequence of the BH3-domain of Bik (Bik BH3)
<400> 227
   Ala Leu Ala Leu Arg Leu Ala Cys Ile Gly Asp Glu Met Asp Val Ser
   1 5 10 15
   Leu Arg
<210> 228
   <211> 18
   <212> PRT
   <213> Homo sapiens
<220>
   <221> misc_feature
   <223> Description of sequence: native L-amino acid sequence of the BH3-domain of Bad (Bad BH3)
<400> 228
   Arg Tyr Gly Arg Glu Leu Arg Arg Met Ser Asp Glu Phe Val Asp Ser
   1 5 10 15
   Phe Lys
<210> 229
   <211> 18
   <212> PRT
   <213> Homo sapiens
<220>
   <221> misc_feature
   <223> Description of sequence: native L-amino acid sequence of the BH3-domain of Bid (Bid BH3)
<400> 229
   Asn Ile Ala Arg His Leu Ala Gln Val Gly Asp Ser Met Asp Arg Ser
   1 5 10 15
   Ile Pro
<210> 230
   <211> 18
   <212> PRT
   <213> Homo sapiens
<220>
   <221> misc_feature
   <223> Description of sequence: native L-amino acid sequence of the BH3-domain of Bmf (Bmf BH3)
<400> 230
   Gln Ile Ala Arg Lys Leu Gln Cys Ile Ala Asp Gln Phe His Arg Leu
   1 5 10 15
   His Val
<210> 231
   <211> 18
   <212> PRT
   <213> Homo sapiens
<220>
   <221> misc_feature
   <223> Description of sequence: native L-amino acid sequence of the BH3-domain of DPS/Hrk (DP5Hrk BH3)
<400> 231
   Leu Thr Ala Ala Arg Leu Lys Ala Ile Gly Asp Glu Leu His Gln Arg
   1 5 10 15
   Thr Met
<210> 232
   <211> 18
   <212> PRT
   <213> Homo sapiens
<220>
   <221> misc_feature
   <223> Description of sequence: native L-amino acid sequence of the BH3-domain of Bim (Bim BH3)
<400> 232
   Trp Ile Ala Gln Glu Leu Arg Arg Ile Gly Asp Glu Phe Asn Ala Tyr
   1 5 10 15
   Tyr Ala
<210> 233
   <211> 18
   <212> PRT
   <213> Homo sapiens
<220>
   <221> misc_feature
   <223> Description of sequence: native L-amino acid sequence of the BH3-domain of Noxa (Noxa BH3)
<400> 233
   Glu Cys Ala Thr Gln Leu Arg Arg Phe Gly Asp Lys Leu Asn Phe Arg
   1 5 10 15
   Gln Lys
<210> 234
   <211> 18
   <212> PRT
   <213> Homo sapiens
<220>
   <221> misc_feature
   <223> Description of sequence: native L-amino acid sequence of the BH3-domain of PUMA (PUMA BH3)
<400> 234
   Glu Ile Gly Ala Gln Leu Arg Arg Met Ala Asp Asp Leu Asn Ala Gln
   1               5                   10                  15
   Tyr Glu
<210> 235
   <211> 18
   <212> PRT
   <213> Homo sapiens
<220>
   <221> misc_feature
   <223> Description of sequence: native L-amino acid sequence of the BH3-domain of Bax (Bax BH3)
<400> 235
   Lys Leu Ser Glu Cys Leu Lys Arg Ile Gly Asp Glu Leu Asp Ser Asn
   1               5                   10                  15
   Met Glu
<210> 236
   <211> 18
   <212> PRT
   <213> Homo sapiens
<220>
   <221> misc_feature
   <223> Description of sequence: native L-amino acid sequence of the BH3-domain of Bak (Bak BH3)
<400> 236
   Gln Val Gly Arg Gln Leu Ala Ile Ile Gly Asp Asp Ile Asn Arg Arg
   1               5                   10                  15
   Tyr Asp
<210> 237
   <211> 18
   <212> PRT
   <213> Homo sapiens
<220>
   <221> misc_feature
   <223> Description of sequence: native L-amino acid sequence of the BH3-domain of Bok (Bok BH3)
<210> 238
   <211> 4
   <212> PRT
   <213> Artificial Sequence
<220>
   <223> Description of sequence: signal sequence or localisation sequence
<400> 238
   Lys Asp Glu Leu
   1
<210> 239
   <211> 4
   <212> PRT
   <213> Artificial Sequence
<220>
   <223> Description of sequence: signal sequence or localisation sequence
<400> 239
   Asp Asp Glu Leu
   1
<210> 240
   <211> 4
   <212> PRT
   <213> Artificial Sequence
<220>
   <223> Description of sequence: signal sequence or localisation sequence
<400> 240
   Asp Glu Glu Leu
   1
<210> 241
   <211> 4
   <212> PRT
   <213> Artificial Sequence
<220>
   <223> Description of sequence: signal sequence or localisation sequence
<400> 241
   Gln Glu Asp Leu
   1
<210> 242
   <211> 4
   <212> PRT
   <213> Artificial Sequence
<220>
   <223> Description of sequence: signal sequence or localisation sequence
<400> 242
   Arg Asp Glu Leu
   1
<210> 243
   <211> 7
   <212> PRT
   <213> Artificial Sequence
<220>
   <223> Description of sequence: signal sequence or localisation sequence
<400> 243
   Pro Lys Lys Lys Arg Lys Val
   1 5
<210> 244
   <211> 7
   <212> PRT
   <213> Artificial Sequence
<220>
   <223> Description of sequence: signal sequence or localisation sequence
<400> 244
   Pro Gln Lys Lys Ile Lys Ser
   1 5
<210> 245
   <211> 5
   <212> PRT
   <213> Artificial sequence
<220>
   <223> Description of sequence: signal sequence or localisation sequence
<400> 245
   Gln Pro Lys Lys Pro
   1 5
<210> 246
   <211> 4
   <212> PRT
   <213> Artificial Sequence
<220>
   <223> Description of sequence: signal sequence or localisation sequence
<400> 246
   Arg Lys Lys Arg
   1
<210> 247
   <211> 12
   <212> PRT
   <213> Artificial sequence
<220>
   <223> Description of sequence: signal sequence or localisation sequence
<400> 247
   Arg Lys Lys Arg Arg Gln Arg Arg Arg Ala His Gln
   1 5 10
<210> 248
   <211> 16
   <212> PRT
   <213> Artificial sequence
<220>
   <223> Description of sequence: signal sequence or localisation sequence
<400> 248
   Arg Gln Ala Arg Arg Asn Arg Arg Arg Arg Trp Arg Glu Arg Gln Arg
   1 5 10 15
<210> 249
   <211> 19
   <212> PRT
   <213> Artificial Sequence
<220>
   <223> Description of sequence: signal sequence or localisation sequence
<400> 249
   Met Pro Leu Thr Arg Arg Arg Pro Ala Ala Ser Gln Ala Leu Ala Pro
   1 5 10 15
   Pro Thr Pro
<210> 250
   <211> 15
   <212> PRT
   <213> Artificial Sequence
<220>
   <223> Description of sequence: signal sequence or localisation sequence
<400> 250
   Met Asp Asp Gln Arg Asp Leu Ile Ser Asn Asn Glu Gln Leu Pro
   1               5                   10                  15
<210> 251
   <211> 9
   <212> PRT
   <213> Artificial Sequence
<220>
   <223> Description of artificial sequence: D-TAT
<220>
   <221> VARIANT
   <222> (1)..(9)
   <223> all amino acids are D-enantiomeric amino acids
<400> 251
   Arg Arg Arg Gln Arg Arg Lys Lys Arg
   1               5
<210> 252
   <211> 9
   <212> PRT
   <213> Artificial
<220>
   <223> Description of sequence: consensus sequence rxxxrxxxr
<220>
   <221> misc_feature
   <222> (1)..(1)
   <223> Arg is D-enantiomeric Arg
<220>
   <221> misc_feature
   <222> (2)..(4)
   <223> Xaa can be any naturally occurring amino acid
<220>
   <221> misc_feature
   <222> (5)..(5)
   <223> Arg is D-enantiomeric Arg
<220>
   <221> misc_feature
   <222> (6)..(8)
   <223> Xaa can be any naturally occurring amino acid
<220>
   <221> misc_feature
   <222> (9)..(9)
   <223> Arg is D-enantiomeric Arg
<400> 252
   Arg Xaa Xaa Xaa Arg Xaa Xaa Xaa Arg
   1               5
<210> 253
   <211> 5
   <212> PRT
   <213> Artificial
<220>
   <223> Description of sequence: Conserved sequence motif Trp-Ser-X-rrp-Ser/cytokines of class I of the family of cytokines
<220>
   <221> misc_feature
   <222> (3)..(3)
   <223> Xaa can be any naturally occurring amino acid
<400> 253
   Trp Ser Xaa Trp Ser
   1               5
<210> 254
   <211> 4
   <212> PRT
   <213> Artificial
<220>
   <223> Description of sequence: Positionally conserved cystein residues cytokines of class I of the family of cytokines
<400> 254
   Cys Cys Cys Cys
   1
<210> 255
   <211> 5
   <212> PRT
   <213> Artificial
<220>
   <223> Description of sequence: Peptidic linker sequence
<400> 255
   Gly Gly Gly Gly Gly
   1               5
<210> 256
   <211> 4
   <212> PRT
   <213> Artificial
<220>
   <223> Description of sequence: Peptidic linker sequence
<400> 256
   Gly Gly Gly Gly
   1
<210> 257
   <211> 9
   <212> PRT
   <213> Artificial
<220>
   <223> Description of sequence: all D transporter construct (all amino acid residues are D-amino acids)
<400> 257
   Arg Arg Arg Arg Arg Arg Arg Arg Arg
   1 5
<210> 258
   <211> 9
   <212> PRT
   <213> Artificial
<220>
   <223> Description of sequence: D/L transporter construct (D and L amino acid residues alternate, beginning wit D amino acids)
<400> 258
   Arg Arg Arg Arg Arg Arg Arg Arg Arg
   1 5
<210> 259
   <211> 9
   <212> PRT
   <213> Artificial
<220>
   <223> Description of sequence: DD/LL transporter construct
<400> 259
   Arg Arg Arg Arg Arg Arg Arg Arg Arg
   1 5
<210> 260
   <211> 9
   <212> PRT
   <213> Artificial
<220>
   <223> Description of sequence: DDD/LLL (r6R3) transporter construct
<400> 260
   Arg Arg Arg Arg Arg Arg Arg Arg Arg
   1 5
<210> 261
   <211> 9
   <212> PRT
   <213> Artificial
<220>
   <223> Description of sequence: DDDD/LLLL transporter construct
<400> 261
   Arg Arg Arg Arg Arg Arg Arg Arg Arg
   1 5
<210> 262
   <211> 21
   <212> DNA
   <213> Artificial
<220>
   <223> Description of sequence: AP-1 doubled labeled probe
<400> 262
   cgcttgatga gtcagccgga a 21

## Description

The present invention relates to novel transporter constructs of the generic formula (1) DₗLLLₓDₘ(LLL_{y}Dₙ)ₐ and variants thereof. The present invention also refers to transporter cargo conjugate molecules, particularly of conjugates of the novel transporter constructs with a cargo moiety, e.g. proteins or peptides, nucleic acids, cytotoxic agents, organic molecules, etc. The present invention furthermore discloses (pharmaceutical) compositions comprising these conjugates and methods of treatment and uses involving such transporter constructs.

Techniques enabling efficient transfer of a substance of interest from the external medium into tissue or cells, and particularly to cellular nuclei, such as nucleic acids, proteins or cytotoxic agents, but also of other (therapeutically useful) compounds, are of considerable interest in the field of biotechnology. These techniques may be suitable for transport and translation of nucleic acids into cells *in vitro* and *in vivo* and thus for protein or peptide production, for regulation of gene expression, for induction of cytotoxic or apoptotic effects, for analysis of intracellular processes and for the analysis of the effect of the transport of a variety of different cargos into a cell (or cell nucleus), etc.

One important application of such a transfer of a cargo of interest from the external medium into tissue or cells is gene therapy, wherein the cargo is typically a nucleic acid or a gene. Although this technique has shown some rather promising developments in the last decades, gene transfer is typically limited by the inability of the gene transfer vectors to effectively transfer the biologically active cargo into the cytoplasm or nuclei of cells in the host to be treated without affecting the host genome or altering the biological properties of the active cargo.

In this respect, several techniques have been developed in an effort to more efficiently transfect e.g. nucleic acids, such as DNA or RNA, into cells. Transfection of nucleic acids into cells or tissues of patients by methods of gene transfer is a central method of molecular medicine and plays a critical role in therapy and prevention of numerous diseases.

Representative examples of gene transfer methods include general (physical or physico-chemical) methods such as coprecipitating nucleic acids with calcium phosphate or DEAE-dextran, a method which enables nucleic acids to penetrate the plasma membrane and then enter the cell and/or nucleus. However, this technique suffers from low transfer efficiency and a high percentage of cell death. Additionally, this method is restricted to *in vitro* or *ex vivo* methods, but is not applicable to *in vivo* situations due to its very nature.

The same holds for methods involving *in vitro* electroporation. *In vitro* electroporation is based on the use of high-voltage current to make cell membranes permeable to allow the introduction of new nucleic acids, e.g. DNA or RNA, into the cell. However, such methods are typically not suitable *in vivo.* Furthermore, this technique also suffers from low transfer efficiency and a high percentage of cell death.

Further well known physical or physico-chemical methods include (direct) injection of (naked) nucleic acids or biolistic gene transfer. Biolistic gene transfer (also known as biolistic particle bombardment) is a method developed at Cornell University that allows introducing genetic material into tissues or culture cells. Biolistic gene transfer is typically accomplished by surface coating metal particles, such as gold or silver particles, and shooting these metal particles, comprising the adsorbed DNA, into cells by using a gene gun. Similar as discussed above this method is restricted to *in vitro* or *ex vivo* methods, but is usually not applicable in *in vivo* situations.

Other methods utilize the transport capabilities of so called transporter molecules. Transporter molecules to be used in this context typically may be divided into viral vectors, i.e. transporter molecules, which involve viral elements, and nonviral vectors.

The most successful gene therapy strategies available today rely on viral vectors, such as adenoviruses, adeno-associated viruses, retroviruses, and herpes viruses. These viral vectors typically employ a conjugate of a virus-related substance with a strong affinity for DNA and a nucleic acid. Due to their infection properties, viruses or viral vectors have a very high transfection rate. The viral vectors typically used are genetically modified in a way that no functional infectious particles are formed in the transfected cell. In spite of this safety precaution, however, there are many problems associated with viral vectors related to immunogenicity, cytotoxicity, and insertional mutagenesis. As an example, the risk of uncontrolled propagation of the introduced therapeutically active genes or viral genes cannot be ruled out, e.g., because of possible recombination events. Additionally, the viral conjugates are difficult to use and typically require a long preparation prior to treatment (see, e. g., US Patent No. 5,521,291).

Although nonviral vectors are not as efficient as viral vectors, many have been developed to provide a safer alternative in gene therapy. Some of the most common nonviral vectors include polyethylenimine, dendrimers, chitosan, polylysine, and peptide based transporter systems, e.g. many types of peptides, which are generally cationic in nature and able to interact with nucleic acids such as plasmid DNA through electrostatic interactions.

For successful delivery, the nonviral vectors, particularly peptide based transporter systems must be able to overcome many barriers. Such barriers include protection of the cargo moiety, e.g. of DNA or other compounds, during transport and prevention of an early degradation or metabolisation of the cargo moiety *in vivo.* In case of nucleic acids, such as DNA and RNA molecules, the nonviral vectors must furthermore be capable to specifically deliver these molecules for efficient gene expression in target cells.

Particularly for nucleic acids such DNA and RNA molecules there are presently 4 barriers nonviral vectors must overcome to achieve successful gene delivery (see e.g. Martin et al., The AAPS Journal 2007; 9 (1) Article 3). The nonviral vector must be able to 1) tightly compact and protect the nucleic acids, 2) it must able to target specific cell-surface receptors, 3) the nonviral vector must be capable to disrupt the endosomal membrane, and 4) it has to deliver the nucleic acid cargo to the nucleus and allow translation of an encoded protein or peptide sequence.

Such nonviral vectors, particularly peptide-based nonviral vectors, are advantageous over other nonviral strategies in that they are in general able to achieve all 4 of these goals, however, with different efficiency regarding the different barriers.

As an example, cationic peptides rich in basic residues such as lysine and/or arginine are able to efficiently condense nucleic acids such as DNA into small, compact particles that can be stabilized in serum. Furthermore, attachment of a peptide ligand to the polyplex allows targeting to specific receptors and/or specific cell types. Polyplexes or cationic polymers as mentioned above typically form a complex with negatively charged nucleic acids leading to a condensation of nucleic acids and protecting these nucleic acids against degradation. Transport into cells using polyplexes (cationic polymers) typically occurs via receptor mediated endocytosis. Thereby, the DNA is coupled to a distinct molecule, such as Transferrin, via e.g. the polyplex poly-L-lysine (PLL), which binds to a surface receptor and triggers endocytosis. Polyplexes (cationic polymers) include e.g. poly-L-lysine (PLL), chitosan, polyethylenimine (PEI), polydimethylaminoethylmethacrylate (PD-MAEMA), polyamidoamine (PAMAM). Such effects are also known from nanoplexes (nanoparticular systems) or lipoplexes (liposomal systems). Nanoplexes (nanoparticular systems) typically involve the use of polyacrylates, polyamides, polystyrene, cyanoacrylates, polylactat (PLA), poly(lactic-co-glycolic acid) (PLGA), etc. Lipoplexes or liposomal systems typically involve the use of cationic lipids, which are capable to mimick a cell membrane. Thereby, the positively charged moiety of the lipid interacts with the negatively charged moiety of the nucleic acid and thus enables fusion with the cell membrane. Lipoplexes or liposomal systems include, e.g. DOTMA, DOPE, DOSPA, DOTAP, DC-Chol, EDMPC, etc.

In this context, receptor-mediated endocytosis is also widely exploited in experimental systems for the targeted delivery of cargos such as nucleic acids or therapeutic agents into cells. During receptor-mediated endocytosis the cargo-containing complexes are either selectively internalized by receptors located in the cell membrane which are specific for the cargos, or by specific antibodies located in membrane constituents. Endocytotic activity has been described for many receptors including IgG Fc, somatostatin, insulin, IGF-I and -II, transferrin, EGF, GLP-1, VLDL or integrin receptors, etc.

Different peptide or protein sequences have been tested widely for their use in gene transfer methods via receptor-mediated endocytosis. Interestingly, the isolation of peptide sequences that direct efficient receptor-mediated endocytosis have been profoundly boosted by the use of phage display technologies. Phage display libraries are extremely powerful tools that provide for a practically unlimited source of molecular variants including modifications of natural ligands or cargo moieties to cell receptors and short peptides. Similar libraries have also been injected directly into mice and peptide sequences have been successfully isolated that show a 13-fold selectivity for brain and kidney.

Proprotein convertases may serve as an example of peptide or protein sequences that may be used for transport of molecules into cells. Proprotein convertases are an example of a cell surface receptor which gets internalized through receptor mediated endocytosis. These proteins have been shown to be responsible for conversion of precursors of peptide hormones, neuropeptides, and many other proteins into their biologically active forms. All cleavage sites for the proprotein convertase family obey to the consensus R-X-X-R. The mammalian proprotein convertases can be classified into three groups on the basis of their tissue distribution. Furin, PACE4, PC5/PC6, and LPCIPC7/PC8/SPC7 are expressed in a broad range of tissues and cell lines. In contrast, expression of PC2 and PC1/PC3 is limited to neuroendocrine tissues, such as pancreatic islets, pituitary, adrenal medulla and many brain areas. Expression of PC4 is highly restricted to testicular spermatogenic cells. The neuroendocrine-specific convertases, PC2 and PC1/PC3, are mainly localized in secretory granules. PC5/PC6A has also been reported to be localized to secretory granules. Furthermore, indirect evidence has suggested that a proportion of proprotein convertases molecules is present on the cell surface, and it has been shown that furin cycles between the TGN and the cell surface. Taken together, these properties indicate that proprotein convertases transport extracellular ligands into the intracellular space.

Advantageous are also so called translocatory proteins or of protein transduction domains (PTDs). Peptide sequences derived from translocatory proteins or protein transduction domains (PTDs) are typically able to selectively lyse the endosomal membrane in its acidic environment leading to cytoplasmic release of the polyplex. Translocatory proteins are considered as a group of peptides capable of effecting transport of macromolecules between cells (translocatory proteins), such as HIV-1 TAT (HIV), antennapedia (*Drosophila antennapedia),* HSV VP22 *(Herpes simplex),* FGF or lactoferrin, etc. In contrast, protein transduction domains (PTDs) are considered as a group of peptides capable of directing proteins and peptides covalently bound to these sequences into a cell via the cell membrane (Leifert and Whitton: Translocatory proteins and protein transduction domains: a critical analysis of their biological effects and the underlying mechanisms. Molecular Therapy Vol. 8 No.1 2003). Common to translocatory proteins as well as to PTDs is a basic region, which is regarded as mainly responsible for transport of the fusion peptides since it is capable of binding polyanions such as nucleic acids. Without being bound thereto, PTDs may act similar to cationic transfection reagents using receptor dependent non-saturatable adsorptive endocytosis. PTDs are typically coupled to proteins or peptides in order to effect or enhance a CTL response when administering a peptide based vaccine (see review: Melikov and Chernomordik, Arginine-rich cell penetrating peptides: from endosomal uptake to nuclear delivery, Cell. Mol. Life Sci. 2005).

US 6740524 B1 discloses a phage expressing in its head a bifunctional protein that has nuclear translocation and cell adhesion activities, whereby the TAT protein of HIV can be used as such a bifunctional protein. The phage is used to package a foreign substance such as a gene and may be useful in gene therapy. Moreover, fusion proteins comprising a TAT protein and a lambda phage head protein are disclosed.

WO 02/065986 A2 discloses compositions and methods for enhancing transport of biologically active compounds across biological membranes and across and into animal epithelial or endothelial structures. The composition includes an active agent and a transport moiety.

Unfortunately, peptide based transporter systems typically undergo proteolytic degradation *in vivo* due to peptidases leading to truncated transporter (and/or cargo) sequences. Such peptidases may be distinguished into exopeptidases and endopeptidases, which are both enzymes capable of catalysing the splitting of proteins into smaller peptide fractions and even into single amino acids by a process known as proteolysis. In this context, endopeptidases are typically proteolytic peptidases that break peptide bonds of nonterminal amino acids (i.e. within the molecule). Endopeptidases are typically specific for certain amino acids. Examples of endopeptidases include e.g. trypsin, chymotrypsin, elastase, thermolysin, pepsin and endopeptidase V, etc. Trypsin is known to cut after Arg or Lys, unless followed by a Pro. Chymotrypsin is known to cut after Phe, Trp, or Tyr, unless followed by a Pro. Chymotrypsin cuts more slowly after Asn, His, Met or Leu. Elastase cuts after Ala, Gly, Ser, or Val, unless followed by a Pro. Thermolysin is a heat stable endoprotease, which cuts *before lie,* Met, Phe, Trp, Tyr, or Val, unless *preceded* by Pro. Thermolysin sometimes cuts after Ala, Asp, His or Thr. Pepsin is known to cut *before* Leu, Phe, Trp or Tyr, unless *preceded* by Pro. Finally, endopeptidase V8 is known to cut after Glu. In contrast to endopeptidases exopeptidases are enzymes that catalyse the removal of an amino acid from the end of a polypeptide chain and thus cleave the end of said polypeptide chain. Exopeptidases may be distinguished from their cleavage site into aminopeptidases and carboxypeptidases. Aminopeptidases are typically zinc-dependent enzymes and are produced by glands of the small intestine. Aminopeptidases usually cleave a single amino acid from the amino-terminal end of a peptide or protein sequence. Carboxypeptidases are typically enzymes that hydrolyze the carboxy-terminal (C-terminal) end of a peptide bond. Humans, animals, and plants contain several types of carboxypeptidases with diverse functions ranging from catabolism to protein maturation, which is a digestive enzyme present in pancreatic juice, will cleave a single amino acid from the carboxylic end of the peptide. A particular example is Carboxypeptidase N (CPN), a plasma zinc metalloprotease comprised of two small subunits that have enzymatic activity, and two large subunits, which protect the enzyme from degradation. CPN cleaves the carboxyl-terminal amino acids arginine and lysine from biologically active peptides such as complement anaphylatoxins, kinins, and fibrinopeptides.

In order to modify proteolytic cleavage by peptide based transporter systems as defined above, the peptide based transporter systems may be composed entirely of D-amino acids, thereby forming "retro-inverso peptide sequences". The term "retro-inverso (peptide) sequences" refers to an isomer of a linear peptide sequence in which the direction of the sequence is reversed and the chirality of each amino acid residue is inverted (see e.g. Jameson et al., Nature, 368,744-746 (1994); Brady et al., Nature, 368,692-693 (1994)). The advantage of combining D-enantiomeric amino acids and reverse synthesis is that the positions of carbonyl and amino groups in each amide bond are exchanged, while the position of the side-chain groups at each alpha carbon is preserved. Due to the conformational change of the naturally occurring L-enantiomeric amino acids of the peptide sequence of a peptide based transporter to D-enantiomeric amino acids the risk of proteolytic cleavage *in vivo* is eliminated, being advantageous and highly efficient for the purpose of transfection of a cargo moiety into a cell. In contrast, the term "reverse sequence" refers to a sequence in which the direction of the sequence is reversed (but the chirality of each amino acid residue is not inverted (e.g. D-Arg-L-Arg-L-Arg → L-Arg-L-Arg-D-Arg).

However, though efficiently working as a transporter molecule as defined above the conformational change of the naturally occurring L-enantiomeric amino acids of the peptide sequence of such a peptide based transporter to D-enantiomeric amino acids entails the risk of a predominant accumulation of these transporters in the cell during the whole lifetime of a cell or even longer in the (surrounding) tissue or organism. Accordingly, such transporters, even if the attached cargo moiety is cleaved off or is metabolised in the meantime, may remain in the cell and participate in further inter- and intracellular processes leading to unknown and unwanted side effects.

Accordingly, there is a need in the art to provide alternative nonviral transporter molecules, preferably peptide based transporter systems as defined above, which avoid such an unwanted accumulation in the cell or tissue but nevertheless allow an efficient transfer of cargo moieties into cells.

The above object is solved by the subject matter as defined in the claims attached hereto, particularly by a novel transporter construct and its conjugates (transporter cargo conjugate molecule) as defined in the claims. The above object is furthermore solved by methods and uses employing the novel transporter construct and its conjugates as defined in the claims.

According to a first aspect of the present invention, the object of the present invention is solved by a novel transporter construct comprising or consisting of at least one D-/L-pattern of the generic formula (I) (SEQ ID NO: 1):

DₗLLₓDₘ(LLL_{y}Dₙ)ₐ

wherein:
- D: is a D-amino acid;
- L: is a L-amino acid;
- a: is 0 - 3, preferably 0-2, more preferably 0, 1, 2 or 3, even more preferably 0, 1, or 2 and most preferably 1;
- l, m and n: are independently from each other 1 or 2, preferably 1;
- x and y: are independently from each other 0, 1 or 2, preferably 1;
in particular wherein the transporter construct comprises at least one D-/L-pattern according to one of the following subformulas (Ia) to (If):

(Ia): DₗLLLₓDₘ ;

(Ib): DₗLLLₓDₘLLL_{y}Dₙ ;

(Ic): DₗLLLₓDₘLLL_{y}DₙLLL_{y}Dₙ ;

(Id): DₗLLLₓDₘLLL_{y}DₙLLL_{y}DₙLLL_{y}Dₙ,

(Ie): DLLLD(LLLD)ₐ,

or

(If): DLLLDLLLD ,

and wherein the D-/L-pattern according to generic formula (I) or according to any of subformulas (Ia), (Ib), (Ic), (Id), (Ie), or (If), is applied to one of the following sequences:

| SEQUENCE/PEPTIDE NAME | SEQ ID NO | AA | SEQUENCE |
|---|---|---|---|
| TAT (1-86) | 8 | 86 | |
| TAT (37-72) | 9 | 36 | |
| TAT (37-58) | 10 | 22 | CFITKALGIS YGRKKRRQRR RP |
| TAT (38-58)GGC | 11 | 24 | FITKALGISY GRKKRRQRRR PGGC |
| TAT CGG(47-58) | 12 | 15 | CGGYGRKKRR QRRRP |
| TAT (47-58)GGC | 13 | 15 | YGRKKRRQRR RPGGC |
| TAT (1-72) Mut Cys/Ala 72 | 14 | 56 | |
| L-generic-TAT | 15 | 17 | XXXXRKKRRQRRRXXXX (NH₂- XXXXRKKRRQRRRXXXX -COOH) |
| L-generic-TAT (s) | 16 | 11 | NH₂-Xₙ^{b}-RKKRRQRRR-Xₙ^{b}-COOH |
| L-TAT (s1a) | 17 | 10 | GRKKRRQRRR (NH₂-GRKKRRQRRR-COOH) |
| L-TAT (s1b) | 18 | 9 | RKKRRQRRR (NH₂-GRKKRRQRRR-COOH) |
| L-TAT (s1c) | 19 | 11 | YDRKKRRQRRR |
| r₃-L-TAT | 20 | 9 | rKKRrQRRr |
| r₃-L-TATi | 21 | 9 | rRRQrRKKr |
| βA-r₃-L-TAT | 22 | 9 | βA-rKKRrQRRr |
| βA-r₃-L-TATi | 23 | 9 | βA-rRRQrRKKr |
| FITC-βA-r₃-L-TAT | 24 | 9 | FITC-βA-rKKRrQRRr |
| FITC-βA-r₃-L-TATi | 25 | 9 | FITC-βA-rRRQrRKKr |
| TAT(s2-1) | 26 | 9 | rAKRrQRRr |
| TAT(s2-2) | 27 | 9 | rKARrQRRr |
| TAT(s2-3) | 28 | 9 | rKKArQRRr |
| TAT(s2-4) | 29 | 9 | rKKRrARRr |
| TAT(s2-5) | 30 | 9 | rKKRrQARr |
| TAT(s2-6) | 31 | 9 | rKKRrQRAr |
| TAT(s2-7) | 32 | 9 | rDKRrQRRr |
| TAT(s2-8) | 33 | 9 | rKDRrQRRr |
| TAT(s2-9) | 34 | 9 | rKKDrQRRr |
| TAT(s2-10) | 35 | 9 | rKKRrDRRr |
| TAT(s2-11) | 36 | 9 | rKKRrQDRr |
| TAT(s2-12) | 37 | 9 | rKKRrQRDr |
| TAT(s2-13) | 38 | 9 | rEKRrQRRr |
| TAT(s2-14) | 39 | 9 | rKERrQRRr |
| TAT(s2-15) | 40 | 9 | rKKErQRRr |
| TAT(s2-16) | 41 | 9 | rKKRrERRr |
| TAT(s2-17) | 42 | 9 | rKKRrQERr |
| TAT(s2-18) | 43 | 9 | rKKRrQREr |
| TAT(s2-19) | 44 | 9 | rFKRrQRRr |
| TAT(s2-20) | 45 | 9 | rKFRrQRRr |
| TAT(s2-21) | 46 | 9 | rKKFrQRRr |
| TAT(s2-22) | 47 | 9 | rKKRrFRRr |
| TAT(s2-23) | 48 | 9 | rKKRrQFRr |
| TAT(s2-24) | 49 | 9 | rKKRrQRFr |
| TAT(s2-25) | 50 | 9 | rRKRrQRRr |
| TAT(s2-26) | 51 | 9 | rKRRrQRRr |
| TAT(s2-27) | 52 | 9 | rKKKrQRRr |
| TAT(s2-28) | 53 | 9 | rKKRrRRRr |
| TAT(s2-29) | 54 | 9 | rKKRrQKRr |
| TAT(s2-30) | 55 | 9 | rKKRrQRKr |
| TAT(s2-31) | 56 | 9 | rHKRrQRRr |
| TAT(s2-32) | 57 | 9 | rKHRrQRRr |
| TAT(s2-33) | 58 | 9 | rKKHrQRRr |
| TAT(s2-34) | 59 | 9 | rKKRrHRRr |
| TAT(s2-35) | 60 | 9 | rKKRrQHRr |
| TAT(s2-36) | 61 | 9 | rKKRrQRHr |
| TAT(s2-37) | 62 | 9 | rIKRrQRRr |
| TAT(s2-38) | 63 | 9 | rKIRrQRRr |
| TAT(s2-39) | 64 | 9 | rKKIrQRRr |
| TAT(s2-40) | 65 | 9 | rKKRrIRRr |
| TAT(s2-41) | 66 | 9 | rKKRrQIRr |
| TAT(s2-42) | 67 | 9 | rKKRrQRIr |
| TAT(s2-43) | 68 | 9 | rLKRrQRRr |
| TAT(s2-44) | 69 | 9 | rKLRrQRRr |
| TAT(s2-45) | 70 | 9 | rKKLrQRRr |
| TAT(s2-46) | 71 | 9 | rKKRrLRRr |
| TAT(s2-47) | 72 | 9 | rKKRrQLRr |
| TAT(s2-48) | 73 | 9 | rKKRrQRLr |
| TAT(s2-49) | 74 | 9 | rMKRrQRRr |
| TAT(s2-50) | 75 | 9 | rKMRrQRRr |
| TAT(s2-51) | 76 | 9 | rKKMrQRRr |
| TAT(s2-52) | 77 | 9 | rKKRrMRRr |
| TAT(s2-53) | 78 | 9 | rKKRrQMRr |
| TAT(s2-54) | 79 | 9 | rKKRrQRMr |
| TAT(s2-55) | 80 | 9 | rNKRrQRRr |
| TAT(s2-56) | 81 | 9 | rKNRrQRRr |
| TAT(s2-57) | 82 | 9 | rKKNrQRRr |
| TAT(s2-58) | 83 | 9 | rKKRrNRRr |
| TAT(s2-59) | 84 | 9 | rKKRrQNRr |
| TAT(s2-60) | 85 | 9 | rKKRrQRNr |
| TAT(s2-61) | 86 | 9 | rQKRrQRRr |
| TAT(s2-62) | 87 | 9 | rKQRrQRRr |
| TAT(s2-63) | 88 | 9 | rKKQrQRRr |
| TAT(s2-64) | 89 | 9 | rKKRrKRRr |
| TAT(s2-65) | 90 | 9 | rKKRrQQRr |
| TAT(s2-66) | 91 | 9 | rKKRrQRQr |
| TAT(s2-67) | 92 | 9 | rSKRrQRRr |
| TAT(s2-68) | 93 | 9 | rKSRrQRRr |
| TAT(s2-69) | 94 | 9 | rKKSrQRRr |
| TAT(s2-70) | 95 | 9 | rKKRrSRRr |
| TAT(s2-71) | 96 | 9 | rKKRrQSRr |
| TAT(s2-72) | 97 | 9 | rKKRrQRSr |
| TAT(s2-73) | 98 | 9 | rTKRrQRRr |
| TAT(s2-74) | 99 | 9 | rKTRrQRRr |
| TAT(s2-75) | 100 | 9 | rKKTrQRRr |
| TAT(s2-76) | 101 | 9 | rKKRrTRRr |
| TAT(s2-77) | 102 | 9 | rKKRrQTRr |
| TAT(s2-78) | 103 | 9 | rKKRrQRTr |
| TAT(s2-79) | 104 | 9 | rVKRrQRRr |
| TAT(s2-80) | 105 | 9 | rKVRrQRRr |
| TAT(s2-81) | 106 | 9 | rKKVrQRRr |
| TAT(s2-82) | 107 | 9 | rKKRrVRRr |
| TAT(s2-83) | 108 | 9 | rKKRrQVRr |
| TAT(s2-84) | 109 | 9 | rKKRrQRVr |
| TAT(s2-85) | 110 | 9 | rWKRrQRRr |
| TAT(s2-86) | 111 | 9 | rKWRrQRRr |
| TAT(s2-87) | 112 | 9 | rKKWrQRRr |
| TAT(s2-88) | 113 | 9 | rKKRrWRRr |
| TAT(s2-89) | 114 | 9 | rKKRrQWRr |
| TAT(s2-90) | 115 | 9 | rKKRrQRWr |
| TAT(s2-91) | 116 | 9 | rYKRrQRRr |
| TAT(s2-92) | 117 | 9 | rKYRrQRRr |
| TAT(s2-93) | 118 | 9 | rKKYrQRRr |
| TAT(s2-94) | 119 | 9 | rKKRrYRRr |
| TAT(s2-95) | 120 | 9 | rKKRrQYRr |
| TAT(s2-96) | 121 | 9 | rKKRrQRYr |
| r₃R₆ | 122 | 9 | rRRRrRRRr |
| L-R₉ | 123 | 9 | RRRRRRRRR |
| L-R₈ | 124 | 8 | RRRRRRRR |
| L-R₇ | 125 | 7 | RRRRRRR |
| L-R₆ | 126 | 6 | RRRRRR |
| L-R₅ | 127 | 5 | RRRRR |
| PTD-4 | 128 | 11 | YARAAARQARA |
| PTD-4 (variant 1) | 129 | 11 | WARAAARQARA |
| PTD-4 (variant 2) | 130 | 11 | WARAQRAAARA |
| L-P1 Penetratin | 131 | 16 | RQVKVWFQNRRMKWKK |
| D-P1 Penetratin | 132 | 16 | KKWKMRRNQFWVKVQR |
| JNKI, bestfit | 133 | 17 | WKRAAARKARAMSLNLF |
| JNKI, bestfit (variant 1) | 134 | 17 | WKRAAARAARAMSLNLF |
| MDCK transcytose sequence | 135 | 9 | RYRGDLGRR |
| YKGL | 136 | 4 | YKGL |
| r₃ (generic) | 252 | 9 | rXXXrXXXr |

or a reverse sequence thereof, or a variant or fragment of these sequences, wherein a variant or fragment of these sequences retains the function to provide for translocation across biological membranes and consists of a peptide sequence having at least 80% sequence identity over the whole length to the respective native sequence.

As used herein, the term "transporter construct" refers to an amino acid containing compound, which is capable of translocation across biological membranes. As used herein, the term "trafficking sequence" (or "transporter sequence") refers to a sequence of amino acids providing translocation across biological membranes. Accordingly, the transporter constructs according to the present invention comprise a trafficking sequence which allow the transporter construct to translocate across biological membranes. Thus, the transporter constructs as defined above according to generic formula (I) effectively allow and may provide for the transport of cargo moieties, e.g. of proteins or peptides, of nucleic acids, of small organic molecules, of antigens, of cytotoxic agents, etc., into an organism, a tissue, a cell (e.g. to be treated), a cellular subcompartiment and/or into the nucleus of a cell.

Advantageously, the inventive transporter construct as defined above according to generic formula (I) is stable enough to prevent degradation by proteases prior to transport of the cargo moiety to its target site. On the other hand, the inventive transporter constructs as defined above according to generic formula (I) are not permanently persistent in the cell and may be degraded by proteases within a considerable time limit so as to avoid negative side effects such as unwanted accumulation of the novel inventive transporter or its conjugate in the cell. As surprisingly found by the inventors, such advantageous properties may be conferred to a trafficking sequence as described above only by the pattern as described above (herein also described as D-/L-pattern) of the above defined generic formula (I), the specific content and position of the D-amino acids in alteration with the specific content of L-amino acids as defined in generic formula (I). This D-/L-pattern as described herein allows a skilled person to define the *in vivo* or *in vitro* persistence of the inventive transporter construct as defined above in the cell precisely enough as a time sufficiently long to ensure administration and entering of the inventive transporter construct as described above into the cell or nucleus prior to degradation of the inventive transporter construct as described above by proteases within a considerable time limit. This *in vivo* or *in vitro* persistence of the inventive transporter construct as defined above in the cell is in fact dependent on the specific content and position of the D-amino acids in alteration with the specific content of L-amino acids as defined in generic formula (I). Furthermore, a transporter construct exhibiting the D-/L-pattern of the above defined generic formula (I) is short enough to avoid a sterical hindrance of a cargo moiety by the inventive transporter construct as defined above in a transporter cargo conjugate molecule such as defined below. It also allows a cost efficient preparation of such inventive transporter constructs as defined above. Additionally, a conjugate of the transporter peptide or protein of the above defined generic formula (I) with either proteins or peptides, nucleic acids such as DNA and RNA molecules or with cytotoxic agents or even small organic molecules, etc., may be formed easily.

According to the above defined generic formula (I), the inventive transporter construct as described above comprises L-amino acids and D-amino acids according to the specific D-/L-pattern as set forth in generic formula (I).

In the context of the present invention L-amino acids, also termed L-enantiomeric amino acids; are preferably amino acids selected from natively occurring amino acids or their derivatives. Naturally occurring amino acids are typically selected from the standard (proteinogenic) amino acids alanine, arginine, asparagine, aspartic acid, cysteine, glutamine, glutaminic acid; glycine, histidine, isoleucine, leucine, lysine, methionine, phenyl alanine, proline, serine, threonine, tryptophane, tyrosine, and valine, as well as from non-standard amino acids such as ornithine, citrulline, homocysteine, S-adenosyl methionione, hydroxyproline, selenocysteine, pyrrolysine, lanthionine, 2-aminoisobutyric acid, dehydroalanine, gamma-aminobutyric acid, etc.

Derivatives from such L-amino acids or L-enantiomeric amino acids typically comprise any naturally or non-naturally occurring derivative of these amino acids, including, without being limited thereto, amino acids as defined above comprising post-translational modifications or synthetic modifications, including acetylation (at the N-terminus of the peptide sequence, at lysine residues, etc.), deacetylation, alkylation, such as methylation, ethylation, etc. (preferably at lysine or arginine residues within the peptide sequence), dealkylation, such as demethylation, deethylation, etc., amidation (preferably at the C-terminus of the peptide sequence), formylation, gamma-carboxylation, glutamylation, glycosylation (preferably at asparagine, lysine, hydroxylysine, serine or threonine residues, etc., within the peptide sequence), addition of a heme or haem moiety, hydroxylation, iodination, isoprenylation addition of an isoprenoid moiety such as farnesyl or geranylgeraniol, etc.), lipoylation (attachment of lipoate functionality), such as prenylation, formation of a GPI anchor, including myristoylation, farnesylation, geranylgernaylation, etc., oxidation, phosphorylation (e.g. to a serine, tytosine, threonine or a histidine moiety, etc., within the peptide sequence), sulfation (e.g. of tyrosine), selenoylation, sulfation, etc.

Derivatives of L-amino acids also include, without being limited thereto, modified L-amino acids, which have been modified by introducing one of the following labels:
(i) radioactive labels, i.e. radioactive phosphorylation or a radioactive label with sulphur, hydrogen, carbon, nitrogen, etc.;
(ii) colored dyes (e.g. digoxygenin; etc.);
(iii) fluorescent groups (e.g. fluorescein, rhodamine, flourochrome proteins as defined below, etc.);
(iv) chemoluminescent groups;
(v) a combination of labels of two or more of the labels mentioned under (i) to (iv).

Particularly specific examples of derivatives of L-amino acids include, without being limited thereto, AMC (aminomethylcoumarin), Dabcyl (dimethylaminophenylazobenzoyl), Dansyl (dimethylaminonaphtalenesulfonyl), FAM (carboxyfluoroscein), Mca (methoxycoumarin acetyl), Xan (xanthyl), Abu (aminobutyric acid), Beta-Ala (beta-alanine), E-Ahx (6-aminohexanoic acid), Alpha-Aib (alpha-aminoisobutyric acid), Ams (aminoserine), Cha (cyclohexylamine), Dab (diaminobutyric acid), Hse (homoserine), Hyp (hydroxyproline), Mpr (mercaptopropionic acid), Nal (naphtylalanine), Nva (Norvaline), Orn (ornithine), Phg (phenylglycine), Sar (sarcosine), Sec (selenocysteine), Thi (thienylalanine), etc.

Furthermore, L-enantiomeric amino acids selected for the inventive transporter construct as defined above furthermore may be selected from specific combinations of the above defined L-enantiomeric amino acids or derivatives thereof. Such combinations may comprise 1, 2, 3, 4, 5, 6, 7, 8, 9 or 10 or even more of the above defined L-enantiomeric amino acids or derivatives thereof. Combinations are also possible between any of the above defined L-enantiomeric amino acids or derivatives thereof and any the above defined D-enantiomeric amino acids or derivatives thereof within the definitions of generic formula (I) or of any of subformulas as defined herein. Such specific combinations of amino acids may exhibit a higher or a lower stability towards peptidases and thus may provide a further possibility to render the *in vivoor in* vitrostability of the inventive transporter construct as defined above towards a higher or a lower stability. As an example, the inventive transporter construct as defined above may contain the dipeptide sequence Arg-Lys in D- and/or L-form (i.e. both as D-enantiomeric amino acids or as L-enantiomeric amino acids or mixed D- and L-enantiomeric amino acids), preferably in L-form, wich exhibits a lower stability towards pepidases and thus may be used to destabilize the peptide sequence of the inventive transporter construct as defined above and therefore to decrease its half life *in vivoto* a further extent.

In the context of the present invention D-amino acids, also termed D-enantiomeric amino acids, are preferably non-native (non-proteinogenic) "retro-inverso" amino acids, wherein these non-native (non-proteinogenic) "retro-inverso" amino acids are preferably derived from naturally occurring L-amino acids and/or their derivatives as defined above. In this context, the term "retro-inverso" refers to an isomer of a naturally occurring L-amino acid as defined above (and peptides made therefrom) in which the chirality of the naturally occurring L-amino acid residue is inverted in the corresponding D-amino acid (see e.g. Jameson et al., Nature, 368,744-746 (1994); Brady et al., Nature, 368,692-693 (1994)). In other words, in the peptide bonds of D-amino acids the positions of carbonyl and amino groups are exchanged, while the position of the side-chain groups at each alpha carbon is preserved. Accordingly, D-amino acids may be inserted into a peptide sequence consisting of or comprising L-amino acids and therefore may be conjugated with L-amino acids as defined above by methods known in the art. Such methods known in the art include e.g., without being limited thereto, liquid phase peptide synthesis methods or solid peptide synthesis methods, e.g. solid peptide synthesis methods according to Merrifield, *t*-Boc solid-phase peptide synthesis, Fmoc solid-phase peptide synthesis, BOP (Benzotriazole-1-yl-oxy-tris-(dimethylamino)-phosphonium hexafluorophosphate) based solid-phase peptide synthesis, etc. The content of D-amino acids in the inventive transporter constructs as described above according to the D-/L-pattern of generic formula (I) above additionally provides a further variety of useful properties. For example, such novel transporter constructs enter cells more efficiently and are more stable (especially *in vivo)* and show lower immunogenicity than corresponding L-amino-acid-sequence based transporter constructs. However, they are not as persistent in the cell as transporter constructs entirely made of D-amino acids, particularly due to the fact that almost all decomposition enzymes, like proteases or peptidases, cleave peptide bonds between adjacent L-amino acids. Consequently, peptides composed of D-enantiomeric amino acids and L-enantiomeric amino acids are largely resistant towards a fast proteolytic breakdown without leading to an accumulation in the cell due to a missing degradation by proteases.

The above defined inventive transporter construct according to generic formula (I), preferably comprises L-amino acids and D-amino acids or their derivatives as defined above. Such derivatives may be contained in the entire inventive transporter construct as defined above in a content of about 0%, about 10%, about 20%, about 30%, about 40%, about 50%, about 60%, about 70%, about 80%, about 90%, or even about 100%. In other words, the entire inventive transporter peptide may contain about 1, 2, 3, 4, 5, 6, 7, 8, 9, 10, 11, 12, 13, 14, 15, 16, 17, 18, 19, 20, 21, 22, 23, 24, 25, 26, or even more, of such derivatives, wherein the maximum number of possible derivatives is, of course, limited by the maximum number of amino acids as contained in the above defined inventive transporter construct as described above according to generic formula (I).

According to the above defined generic formula (I), the inventive transporter construct comprises a specific D-/L-pattern of L-amino acids and D-amino acids, which is defined by integers a, I, m, n, x and y.

According to the above definition of generic formula (I), a is a determinant defining the number of repetitions of the subgroup (LLL_{y}D) as defined in the generic formula (I) DₗLLLₓDₘ(LLL_{y}Dₙ)ₐ. According to the definition above, a may be any number selected from the range 0 - 3, preferably selected from the range 0-2, more preferably selected from the range 0-1, or may be selected from individual numbers 0, 1, 2 or 3, even more preferably from individual numbers 0, 1, or 2 and most preferably a = 1. According to the specific number of repetitions of a = 0, 1, 2 or 3, the inventive transporter construct as defined above according to generic formula (I) DₗLLLₓDₘ(LLL_{y}Dₙ)ₐ, may consist or comprise at least one of the following subformulas (Ia) to (Id):

(Ia): DₗLLLₓDₘ (SEQ ID NO: 2);

(Ib): DₗLLLₓDₘLLL_{y}Dₙ (SEQ ID NO: 3);

(Ic): DₗLLLₓDₘLLL_{y}DₙLLL_{y}Dₙ (SEQ ID NO: 4)

or

(Id): DₗLLLₓDₘLLL_{y}DₙLLL_{y}DₙLLL_{y}Dₙ (SEQ ID NO: 5).

Furthermore, according to the above definition of generic formula (I), I, m and n are integers defining the number of D-amino acids occurring in generic formula (I), but also in the subformulas (Ia) to (Id) as defined herein. Integers I, m and n may be selected independently from each other. This particularly holds for determinant n, which may occur several times in generic formula (I) or subformulas (Ia) to (Id) as defined herein, i.e. if n occurs several times, each n may be selected independently from each other. According to the definition above, integers I, m and n independently of each other may be any number selected from the range 1 - 2, or may be selected from individual numbers 1 or 2, even more preferably I, m and/or n = 1.

Additionally, according to the above definition of generic formula (I), x and y are integers defining the number of L-amino acids occurring in the generic formula (I), but also in the subformulas (Ia) to (Id) as defined herein. Integers x and y may be selected independently from each other. According to the definition above, integers x and y independently of each other may be any number selected from the range 0 - 2, preferably selected from the range 0-1, or may be selected from individual numbers 0, 1 or 2, even more preferably from individual numbers 0 or 1 and most preferably x and/or y = 1.

According to one particularly preferred embodiment, the object of the present invention is solved by a novel transporter construct comprising or consisting at least one sequence of the specific subformula (Ie):

DLLLD(LLLD)ₐ (SEQ ID NO: 6);

wherein D, L, and a are as defined above for generic formula (I) or subformulas (Ia) to (Id).

According to another particularly preferred embodiment, the object of the present invention is solved by a transporter construct as defined above comprising or consisting at least one sequence of the specific subformula (If):

DLLLDLLLD (SEQ ID NO: 7);

wherein D and L are as defined above for generic formula (I) or subformulas (Ia) to (Id).

The inventive transporter construct as described above according to generic formula (I), in particular according to any of subformulas (Ia), (Ib), (Ic), (Id), (Ie), or (If), particularly transporter constructs comprising the D-/L-pattern as described herein, are applied to any trafficking sequence of Table 1, as described above, wherein the selected number of contiguous amino acids of those trafficking sequences is determined by the number of amino acids as defined by generic formula (I) or any of subformulas (Ia), (Ib), (Ic), (Id), (Ie), or (If). Trafficking sequences typically direct the transport of a cargo moiety into a cell or the nucleus or a further specific target region and may comprise, without being limited thereto, translocatory proteins as defined above, e.g. derived from HIV TAT (HIV), e.g. native proteins such as e.g. the TAT protein (e.g. as described in U.S. Patent Nos. 5,804,604 and 5,674,980, each of these references being incorporated herein by reference), e.g. derived from HIV tat (HIV), HSV VP22 (*Herpes simplex*) (described in e.g. WO 97/05265; Elliott and O'Hare, Cell 88 : 223-233 (1997)), non-viral proteins (Jackson et al, Proc. Natl. Acad. Sci. USA 89 : 10691-10695 (1992)), trafficking sequences derived from Antennapedia, particularly from *Drosophila antennapedia* (e.g. the antennapedia carrier sequence thereof), FGF, lactoferrin, etc. or derived from basic peptides, e.g. peptides having a length of 5 to 15 amino acids, preferably 10 to 12 amino acids and comprising at least 80 %, more preferably 85 % or even 90 % basic amino acids, such as e.g. arginine, lysine and/or histidine, or may be selected from e.g. arginine rich peptide sequences, such as R₉, R₈, R₇, R₆, Rs, etc., from VP22, from PTD-4 derived proteins or peptides, from RGD-K₁₆, from PEPT1/2 or PEPT1/2 derived proteins or peptides, from SynB3 or SynB3 derived proteins or peptides, from PC inhibitors, from P21 derived proteins or peptides, or from JNKI derived proteins or peptides. Furthermore, variants, fragments and derivatives of one of the native proteins used as trafficking sequences are disclosed herewith.

Particular examples of trafficking sequences forming a basis for the transporter construct as defined above according to generic formula (I) or to any of subformulas (Ia), (Ib), (Ic), (Id), (Ie), or (If), as defined above, may be selected from, without being limited thereto, a so-called TAT cell permeation sequence shown in Table 1, which is derived from the basic trafficking sequence of the HIV-1 TAT protein. Preferably, the basic trafficking sequence of the HIV-1 TAT protein may include sequences from the human immunodeficiency virus HIV-1 TAT protein, e.g. as described in, e.g., U.S. Patent Nos. 5,804,604 and 5,674,980. In this context, the full-length HIV-1 TAT protein has 86 amino acid residues [SEQ ID NO: 8] encoded by two exons of the HIV TAT gene. TAT amino acids 1-72 are encoded by exon 1, whereas amino acids 73-86 are encoded by exon 2. The full-length TAT protein is characterized by a basic region which contains two lysines and six arginines (amino acids 49-57) and a cysteine-rich region which contains seven cysteine residues (amino acids 22-37). The basic region (i.e., amino acids 49-57) was thought to be important for nuclear localization. Ruben, S. et al., J. Virol. 63: 1-8 (1989); Hauber, J. et al., J. Virol. 63 1181-1187 (1989). The cysteine-rich region mediates the formation of metal-linked dimers in vitro (Frankel, A. D. et al, Science 240: 70-73 (1988); Frankel, A. D. et al., Proc. Natl. Acad. Sci USA 85: 6297-6300 (1988)) and is essential for its activity as a transactivator (Garcia, J. A. et al., EMBO J. 7: 3143 (1988); Sadaie, M. R. et al., J. Virol. 63:1 (1989)). As in other regulatory proteins, the N-terminal region may be involved in protection against intracellular proteases (Bachmair, A. et al., Cell 56: 1019-1032 (1989)). Preferred TAT trafficking sequences utilized with generic formula (I) or any of subformulas (Ia), (Ib), (Ic), (Id), (Ie), or (If), are preferably characterized by the presence of the TAT basic region amino acid sequence (amino acids 49-57 of naturally-occurring tat protein); the absence of the TAT cysteine-rich region amino acid sequence (amino acids 22-36 of naturally-occurring TAT protein) and the absence of the TAT exon 2-encoded carboxy-terminal domain (amino acids 73-86 of naturally-occurring TAT protein).

According to a more preferred embodiment the trafficking sequences forming a basis for the transporter construct as defined above according to generic formula (I) or to any of subformulas (Ia), (Ib), (Ic), (Id), (Ie), or (If), as defined above, may be selected from an amino acid sequence shown in Table 1 containing TAT residues 48-57 or 49 to 57, and most preferably a TAT sequence according to any of SEQ ID NOs: 8 to 14, or from a generic TAT sequence NH₂-Xₙ^{b}-RKKRRQRRR-Xₙ^{b}-COOH (L-generic-TAT (s)) [SEQ ID NO: 16] and/or XXXXRKKRRQ RRRXXXX (L-generic-TAT) [SEQ ID NO: 15]. In this context, each X typically represents an amino acid residue, preferably selected from any (naturally occurring) amino acid residue as defined herein. Furthermore, each Xₙ^{b} may be selected from any amino acid residue as defined herein, wherein n (the number of repetitions of X) is 0-5, 5-10, 10-15, 15-20, 20-30 or more. Preferably, Xₙ^{b} represents a contiguous stretch of peptide residues derived from the sequence according to SEQ ID NO: 8 (TAT (1-86)). Alternatively, the trafficking sequences forming a basis for the transporter construct as defined above according to generic formula (I) or to any of subformulas (Ia), (Ib), (Ic), (Id), (Ie), or (If), as defined above, may be selected from, e.g., a peptide containing the amino acid sequence NH₂-GRKKRRQRRR-COOH (L-TAT (s1 a)) [SEQ ID NO: 17] or the amino acid sequence NH₂-RKKRRQRRR-COOH (L-TAT (s1b)) [SEQ ID NO: 18].

The person skilled in the art will understand that phrases like that a D-/L-pattern according to generic formula (I) or according to any of subformulas (Ia), (Ib), (Ic), (Id), (Ie), or (If) may be used with or be applied to a particular (trafficking) sequence or may form a basis for a transporter peptide construct according to generic formula (I) or any of subformulas (Ia), (Ib), (Ic), (Id), (Ie), or (If), etc. is intended to illustrate that a sequence is claimed which exhibits certain characterisitics with regard to:
i) the sequence of side chain residues characterizing specific amino acid entities, and
ii) the sequence of D- and L- amino acids in said sequence.

To give an illustrative example: If subformula (If) is used with or applied to TAT (1-86) (SEQ ID NO:8), the sequence of the side chain residues (i) is as indicated in SEQ ID NO: 8. However, this claimed sequence is not a pure L-amino acid sequence, but comprises somewhere the motif of subformula (If). An example for such embodiment would be the following sequence (D- amino acids indicated in small letters, L amino acids indicated in capital letters):

Since SEQ ID NO:8 comprises 86 amino acids, there are of course several further possibilites of placing the motif of subformula (If) elsewhere in this sequence. It is also envisioned that the sequence may comprise in particular embodiments more than one motif of the generic formula and/or subformulas.

Trafficking sequences forming a basis for a transporter peptide construct to generic formula (I) or any of subformulas (Ia), (Ib), (Ic), (Id), (Ie), or (If), as defined above, are selected from sequences or a part thereof as defined according to Table 1 below, or any fragment or variant or derivative thereof (as long as it retains the function of translocating across a biological membrane).

**TABLE 1**

| SEQUENCE/PEPTIDE NAME | SEQ ID NO | AA | SEQUENCE |
|---|---|---|---|
| TAT (1-86) | 8 | 86 | MEPVDPRLEP WKHPGSQPKT ACTNCYCKKC CFHCQVCFIT KALGISYGRK KRRQRRRPPQ GSQTHQVSLS KQPTSQSRGD PTGPKE |
| TAT (37-72) | 9 | 36 | CFITKALGIS YGRKKRRQRR RPPQGSQTHQ VSLSKQ |
| TAT (37-58) | 10 | 22 | CFITKALGIS YGRKKRRQRR RP |
| TAT (38-58)GGC | 11 | 24 | FITKALGISY GRKKRRQRRR PGGC |
| TAT CGG(47-58) | 12 | 15 | CGGYGRKKRR QRRRP |
| TAT (47-58)GGC | 13 | 15 | YGRKKRRQRR RPGGC |
| TAT (1-72) Mut Cys/Ala 72 | 14 | 56 | MEPVDPRLEP WKHPGSQPKT AFITKALGIS YGRKKRRQRR RPPQGSQTHQ VSLSKQ |
| L-generic-TAT | 15 | 17 | XXXXRKKRRQRRRXXXX (NH₂- XXXXRKKRRQRRRXXXX -COOH) |
| L-generic-TAT (s) | 16 | 11 | NH₂-Xₙ^{b}-RKKRRQRRR-Xₙ^{b}-COOH |
| L-TAT (s1a) | 17 | 10 | GRKKRRQRRR (NH₂-GRKKRRQRRR-COOH) |
| L-TAT (s1b) | 18 | 9 | RKKRRQRRR (NH₂-GRKKRRQRRR-COOH) |
| L-TAT (s1c) | 19 | 11 | YDRKKRRQRRR |
| r₃-L-TAT | 20 | 9 | rKKRrQRRr |
| r₃-L-TATi | 21 | 9 | rRRQrRKKr |
| βA-r₃-L-TAT | 22 | 9 | βA-rKKRrQRRr |
| βA-r₃-L-TATi | 23 | 9 | βA-rRRQrRKKr |
| FITC-βA-r₃-L-TAT | 24 | 9 | FITC-βA-rKKRrQRRr |
| FITC-βA-r₃-L-TATi | 25 | 9 | FITC-βA-rRRQrRKKr |
| TAT(s2-1) | 26 | 9 | rAKRrQRRr |
| TAT(s2-2) | 27 | 9 | rKARrQRRr |
| TAT(s2-3) | 28 | 9 | rKKArQRRr |
| TAT(s2-4) | 29 | 9 | rKKRrARRr |
| TAT(s2-5) | 30 | 9 | rKKRrQARr |
| TAT(s2-6) | 31 | 9 | rKKRrQRAr |
| TAT(s2-7) | 32 | 9 | rDKRrQRRr |
| TAT(s2-8) | 33 | 9 | rKDRrQRRr |
| TAT(s2-9) | 34 | 9 | rKKDrQRRr |
| TAT(s2-10) | 35 | 9 | rKKRrDRRr |
| TAT(s2-11) | 36 | 9 | rKKRrQDRr |
| TAT(s2-12) | 37 | 9 | rKKRrQRDr |
| TAT(s2-13) | 38 | 9 | rEKRrQRRr |
| TAT(s2-14) | 39 | 9 | rKERrQRRr |
| TAT(s2-15) | 40 | 9 | rKKErQRRr |
| TAT(s2-16) | 41 | 9 | rKKRrERRr |
| TAT(s2-17) | 42 | 9 | rKKRrQERr |
| TAT(s2-18) | 43 | 9 | rKKRrQREr |
| TAT(s2-19) | 44 | 9 | rFKRrQRRr |
| TAT(s2-20) | 45 | 9 | rKFRrQRRr |
| TAT(s2-21) | 46 | 9 | rKKFrQRRr |
| TAT(s2-22) | 47 | 9 | rKKRrFRRr |
| TAT(s2-23) | 48 | 9 | rKKRrQFRr |
| TAT(s2-24) | 49 | 9 | rKKRrQRFr |
| TAT(s2-25) | 50 | 9 | rRKRrQRRr |
| TAT(s2-26) | 51 | 9 | rKRRrQRRr |
| TAT(s2-27) | 52 | 9 | rKKKrQRRr |
| TAT(s2-28) | 53 | 9 | rKKRrRRRr |
| TAT(s2-29) | 54 | 9 | rKKRrQKRr |
| TAT(s2-30) | 55 | 9 | rKKRrQRKr |
| TAT(s2-31) | 56 | 9 | rHKRrQRRr |
| TAT(s2-32) | 57 | 9 | rKHRrQRRr |
| TAT(s2-33) | 58 | 9 | rKKHrQRRr |
| TAT(s2-34) | 59 | 9 | rKKRrHRRr |
| TAT(s2-35) | 60 | 9 | rKKRrQHRr |
| TAT(s2-36) | 61 | 9 | rKKRrQRHr |
| TAT(s2-37) | 62 | 9 | rIKRrQRRr |
| TAT(s2-38) | 63 | 9 | rKIRrQRRr |
| TAT(s2-39) | 64 | 9 | rKKIrQRRr |
| TAT(s2-40) | 65 | 9 | rKKRrIRRr |
| TAT(s2-41) | 66 | 9 | rKKRrQIRr |
| TAT(s2-42) | 67 | 9 | rKKRrQRIr |
| TAT(s2-43) | 68 | 9 | rLKRrQRRr |
| TAT(s2-44) | 69 | 9 | rKLRrQRRr |
| TAT(s2-45) | 70 | 9 | rKKLrQRRr |
| TAT(s2-46) | 71 | 9 | rKKRrLRRr |
| TAT(s2-47) | 72 | 9 | rKKRrQLRr |
| TAT(s2-48) | 73 | 9 | rKKRrQRLr |
| TAT(s2-49) | 74 | 9 | rMKRrQRRr |
| TAT(s2-50) | 75 | 9 | rKMRrQRRr |
| TAT(s2-51) | 76 | 9 | rKKMrQRRr |
| TAT(s2-52) | 77 | 9 | rKKRrMRRr |
| TAT(s2-53) | 78 | 9 | rKKRrQMRr |
| TAT(s2-54) | 79 | 9 | rKKRrQRMr |
| TAT(s2-55) | 80 | 9 | rNKRrQRRr |
| TAT(s2-56) | 81 | 9 | rKNRrQRRr |
| TAT(s2-57) | 82 | 9 | rKKNrQRRr |
| TAT(s2-58) | 83 | 9 | rKKRrNRRr |
| TAT(s2-59) | 84 | 9 | rKKRrQNRr |
| TAT(s2-60) | 85 | 9 | rKKRrQRNr |
| TAT(s2-61) | 86 | 9 | rQKRrQRRr |
| TAT(s2-62) | 87 | 9 | rKQRrQRRr |
| TAT(s2-63) | 88 | 9 | rKKQrQRRr |
| TAT(s2-64) | 89 | 9 | rKKRrKRRr |
| TAT(s2-65) | 90 | 9 | rKKRrQQRr |
| TAT(s2-66) | 91 | 9 | rKKRrQRQr |
| TAT(s2-67) | 92 | 9 | rSKRrQRRr |
| TAT(s2-68) | 93 | 9 | rKSRrQRRr |
| TAT(s2-69) | 94 | 9 | rKKSrQRRr |
| TAT(s2-70) | 95 | 9 | rKKRrSRRr |
| TAT(s2-71) | 96 | 9 | rKKRrQSRr |
| TAT(s2-72) | 97 | 9 | rKKRrQRSr |
| TAT(s2-73) | 98 | 9 | rTKRrQRRr |
| TAT(s2-74) | 99 | 9 | rKTRrQRRr |
| TAT(s2-75) | 100 | 9 | rKKTrQRRr |
| TAT(s2-76) | 101 | 9 | rKKRrTRRr |
| TAT(s2-77) | 102 | 9 | rKKRrQTRr |
| TAT(s2-78) | 103 | 9 | rKKRrQRTr |
| TAT(s2-79) | 104 | 9 | rVKRrQRRr |
| TAT(s2-80) | 105 | 9 | rKVRrQRRr |
| TAT(s2-81) | 106 | 9 | rKKVrQRRr |
| TAT(s2-82) | 107 | 9 | rKKRrVRRr |
| TAT(s2-83) | 108 | 9 | rKKRrQVRr |
| TAT(s2-84) | 109 | 9 . | rKKRrQRVr |
| TAT(s2-85) | 110 | 9 | rWKRrQRRr |
| TAT(s2-86) | 111 | 9 | rKWRrQRRr |
| TAT(s2-87) | 112 | 9 | rKKWrQRRr |
| TAT(s2-88) | 113 | 9 | rKKRrWRRr |
| TAT(s2-89) | 114 | 9 | rKKRrQWRr |
| TAT(s2-90) | 115 | 9 | rKKRrQRWr |
| TAT(s2-91) | 116 | 9 | rYKRrQRRr |
| TAT(s2-92) | 117 | 9 | rKYRrQRRr |
| TAT(s2-93) | 118 | 9 | rKKYrQRRr |
| TAT(s2-94) | 119 | 9 | rKKRrYRRr |
| TAT(s2-95) | 120 | 9 | rKKRrQYRr |
| TAT(s2-96) | 121 | 9 | rKKRrQRYr |
| r₃R⁶ | 122 | 9 | rRRRrRRRr |
| L-R₉ | 123 | 9 | RRRRRRRRR |
| L-R₈ | 124 | 8 | RRRRRRRR |
| L-R₇ | 125 | 7 | RRRRRRR |
| L-R₆ | 126 | 6 | RRRRRR |
| L-R₅ | 127 | 5 | RRRRR |
| PTD-4 | 128 | 11 | YARAAARQARA |
| PTD-4 (variant 1) | 129 | 11 | WARAAARQARA |
| PTD-4 (variant 2) | 130 | 11 | WARAQRAAARA |
| L-P1 Penetratin | 131 | 16 | RQVKVWFQNRRMKWKK |
| D-P1 Penetratin | 132 | 16 | KKWKMRRNQFWVKVQR |
| JNKI, bestfit | 133 | 17 | WKRAAARKARAMSLNLF |
| JNKI, bestfit (variant 1) | 134 | 17 | WKRAAARAARAMSLNLF |
| MDCK transcytose sequence | 135 | 9 | RYRGDLGRR |
| YKGL | 136 | 4 | YKGL |
| r3 (generic) | 252 | 9 | rXXXrXXXr |

Particular examples of trafficking sequences forming a basis for a transporter construct according to generic formula (I) or according to any of subformulas (Ia), (Ib), (Ic), (Id), (Ie), or (If), as defined above, may also be selected from sequences as shown in Table 1, which exhibit the reverse sequence of this specific sequence, i.e., wherein the order of amino acids in the sequence from N- to C-terminal end is reversed.

Particular preferred examples of trafficking sequences forming a basis for a transporter construct as defined above according to generic formula (I) or according to any of subformulas (Ia), (Ib), (Ic), (Id), (Ie), or (If), as defined above, may be selected from fragments or variants of the above sequences (with the proviso that such fragment or variant retain the function to provide for translocation across biological membranes). In this specific context, variants and/or fragments of those trafficking sequences preferably comprise or consist of a peptide sequence having at least 80% or 85%, preferably at least 90%, more preferably at least 95% and most preferably at least 99% over the whole length of the native sequence of such a trafficking sequence as defined above. Additionally, a fragment of such a trafficking sequence may furthermore comprise epitopes (also called "antigen determinants") of the full-length trafficking sequence. Epitopes in the context of the present invention are typically fragments located on the outer surface of a (native) protein or peptide sequence as defined herein, preferably having 5 to 15 amino acids, more preferably having 5 to 12 amino acids, even more preferably having 6 to 9 amino acids, which may be recognized by antibodies, i.e. in their native form.

In this specific context, a "fragment" of a trafficking sequence as defined above, particularly in Table 1 or 3, is preferably to be understood as a truncated sequence thereof, i.e. an amino acid sequence, which is N-terminally, C-terminally and/or intrasequentially truncated compared to the amino acid sequence of the native sequence.

Furthermore, in the specific context of the present invention, a "variant" of a trafficking sequence or its fragment as defined above, particularly as defined in Table 1 or 3, is preferably to be understood as a sequence wherein the amino acid sequence of the variant differs from the native trafficking sequence or a fragment thereof as defined herein in one or more mutation(s), such as one or more substituted, (or, if necessary, inserted and/or deleted) amino acid(s). Preferably, variants of such a trafficking sequence as defined above have the same biological function or specific activity compared to the full-length native sequence, i.e. transport into cells and the nucleus. More preferably, a variant of such a trafficking sequence as defined above may comprise about 1 to 50, 1 to 20, even more preferably 1 to 10 and most preferably 1 to 5, 4, 3, 2 or 1 amino acid alterations within the above meaning. Variants of such a trafficking sequence as defined above may also comprise conservative amino acid substitutions. Conservative amino acid substitutions may include synonymous amino acid residues within a group which have sufficiently similar physicochemical properties, so that a substitution between members of the group will preserve the biological activity of the molecule (see e.g. Grantham, R. (1974), Science 185, 862-864). It is evident to the skilled person that amino acids may also be inserted and/or deleted in the above-defined sequences without altering their function, particularly if the insertions and/or deletions only involve a few amino acids, e.g. less than twenty, and preferably less than ten, and do not remove or displace amino acids which are critical to functional activity. Particularly, conservative amino acid substitutions are preferably substitutions in which the amino acids, which originate from the same class of amino acids (basic amino acids, acidic amino acids, polar amino acids, etc.), are exchanged for one another. In particular, these are amino acids, aliphatic side chains, positively or negatively charged side chains, aromatic groups in the side chains of amino acids, the side chains of which can enter into hydrogen bridges, e.g. side chains which have a hydroxyl function. This means that e.g. an amino acid having a polar side chain is replaced by another amino acid having a likewise polar side chain, or, for example, an amino acid characterized by a hydrophobic side chain is substituted by another amino acid having a likewise hydrophobic side chain (e.g. serine (threonine) by threonine (serine) or leucine (isoleucine) by isoleucine (leucine)). Preferably, synonymous amino acid residues, which are classified into the same groups and are typically exchangeable by conservative amino acid substitutions, are defined in Table 2.

**TABLE 2**

| Preferred Groups of Synonymous Amino Acid Residues | |
|---|---|
| Amino Acid | Synonymous Residue |
| Ser | Ser, Thr, Gly, Asn |
| Arg | Arg, Gln, Lys, Glu, His |
| Leu | Ile, Phe, Tyr, Met, Val, Leu |
| Pro | Gly, Ala, (Thr), Pro |
| Thr | Pro, Ser, Ala, Gly, His, Gln, Thr |
| Ala | Gly, Thr, Pro, Ala |
| Val | Met, Tyr, Phe, Ile, Leu, Val |
| Gly | Ala, (Thr), Pro, Ser, Gly |
| Ile | Met, Tyr, Phe, Val, Leu, Ile |
| Phe | Trp, Met, Tyr, Ile, Val, Leu, Phe |
| Tyr | Trp, Met, Phe, Ile, Val, Leu, Tyr |
| Cys | Ser, Thr, Cys |
| His | Glu, Lys, Gln, Thr, Arg, His |
| Gln | Glu, Lys, Asn, His, (Thr), Arg, Gln |
| Asn | Gln, Asp, Ser, Asn |
| Lys | Glu, Gln, His, Arg, Lys |
| Asp | Glu, Asn, Asp |
| Glu | Asp, Lys, Asn, Gln, His, Arg, Glu |
| Met | Phe, lie, Val, Leu, Met |
| Trp | Trp |

The length of a trafficking sequences used for a transporter construct as defined above, i.e. the number of contiguous amino acids selected from any of the trafficking sequences as defined above, may in a particular embdoiment be determined by the number of amino acids of generic formula (I) or by any of subformulas (Ia), (Ib), (Ic), (Id), (Ie), or (If) as defined above. The (sequence) length of an inventive transporter construct as defined above according to generic formula (I) or according to any of subformulas (Ia), (Ib), (Ic), (Id), (Ie), or (If), may therefore deviate from the length of the trafficking sequences as shown herein, e.g. in Table 1 or 3. In other words, the length of the transporter construct as defined above according to generic formula (I) or according to any of subformulas (Ia), (Ib), (Ic), (Id), (Ie), or (If) as defined above may determine the length of the amino acid sequence, which is taken from a trafficking sequence as defined herein, provided that the amino acid sequence is taken from a contiguous stretch of amino acids of said trafficking sequence. As an example, if the length of a transporter construct as defined above according to generic formula (I) or according to any of subformulas (Ia), (Ib), (Ic), (Id), (Ie), or (If), as defined above, is 9 amino acids, a sequence suitable as a transporter construct is derived from 9 contiguous amino acids of a trafficking sequence as defined above, wherein the selected amino acid sequence may be derived from any position or region of said trafficking sequence. The same holds for any other length determined by generic formula (I) or by any of subformulas (Ia), (Ib), (Ic), (Id), (Ie), or (If), as defined above. It is also contemplated that in some embodiments the trafficking sequence of the transporter construct according to the present invention is less than 150, 140, 130, 120, 110, 100, 90, 80, 70, 60, 50, 40, 30, 20, and/or less than 10 amino acids in length.

According to another preferred embodiment, an inventive transporter construct as defined above according to generic formula (I) or according to any of subformulas (Ia), (Ib), (Ic), (Id), (Ie), or (If), comprises or consists of at least one variant and/or fragment of the above defined sequences. These variants and/or fragments retain biological activity of the inventive transporter constructs as defined above according to generic formula (I) or according to any of subformulas (Ia), (Ib), (Ic), (Id), (Ie), or (If), as disclosed above. Functionality of such fragments or variants may be tested by various tests, e.g. transfection efficacy, correct expression of proteins encoded by cargo nucleic acids, or by biophysical methods, e.g. spectroscopy, computer modeling, structural analysis, etc. Particularly, the inventive transporter constructs as described above according to generic formula (I) or according to any of subformulas (Ia), (Ib), (Ic), (Id), (Ie), or (If), as disclosed above or variants and/or fragments thereof may be analyzed by hydrophilicity analysis (see e.g. Hopp and Woods, 1981. Proc Natl Acad Sci USA 78: 3824-3828) that can be utilized to identify the hydrophobic and hydrophilic regions of the peptides, thus aiding in the design of substrates for experimental manipulation. Secondary structural analysis may also be performed to identify regions of the inventive transporter construct as defined above according to generic formula (I) or according to any of subformulas (Ia), (Ib), (Ic), (Id), (Ie), or (If), as disclosed above or of variants and/or fragments thereof that assume specific structural motifs (see e.g. Chou and Fasman, 1974, Biochem 13: 222-223). Manipulation, translation, secondary structure prediction, hydrophilicity and hydrophobicity profiles, open reading frame prediction and plotting, and determination of sequence homologies can be accomplished using computer software programs available in the art. Other methods of structural analysis include, e.g. X-ray crystallography (see e.g. Engstrom, 1974. Biochem Exp Biol 11:7-13), mass spectroscopy and gas chromatography (see e.g. METHODS IN PROTEIN SCIENCE, 1997, J. Wiley and Sons, New York, NY) and computer modeling (see e.g. Fletterick and Zoller, eds., 1986. Computer Graphics and Molecular Modeling, In: CURRENT COMMUNICATIONS IN MOLECULAR BIOLOGY, Cold Spring Harbor Laboratory Press, Cold Spring Harbor, NY) may also be employed.

Likewise, the inventive transporter construct as defined above according to generic formula (I) or according to any of subformulas (Ia), (Ib), (Ic), (Id), (Ie), or (If) above, comprises or consists of at least one variant (and/or fragment) of the above defined transporter constructs. In the context of the invention variants (and/or fragments) of these transporter constructs as described above may have a sequence identity to their native transporter construct as defined above according to generic formula (I) or according to any of subformulas (Ia), (Ib), (Ic), (Id), (Ie), or (If) of at least 80% or 85%, preferably at least 90%, more preferably at least 95% and most preferably at least 99% over the whole length of the native transporter construct as defined above.

A "fragment" of an inventive transporter construct as defined above according to generic formula (I) or according to any of subformulas (Ia), (Ib), (Ic), (Id), (Ie), or (If) above, is preferably to be understood as a truncated sequence thereof, i.e. an amino acid sequence of the transporter construct, which is N-terminally, C-terminally and/or intrasequentially truncated compared to the amino acid sequence of the native sequence, e.g. the native inventive transporter construct as described above according to generic formula (I) or according to any of subformulas (Ia), (Ib), (Ic), (Id), (Ie), or (If) above.

A "variant" of an inventive transporter construct as described above according to generic formula (I) or according to any of subformulas (Ia), (Ib), (Ic), (Id), (Ie), or (If) above preferably comprises a sequence wherein the amino acid sequence of the transporter construct variant differs from the native sequence of the transporter construct as defined herein in one or more mutation(s), such as one or more substituted, (or, if necessary, inserted and/or deleted) amino acid(s). Preferably, variants of such inventive transporter constructs have the same biological function or specific activity compared to the full-length native inventive transporter constructs as defined above. Preferably, a variant of inventive transporter constructs may comprise about 1 to 50, 1 to 20, preferably 1 to 10 and more preferably 1 to 5, 1 to 4, 1 to 3, 1 to 2 or 1 amino acid alteration(s) within the above meaning. Such alterations may comprise *inter alia* modifications of amino acids as defined above, introduction of labels into amino acids as defined above, substituting an amino acid with any of the (modified or labelled) amino acids mentioned herein, deletions or insertions of amino acids. Variants as defined herein furthermore preferably comprise conservative amino acid substitutions, preferably such as already defined above.

In order to determine the percentage to which two amino acid sequences are identical, particularly the amino acid sequence of an inventive transporter construct as described above according to generic formula (I) or according to any of subformulas (Ia), (Ib), (Ic), (Id), (Ie), or (If) above, or of any further amino acid sequence as defined herein, the amino acid sequences can be aligned in order to be subsequently compared to one another. Therefore, as an example, e.g. gaps can be inserted into the sequence of the first amino acid sequence and the component at the corresponding position of the second amino acid sequence can be compared. If a position in the first amino acid sequence is occupied by the same component as is the case at a position in the second amino acid sequence, the two sequences are identical at this position. The percentage to which two sequences are identical is a function of the number of identical positions divided by the total number of positions. The same, of course also applies accordingly to nucleic acid sequences. In the above context, an amino acid sequence having a sequence "sharing a sequence identity" of at least, for example, 95% to a query amino acid sequence of the present invention, is intended to mean that the sequence of the subject amino acid sequence is identical to the query sequence except that the subject amino acid sequence may include up to five amino acid alterations per each 100 amino acids of the query amino acid sequence. In other words, to obtain an amino acid sequence having a sequence of at least 95% identity to a query amino acid sequence, up to 5% (5 of 100) of the amino acid residues in the subject sequence may be inserted or substituted with another amino acid or deleted, preferably within the above definitions of variants or fragments. The same, of course, also applies similarly to nucleic acid sequences.

For (amino acid or nucleic acid) sequences without exact correspondence, a "% identity" of a first sequence may be determined with respect to a second sequence. In general, these two sequences to be compared are aligned to give a maximum correlation between the sequences. This may include inserting "gaps" in either one or both sequences, to enhance the degree of alignment. A % identity may then be determined over the whole length of each of the sequences being compared (so-called global alignment), that is particularly suitable for sequences of the same or similar length, or over shorter, defined lengths (so-called local alignment), that is more suitable for sequences of unequal length.

Methods for comparing the identity and homology of two or more sequences are well known in the art. The percentage to which two sequences are identical can e.g. be determined using a mathematical algorithm. A preferred, but not limiting, example of a mathematical algorithm which can be used is the algorithm of Karlin et al. (1993), PNAS USA, 90:5873-5877. Such an algorithm is integrated in the BLAST family of programs, e.g. BLAST or NBLAST program (see also Altschul et al., 1990, J. Mol. Biol. 215, 403-410 or Altschul et al. (1997), Nucleic Acids Res, 25:3389-3402), accessible through the home page of the NCBI at world wide web site ncbi.nlm.nih.gov) and FASTA (Pearson (1990), Methods Enzymol. 183, 63-98; Pearson and Lipman (1988), Proc. Natl. Acad. Sci. U. S. A 85, 2444-2448.). Sequences which are identical to other sequences to a certain extent can be identified by these programmes. Furthermore, programs available in the Wisconsin Sequence Analysis Package, version 9.1 (Devereux et al., 1984, Nucleic Acids Res., 387-395), for example the programs BESTFIT and GAP, may be used to determine the % identity between two polynucleotides and the % identity and the % homology or identity between two polypeptide sequences. BESTFIT uses the "local homology" algorithm of (Smith and Waterman (1981), J. Mol. Biol. 147, 195-197.) and finds the best single region of similarity between two sequences.

According to a particularly preferred embodiment the inventive transporter construct as defined above comprises the subformula (If) DLLLDLLLD (SEQ ID NO: 7) as defined above, wherein the transporter sequence is selected from any of the above mentioned (specific) sequences, even more preferably from following sequences:

**TABLE 3**

| SEQUENCE/PEPTIDE NAME | SEQ ID NO | AA | SEQUENCE |
|---|---|---|---|
| r₃-L-TAT | 20 | 9 | rKKRrQRRr |
| r₃-L-TATi | 21 | 9 | rRRQrRKKr |
| βA-r₃-L-TAT | 22 | 9 | βA-rKKRrQRRr |
| βA-r₃-L-TATi | 23 | 9 | βA-rRRQrRKKr |
| FITC-βA-r₃-L-TAT | 24 | 9 | FITC-βA-rKKRrQRRr |
| FITC-βA-r₃-L-TATi | 25 | 9 | FITC-βA-rRRQrRKKr |
| TAT(s2-1) | 26 | 9 | rAKRrQRRr |
| TAT(s2-2) | 27 | 9 | rKARrQRRr |
| TAT(s2-3) | 28 | 9 | rKKArQRRr |
| TAT(s2-4) | 29 | 9 | rKKRrARRr |
| TAT(s2-5) | 30 | 9 | rKKRrQARr |
| TAT(s2-6) | 31 | 9 | rKKRrQRAr |
| TAT(s2-7) | 32 | 9 | rDKRrQRRr |
| TAT(s2-8) | 33 | 9 | rKDRrQRRr |
| TAT(s2-9) | 34 | 9 | rKKDrQRRr |
| TAT(s2-10) | 35 | 9 | rKKRrDRRr |
| TAT(s2-11) | 36 | 9 | rKKRrQDRr |
| TAT(s2-12) | 37 | 9 | rKKRrORDr |
| TAT(s2-13) | 38 | 9 | rEKRrQRRr |
| TAT(s2-14) | 39 | 9 | rKERrQRRr |
| TAT(s2-15) | 40 | 9 | rKKErQRRr |
| TAT(s2-16) | 41 | 9 | rKKRrERRr |
| TAT(s2-17) | 42 | 9 | rKKRrQERr |
| TAT(s2-18) | 43 | 9 | rKKRrQREr |
| TAT(s2-19) | 44 | 9 | rFKRrQRRr |
| TAT(s2-20) | 45 | 9 | rKFRrQRRr |
| TAT(s2-21) | 46 | 9 | rKKFrQRRr |
| TAT(s2-22) | 47 | 9 | rKKRrFRRr |
| TAT(s2-23) | 46 | 9 | rKKRrQFRr |
| TAT(s2-24) | 49 | 9 | rKKRrQRFr |
| TAT(s2-25) | 50 | 9 | rRKRrQRRr |
| TAT(s2-26) | 51 | 9 | rKRRrQRRr |
| TAT(s2-27) | 52 | 9 | rKKKrQRRr |
| TAT(s2-28) | 53 | 9 | rKKRrRRRr |
| TAT(s2-29) | 54 | 9 | rKKRrQKRr |
| TAT(s2-30) | 55 | 9 | rKKRrQRKr |
| TAT(s2-31) | 56 | 9 | rHKRrQRRr |
| TAT(s2-32) | 57 | 9 | rKHRrQRRr |
| TAT(s2-33) | 58 | 9 | rKKHrQRRr |
| TAT(s2-34) | 59 | 9 | rKKRrHRRr |
| TAT(s2-35) | 60 | 9 | rKKRrQHRr |
| TAT(s2-36) | 61 | 9 | rKKRrQRHr |
| TAT(s2-37) | 62 | 9 | rIKRrQRRr |
| TAT(s2-38) | 63 | 9 | rKIRrQRRr |
| TAT(s2-39) | 64 | 9 | rKKIrQRRr |
| TAT(s2-40) | 65 | 9 | rKKRrIRRr |
| TAT(s2-41) | 66 | 9 | rKKRrQIRr |
| TAT(s2-42) | 67 | 9 | rKKRrQRIr |
| TAT(s2-43) | 68 | 9 | rLKRrQRRr |
| TAT(s2-44) | 69 | 9 | rKLRrQRRr |
| TAT(s2-45) | 70 | 9 | rKKLrQRRr |
| TAT(s2-46) | 71 | 9 | rKKRrLRRr |
| TAT(s2-47) | 72 | 9 | rKKRrQLRr |
| TAT(s2-48) | 73 | 9 | rKKRrQRLr |
| TAT(s2-49) | 74 | 9 | rMKRrQRRr |
| TAT(s2-50) | 75 | 9 | rKMRrQRRr |
| TAT(s2-51) | 76 | 9 | rKKMrQRRr |
| TAT(s2-52) | 77 | 9 | rKKRrMRRr |
| TAT(s2-53) | 78 | 9 | rKKRrQMRr |
| TAT(s2-54) | 79 | 9 | rKKRrQRMr |
| TAT(s2-55) | 80 | 9 | rNKRrQRRr |
| TAT(s2-56) | 81 | 9 | rKNRrQRRr |
| TAT(s2-57) | 82 | 9 | rKKNrQRRr |
| TAT(s2-58) | 83 | 9 | rKKRrNRRr |
| TAT(s2-59) | 84 | 9 | rKKRrQNRr |
| TAT(s2-60) | 85 | 9 | rKKRrQRNr |
| TAT(s2-61) | 86 | 9 | rQKRrQRRr |
| TAT(s2-62) | 87 | 9 | rKQRrQRRr |
| TAT(s2-63) | 88 | 9 | rKKQrQRRr |
| TAT(s2-64) | 89 | 9 | rKKRrKRRr |
| TAT(s2-65) | 90 | 9 | rKKRrQQRr |
| TAT(s2-66) | 91 | 9 | rKKRrQRQr |
| TAT(s2-67) | 92 | 9 | rSKRrQRRr |
| TAT(s2-68) | 93 | 9 | rKSRrQRRr |
| TAT(s2-69) | 94 | 9 | rKKSrQRRr |
| TAT(s2-70) | 95 | 9 | rKKRrSRRr |
| TAT(s2-71) | 96 | 9 | rKKRrQSRr |
| TAT(s2-72) | 97 | 9 | rKKRrQRSr |
| TAT(s2-73) | 98 | 9 | rTKRrQRRr |
| TAT(s2-74) | 99 | 9 | rKTRrQRRr |
| TAT(s2-75) | 100 | 9 | rKKTrQRRr |
| TAT(s2-76) | 101 | 9 | rKKRrTRRr |
| TAT(s2-77) | 102 | 9 | rKKRrQTRr |
| TAT(s2-78) | 103 | 9 | rKKRrQRTr |
| TAT(s2-79) | 104 | 9 | rVKRrQRRr |
| TAT(s2-80) | 105 | 9 | rKVRrQRRr |
| TAT(s2-81) | 106 | 9 | rKKVrQRRr |
| TAT(s2-82) | 107 | 9 | rKKRrVRRr |
| TAT(s2-83) | 108 | 9 | rKKRrQVRr |
| TAT(s2-84) | 109 | 9 | rKKRrQRVr |
| TAT(s2-85) | 110 | 9 | rWKRrQRRr |
| TAT(s2-86) | 111 | 9 | rKWRrQRRr |
| TAT(s2-87) | 112 | 9 | rKKWrQRRr |
| TAT(s2-88) | 113 | 9 | rKKRrWRRr |
| TAT(s2-89) | 114 | 9. | rKKRrQWRr |
| TAT(s2-90) | 115 | 9 | rKKRrQRWr |
| TAT(s2-91) | 116 | 9 | rYKRrQRRr |
| TAT(s2-92) | 117 | 9 | rKYRrQRRr |
| TAT(s2-93) | 118 | 9 | rKKYrQRRr |
| TAT(s2-94) | 119 | 9 | rKKRrYRRr |
| TAT(s2-95) | 120 | 9 | rKKRrQYRr |
| TAT(s2-96) | 121 | 9 | rKKRrQRYr |
| r₃R₆ | 122 | 9 | rRRRrRRRr |
| L-R₉ | 123 | 9 | RRRRRRRRR |

In the above table, the subformula (If) DLLLDLLLD (SEQ ID NO: 7) is applied to the above specific sequences, i.e. inventive transporter construct as defined above comprises 9 amino acids, wherein the D-enatiomeric amino acids are indicated herein with a small character and the L-enatiomeric amino acids are indicated with a capital letter.

In a particular embodiment the inventive transporter contruct comprise or consist of at least one sequence according to rXXXrXXXr (SEQ ID NO: 252), wherein:
r represents an D-enatiomeric arginine;
X is any L-amino acid;
and wherein each X may be selected individually and independently of any other X within SEQ ID NO: 252. Preferably at least 4 out of said 6 X L-amino acids within SEQ ID NO: 252 are K or R. In another embodiment the transporter construct according to the present invention comprises or consists of the sequence rX₁X₂X₃rX₄X₅X₆r (SEQ ID NO: 252), wherein X₁ is K, X₂ is K, X₃ is R and X₄, X₅, and X₆ are any L-amino acid selected independently from each other. Similarly, the transporter construct according to the present invention may comprise or consist of the sequence rX₁X₂X₃rX₄X₅X₆r (SEQ ID NO: 252), wherein X₄ is Q, X₅ is R, X₆ is R and X₁, X₂, and X₃ are any L-amino acid selected independently from each other. The inventive transporter construct may also comprises or consist of the sequence rX₁X₂X₃rX₄X₅X₆r (SEQ ID NO: 252), wherein one, two, three, four, five or six X amino acid residues are chosen from the group consisting of: X₁ is K, X₂ is K, X₃ is R, X₄ is Q, X₅ is R, X₆ is R, while the remaining X amino acid residues not selected from above group may be any L-amino acid and are selected independently from each other. X₁ is then preferably Y and/or X₄ is preferably K or R. Similarly considered are reverse sequences of the above mentioned sequences and embodiments of SEQ ID NO: 252.

Surprisingly, the present inventors found, that a tyrosine (Y) at position 2 of formula (I) above not only significantly increases uptake but also a significantly increased intracellular concentration after inoculation for 25 hours. This is particularly observed for transporter constructs which comprise a trafficking sequence derived from HIV-1 TAT protein as shown in Table 1, which show an Y in position 2, preferably have 9 aa and exhibiting the consensus sequence rXXXrXXXr (SEQ ID NO: 252). According to a particularly preferred embodiment of the present invention, the inventive transporter construct as defined above therefore comprises transporter constructs according to subformula (If) as defined above, wherein the subformula (If) is applied to a trafficking sequence derived from HIV-1 TAT protein having a Tyrosine (Y) at position 2 of the TAT derived sequence. Even more preferably, the subformula (If) is applied to a trafficking sequence derived from HIV-1 TAT protein having a Tyrosine (Y) at position 2 of the TAT derived sequence, wherein the transporter construct as defined above according to subformula (If) as defined above preferably has 9 aa and the consensus sequence rXXXrXXXr (SEQ ID NO: 252 wherein the D-enatiomeric amino acids are indicated herein with a small character and the L-enatiomeric amino acids are indicated with a capital letter). Most preferably, the subformula (If) is applied to a trafficking sequence derived from HIV-1 TAT, wherein the transporter construct as defined above according to subformula (If) as defined above preferably has 9 aa and the consensus sequence rXXXrXXXr (SEQ ID NO: 252) and the trafficking sequence is selected from the sequence TAT(s2-91) (SEQ ID NO: 116). The inventive transporter constructs as defined above according to generic formula (I) or according to any of subformulas (Ia), (Ib), (Ic), (Id), (Ie), or (If) above, may furthermore comprise at least one modification, preferably at their termini, either at the C- or the N-terminus or both. The C-Terminus may preferably be modified by an amide modification, whereas the N-terminus may be modified by any suitable NH₂-protection group, such as e.g. acylation, or any further modification as already indicated above for L-amino acids and D-amino acids. Such modifications also includes introduction of labels as defined above.

The inventive transporter constructs as defined above according to generic formula (I) or according to any of subformulas (Ia), (Ib), (Ic), (Id), (Ie), or (If) above, may be obtained or produced by methods well-known in the art, e.g. by chemical synthesis as defined above or by genetic engineering methods.

According to a second aspect of the present invention the underlying object is solved by an inventive transporter cargo conjugate molecule, comprising as a component (A) the inventive transporter construct as defined above according to generic formula (I) or according to any of subformulas (Ia), (Ib), (Ic), (Id), (Ie), or (If) above, and as a component (B) an effector molecule selected from therapeutically active proteins and peptides, protein kinase inhibitors, particularly inhibitors of the protein kinase c-Jun amino terminal kinase, antigens, antibodies, apoptotic factors, proteases implicated in pathological states, preferably peptidic protease inhibitors, BH3-domains, BH3-only proteins, nucleic acids encoding these proteins, siRNAs, antisense RNAs, cytotoxic agents, and small organic compounds.

In the context of the present invention, a therapeutically active protein or peptide suitable as the effector molecule for component (B) of the inventive transporter cargo conjugate molecule may be selected from, without being limited thereto, proteins, capable of stimulating or inhibiting the signal transduction in the cell, e.g. cytokines, antibodies, etc. Therapeutically active proteins may thus comprise cytokines of class I of the family of cytokines, having 4 positionally conserved cysteine residues (CCCC) and comprising a conserved sequence motif Trp-Ser-X-Trp-Ser (WSXWS; SEQ ID NO: 253), wherein X is a non-conserved amino acid. Cytokines of class I of the family of cytokines comprise the GM-CSF subfamily, e.g. IL-3, IL-5, GM-CSF, the IL-6-subfamily, e.g. IL-6, IL-11, IL-12, or the IL-2-subfamily, e.g. IL-2, IL-4, IL-7, IL-9, IL-15, etc., or the cytokines IL-1alpha, IL-1beta, IL-10 etc. Therapeutically active proteins may also comprise cytokines of class II of the family of cytokines, which also comprise 4 positionally conserved cystein residues (CCCC; SEQ ID NO: 254), but no conserved sequence motif Trp-Ser-X-Trp-Ser (WSXWS; SEQ ID NO: 253). Cytokines of class II of the family of cytokines comprise e.g. IFN-alpha, IFN-beta, IFN-gamma, etc. Therapeutically active proteins may additionally comprise cytokines of the family of tumor necrose factors, e.g. TNF-alpha, TNF-beta, etc., or cytokines of the family of chemokines, which comprise 7 transmembrane helices and interact with G-protein, e.g. IL-8, MIP-1, RANTES, CCR5, CXR4, etc., or cytokine specific receptors, such as TNF-RI, TNF-RII, CD40, OX40 (CD134), Fas, or from fragments or variants thereof. Preferably, such fragments as well as variants exhibit a sequence homology or identity of about 10 %, about 20 %, about 30 %, about 40 %, about 50 %, about 60 %, about 70 %, about 80 %, or about 90 % with one of the protein or peptide sequences as shown or described above. In this context, fragments and variants are preferably as defined above for component (A) of the inventive transporter cargo conjugate molecule.

Therapeutically active proteins suitable as the effector molecule of component (B) of the inventive transporter cargo conjugate molecule may also be selected from any of the proteins given in the following: 0ATL3, 0FC3, 0PA3, 0PD2, 4-1BBL, 5T4, 6Ckine, 707-AP, 9D7, A2M, AA, AAAS, AACT, AASS, ABAT, ABCA1, ABCA4, ABCB1, ABCB11, ABCB2, ABCB4, ABCB7, ABCC2, ABCC6, ABCC8, ABCD1, ABCD3, ABCG5, ABCG8, ABL1, ABO, ABR ACAA1, ACACA, ACADL, ACADM, ACADS, ACADVL, ACAT1, ACCPN, ACE, ACHE, ACHM3, ACHM1, ACLS, ACPI, ACTA1, ACTC, ACTN4, ACVRL1, AD2, ADA, ADAMTS13, ADAMTS2, ADFN, ADH1B, ADH1C, ADLDH3A2, ADRB2, ADRB3, ADSL, AEZ, AFA, AFD1, AFP, AGA, AGL, AGMX2, AGPS, AGS1, AGT, AGTR1, AGXT, AH02, AHCY, AHDS, AHHR, AHSG, AIC, AIED, AIH2, AIH3, AIM-2, AIPL1, AIRE, AK1, ALAD, ALAS2, ALB, HPG1, ALDH2, ALDH3A2, ALDH4A1, ALDH5A1, ALDH1A1, ALDOA, ALDOB, ALMS1, ALPL, ALPP, ALS2, ALX4, AMACR, AMBP, AMCD, AMCD1, AMCN, AMELX, AMELY, AMGL, AMH, AMHR2, AMPD3, AMPD1, AMT, ANC, ANCR, ANK1, ANOP1, AOM, AP0A4, AP0C2, AP0C3, AP3B1, APC, aPKC, APOA2, APOA1, APOB, APOC3, APOC2, APOE, APOH, APP, APRT, APS1, AQP2, AR, ARAF1, ARG1, ARHGEF12, ARMET, ARSA, ARSB, ARSC2, ARSE, ART-4, ARTC1/m, ARTS, ARVD1, ARX, AS, ASAH, ASAT, ASD1, ASL, ASMD, ASMT, ASNS, ASPA, ASS, ASSP2, ASSP5, ASSP6, AT3, ATD, ATHS, ATM, ATP2A1, ATP2A2, ATP2C1, ATP6B1, ATP7A, ATP7B, ATP8B1, ATPSK2, ATRX, ATXN1, ATXN2, ATXN3, AUTS1, AVMD, AVP, AVPR2, AVSD1, AXIN1, AXIN2, AZF2, B2M, B4GALT7, B7H4, BAGE, BAGE-1, BAX, BBS2, BBS3, BBS4, BCA225, BCAA, BCH, BCHE, BCKDHA, BCKDHB, BCL10, BCL2, BCL3, BCL5, BCL6, BCPM, BCR, BCR/ABL, BDC, BDE, BDMF, BDMR, BEST1, beta-Catenin/m, BF, BFHD, BFIC, BFLS, BFSP2, BGLAP,BGN, BHD, BHR1, BING-4, BIRC5, BJS, BLM, BLMH, BLNK, BMPR2, BPGM, BRAF, BRCA1, BRCA1/m, BRCA2, BRCA2/m, BRCD2, BRCD1, BRDT, BSCL, BSCL2, BTAA, BTD, BTK, BUB1, BWS, BZX, C0L2A1, C0L6A1, C1NH, C1QA, C1 QB, C1QG, C1S, C2, C3, C4A, C4B, C5, C6, C7, C7orf2, C8A, C8B, C9, CA125, CA15-3/CA 27-29, CA195, CA19-9, CA72-4, CA2, CA242, CA50, CABYR, CACD, CACNA2D1, CACNA1 A, CACNA1F, CACNA1S, CACNB2, CACNB4, CAGE, CA1, CALB3, CALCA, CALCR, CALM, CALR, CAM43, CAMEL, CAP-1, CAPN3, CARD15, CASP-5/m, CASP-8, CASP-8/m, CASR, CAT, CATM, CAV3, CB1, CBBM, CBS, CCA1, CCAL2, CCAL1, CCAT, CCL-1, CCL-11, CCL-12, CCL-13, CCL-14, CCL-15, CCL-16, CCL-17, CCL-18, CCL-19, CCL-2, CCL-20, CCL-21, CCL-22, CCL-23, CCL-24, CCL-25, CCL-27, CCL-3, CCL-4, CCL-5, CCL-7, CCL-8, CCM1, CCNB1, CCND1, CCO, CCR2, CCR5, CCT, CCV, CCZS, CD1, CD19, CD20, CD22, CD25, CD27, CD27L, cD3, CD30, CD30, CD30L, CD33, CD36, CD3E, CD3G, CD3Z, CD4, CD40, CD40L, CD44, CD44v, CD44v6, CD52, CD55, CD56, CD59, CD80, CD86, CDAN1, CDAN2, CDAN3, CDC27, CDC27/m, CDC2L1, CDH1, CDK4, CDK4/m, CDKN1C, CDKN2A, CDKN2A/m, CDKN1A, CDKN1C, CDL1, CDPD1, CDR1, CEA, CEACAM1, CEACAM5, CECR, CECR9, CEPA, CETP, CFNS, CFTR, CGF1, CHAC, CHED2, CHED1, CHEK2, CHM, CHML, CHR39C, CHRNA4, CHRNA1, CHRNB1, CHRNE, CHS, CHS1, CHST6, CHX10, CIAS1, CIDX, CKN1, CLA2, CLA3, CLA1, CLCA2, CLCN1, CLCN5, CLCNKB, CLDN16, CLP, CLN2, CLN3, CLN4, CLN5, CLN6, CLN8, C1QA, C1QB, C1QG, C1 R, CLS, CMCWTD, CMDJ, CMD1 A, CMD1B, CMH2, MH3, CMH6, CMKBR2, CMKBR5, CML28, CML66, CMM, CMT2B, CMT2D, CMT4A, CMT1A, CMTX2, CMTX3, C-MYC, CNA1, CND, CNGA3, CNGA1, CNGB3, CNSN, CNTF, COA-1/m, COCH, COD2, COD1, COH1, COL10A, COL2A2, COL11A2, COL17A1, COL1A1, COL1A2, COL2A1, COL3A1, COL4A3, COL4A4, COL4A5, COL4A6, COL5A1, COL5A2, COL6A1, COL6A2, COL6A3, COL7A1, COL8A2, COL9A2, COL9A3, COL11A1, COL1A2, COL23A1, COL1A1, COLQ, COMP, COMT, CORDS, CORD1, COX10, COX-2, CP, CPB2, CPO, CPP, CPS1, CPT2, CPT1A, CPX, CRAT, CRB1, CRBM, CREBBP, CRH, CRHBP, CRS, CRV, CRX, CRYAB, CRYBA1, CRYBB2, CRYGA, CRYGC, CRYGD, CSA, CSE, CSF1 R, CSF2RA, CSF2RB, CSF3R, CSF1R, CST3, CSTB, CT, CT7, CT-9/BRD6, CTAA1, CTACK, CTEN, CTH, CTHM, CTLA4, CTM, CTNNB1, CTNS, CTPA, CTSB, CTSC, CTSK, CTSL, CTS1, CUBN, CVD1, CX3CL1, CXCL1, CXCL10, CXCL11, CXCL12, CXCL13, CXCL16, CXCL2, CXCL3, CXCL4, CXCL5, CXCL6, CXCL7, CXCL8, CXCL9, CYB5, CYBA, CYBB, CYBB5,, CYFRA21-1, CYLD, CYLD1, CYMD, CYP11B1, CYP11B2, CYP17, CYP17A1, CYP19, CYP19A1, CYP1A2, CYP1B1, CYP21A2, CYP27A1, CYP27B1, CYP2A6, CYP2C, CYP2C19, CYP2C9, CYP2D, CYP2D6, CYP2D7P1, CYP3A4, CYP7B1, CYPB1, CYP11B1, CYP1A1, CYP1B1, CYRAA, D40,DADI, DAM, DAM-10/MAGE-B1, DAM-6/MAGE-B2, DAX1, DAZ, DBA, DBH, DBI, DBT, DCC, DC-CK1, DCK, DCR, DCX, DDB 1, DDB2, DDIT3, DDU, DECR1, DEK-CAN, DEM, DES, DF,DFN2, DFN4, DFN6, DFNA4, DFNA5, DFNB5, DGCR, DHCR7, DHFR, DHOF, DHS, DIA1, DIAPH2, DIAPH1, DIH1, DIO1, DISCI, DKC1, DLAT, DLD, DLL3, DLX3, DMBT1, DMD, DM1, DMPK, DMWD, DNAI1, DNASE1, DNMT3B, DPEP1, DPYD, DPYS, DRD2, DRD4, DRPLA, DSCR1, DSG1, DSP, DSPP, DSS, DTDP2, DTR, DURS1, DWS, DYS, DYSF, DYT2, DYT3, DYT4, DYT2, DYT1, DYX1, EBAF, EBM, EBNA, EBP, EBR3, EBS1, ECA1, ECB2, ECE1, ECGF1, ECT, ED2, ED4, EDA, EDAR, ECA1, EDN3, EDNRB, EEC1, EEF1A1L14, EEGV1, EFEMP1, EFTUD2/m, EGFR, EGFR/Her1, EGI, EGR2, EIF2AK3, eIF4G, EKV, EI IS, ELA2, ELF2, ELF2M, ELK1, ELN, ELONG, EMD, EML1, EMMPRIN, EMX2, ENA-78, ENAM, END3, ENG, ENO1, ENPP1, ENUR2, ENUR1, EOS, EP300, EPB41, EPB42, EPCAM, EPD, EphA1, EphA2, EphA3, EphrinA2, EphrinA3, EPHX1, EPM2A, EPO,EPOR, EPX, ERBB2, ERCC2 ERCC3,ERCC4, ERCC5, ERCC6, ERVR, ESR1, ETFA, ETFB, ETFDH, ETM1, ETV6-AML1, ETV1, EVC, EVR2, EVR1, EWSR1, EXT2, EXT3, EXT1, EYA1, EYCL2, EYCL3, EYCL1, EZH2, F10, F11, F12, F13A1, F13B, F2, F5, F5F8D, F7, F8, F8C, F9, FABP2, FACL6, FAH, FANCA, FANCB, FANCC, FANCD2, FANCF, FasL,FBN2, FBN1, FBP1, FCG3RA,FCGR2A, FCGR2B, FCGR3A, FCHL, FCMD, FCP1, FDPSL5, FECH, FEO, FEOM1, FES, FGA, FGB, FGD1, FGF2, FGF23, FGF5, FGFR2, FGFR3, FGFR1, FGG, FGS1, FH, FIC1, FIH, F2, FKBP6, FLNA, FLT4, FMO3,FMO4, FMR2, FMR1, FN, FN1/m, FOXC1, FOXE1, FOXL2, FOXO1A, FPDMM, FPF, Fra-1, FRAXF, FRDA, FSHB, FSHMD1A, FSHR, FTH1, FTHL17, FTL, FTZF1, FUCA1, FUT2, FUT6, FUT1, FY, G250, G250/CAIX, G6PC, G6PD, G6PT1, G6PT2, GAA, GABRA3, GAGE-1, GAGE-2, GAGE-3, GAGE-4, GAGE-5, GAGE-6, GAGE-7b, GAGE-8, GALC, GALE, GALK1, GALNS, GALT, GAMT, GAN, GAST, GASTRIN17, GATA3, GATA, GBA, GBE, GC, GCDH, GCGR, GCH1, GCK, GCP-2, GCS1, G-CSF, GCSH, GCSL, GCY, GDEP,GDF5, GDI1, GDNF, GDXY, GFAP, GFND, GGCX, GGT1, GH2, GH1, GHR, GHRHR, GHS, GIF, GINGF, GIP, GJA3, GJA8, GJB2, GJB3, GJB6, GJB1, GK, GLA, GLB, GLB1, GLC3B, GLC1B, GLC1C, GLDC, GLI3, GLP1, GLRA1, GLUD1, GM1 (fuc-GM1), GM2A, GM-CSF, GMPR, GNAI2, GNAS, GNAT1, GNB3, GNE, GNPTA, GNRH, GNRH1, GNRHR, GNS, GnT-V, gp100, GP1BA, GP1BB, GP9, GPC3, GPD2, GPDS1, GPI, GP1BA, GPN1LW, GPNMB/m, GPSC, GPX1, GRHPR, GRK1, GROα, GROβ, GROγ, GRPR, GSE, GSM1, GSN, GSR, GSS, GTD, GTS, GUCA1A, GUCY2D, GULOP, GUSB, GUSM, GUST, GYPA, GYPC, GYS1, GYS2, HOKPP2, H0MG2, HADHA, HADHB, HAGE, HAGH, HAL, HAST-2, HB 1, HBA2, HBA1, HBB, HBBP1, HBD, HBE1, HBG2, HBG1, HBHR, HBP1, HBQ1, HBZ, HBZP, HCA, HCC-1, HCC-4, HCF2, HCG, HCL2, HCL1, HCR, HCVS, HD, HPN, HER2, HER2/NEU, HER3, HERV-K-MEL, HESX1, HEXA, HEXB, HF1, HFE, HF1, HGD, HHC2, HHC3, HHG, HK1 HLA-A, HLA-A*0201-R170I, HLA-A11/m, HLA-A2/m, HLA-DPB1 HLA-DRA, HLCS, HLXB9, HMBS, HMGA2, HMGCL, HMI, HMN2, HMOX1, HMS1 HMW-MAA, HND, HNE, HNF4A, HOAC, HOMEOBOX NKX 3.1, HOM-TES-14/SCP-1, HOM-TES-85, HOXA1 HOXD13, HP, HPC1, HPD, HPE2, HPE1, HPFH, HPFH2, HPRT1, HPS1, HPT, HPV-E6, HPV-E7, HR, HRAS, HRD, HRG, HRPT2, HRPT1, HRX, HSD11B2, HSD17B3, HSD17B4, HSD3B2, HSD3B3, HSN1, HSP70-2M, HSPG2, HST-2, HTC2, HTC1, hTERT, HTN3, HTR2C, HVBS6, HVBS1, HVEC, HV1S, HYAL1, HYR, I-309, IAB, IBGC1, IBM2, ICAM1, ICAM3, iCE, ICHQ, ICR5, ICR1, ICS 1, IDDM2, IDDM1, IDS, IDUA, IF, □IFNa/b, □IFNGR1, IGAD1, IGER, IGF-1R, IGF2R, IGF1, IGH, IGHC, IGHG2, IGHG1, IGHM, IGHR, IGKC, IHG1, IHH, IKBKG, IL1, IL-1 RA, IL10, IL-11, IL12, IL12RB1, IL13, IL-13Rα2, IL-15, IL-16, IL-17, IL18, IL-1a, IL-1α, IL-1b, IL-1β, IL1RAPL1, IL2, IL24, IL-2R, IL2RA, IL2RG, IL3, IL3RA,IL4, IL4R,IL4R, IL-5, IL6, IL-7, IL7R, IL-8, IL-9, Immature laminin receptor, IMMP2L, INDX, INFGR1, INFGR2, INFα, IFN □□INFγ, INS, INSR, INVS, IP-10, IP2, IPF1, IP1, IRF6, IRS1, ISCW, ITGA2, ITGA2B, ITGA6, ITGA7, ITGB2, ITGB3, ITGB4, ITIH1, ITM2B, IV, IVD, JAG1, JAK3, JBS, JBTS1, JMS, JPD, KAL1, KAL2, KALI, KLK2, KLK4, KCNA1, KCNE2, KCNE1, KCNH2, KCNJ1, KCNJ2, KCNJ1, KCNQ2, KCNQ3, KCNQ4, KCNQ1, KCS, KERA, KFM, KFS, KFSD, KHK, ki-67, KIAA0020, KIAA0205, KIAA0205/m, KIF1B, KIT, KK-LC-1, KLK3, KLKB1, KM-HN-1, KMS, KNG, KNO, K-RAS/m, KRAS2, KREV1, KRT1, KRT10, KRT12, KRT13, KRT14, KRT14L1, KRT14L2, KRT14L3,KRT16, KRT16L1, KRT16L2, KRT17, KRT18, KRT2A, KRT3, KRT4, KRT5, KRT6 A, KRT6B, KRT9, KRTHB1, KRTHB6, KRT1, KSA, KSS, KWE, KYNU, L0H19CR1, L1CAM, LAGE, LAGE-1, LALL, LAMA2, LAMA3, LAMB3, LAMB1, LAMC2, LAMP2, LAP, LCA5, LCAT, LCCS, LCCS 1, LCFS2, LCS1, LCT, LDHA, LDHB, LDHC, LDLR, LDLR/FUT, LEP, LEWISY, LGCR, LGGF-PBP, LGI1, LGMD2H, LGMD1A, LGMD1B, LHB, LHCGR, LHON, LHRH, LHX3, LIF, LIG1, LIMM, LIMP2, LIPA, LIPA, LIPB, LIPC, LIVIN, L1CAM, LMAN1, LMNA, LMX1B, LOLR, LOR, LOX, LPA, LPL, LPP, LQT4, LRP5, LRS 1, LSFC, LT- p , LTBP2, LTC4S, LYL1, XCL1, LYZ, M344, MA50, MAA, MADH4, MAFD2, MAFD1, MAGE, MAGE-A1, MAGE-A10, MAGE-A12, MAGE-A2, MAGE-A3, MAGE-A4, MAGE-A6, MAGE-A9, MAGEB1, MAGE-B10, MAGE-B16, MAGE-B17, MAGE-B2, MAGE-B3, MAGE-B4, MAGE-B5, MAGE-B6, MAGE-C1, MAGE-C2, MAGE-C3, MAGE-D1, MAGE-D2, MAGE-D4, MAGE-E1, MAGE-E2, MAGE-F1,MAGE-H1, MAGEL2, MGB1, MGB2, MAN2A1, MAN2B1, MANBA, MANBB, MAOA, MAOB, MAPK8IP1, MAPT, MART-1, MART-2, MART2/m, MAT1A, MBL2, MBP, MBS1, MC1R, MC2R, MC4R, MCC, MCCC2, MCCC1, MCDR1, MCF2, MCKD, MCL1, MC1R, MCOLN1, MCOP, MCOR, MCP-1, MCP-2, MCP-3, MCP-4, MCPH2, MCPH1, MCS, M-CSF, MDB, MDCR, MDM2, MDRV, MDS 1, ME1, ME1/m, ME2, ME20, ME3, MEAX, MEB, MEC CCL-28, MECP2, MEFV, MELANA, MELAS, MEN1 MSLN, MET, MF4, MG50, MG50/PXDN, MGAT2, MGAT5, MGC1 MGCR, MGCT, MGI, MGP, MHC2TA, MHS2, MHS4, MIC2, MIC5, MIDI, MIF, MIP, MIP-5/HCC-2, MITF, MJD, MKI67, MKKS, MKS1, MLH1, MLL, MLLT2, MLLT3, MLLT7, MLLT1, MLS, MLYCD, MMA1a, MMP 11, MMVP1, MN/CA IX-Antigen, MNG1, MN1, MOC31, MOCS2, MOCS1, MOG, MORC, MOS, MOV18, MPD1, MPE, MPFD, MPI, MPIF-1, MPL, MPO, MPS3C, MPZ, MRE11A, MROS, MRP1, MRP2, MRP3, MRSD, MRX14, MRX2, MRX20, MRX3, MRX40, MRXA, MRX1, MS, MS4A2, MSD, MSH2, MSH3, MSH6, MSS, MSSE, MSX2, MSX1, MTATP6, MTC03, MTCO1, MTCYB, MTHFR, MTM1, MTMR2, MTND2, MTND4, MTND5, MTND6, MTND1, MTP, MTR, MTRNR2, MTRNR1, MTRR,MTTE, MTTG, MTTI, MTTK, MTTL2, MTTL1, MTTN, MTTP, MTTS1, MUC1,MUC2, MUC4, MUC5AC, MUM-1, MUM-1/m, MUM-2, MUM-2/m, MUM-3, MUM-3/m, MUT, mutant p21 ras, MUTYH, MVK, MX2, MXI1, MY05A, MYB, MYBPC3, MYC, MYCL2, MYH6, MYH7, MYL2, MYL3, MYMY, MYO15A, MYO1G, MYO5A, MYO7A, MYOC, Myosin/m, MYP2, MYP1, NA88-A, N-acetylglucosaminyltransferase-V, NAGA, NAGLU, NAMSD, NAPB, NAT2, NAT, NBIA1, NBS1, NCAM, NCF2, NCF1, NDN, NDP, NDUFS4, NDUFS7, NDUFS8, NDUFV1, NDUFV2, NEB, NEFH, NEM1, Neo-PAP, neo-PAP/m, NEU1, NEUROD1, NF2, NF1, NFYC/m, NGEP, NHS, NKS1, NKX2E, NM, NME1, NMP22, NMTC, NODAL, NOG, NOS3, NOTCH3, NOTCH1, NP, NPC2, NPC1, NPHL2, NPHP1, NPHS2, NPHS1, NPM/ALK, NPPA, NQO1, NR2E3, NR3C1, NR3C2, NRAS, NRAS/m, NRL, NROB1, NRTN, NSE, NSX, NTRK1, NUMA1, NXF2, NY-CO1, NY-ESO1, NY-ESO-B, NY-LU-12, ALDOA, NYS2, NYS4, NY-SAR-35, NYS1, NYX, OA3, OA1, OAP, OASD, OAT, OCA1, OCA2, OCD1, OCRL, OCRL1, OCT, ODDD, ODT1, OFC1, OFD1, OGDH, OGT, OGT/m, OPA2, OPA1, OPD1, OPEM, OPG, OPN, OPN1 LW, OPN1MW, OPN1 SW, OPPG, OPTB1, TTD, ORM1, ORP1, OS-9, OS-9/m, OSM LIF, OTC, OTOF, OTSC1, OXCT1, OYTES1, P15, P190 MINOR BCR-ABL, P2RY12, P3, P16, P40, P4HB, P-501, P53, P53/m, P97, PABPN1, PAFAH1B1, PAFAH1P1, PAGE-4, PAGE-5, PAH, PAI-1, PAI-2, PAK3, PAP, PAPPA, PARK2, PART-1, PATE, PAX2, PAX3, PAX6, PAX7, PAX8, PAX9, PBCA, PBCRA1, PBT, PBX1, PBXP1, PC, PCBD, PCCA, PCCB, PCK2, PCK1, PCLD, PCOS1, PCSK1, PDB1, PDCN, PDE6A, PDE6B, PDEF, PDGFB, PDGFR, PDGFRL, PDHA1, PDR, PDX1, PECAM1, PEE1, PEO1, PEPD, PEX10, PEX12, PEX13, PEX3, PEX5, PEX6, PEX7, PEX1, PF4, PFBI, PFC, PFKFB1, PFKM, PGAM2, PGD, PGK1, PGK1P1, PGL2, PGR, PGS, PHA2A, PHB, PHEX, PHGDH, PHKA2, PHKA1, PHKB, PHKG2, PHP, PHYH, PI, PI3, PIGA, PIM1-KINASE, PIN1, PIP5K1B, PITX2, PITX3, PKD2, PKD3, PKD1, PKDTS, PKHD1, PKLR, PKP1, PKU1, PLA2G2A, PLA2G7, PLAT, PLEC1, PLG, PLI, PLOD, PLP1, PMEL17, PML, PML/RARα, PMM2, PMP22, PMS2, PMS1, PNKD, PNL1P, POF1, POLA, POLH, POMC, PON2, PON1, PORC, POTE, POU1F1, POU3F4, POU4F3, POU1F1, PPAC, PPARG, PPCD, PPGB, PPH1, PPKB, PPMX, PPOX, PPP1 R3A, PPP2R2B, PPT1, PRAME, PRB, PRB3, PRCA1, PRCC, PRD, PRDX5/m, PRF1, PRG4, PRKAR1A, PRKCA, PRKDC, PRKWNK4, PRNP, PROC, PRODH, PROM1, PROP1, PROS1, PRST, PRP8, PRPF31, PRPF8, PRPH2, PRPS2, PRPS1, PRS, PRSS7, PRSS1, PRTN3, PRX, PSA, PSAP, PSCA, PSEN2, PSEN1, PSG1, PSGR, PSM, PSMA, PSORS1, PTC, PTCH, PTCH1, PTCH2, PTEN, PTGS1, PTH, PTHR1, PTLAH, PTOS1, PTPN12, PTPNI I, PTPRK, PTPRK/m, PTS, PUJO, PVR, PVRL1, PWCR, PXE, PXMP3, PXR1, PYGL, PYGM, QDPR, RAB27A, RAD54B, RAD54L, RAG2, RAGE, RAGE-1, RAG1, RAP1, RARA, RASA1, RBAF600/m, RB1, RBP4, RBP4, RBS, RCA1, RCAS1, RCCP2, RCD1, RCV1, RDH5, RDPA, RDS, RECQL2, RECQL3, RECQL4, REG1A, REHOBE, REN, RENBP, RENS1, RET, RFX5, RFXANK, RFXAP, RGR, RHAG, RHAMM/CD168, RHD, RHO, Rip-1, RLBP1, RLN2, RLN1, RLS, RMD1, RMRP, ROM1, ROR2, RP, RP1, RP14, RP17, RP2, RP6, RP9, RPD1, RPE65, RPGR, RPGRIP1, RP1, RP10, RPS19, RPS2, RPS4X, RPS4Y, RPS6KA3, RRAS2, RS1, RSN, RSS, RU1, RU2, RUNX2,RUNXI, RWS, RYR1, S-100, SAA1, SACS, SAG, SAGE, SALL1, SARDH, SART1, SART2 , SART3, SAS, SAX1, SCA2, SCA4, SCA5, SCA7, SCA8, SCA1, SCC, SCCD, SCF, SCLC1, SCN1A, SCN1B, SCN4A, SCN5A, SCNN1 A, SCNN1B, SCNN1G, SCO2, SCP1, SCZD2, SCZD3, SCZD4, SCZD6, SCZD1, SDF-1α/ □□SDHA, SDHD, SDYS, SEDL, SERPENA7, SERPINA3, SERPINA6, SERPINA1, SERPINC1, SERPIND1, SERPINE1, SERPINF2, SERPING1, SERPINI1, SFTPA1, SFTPB, SFTPC, SFTPD, SGCA, SGCB, SGCD, SGCE, SGM1, SGSH, SGY-1, SH2D1A, SHBG, SHFM2, SHFM3, SHFM1, SHH, SHOX, SI, SIAL, SIALYL LEWISX , SIASD, S11, SIM1, SIRT2/m, SIX3, SJS1, SKP2, SLC10A2, SLC12A1, SLC12A3, SLC17A5, SLC19A2, SLC22A1L, SLC22A5, SLC25A13, SLC25A15, SLC25A20, SLC25A4, SLC25A5, SLC25A6, SLC26A2, SLC26A3, SLC26A4, SLC2A1, SLC2A2, SLC2A4, SLC3A1, SLC4A1, SLC4A4, SLC5A1, SLC5A5, SLC6A2, SLC6A3, SLC6A4, SLC7A7, SLC7A9, SLC11A1, SLOS, SMA, SMAD1, SMAL, SMARCB1, SMAX2, SMCR, SMCY, SM1, SMN2, SMN1, SMPD1, SNCA, SNRPN, SOD2, SOD3, SOD1, SOS1, SOST, SOX9, SOX10, Sp17, SPANXC, SPG23, SPG3A, SPG4, SPG5A, SPG5B, SPG6, SPG7, SPINK1, SPINK5, SPPK, SPPM, SPSMA, SPTA1, SPTB, SPTLC1, SRC, SRD5A2, SRPX, SRS, SRY, ßhCG, SSTR2, SSX1, SSX2 (HOM-MEL-40/SSX2), SSX4, ST8, STAMP-1, STAR, STARP1, STATH, STEAP, STK2, STK11, STn/ KLH, STO, STOM, STS, SUOX, SURF1, SURVIVIN-2B, SYCP1, SYM1, SYN1, SYNS1, SYP, SYT/SSX, SYT-SSX-1, SYT-SSX-2, TA-90, TAAL6, TACSTD1, TACSTD2, TAG72, TAF7L, TAF1, TAGE, TAG-72, TALI, TAM, TAP2, TAP1, TAPVR1, TARC, TARP, TAT, TAZ, TBP, TBX22, TBX3, TBX5, TBXA2R, TBXAS1, TCAP, TCF2, TCF1, TCIRG1, TCL2, TCL4, TCL1A, TCN2, TCOF1, TCR, TCRA, TDD, TDFA, TDRD1, TECK, TECTA, TEK, TEL/AML1, TELAB1, TEX15, TF, TFAP2B, TFE3, TFR2, TG, TGFA, TGF-□□β, TGFBI, TGFB1, TGFBR2, TGFBRE, TGFβ, TGFβRII, TGIF, TGM-4, TGM1, TH, THAS, THBD, THC, THC2, THM, THPO, THRA, THRB, TIMM8A, TIMP2, TIMP3, TIMP1, TITF1, TKCR, TKT, TLP, TLR1, TLR10, TLR2, TLR3, TLR4, TLR4, TLR5, TLR6, TLR7, TLR8, TLR9, TLX1, TM4SF1, TM4SF2, TMC1, TMD, TMIP, TNDM, TNF, TNFRSF11A, TNFRSF1A, TNFRSF6, TNFSF5, TNFSF6, TNFα, INFβ, TNNI3, TNNT2, TOC, TOP2A, TOP1, TP53, TP63, TPA, TPBG, TPI, TPI/m, TPI1, TPM3, TPM1, TPMT, TPO, TPS, TPTA, TRA, TRAG3, TRAPPC2, TRC8, TREH, TRG, TRH, TRIM32, TRIM37, TRP1, TRP2, TRP-2/6b, TRP-2/INT2, Trp-p8, TRPS1, TS, TSC2, TSC3, TSC1, TSG101, TSHB, TSHR, TSP-180, TST, TTGA2B, TTN, TTPA, TTR, TU M2-PK, TULP1, TWIST, TYH, TYR, TYROBP, TYROBP, TYRP1, TYS, UBE2A, UBE3A, UBE1, UCHL1, UFS, UGT1A, ULR, UMPK, UMPS, UOX, UPA, UQCRC1, URO5, UROD, UPK1B, UROS, USH2A, USH3A, USH1A, USH1C, USP9Y, UV24, VBCH, VCF, VDI, VDR, VEGF, VEGFR-2, VEGFR-1, VEGFR-2/FLK-1, VHL, VIM, VMD2, VMD1, VMGLOM, VNEZ, VNF, VP, VRNI, VWF, VWS, WAS, WBS2, WFS2, WFS1, WHCR, WHN, WISP3, WMS, WRN, WS2A, WS2B, WSN, WSS, WT2, WT3, WT1, WTS, WWS, XAGE, XDH, XIC, XIST, XK, XM, XPA, XPC, XRCC9, XS, ZAP70, ZFHX1B, ZFX, ZFY, ZIC2, ZIC3, ZNF145, ZNF261, ZNF35, ZNF41, ZNF6, ZNF198, ZWS1, or from fragments or variants thereof. Preferably, such fragments as well as variants exhibit a sequence homology or identity of about 10 %, about 20 %, about 30 %, about 40 %, about 50 %, about 60 %, about 70 %, about 80 %, or about 90 % with one of the proteins or peptides or protein or peptide sequences as shown or described above. In this context, the definition of fragments and variants similarly applies as defined above for component (A) of the inventive transporter cargo conjugate molecule.

Effector molecules suitable as component (B) of the inventive transporter cargo conjugate molecule may also be selected from protein kinase inhibitors, particularly inhibitors of the protein kinase c-Jun amino terminal kinase, i.e. a JNK inhibitor. Typically, a JNK inhibitor suitable as component (B) of the inventive transporter cargo conjugate molecule may be derived from a human or rat IB1 sequence, preferably from an amino acid sequence as defined or encoded by any of sequences according to SEQ ID NO: 137 (depicts the IB1 cDNA sequence from rat and its predicted amino acid sequence), SEQ ID NO: 138 (depicts the IB1 protein sequence from rat encoded by the exon-intron boundary of the rIB1 gene - splice donor), SEQ ID NO: 139 (depicts the IB1 protein sequence from *Homo sapiens),* or SEQ ID NO: 140 (depicts the IB1 cDNA sequence from *Homo sapiens),* more preferably from an amino acid sequence as defined or encoded by any of sequences according to SEQ ID NO: 139 (depicts the IB1 protein sequence from *Homo sapiens),* or SEQ ID NO: 140 (depicts the IB1 cDNA sequence from *Homo sapiens),* or from any fragments or variants thereof. In this context, the definition of fragments and variants similarly applies as defined above for component (A) of the inventive transporter cargo conjugate molecule.

Preferably, a JNK inhibitor sequence suitable as component (B) of the inventive transporter cargo conjugate molecule comprises a total length of less than 150 amino acid residues, preferably a range of 5 to 150 amino acid residues, more preferably 10 to 100 amino acid residues, even more preferably 10 to 75 amino acid residues and most preferably a range of 10 to 50 amino acid residues, e.g. 10 to 30, 10 to 20, or 10 to 15 amino acid residues. More preferably, such a JNK inhibitor sequence and the above ranges may be selected from any of the herein mentioned JNK inhibitor sequence, even more preferably from an amino acid sequence as defined according to SEQ ID NO: 139 or as encoded by SEQ ID NO: 140, even more preferably in the region between nucleotides 420 and 980 of SEQ ID NO: 140 or amino acids 105 and 291 of SEQ ID NO: 139, and most preferably in the region between nucleotides 561 and 647 of SEQ ID NO: 140 or amino acids 152 and 180 of SEQ ID NO: 139.

According to a particular embodiment, a JNK inhibitor sequence suitable as component (B) of the inventive transporter cargo conjugate molecule typically binds JNK and/or inhibits the activation of at least one JNK activated transcription factor, e.g. c-Jun or ATF2 (see e.g. SEQ ID NOs: 147 and 148, respectively) or Elk1.

Likewise, a JNK inhibitor sequence suitable as component (B) of the inventive transporter cargo conjugate molecule preferably comprises or consists of at least one amino acid sequence according to any one of SEQ ID NOs: 137 to 220, or a fragment, derivative or variant thereof. More preferably, the JNK inhibitor sequence as used herein may contain 1, 2, 3, 4 or even more copies of an amino acid sequence according to SEQ ID NOs: 137 to 220, or a variant, fragment or derivative thereof. If present in more than one copy, these amino acid sequences according to SEQ ID NOs: 137 to 220, or variants, fragments, or derivatives thereof as used herein may be directly linked with each other without any linker sequence or via a linker sequence comprising 1 to 10, preferably 1 to 5 amino acids. Amino acids forming the linker sequence are preferably selected from glycine or proline as amino acid residues. More preferably, these amino acid sequences according to SEQ ID NOs: 137 to 220, or fragments, variants or derivatives thereof, as used herein, may be separated by each other by a hinge of two, three or more proline residues.

The JNK inhibitor sequence suitable as component (B) of the inventive transporter cargo conjugate molecule may be composed of L-amino acids, D-amino acids, or a combination of both. Preferably, the JNK inhibitor sequences as used herein comprise at least 1 or even 2, preferably at least 3, 4 or 5, more preferably at least 6, 7, 8 or 9 and even more preferably at least 10 or more D- and/or L-amino acids, wherein the D- and/or L-amino acids may be arranged in the JNK inhibitor sequences as used herein in a blockwise, a non-blockwise or in an alternate manner.

According to one preferred embodiment the JNK inhibitor sequence suitable as component (B) of the inventive transporter cargo conjugate molecule may be exclusively composed of L-amino acids. The JNK inhibitor sequences as used herein may then comprise or consist of at least one "native JNK inhibitor sequence" according to SEQ ID NO: 141 or 143. In this context, the term "native" or "native JNK inhibitor sequence(s)" is referred to non-altered JNK inhibitor sequences according to any of SEQ ID NOs: 141 or 143, as used herein, entirely composed of L-amino acids.

Accordingly, the JNK inhibitor sequence suitable as component (B) of the inventive transporter cargo conjugate molecule may comprise or consist of at least one (native) amino acid sequence NH₂-Xₙ^{b}-Xₙ^{a}-RPTTLXLXXXXXXXQD-Xₙ^{b}-COOH (L-IB generic (s)) [SEQ ID NO: 143] and/or the JNK binding domain (JBDs) of IB1 XRPTTLXLXXXXXXXQDS/TX (L-IB (generic)) [SEQ ID NO: 151]. In this context, each X typically represents an amino acid residue, preferably selected from any (native) amino acid residue. Xₙ^{a} typically represents one amino acid residue, preferably selected from any amino acid residue except serine or threonine, wherein n (the number of repetitions of X) is 0 or 1. Furthermore, each Xₙ^{b} may be selected from any amino acid residue, wherein n (the number of repetitions of X) is 0-5, 5-10, 10-15, 15-20, 20-30 or more, provided that if n (the number of repetitions of X) is 0 for Xₙ^{a}, Xₙ^{b} does preferably not comprise a serine or threonine at its C-terminus, in order to avoid a serine or threonine at this position. Preferably, Xₙ^{b} represents a contiguous stretch of peptide residues derived from SEQ ID NOs: 141 or 143. Xₙ^{a} and Xₙ^{b} may represent either D or L amino acids. Additionally, the JNK inhibitor sequence as used herein may comprise or consist of at least one (native) amino acid sequence selected from the group comprising the JNK binding domain of IB1 DTYRPKRPTTLNLFPQVPRSQDT (L-IB1) [SEQ ID NO: 149]. More preferably, the JNK inhibitor sequence as used herein further may comprise or consist of at least one (native) amino acid sequence NH₂-RPKRPTTLNLFPQVPRSQD-COOH (L-IB1(s)) [SEQ ID NO: 141]. Furthermore, the JNK inhibitor sequence as used herein may comprise or consist of at least one (native) amino acid sequence selected from the group comprising the JNK binding domain of IB1 L-IB1(s1) (NH₂-TLNLFPQVPRSQD-COOH, SEQ ID NO: 153); LIB1(s2) (NH₂-TTLNLFPQVPRSQ-COOH, SEQ ID NO: 154); L-IB1(s3) (NH₂-PTTLNLFPQVPRS-COOH, SEQ ID NO: 155); L-IB1(s4) (NH₂-RPTTLNLFPQVPR-COOH, SEQ ID NO: 156); L-IB1(s5) (NH₂-KRPTTLNLFPQVP-COOH, SEQ ID NO: 157); L-IB1(s6) (NH₂-PKRPTTLNLFPQV-COOH, SEQ ID NO: 158); L-IB1 (s7) (NH₂-RPKRPTTLNLFPQ-COOH, SEQ ID NO: 159); L-IB1(s8) (NH₂-LNLFPQVPRSQD-COOH, SEQ ID NO: 160); L-IB1(s9) (NH₂-TLNLFPQVPRSQ-COOH, SEQ ID NO: 161); L-IB1(s10) (NH₂-TTLNLFPQVPRS-COOH, SEQ ID NO: 162); L-IB1(s11) (NH₂-PTTLNLFPQVPR-COOH, SEQ ID NO: 163); L-IB1(s12) (NH₂-RPTTLNLFPQVP-COOH, SEQ ID NO: 164); L-IB1(s13) (NH₂-KRPTTLNLFPQV-COOH, SEQ ID NO: 165); L-IB1(s14) (NH₂-PKRPTTLNLFPQ-COOH, SEQ ID NO: 166); L-IB1(s15) (NH₂-RPKRPTTLNLFP-COOH, SEQ ID NO: 167); L-IB1(s16) (NH₂-NLFPQVPRSQD-COOH, SEQ ID NO: 168); L-IB1(s17) (NH₂-LNLFPQVPRSQ-COOH, SEQ ID NO: 169); L-IB1(s18) (NH₂-TLNLFPQVPRS-COOH, SEQ ID NO: 170); L-IB1(s19) (NH₂-TTLNLFPQVPR-COOH, SEQ 1D NO: 171); L-IB1(s20) (NH₂-PTTLNLFPQVP-COOH, SEQ ID NO: 172); L-IB1(s21) (NH₂-RPTTLNLFPQV-COOH, SEQ ID NO: 173); L-IB1(s22) (NH₂-KRPTTLNLFPQ-COOH, SEQ ID NO: 174); L-IB1(s23) (NH₂-PKRPTTLNLFP-COOH, SEQ ID NO: 175); L-IB1(s24) (NH₂-RPKRPTTLNLF-COOH, SEQ ID NO: 176); L-IB1(s25) (NH₂-LFPQVPRSQD-COOH, SEQ ID NO: 177); L-IB1(s26) (NH₂-NLFPQVPRSQ-COOH, SEQ ID NO: 178); L-IB1(s27) (NH₂-LNLFPQVPRS-COOH, SEQ ID NO: 179); L-IB1(s28) (NH₂-TLNLFPQVPR-COOH, SEQ ID NO: 180); L-IB1(s29) (NH₂-TTLNLFPQVP-COOH, SEQ ID NO: 181); L-IB1(s30) (NH₂-PTTLNLFPQV-COOH, SEQ ID NO: 182); L-IB1(s31) (NH₂-RPTTLNLFPQ-COOH, SEQ ID NO: 183); L-IB1(s32) (NH₂-KRPTTLNLFP-COOH, SEQ ID NO: 184); L-IB1(s33) (NH₂-PKRPTTLNLF-COOH, SEQ ID NO: 185); and L-IB1(s34) (NH₂-RPKRPTTLNL-COOH, SEQ ID NO: 186).

Additionally, the JNK inhibitor sequence suitable as component (B) of the inventive transporter cargo conjugate molecule may comprise or consist of at least one (native) amino acid sequence selected from the group comprising the (long) JNK binding domain (JBDs) of IB1 PGTGCGDTYRPKRPTTLNLFPQVPRSQDT (IB1-long) [SEQ ID NO: 145], the (long) JNK binding domain of IB2 IPSPSVEEPHKHRPTTLRLTTLGAQDS (IB2-long) [SEQ ID NO: 146], the JNK binding domain of c-Jun GAYGYSNPKILKQSMTLNLADPVGNLKPH (c-Jun) [SEQ ID NO: 147], the JNK binding domain of ATF2 TNEDHLAVHKHKHEMTLKFGPARNDSVIV (ATF2) [SEQ ID NO: 148]). In this context, an alignment revealed a partially conserved 8 amino acid sequence and a further comparison of the JBDs of IB1 and IB2 revealed two blocks of seven and three amino acids that are highly conserved between the two sequences.

According to another preferred embodiment the JNK inhibitor sequence suitable as component (B) of the inventive transporter cargo conjugate molecule may be composed in part or exclusively of D-amino acids as defined above. More preferably, these JNK inhibitor sequences composed of D-amino acids are non-native D retro-inverso sequences of the above (native) JNK inhibitor sequences. The term "retro-inverso sequences" refers to an isomer of a linear peptide sequence in which the direction of the sequence is reversed and the chirality of each amino acid residue is inverted (see e.g. Jameson et al., Nature, 368,744-746 (1994); Brady et al., Nature, 368, 692-693 (1994)). The advantage of combining D-enantiomers and reverse synthesis is that the positions of carbonyl and amino groups in each amide bond are exchanged, while the position of the side-chain groups at each alpha carbon is preserved. Unless specifically stated otherwise, it is presumed that any given L-amino acid sequence or peptide as used according to the present invention may be converted into an D retro-inverso sequence or peptide by synthesizing a reverse of the sequence or peptide for the corresponding native L-amino acid sequence or peptide.

Accordingly, the JNK inhibitor sequence suitable as component (B) of the inventive transporter cargo conjugate molecule may comprise or consist of at least one D retro-inverso sequence according to the amino acid sequence NH₂-Xₙ^{b}-DQXXXXXXXLXLTTPR-Xₙ^{a}-Xₙ^{b}-COOH (D-IB1 generic (s)) [SEQ ID NO: 144] and/or XS/TDQXXXXXXXLXLTTPRX (D-IB (generic)) [SEQ ID NO: 152]. As used in this context, X, Xₙ^{a} and Xₙ^{b} are as defined above (preferably, representing D amino acids), wherein Xₙ^{b} preferably represents a contiguous stretch of residues derived from SEQ ID NO: 142 or 144. Additionally, the JNK inhibitor sequences as used herein may comprise or consist of at least one D retro-inverso sequence according to the amino acid sequence comprising the JNK binding domain (JBDs) of IB1 TDQSRPVQPFLNLTTPRKPRYTD (D-IB1) [SEQ ID NO: 150]. More preferably, the JNK inhibitor sequences as used herein may comprise or consist of at least one D retro-inverso sequence according to the amino acid sequence NH₂-DQSRPVQPFLNLTTPRKPR-COOH (D-IB1(s)) [SEQ ID NO: 142]. Furthermore, the JNK inhibitor sequences as used herein may comprise or consist of at least one D retro-inverso sequence according to the amino acid sequence comprising the JNK binding domain (JBDs) of IB1 D-IB1(s1) (NH₂-QPFLNLTTPRKPR-COOH, SEQ ID NO: 187); D-IB1(s2) (NH₂-VQPFLNLTTPRKP-COOH, SEQ ID NO: 188); D-IB1(s3) (NH₂-PVQPFLNLTTPRK-COOH, SEQ ID NO: 189); D-IB1(s4) (NH₂-RPVQPFLNLTTPR-COOH, SEQ ID NO: 190); D-IB1(s5) (NH₂-SRPVQPFLNLTTP-COOH, SEQ ID NO: 191); D-IB1(s6) (NH₂-QSRPVQPFLNLTT-COOH, SEQ ID NO: 192); D-IB1(s7) (NH₂-DQSRPVQPFLNLT-COOH, SEQ ID NO: 193); D-IB1(s8) (NH₂-PFLNLTTPRKPR-COOH, SEQ ID NO: 194); D-IB1(s9) (NH₂-QPFLNLTTPRKP-COOH, SEQ ID NO: 195); D-IB1(s10) (NH₂-VQPFLNLTTPRK-COOH, SEQ ID NO: 196); D-IB1(s11) (NH₂-PVQPFLNLTTPR-COOH, SEQ ID NO: 197); D-IB1(s12) (NH₂-RPVQPFLNLTTP-COOH, SEQ ID NO: 198); D-IB1(s13) (NH₂-SRPVQPFLNLTT-COOH, SEQ ID NO: 199); D-IB1(s14) (NH₂-QSRPVQPFLNLT-COOH, SEQ ID NO: 200); D-IB1(s15) (NH₂-DQSRPVQPFLNL-COOH, SEQ ID NO: 201); D-IB1(s16) (NH₂-FLNLTTPRKPR-COOH, SEQ ID NO: 202); D-IB1(s17) (NH₂-PFLNLTTPRKP-COOH, SEQ ID NO: 203); D-IB1(s18) (NH₂-QPFLNLTTPRK-COOH, SEQ ID NO: 204); D-IB1(s19) (NH₂-VQPFLNLTTPR-COOH, SEQ ID NO: 205); D-IB1(s20) (NH₂-PVQPFLNLTTP-COOH, SEQ ID NO: 206); D-IB1(s21) (NH₂-RPVQPFLNLTT-COOH, SEQ ID NO: 207); D-IB1(s22) (NH₂-SRPVQPFLNLT-COOH, SEQ ID NO: 208); D-IB1(s23) (NH₂-QSRPVQPFLNL-COOH, SEQ ID NO: 209); D-IB1(s24) (NH₂-DQSRPVQPFLN-COOH, SEQ ID NO: 210); D-IB1(s25) (NH₂-DQSRPVQPFLCOOH, SEQ ID NO: 211); D-IB1(s26) (NH₂-QSRPVQPFLN-COOH, SEQ ID NO: 212); D-IB1 (s27) (NH₂-SRPVQPFLNL-COOH, SEQ ID NO: 213); D-IB1(s28) (NH₂-RPVQPFLNLT-COOH, SEQ ID NO: 214); D-IB1(s29) (NH₂-PVQPFLNLTT-COOH, SEQ ID NO: 215); D-IB1(s30) (NH₂-VQPFLNLTTP-COOH, SEQ ID NO: 216); D-IB1(s31) (NH₂-QPFLNLTTPR-COOH, SEQ ID NO: 217); D-IB1(s32) (NH₂-PFLNLTTPRK-COOH, SEQ ID NO: 218); D-IB1(s33) (NH₂-FLNLTTPRKP-COOH, SEQ ID NO: 219); and D-IB1(s34) (NH₂-LNLTTPRKPR-COOH, SEQ ID NO: 220).

Exemplary JNK inhibitor sequence suitable as component (B) of the inventive transporter cargo conjugate molecule and are presented in Table 4 (SEQ ID NO:s 141 to 220). The table presents the name of the JNK inhibitor sequences as used herein, as well as their sequence identifier number, their length, and amino acid sequence. Furthermore, Table 4 shows IB1 derived sequences as well as their generic formulas, e.g. for SEQ ID NO's: 141 and 142 and SEQ ID NO's: 143 and 144, respectively. Table 4 furthermore discloses L-IB1 sequences according to SEQ ID NOs: 153 to 186 and D-IB1 sequences SEQ ID NOs: 187 to 220.

**TABLE 4**

| SEQUENCE/PEPTIDE NAME | SEQ ID NO | AA | SEQUENCE |
|---|---|---|---|
| L-IB1(s) | 141 | 19 | RPKRPTTLNLFPQVPRSQD |
| | | | (NH₂-RPKRPTTLNLFPQVPRSQD-COOH) |
| D-IB1(s) | 142 | 19 | DQSRPVQPFLNLTTPRKPR |
| | | | (NH₂-DQSRPVQPFLNLTTPRKPR-COOH) |
| L-IB (generic) (s) | 143 | 19 | NH₂-Xₙ^{b}-Xₙ^{a}-RPTTLXLXXXXXXXQD-Xₙ^{b}-COOH |
| D-IB (generic) (s) | 144 | 19 | NH₂-Xₙ^{b}-DQXXXXXXXLXLTTPR-Xₙ^{a}-Xₙ^{b}-COOH |
| IB1-long | 145 | 29 | PGTGCGDTYRPKRPTTLNLFPQVPRSQDT |
| | | | (NH₂- PGTGCGDTYRPKRPTTLNLFPQVPRSQDT -COOH) |
| IB2-long | 146 | 27 | IPSPSVEEPHKHRPTTLRLTTLGAQDS |
| | | | (NH₂- IPSPSVEEPHKHRPTTLRLTTLGAQDS -COOH) |
| c-Jun | 147 | 29 | GAYGYSNPKILKQSMTLNLADPVGNLKPH |
| | | | (NH₂- GAYGYSNPKILKQSMTLNLADPVGNLKPH -COOH) |
| ATF2 | 148 | 29 | TNEDHLAVHKHKHEMTLKFGPARNDSVIV |
| | | | (NH₂- TNEDHLAVHKHKHEMTLKFGPARNDSVIV -COOH) |
| L-IB1 | 149 | 23 | DTYRPKRPTTLNLFPQVPRSQDT |
| | | | (NH₂- DTYRPKRPTTLNLFPQVPRSQDT -COOH) |
| D-I81 | 150 | 23 | TDQSRPVQPFLNLTTPRKPRYTD |
| | | | (NH₂- TDQSRPVQPFLNLTTPRKPRYTD -COOH) |
| L-IB (generic) | 151 | 19 | XRPTTLXLXXXXXXXQDS/TX |
| | | | (NH₂- XRPTTLXLXXXXXXXQDS/TX -COOH) |
| D-IB (generic) | 152 | 19 | XS/TDQXXXXXXXLXLTTPRX |
| | | | (NH₂- XS/TDQXXXXXXXLXLTTPRX -COOH) |
| L-IB1(s1) | 153 | 13 | TLNLFPQVPRSQD |
| | | | (NH₂-TLNLFPQVPRSQD-COOH) |
| L-IB1(s2) | 154 | 13 | TTLNLFPQVPRSQ |
| | | | (NH₂-TTLNLFPQVPRSQ-COOH) |
| L-IB1(s3) | 155 | 13 | PTTLNLFPQVPRS |
| | | | (NH₂-PTTLNLFPQVPRS-COOH) |
| L-IB1(s4) | 156 | 13 | RPTTLNLFPQVPR |
| | | | (NH₂-RPTTLNLFPQVPR-COOH) |
| L-IB1(s5) | 157 | 13 | KRPTTLNLFPQVP |
| | | | (N H₂-KRPTTLNLFPQVP-COOH) |
| L-IB1 (s6) | 158 | 13 | PKRPTTLNLFPQV |
| | | | (NH₂-PKRPTTLNLFPQV-COOH) |
| L-IB1 (s7) | 159 | 13 | RPKRPTTLNLFPQ |
| | | | (NH₂-RPKRPTTLNLFPQ-COOH) |
| L-IB1 (s8) | 160 | 12 | LNLFPQVPRSQD |
| | | | (NH₂-LNLFPQVPRSQD-COOH) |
| L-IB1 (s9) | 161 | 12 | TLNLFPQVPRSQ |
| | | | (NH₂-TLNLFPQVPRSQ-COOH) |
| L-IB1 (s10) | 162 | 12 | TTLNLFPQVPRS |
| | | | (NH₂-TTLNLFPQVPRS-COOH) |
| L-1B1(s11) | 163 | 12 | PTTLNLFPQVPR |
| | | | (NH₂-PTTLNLFPQVPR-COOH) |
| L-IB1(s12) | 164 | 12 | RPTTLNLFPQVP |
| | | | (NH₂-RPTTLNLFPQVP-COOH) |
| L-IB1(s13) | 165 | 12 | KRPTTLNLFPQV |
| | | | (NH₂-KRPTTLNLFPQV-COOH) |
| L-IB1(s14) | 166 | 12 | PKRPTTLNLFPQ |
| | | | (NH₂-PKRPTTLNLFPQ-COOH) |
| L-IB1(s15) | 167 | 12 | RPKRPTTLNLFP |
| | | | (NH₂-RPKRPTTLNLFP-COOH) |
| L-IB1 (s16) | 168 | 11 | NLFPQVPRSQD |
| | | | (NH₂-NLFPQVPRSQD-COOH) |
| L-IB1 (s17) | 169 | 11 | LNLFPQVPRSQ |
| | | | (NH₂-LNLFPQVPRSQ-COOH) |
| L-IB1 (s18) | 170 | 11 | TLNLFPQVPRS |
| | | | (NH₂-TLNLFPQVPRS-COOH) |
| L-IB1 (s19) | 171 | 11 | TTLNLFPQVPR |
| | | | (NH₂-TTLNLFPQVPR-COOH) |
| L-181 (s20) | 172 | 11 | PTTLNLFPQVP |
| | | | (NH₂-PTTLNLFPQVP-COOH) |
| L-IB1 (s21) | 173 | 11 | RPTTLNLFPQV |
| | | | (NH₂-RPTTLNLFPQV-COOH) |
| L-IB1(s22) | 174 | 11 | KRPTTLNLFPQ |
| | | | (NH₂-KRPTTLNLFPQ-COOH) |
| L-IB1 (s23) | 175 | 11 | PKRPTTLNLFP |
| | | | (NH₂-PKRPTTLNLFP-COOH) |
| L-1B1 (s24) | 176 | 11 | RPKRPTTLNLF |
| | | | (NH₂-RPKRPTTLNLF-COOH) |
| L-1B1 (s25) | 177 | 10 | LFPQVPRSQD |
| | | | (NH₂-LFPQVPRSQD-COOH) |
| L-1B1 (s26) | 178 | 10 | NLFPQVPRSQ |
| | | | (NH₂-NLFPQVPRSQ-COOH) |
| L-IB1 (s27) | 179 | 10 | LNLFPQVPRS |
| | | | (NH₂-LNLFPQVPRS-COOH) |
| L-IB1 (s28) | 180 | 10 | TLNLFPQVPR |
| | | | (N H₂-TLNLFPQVPR-COOH) |
| L-IB1 (s29) | 181 | 10 | TTLNLFPQVP |
| | | | (NH₂-TTLNLFPQVP-COOH) |
| L-IB1 (s30) | 182 | 10 | PTTLNLFPQV |
| | | | (NH₂-PTTLNLFPQV-COOH) |
| L-IB1 (s31) | 183 | 10 | RPTTLNLFPQ |
| | | | (NH₂-RPTTLNLFPQ-COOH) |
| L-I81 (s32) | 184 | 10 | KRPTTLNLFP |
| | | | (NH₂-KRPTTLNLFP-COOH) |
| L-IB1 (s33) | 185 | 10 | PKRPTTLNLF |
| | | | (NH₂-PKRPTTLNLF-COOH) |
| L-IB1 (s34) | 186 | 10 | RPKRPTTLNL |
| | | | (NH₂-RPKRPTTLNL-COOH) |
| D-IB1 (s1) | 187 | 13 | QPFLNLTTPRKPR |
| | | | (NH₂-QPFLNLTTPRKPR-COOH) |
| D-IB1 (s2) | 188 | 13 | VQPFLNLTTPRKP |
| | | | (NH₂-VQPFLNLTTPRKP-COOH) |
| D-I81 (s3) | 189 | 13 | PVQPFLNLTTPRK |
| | | | (NH₂-PVQPFLNLTTPRK-COOH) |
| D-IB1 (s4) | 190 | 13 | RPVQPFLNLTTPR |
| | | | (NH₂-RPVQPFLNLTTPR-COOH) |
| D-IB1 (s5) | 191 | 13 | SRPVQPFLNLTTP |
| | | | (NH₂-SRPVQPFLNLTTP-COOH) |
| D-IB1 (s6) | 192 | 13 | QSRPVQPFLNLTT |
| | | | (NH₂-QSRPVQPFLNLTT-COOH) |
| D-IB1 (s7) | 193 | 13 | DQSRPVQPFLNLT |
| | | | (NH₂-DQSRPVQPFLNLT-COOH) |
| D-IB1 (s8) | 194 | 12 | PFLNLTTPRKPR |
| | | | (NH₂-PFLNLTTPRKPR-COOH) |
| D-IB1 (s9) | 195 | 12 | QPFLNLTTPRKP |
| | | | (NH₂-QPFLNLTTPRKP-COOH) |
| D-IB1 (s10) | 196 | 12 | VQPFLNLTTPRK |
| | | | (NH₂-VQPFLNLTTPRK-COOH) |
| D-I81 (s11) | 197 | 12 | PVQPFLNLTTPR |
| | | | (NH₂-PVQPFLNLTTPR-COOH) |
| D-IB1 (s12) | 198 | 12 | RPVQPFLNLTTP |
| | | | (NH₂-RPVQPFLNLTTP-COOH) |
| D-IB1 (s13) | 199 | 12 | SRPVQPFLNLTT |
| | | | (NH₂-SRPVQPFLNLTT-COOH) |
| D-IB1 (s14) | 200 | 12 | QSRPVQPFLNLT |
| | | | (NH₂-QSRPVQPFLNLT-COOH) |
| D-IB1 (s15) | 201 | 12 | DQSRPVQPFLNL |
| | | | (NH₂-DQSRPVQPFLNL-COOH) |
| D-IB1 (s16) | 202 | 11 | FLNLTTPRKPR |
| | | | (NH₂-FLNLTTPRKPR-COOH) |
| D-IB1 (s17) | 203 | 11 | PFLNLTTPRKP |
| | | | (N H₂-PFLNLTTPRKP-COOH) |
| D-IB1 (s18) | 204 | 11 | QPFLNLTTPRK |
| | | | (NH₂-QPFLNLTTPRK-COOH) |
| D-IB1 (s19) | 205 | 11 | VQPFLNLTTPR |
| | | | (NH₂-VQPFLNLTTPR-COOH) |
| D-IB1 (s20) | 206 | 11 | PVQPFLNLTTP |
| | | | (NH₂-PVQPFLNLTTP-COOH) |
| D-IB1 (s21) | 207 | 11 | RPVQPFLNLTT |
| | | | (NH₂-RPVQPFLNLTT-COOH) |
| D-IB1 (s22) | 208 | 11 | SRPVQPFLNLT |
| | | | (NH₂-SRPVQPFLNLT-COOH) |
| D-IB1 (s23) | 209 | 11 | QSRPVQPFLNL |
| | | | (NH₂-QSRPVQPFLNL-COOH) |
| D-IB1 (s24) | 210 | 11 | DQSRPVQPFLN |
| | | | (NH₂-DQSRPVQPFLN-COOH) |
| D-IB1 (s25) | 211 | 10 | DQSRPVQPFL |
| | | | (NH₂-DQSRPVQPFL-COOH) |
| D-IB1 (s26) | 212 | 10 | QSRPVQPFLN |
| | | | (NH₂-QSRPVQPFLN-COOH) |
| D-I81 (s27) | 213 | 10 | SRPVQPFLNL |
| | | | (NH₂-SRPVQPFLNL-COOH) |
| D-IB1 (s28) | 214 | 10 | RPVQPFLNLT |
| | | | (NH₂-RPVQPFLNLT-COOH) |
| D-IB1 (s29) | 215 | 10 | PVQPFLNLTT |
| | | | (NH₂-PVQPFLNLTT-COOH) |
| D-IB1 (s30) | 216 | 10 | VQPFLNLTTP |
| | | | (NH₂-VQPFLNLTTP-COOH) |
| D-IB1 (s31) | 217 | 10 | QPFLNLTTPR |
| | | | (NH₂-QPFLNLTTPR-COOH) |
| D-IB1 (s32) | 218 | 10 | PFLNLTTPRK |
| | | | (NH₂-PFLNLTTPRK-COOH) |
| D-IB1 (s33) | 219 | 10 | FLNLTTPRKP |
| | | | (NH₂-FLNLTTPRKP-COOH) |
| D-IB1 (s34) | 220 | 10 | LNLTTPRKPR |
| | | | (NH₂-LNLTTPRKPR-COOH) |

The JNK inhibitor sequences suitable as component (B) of the inventive transporter cargo conjugate molecule may furthermore comprises or consists of at least one variant, fragment and/or derivative of the above defined native or non-native amino acid sequences according to SEQ ID NOs: 141 to 220. Preferably, these variants, fragments and/or derivatives retain biological activity of the above disclosed native or non-native JNK inhibitor sequences as used herein, particularly of native or non-native amino acid sequences according to SEQ ID NOs: 141 to 220, i.e. binding JNK and/or inhibiting the activation of at least one JNK activated transcription factor, e.g. c-Jun, ATF2 or Elk1. Functionality may be tested by various tests, e.g. binding tests of the peptide to its target molecule or by biophysical methods, e.g. spectroscopy, computer modeling, structural analysis, etc. Particularly, an JNK inhibitor sequence or variants, fragments and/or derivatives thereof as defined above may be analyzed by hydrophilicity analysis (see e.g. Hopp and Woods, 1981. Proc Natl Acad Sci USA 78: 3824-3828) that can be utilized to identify the hydrophobic and hydrophilic regions of the peptides, thus aiding in the design of substrates for experimental manipulation, such as in binding experiments, or for antibody synthesis. Secondary structural analysis may also be performed to identify regions of an JNK inhibitor sequence or of variants, fragments and/or derivatives thereof as used herein that assume specific structural motifs (see e.g. Chou and Fasman, 1974, Biochem 13: 222-223). Manipulation, translation, secondary structure prediction, hydrophilicity and hydrophobicity profiles, open reading frame prediction and plotting, and determination of sequence homologies can be accomplished using computer software programs available in the art. Other methods of structural analysis include, e.g. X-ray crystallography (see e.g. Engstrom, 1974. Biochem Exp Biol 11: 7-13), mass spectroscopy and gas chromatography (see e.g. METHODS IN PROTEIN SCIENCE, 1997, J. Wiley and Sons, New York, NY) and computer modeling (see e.g. Fletterick and Zoller, eds., 1986. Computer Graphics and Molecular Modeling, In: CURRENT COMMUNICATIONS IN MOLECULAR BIOLOGY, Cold Spring Harbor Laboratory Press, Cold Spring Harbor, NY) may also be employed.

Accordingly, the JNK inhibitor sequence suitable as component (B) of the inventive transporter cargo conjugate molecule may comprise or consist of at least one variant of (native or non-native) amino acid sequences according to SEQ ID NOs: 141 to 220. In the context of the present invention, a "variant of a (native or non-native) amino acid sequence according to SEQ ID NOs: 141 to 220" is preferably a sequence derived from any of the sequences according to SEQ ID NOs: 141 to 220, wherein the variant comprises amino acid alterations of the amino acid sequences according to SEQ ID NOs: 141 to 220. Such alterations typically comprise 1 to 20, preferably 1 to 10 and more preferably 1 to 5 substitutions, additions and/or deletions of amino acids according to SEQ ID NOs: 141 to 220, wherein the variant exhibits a sequence identity with any of the sequences according to SEQ ID NOs: 141 to 220 of at least about 30%, 50%, 70%, 80%, 90%, 95%, 98% or even 99%. If variants of (native or non-native) amino acid sequences according to SEQ ID NOs: 141 to 220 as defined above and used herein are obtained by substitution of specific amino acids, such substitutions preferably comprise conservative amino acid substitutions as already defined above.

Effector molecules suitable as component (B) of the inventive transporter cargo conjugate molecule may furthermore be selected from antigens, preferably protein and peptide antigens, e.g. tumor antigens, allergy antigens, auto-immune self-antigens, pathogenic antigens, and antigens from viruses, preferably from cytomegalovirus (CMV), orthopox variola virus, orthopox alastrim virus, parapox ovis virus, molluscum contagiosum virus, herpes simplex virus 1, herpes simplex virus 2, herpes B virus, varicella zoster virus, pseudorabies virus, human cytomegaly virus, human herpes virus 6, human herpes virus 7, Epstein-Barr virus, human herpes virus 8, hepatitis B virus, chikungunya virus, O'nyong'nyong virus, rubivirus, hepatitis C virus, GB virus C, West Nile virus, dengue virus, yellow fever virus, louping ill virus, St. Louis encephalitis virus, Japan B encephalitis virus, Powassan virus, FSME virus, SARS, SARS-associated corona virus, human corona virus 229E, human corona virus Oc43, Torovirus, human T cell lymphotropic virus type I, human T cell lymphotropic virus type II, HIV (AIDS), i.e. human immunodeficiency virus type 1 or human immunodeficiency virus type 2, influenza virus, Lassa virus, lymphocytic choriomeningitis virus, Tacaribe virus, Junin virus, Machupo virus, Borna disease virus, Bunyamwera virus, California encephalitis virus, Rift Valley fever virus, sand fly fever virus, Toscana virus, Crimean-Congo haemorrhagic fever virus, Hazara virus, Khasan virus, Hantaan virus, Seoul virus, Prospect Hill virus, Puumala virus, Dobrava Belgrade virus, Tula virus, sin nombre virus, Lake Victoria Marburg virus, Zaire Ebola virus, Sudan Ebola virus, Ivory Coast Ebola virus, influenza virus A, influenza virus B, influenza viruses C, parainfluenza virus, malaria virus, Marburg virus, measles virus, mumps virus, respiratory syncytial virus, human metapneumovirus, vesicular stomatitis Indiana virus, rabies virus, Mokola virus, Duvenhage virus, European bat lyssavirus 1 + 2, Australian bat lyssavirus, adenoviruses A-F, human papilloma viruses, condyloma virus 6, condyloma virus 11, polyoma viruses, adeno-associated virus 2, rotaviruses, orbiviruses, Varicella including Varizella zoster, etc., or antigens from leishmania, typanosomes, amibes, bacteria, etc., or may be selected from variants of the above antigens. Preferably, fragments as well as variants of antigens as defined above exhibit a sequence homology or identity of about 10 %, about 20 %, about 30 %, about 40 %, about 50 %, about 60 %, about 70 %, about 80 %, or about 90 % with one of the antigens or antigen sequences as shown or described above. In this context, the definition of fragments and variants similarly applies as defined above for component (A) of the inventive transporter cargo conjugate molecule. Furthermore, epitopes (also called "antigen determinants") of antigens or antigenic fragments as defined above are described herein, epitopes in the context of the present invention are typically fragments located on the outer surface of (native) protein or peptide antigens as defined herein, preferably having 5 to 15 amino acids, more preferably having 5 to 12 amino acids, even more preferably having 6 to 9 amino acids, which may be recognized by antibodies, i.e. in their native form.

Furthermore, effector molecules suitable as component (B) of the inventive transporter cargo conjugate molecule may be selected from antibodies. According to the present invention, such an antibody may be selected from any antibody, e.g. any recombinantly produced or naturally occurring antibodies, known in the art, in particular antibodies suitable for therapeutic, diagnostic or scientific purposes, or antibodies which have been identified in relation to specific cancer diseases. Herein, the term "antibody" is used in its broadest sense and specifically covers monoclonal and polyclonal antibodies (including agonist, antagonist, and blocking or neutralizing antibodies) and antibody species with polyepitopic specificity. According to the invention, "antibody" typically comprises any antibody known in the art (e.g. IgM, IgD, IgG, IgA and IgE antibodies), such as naturally occurring antibodies, antibodies generated by immunization in a host organism, antibodies which were isolated and identified from naturally occurring antibodies or antibodies generated by immunization in a host organism and recombinantly produced by biomolecular methods known in the art, as well as chimeric antibodies, human antibodies, humanized antibodies, bispecific antibodies, intrabodies, i.e. antibodies expressed in cells and optionally localized in specific cell compartments, and fragments and variants of the aforementioned antibodies. In general, an antibody consists of a light chain and a heavy chain both having variable and constant domains. The light chain consists of an N-terminal variable domain, V_{L}, and a C-terminal constant domain, C_{L}. In contrast, the heavy chain of the IgG antibody, for example, is comprised of an N-terminal variable domain, V_{H}, and three constant domains, C_{H}1, C_{H}2 und C_{H}3. Antibodies in this context also comprise fragments and variants of antibodies as described above, e.g. an F_{ab} fragment, an F_{c} fragment, etc. Preferably, such fragments as well as variants exhibit a sequence homology or identity of about 10 %, about 20 %, about 30 %, about 40 %, about 50 %, about 60 %, about 70 %, about 80 %, or about 90 % with one of the antibodies as described above. In this context, the definition of fragments and variants similarly applies as defined above for component (A) of the inventive transporter cargo conjugate molecule..

Additionally, effector molecules suitable as component (B) of the inventive transporter cargo conjugate molecule may be selected from apoptotic factors including AIF, Apaf e.g. Apaf-1, Apaf-2, Apaf-3, oder APO-2 (L), APO-3 (L), Apopain, Bad, Bak, Bax, Bcl-2, Bcl-x_{L}, Bcl-xₛ, bik, Bok, CAD, Calpain, Caspase e.g. Caspase-1, Caspase-2, Caspase-3, Caspase-4, Caspase-5, Caspase-6, Caspase-7, Caspase-8, Caspase-9, Caspase-10, Caspase-11, ced-3, ced-9, c-Jun, c-Myc, crm A, cytochrom C, CdR1, DcR1, DD, DED, DISC, DNA-PKcs, DR3, DR4, DR5, FADD/MORT-1, FAK, Fas (Fas-ligand CD95/fas (receptor)), FLICE/MACH, FLIP, fodrin, fos, G-Actin, Gas-2, gelsolin, granzyme A/B, ICAD, ICE, JNK, lamin A/B, MAP, Max, MCL-1, Mdm-2, MEKK-1, MORT-1, Myd88, NEDD, NF-ₖₐₚₚₐB, NuMa, p38, p53, PAK-2, PARP, perforin, PITSLRE, PKCdelta, pRb, presenilin, prICE, RAIDD, Ras, RIP, sphingomyelinase, thymidinkinase from herpes simplex, TRADD, TRAF2, TRAIL-R1, TRAIL-R2, TRAIL-R3, transglutaminase, etc., or from fragments or variants thereof, or from components of the wnt-signalling pathway, such as β-catenine, or the ICF-family, pololike kinases, CiP2A, PP2A, etc., or from fragments or variants thereof. Preferably, such fragments as well as variants exhibit a sequence homology or identity of about 10 %, about 20 %, about 30 %, about 40 %, about 50 %, about 60 %, about 70 %, about 80 %, or about 90 % with one of the sequences as shown or described above. In this context, the definition of fragments and variants similarly applies as defined above for component (A) of the inventive transporter cargo conjugate molecule.

Effector molecules suitable as component (B) of the inventive transporter cargo conjugate molecule may furthermore be selected from at least one or more partial or full-length BH3-domain and/or at least one partial or full-length BH3-only protein. In this context, BH3-only proteins are preferably defined as members of the Bcl-2 family representing regulators of apoptosis by interacting with other members of Bcl-2 family. In the context of the present invention component (B) of the inventive transporter cargo conjugate molecule may thus be selected from an amino acid sequence comprising at least one or more partial or full-length BH3-domain sequence(s) of a BH3-only protein or a partial or full-length BH3-only protein (defined as a subclass of the Bcl-2 family proteins), which is (are) capable of inducing apoptosis by either interacting with at least one Bcl-2 family protein or by activating or sensitising at least one pro-apoptotic member of the Bcl-2 family. Their functional activity can be tested by suitable assay methods, e.g. by binding assays or by assaying its pro-apoptotic activity by apoptosis assays. Preferably, an amino acid sequence used as component (B) of the inventive transporter cargo conjugate molecule may comprise or consist of at least one partial or full-length BH3-domain sequence and/or at least one partial or full-length BH3-only protein sequence selected from the group consisting of Bid, Bad, Noxa, Puma, Bim, Bik, Bmf, DP5 / Hrk and Bok. Alternatively, an amino acid sequence used as component (B) of the inventive transporter cargo conjugate molecule may comprise or consist of a combination of at least one partial or full-length BH3-domain sequence and/or at least one partial or full-length BH3-only protein sequence, the combinations preferably selected from the group consisting of e.g. Bid and Bad, Bim and Bad, Bik and Bad, Puma and Bad, Noxa and Bad, Bmf and Bad, DP5 / Hrk and Bad, Bok and Bad, Bik and Bim, Bik and Bid, Bik and Puma, Bik and Noxa, Bik and Bmf, Bik and DP5 / Hrk, Bik and Bok, Bid and Puma, Bid and Noxa, Bid and Bim, Bid and Bmf, Bid and DP5 / Hrk, Bid and Bok, Bim and Noxa, Bim and Puma, Bim and Bmf, Bim and DP5 / Hrk, Bim and Bok, Puma and Noxa, Puma and Bmf, Puma and DP5 / Hrk, Puma and Bok, Noxa and Bmf, Noxa and DP5 / Hrk and Noxa and Bok. The (full-length or partial) BH3-sequences or BH3-only protein sequences defined above may be selected from e.g. any mammalian BH3-only protein, in particular from the human isoforms. Accordingly, component (B) of the inventive transporter cargo conjugate molecule may comprise or consist of at least one partial or full-length BH3-domain sequence and/or at least one BH3-only protein sequence as defined by any of SEQ ID NOs: 221 to 237 (see Table 5). Preferably, an amino acid sequence used as component (B) of the inventive transporter cargo conjugate molecule may further comprise or consist of at least one fragment or variant of at least one partial or full-length BH3-domain sequence and/or at least one BH3-only protein sequence as defined by any of SEQ ID NOs: 221 to 237. Such fragments as well as variants preferably have a sequence length of less than 50, preferably of less than 40 and even more preferably of less than 30 amino acids, or exhibit a sequence homology or identity of about 10 %, about 20 %, about 30 %, about 40 %, about 50 %, about 60 %, about 70 %, about 80 %, or about 90 % with one of the sequences described above or as shown in any of SEQ ID NOs: 221 to 237. In this context, the definition of fragments and variants similarly applies as defined above for component (A) of the inventive transporter cargo conjugate molecule. Furthermore, fragments or variants of the native sequences typically comprise a BH3-domain sequence or at least partially comprise a BH3-domain sequence (at least 7 amino acids of the BH3-domain sequence).

**TABLE 5**

| SEQUENCE/ PEPTIDE NAME | SEQ ID NO | AA | SEQUENCE |
|---|---|---|---|
| Bid (human) (transcript variant 1) | 221 | 241 | |
| | | | |
| Bad (human) | 222 | 168 | |
| Noxa1 (human) | 223 | 483 | |
| Puma (human) | 224 | 193 | |
| Bim (human) (transcript variant 1) | 225 | 198 | |
| Bik (human) | 226 | 160 | |
| BH3-domain of Bik (Bik BH3) | 227 | 18 | ALALRLACIG DEMDVSLR |
| BH3-domain of Bad (Bad BH3) | 228 | 18 | RYGRELRRMS DEFVDSFK |
| BH3-domain of Bid (Bid BH3) | 229 | 18 | NIARHLAQVG DSMDRSIP |
| BH3-domain of Bmf (Bmf BH3) | 230 | 18 | QIARKLQCIA DQFHRLHV |
| BH3-domain of DP5/Hrk (DP5Hrk BH3) | 231 | 18 | LTAARLKAIG DELHQRTM |
| BH3-domain of Bim (Bim BH3) | 232 | 18 | WIAQELRRIG DEFNAYYA |
| BH3-domain of Noxa (Noxa BH3) | 233 | 18 | ECATQLRRFG DKLNFRQK |
| BH3-domain of PUMA (PUMA BH3) | 234 | 18 | EIGAQLRRMA DDLNAQYE |
| BH3-domain of Bax (Bax BH3) | 235 | 18 | KLSECLKRIG DELDSNME |
| BH3-domain of Bak (Bak BH3) | 236 | 18 | QVGRQLAIIG DDINRRYD |
| BH3-domain of Bok (Bok BH3) | 237 | 18 | EVCTVLLRLG DELEQIRP |

The proteins or peptide sequences as described above, e.g. of therapeutically active proteins, antigens, antibodies, apoptotic factors, proteases implicated in pathological states, preferably peptidic protease inhibitors, BH3 domains, etc., which are used as effector molecules for component (B) of the inventive transporter cargo conjugate molecule, may be provided in as a protein or peptide sequence either in the native form composed of L-amino acids or in the retro-inverso D-form (entirely) composed of D amino acids, which means that these sequences have to be inverted by reverting the termini: native C-terminus is the N-terminus of the inverted form and the native N-terminus is the C-Terminus of the inverted form). Alternatively, these proteins or peptide sequences as described above, may provide their protein or peptide sequence as a mixture of L-amino acids and D-amino acids.

Effector molecules suitable as component (B) of the inventive transporter cargo conjugate molecule may additionally be selected from nucleic acids encoding the above defined proteins or peptides, such as therapeutically active proteins and peptides, antigens, antibodies, apoptotic factors, proteases implicated in pathological states, preferably peptidic protease inhibitors, BH3-domains or partial or full-length BH3-only proteins or their variants of fragments. In this context, nucleic acids preferably comprise single stranded, double stranded or partially double stranded nucleic acids, preferably selected from genomic DNA, cDNA, RNA, siRNA, antisense DNA, antisense RNA, ribozyme, complimentary RNA/DNA sequences with or without expression elements, a mini-gene, gene fragments, regulatory elements, promoters, and combinations thereof.

As a further particular example, effector molecules suitable as component (B) of the inventive transporter cargo conjugate molecule may be selected from siRNAs. In this context, an siRNAs is of interest particularly in connection with the phenomenon of RNA interference. Attention was drawn to the phenomenon of RNA interference in the course of immunological research. In recent years, a RNA-based defence mechanism has been discovered, which occurs both in the kingdom of the fungi and in the plant and animal kingdom and acts as an "immune system of the genome". The system was originally described in various species independently of one another, first in *C. elegans,* before it was possible to identify the underlying mechanisms of the processes as being identical: RNA-mediated virus resistance in plants, PTGS (posttranscriptional gene silencing) in plants, and RNA interference in eukaryotes are accordingly based on a common procedure. The *in vitro* technique of RNA interference (RNAi) is based on double-stranded RNA molecules (dsRNA), which trigger the sequence-specific suppression of gene expression (Zamore (2001) Nat. Struct. Biol. 9: 746-750; Sharp (2001) Genes Dev. 5:485-490: Hannon (2002) Nature 41: 244-251). In the transfection of mammalian cells with long dsRNA, the activation of protein kinase R and RnaseL brings about unspecific effects, such as, for example, an interferon response (Stark et al. (1998) Annu. Rev. Biochem. 67: 227-264; He and Katze (2002) Viral Immunol. 15: 95-119). These unspecific effects are avoided when shorter, for example 21- to 23-mer, so-called siRNA (small interfering RNA), is used, because unspecific effects are not triggered by siRNA that is shorter than 30 bp (Elbashir et al. (2001) Nature 411: 494-498). Recently, dsRNA molecules have also been used *in vivo*(McCaffrey et al. (2002), Nature 418: 38-39; Xia et al. (2002), Nature Biotech. 20: 1006-1010; Brummelkamp et al. (2002), Cancer Cell 2: 243-247). Thus, an siRNA used as an effector molecule suitable as component (B) of the inventive transporter cargo conjugate molecule typically comprises a (single- or) double stranded, preferably a double-stranded, RNA sequence with about 8 to 30 nucleotides, preferably 1 7 to 25 nucleotides, even more preferably from 20 to 25 and most preferably from 21 to 23 nucleotides. In principle, all the sections having a length of from 17 to 29, preferably from 19 to 25, most preferably from 21 to 23 base pairs that occur in the coding region of a protein (sequence) as mentioned above, can serve as target sequence for a siRNA. Equally, siRNAs can also be directed against nucleotide sequences of a protein (sequence) described hereinbefore that do not lie in the coding region, in particular in the 5' non-coding region of the RNA, for example, therefore, against non-coding regions of the RNA having a regulatory function. The target sequence of the siRNA can therefore lie in the translated and/or untranslated region of the RNA and/or in the region of the control elements. The target sequence of a siRNA can also lie in the overlapping region of untranslated and translated sequence; in particular, the target sequence can comprise at least one nucleotide upstream of the start triplet of the coding region.

As another particular example, effector molecules suitable as component (B) of the inventive transporter cargo conjugate molecule may be selected from antisense RNA. In this context, an antisense RNA is preferably a (single-stranded) RNA molecule transcribed on the basis of the coding, rather than the template, strand of (genomic) DNA, so that it is complementary to the sense (messenger) RNA. An antisense RNA suitable as component (B) of the inventive transporter cargo conjugate molecule typically forms a duplex between the sense and antisense RNA molecules and is thus capable to block translation of the corresponding mRNA. An antisense RNA as used herein can be directed against any portion of an mRNA sequence, e.g. derived from genomic DNA and/or which may encode any protein, e.g. a protein peptide as defined herein such as therapeutically active proteins and peptides, antigens, antibodies, apoptotic factors, proteases implicated in pathological states, preferably peptidic protease inhibitors, BH3-domains or partial or full-length BH3-only proteins or their variants of fragments as described hereinbefore, if thereby translation of the encoded protein or peptide is reduced/suppressed. Accordingly, the target sequence of the antisense RNA on the targeted mRNA (or the targeted (genomic) DNA) may be located in the translated and/or untranslated region of the mRNA (or the targeted (genomic) DNA), e.g. in the region of the control elements, in particular in the 5' non-coding region of the mRNA (or the targeted (genomic) DNA) exerting a regulatory function. The target sequence of an antisense RNA on the targeted mRNA (or the targeted (genomic) DNA) may also be constructed such that the antisense RNA binds to the mRNA (or the targeted (genomic) DNA) by covering with its sequence a region which is partially complementary to the untranslated and to translated (coding) sequence of the targeted mRNA (or the targeted (genomic) DNA); in particular, the antisense RNA may be complementary to the target mRNA (or the targeted (genomic) DNA) sequence by at least one nucleotide upstream of the start triplet of the coding region of the targeted mRNA. Preferably, the antisense RNA as used herein comprises a length of about 5 to about 5000, of about 500 to about 5000, and, more preferably, of about 1000 to about 5000 or, alternatively, of about 5 to about 1000, about 5 to about 500, about 5 to about 250, of about 5 to about 100, of about 5 to about 50 or of about 5 to about 30 nucleotides, or, alternatively, and even more preferably a length of about 20 to about 100, of about 20 to about 80, or of about 20 to about 60 nucleotides.

As a further particular example, effector molecules suitable as component (B) of the inventive transporter cargo conjugate molecule may additionally be selected from a cytotoxic or anti-tumor drug, suitable as a chemotherapy drug. In general, chemotherapy drugs suitable for component (B) of the inventive transporter cargo conjugate molecule can be divided into three main categories based on their mechanism of action. They may (a) stop the synthesis of preDNA molecule building blocks: These agents work in a number of different ways. DNA building blocks are folic acid, heterocyclic bases, and nucleotides, which are made naturally within cells. All of these agents work to block some step in the formation of nucleotides or deoxyribonucleotides (necessary for making DNA). When these steps are blocked, the nucleotides, which are the building blocks of DNA and RNA, cannot be synthesized. Thus the cells cannot replicate because they cannot make DNA without the nucleotides. Examples of drugs in this class include methotrexate (Abitrexate^{®}), fluorouracil (Adrucil^{®}), hydroxyurea (Hydrea^{®}), and mercaptopurine (Purinethol^{®}), thioguanine, tocoferol, or, more generally, also any nucleotide analogue, e.g. 2'-deoxycytidine analogues. Alternatively, chemotherapy drugs may (b) directly damage the DNA in the nucleus of the cell. These agents chemically damage DNA and RNA. They disrupt replication of the DNA and either totally halt replication or cause the manufacture of nonsense DNA or RNA (i.e. the new DNA or RNA does not code for anything useful). Examples of drugs in this class include cisplatin (Platinol^{®}) and antibiotics - daunorubicin (Cerubidine^{®}), doxorubicin (Adriamycin^{®}) belonging to the class of anthracycline antitumor agents (the members of which may be used as component (B) of the inventive transporter cargo conjugate molecule), and etoposide (VePesid^{®}) or any intercalator. Finally, chemotherapy drugs may (c) effect the synthesis or breakdown of the mitotic spindles: Mitotic spindles serve as molecular railroads with "North and South Poles" in the cell when a cell starts to divide itself into two new cells. These spindles are very important because they help to split the newly copied DNA such that a copy goes to each of the two new cells during cell division. These drugs disrupt the formation of these spindles and therefore interrupt cell division. Examples of drugs in this class of mitotic disrupters include: Vinblastine (Velban^{®}), Vincristine (Oncovin^{®}) and Paclitaxel (Taxol^{®}). Component (B) of the inventive transporter cargo conjugate molecule may act according to one of the above modes of action. In other terms, each of the classes of anti-tumor drugs, i.e. alkylating agents, nitrosoureas, antimetabolites, plant alkaloids, antitumor antibiotics, and steroid hormones may be used as component (B) of the inventive transporter cargo conjugate molecule. To describe these drug classes in more detail it is emphasized that each anti cancer drug may also be categorized according to its effect on the cell cycle and cell chemistry as disclosed above. Alkylating agents kill cells by directly attacking DNA. Alkylating agents may be used in the treatment of chronic leukemias, Hodgkin's disease, lymphomas, and certain carcinomas of the lung, breast, prostate and ovary. Cyclophosphamide is an example of a commonly used alkylating agent. Nitrosoureas act similarly to akylating agents and also inhibit changes necessary for DNA repair. These agents cross the blood-brain barrier and are therefore used to treat brain tumors, lymphomas, multiple myeloma, and malignant melanoma. Carmustine and lomustine are the major drugs in this category. Antimetabolites are that drugs block cell growth by interfering with certain activities, usually DNA synthesis. Once ingested into the cell they halt normal development and reproduction. All drugs in this category affect the cell during the "S" phase of the cell cycle. Antimetabolites may be used in the treatment of acute and chronic leukemias, choriocarcinoma, and some tumors of the gastrointestinal tract, breast and ovary. Examples of commonly used antimetabolites are 6-mercaptopurine and 5-fluorouracil (5FU). Antitumor antibiotics are a diverse group of compounds. In general, they act by binding with DNA and preventing RNA synthesis. These agents are widely used in the treatment of a variety of cancers. The most commonly used drugs in this group are doxorubicin (Adriamycin), mitomycin-C, and bleomycin. Plant (vinca)alkaloids are anti-tumor agents derived from plants. These drugs act specifically by blocking cell division during mitosis. They are commonly used in the treatment of acute lymphoblastic leukemia, Hodgkin's and non-Hodgkin's lymphomas, neuroblastomas, Wilms' tumor, and cancers of the lung, breast and testes. Vincristine and vinblastine are commonly used agents in this group. Steroid hormones are useful in treating some types of tumors. This class includes adrenocorticosteroids, estrogens, antiestrogens, progesterones, and androgens. Although their specific mechanism of action is not clear, steroid hormones modify the growth of certain hormone-dependent cancers. Tamoxifen is an example, which is used for estrogen dependent breast cancer. All of the above-mentioned tumor species may be treated by the inventive transporter cargo conjugate molecules comprising as component (B) any of the above antitumor agents.

One group of cytotoxic or anti-tumor drugs, which may be used as effector molecules for component (B) of the inventive transporter cargo conjugate molecule is preferably selected from alkylating drugs, antimetabolica, cytostatics or drugs related to hormone treatment. In this context, it it is preferred to select as cytotoxic or anti-tumor drugs compounds of metal, in particular platin (derivative) and taxol classes. In particular, the drug moiety is selected from the group of drugs consisting of, for example, cisplatin, transplatin, satraplatin, oxaliplatin, carboplatin, nedaplatin, chlorambucil, cyclophosphamide, mephalan, azathioprin, fluorouracil, (6)-mercaptopurine, methrexate, nandrolone, aminogluthemide, medroxyprogesteron, megestrolacetate, procarbazin, docetaxel, paclitaxel, irinotecan, epipodophyllotoxin, podophyllotoxin, vincristine, vinblastine, docetaxel, daunomycin, daunorubicin, doxorubicin, mitoxantrone, topotecan, bleomycin, gemcitabine, fludarabine, navelbine and 5-FUDR. Particularly preferred is the class of metal containing anticancer drugs, e.g. the class of platinum compounds.

Further cytotoxic or anti-tumor drugs, which may be used as effector molecules for component (B) of the inventive transporter cargo conjugate molecule are (identified by their generic name) Alitretinoin, Altretamine, Azathioprine, Bicalutamide, Busulfan, Capecitabine, Cyclophosphamide, Exemestane, Letrozole, Finasteride, Megestrol Acetate, Triptorelin, Temozolomide, Mifepristone, Tretinoin, Oral, Tamoxifen, Teniposide, Imatinib (Gleevec^{®}), Gefitinib (IRESSA^{®}), Peplomycin sulfate or the class of camptothecins.

Another group of cytotoxic or anti-tumor drugs, which may be used as effector molecules for component (B) of the inventive transporter cargo conjugate molecule are indolocarbazole compounds, e.g. staurosporin (and its analogues) and rebeccamycin. It is to be mentioned that compounds belonging to the class of anilinoquinazolines (e.g. gefitinib) are also particularly preferred as component (B).

A further group of cytotoxic or anti-tumor drugs, which may be used as effector molecules for component (B) of the inventive transporter cargo conjugate molecule may additionally be selected from inhibitors of topoisomerases, such as irinotecan, or mitotic kinesins or DHFR.

Additionally, cytotoxic or anti-tumor drugs, which may be used as effector molecules for component (B) of the inventive transporter cargo conjugate molecule can be selected from factors inhibiting or stimulating cell proliferation (PDGF), intracellular pathways, e.g the RAS/RAF signaling pathway, such as a member of the RAF/MEK/ERK signaling pathway (e.g. RAF-1) or mitogen-activated protein kinase pathway, CMGC kinase family (containing CDK (cyclin dependent-kinases), MAPK, GSK3, CLK), Ser/Thr kinases that belong to the AGC kinase family containing PKA, PKG, PKC kinase families, receptor tyrosine kinases involved e.g. in neovascularization and tumor progression, including vascular endothelial growth factor receptor (VEGFR)-2, VEGFR-3, platelet-derived growth factor receptor ß, Flt-3, the endothelin (ET) system, that includes ET-1, ET-2, ET-3, and the ET_{A} receptor (ET_{A}R) and ET_{B}R, and c-KIT, which are targeted by e.g. inhibiting their function, and members of the IGF-family, such as IGF-1, IGF-2, IGF-1R, IGF2R, etc.

Another group of cytotoxic or anti-tumor drugs, which may be used as effector molecules for component (B) of the inventive transporter cargo conjugate molecule may may be selected from inhibitors that target tumor cell proliferation and tumor angiogenesis. Particularly preferred in this context are small molecule antitumor kinase inhibitors directed toward targets on malignant cells and/or vascular cells have antiangiogenic activity. Kinase inhibitors such as those directed toward EGFR, Her2/neu, BCR-ABL, c-KIT, PKC, Raf and Pl3, are antiangiogenic by virtue of blocking secretion of angiogenic factors by affected malignant cells. Kinase inhibitors such as those directed toward VEGFR2, VEGFR1, PDGFR, PKC, Raf and Pl3, are antiangiogenic by effects on vascular cells. Examples of synthetic inhibitors of cyclin dependent kinases (CDKls) are e.g. olomoucine, flavopiridol, butyrolactone and their derivatives and thus constrain tumor cell proliferation. On the other hand, antitumor compounds suitable as component (B) of the inventive transporter cargo conjugate molecule may be selected from activators of apoptosis programs in cancer cells (e.g. staurosporine) or by downregulating antiapoptotic proteins, e.g. Bcl-2.

It is common to all of the above compounds that they have to cross the cell membrane in order to act as anticancer drugs. By coupling compounds belonging to each of these classes (compounds directly damaging the DNA in the nucleus of the cell, effecting the synthesis or breakdown of the mitotic spindles or stopping the synthesis of pre-DNA molecule building blocks) as component (B) to component (A) to form the inventive transporter cargo conjugate molecule, the entry of the anticancer compounds into the cell is enhanced and/or their solubility is enhanced, thereby increasing the efficacy of these therapeutic compounds. In turn, increased cell take-up and, preferably, better solubility of these compounds in the aqueous environment (e.g. the cytosol) allows to lower the dosage of the therapeutic anticancer compound.

Additionally, component (B) of the inventive transporter cargo conjugate molecule may also comprise small organic comounds, such as protease inhibitors which inhibit proteases, in particular proteases which are involved in the infection cycle of infectious agents, e.g. viral, bacterial or protozoological proteases. In a preferred embodiment, these protease inhibitors (organic compounds or drug molecules) as part of an inventive conjugate molecule may serve to treat viral, bacterial infections or protozoological infections, e.g. malaria. In particular, virus infections may be treated by protease inhibitors, e.g. retroviral diseases. The use of conjugate molecules comprising protease inhibitors are strongly preferred for the treatment of HIV infections. The protease inhibitors to be used for coupling to carrier sequence as disclosed herein may be selected from a group containing the 640385, abacavir sulfate, AG1776, amprenavir (141W94 or VX-478), atazanavir (BMS-232632), Cathepsin S protease inhibitor, D1927, D9120, efavirenz, emtricitabine, enfuvirtide (T-20), fosamprenavir (GW-433908 or VX-175), GS 9005, GW640385 (VX-385), HCV protease inhibitor, indinavir (MK-639), L-756, 423, levoprin-ZG, lopinavir(ABT-378), lopinavir/ritonavir(LPV ABT-378/r), MK-944A, mozenavir (DMP450), nelfinavir (AG-1343), nevirapine, P-1946, PL-100, prinomastat, ritonavir (ABT-538), RO033-4649, TMC114, saquinavir (Ro-31-8959), tenofovir disoproxil fumarate, tipranavir (PNU-140690), TLK 19781, TMC-114, Vertex 385, VX-950.

Finally, effector molecules suitable but not claimed as component (B) of the inventive transporter cargo conjugate molecule may be selected as a separate component from a label as defined above for the inventive transporter cargo conjugate molecule. Such a transporter cargo conjugate molecule is particularly suitable for *in vitro* or *in vivo* assays. In this context, labels may comprise radioactive labels, i.e. radioactive phosphorylation or a radioactive label with sulphur, hydrogen, carbon, nitrogen, etc.; colored dyes (e.g. digoxygenin, etc.); fluorescent groups (e.g. fluorescein, rhodamine, flourochrome proteins as defined below, etc.); chemoluminescent groups; or combination of these labels. Preferably, flourochrome proteins comprise any fluorochrome protein, which can be activated such as to emit a fluorescence signal. More preferably, the fluorochrome protein is selected from any fluorescent protein, e.g. from a group comprising the Green Fluorescent Protein (GFP), derivatives of the Green Fluorescent Protein (GFP), e.g. EGFP, AcGFP, TurboGFP, Emerald, Azami Green, the photo activatable-GFP (PA-GFP), or Blue Fluorescent Protein (BFP) including EBFP, Sapphire, T-Sapphire, or Cyan Fluorescent Proteins (CFP) including the enhanced cyan fluorescent protein (ECFP), mCFP, Cerulan, CyPet, or Yellow Fluorescent Proteins (YFP), including Topaz, Venus, mCitrine, Ypet, PhiYFP, mBanana, the yellow shifted green fluorescent protein (Yellow GFP), the enhanced yellow fluorescent protein (EYFP), or Orange and Red Flourescent Proteins (RFP) including Kusibara Orange, mOrange, dTomato-Tandem, DsRed-Monomer, mTangerine, mStrawberry, monomeric red fluorescent protein (mRFP1) (also designated herein as mRFP), mCherry, mRaspberry, HcRed-Tandem,mPlum, as well as optical highlighters selected from PA-GFP, CoralHue Dronpa (G), PS-CFP (C), PS-CFP (G), mEosFP (G), mEosFP (G), or other monomeric fluorescent proteins such as or the kindling fluorescent protein (KFP1), aequorin, the autofluorescent proteins (AFPs), or the fluorescent proteins JRed, TurboGFP, PhiYFP and PhiYFP-m, tHc-Red (HcRed-Tandem), PS-CFP2 and KFP-Red (as available from EVRΩGEN, see also www.evrogen.com), or other suitable fluorescent proteins.

The inventive transporter cargo conjugate molecule comprising components (A) and (B) may furthermore comprise at least one optional additional component (C), (D) and/or (E), etc., preferably different to component (B). This at least one optional additional portion may award additional functions to the inventive fusion protein and may be selected independent from other components (B), (C), (D) and/or (E).

For example, the at least one optional additional component (C), (D) and/or (E), etc., of the inventive transporter cargo conjugate molecule may be any of the effector molecules as described for component (B) above. Preferably, the at least one optional additional component (C), (D) and/or (E), etc., of the inventive transporter cargo conjugate molecule is not identical to the specifically selected component (B) of the inventive transporter cargo conjugate molecule, i.e., the at least one optional additional component (C), (D) and/or (E), etc., preferably may be selected independent from each other from different effector molecules or their fragments or variants as described above. The at least one optional additional component (C), (D) and/or (E), etc., of the inventive transporter cargo conjugate molecule can furthermore be an amino acid, oligopeptide or polypeptide or a (small) organo-chemical compound and can be linked to the inventive transporter cargo conjugate molecule at a suitable position, for example, the N-terminus, the C-terminus of the inventive transporter cargo conjugate molecule or may be internally coupled to amino acids, e.g. amino acid side chains, or to nucleic acids or any suitable position of a component (B) (or (A)). The at least one optional additional component (C), (D) and/or (E), etc. of the inventive transporter cargo conjugate molecule can also be a portion (e.g, HA, HSV-Tag, His6-Tag, FLAG-Tag), which may render the inventive transporter cargo conjugate molecule amenable to purification and/or isolation. If desired, the component needed for purification can then be removed from the other components of the inventive transporter cargo conjugate molecule (e.g., by proteolytic cleavage or other methods known in the art) at the end of the production process. Furthermore, the at least one optional additional component (C), (D) and/or (E), etc. of the inventive transporter cargo conjugate molecule may be a signal sequence or localisation sequence, which efficiently directs the inventive transporter cargo conjugate molecule to a particular intracellular target localization, preferably without loss of the enhanced cell permeability properties of the inventive transporter cargo conjugate molecule. Typically, such a signal sequence or localisation sequence directs the inventive transporter cargo conjugate molecule to specific cell compartments, e.g., endoplasmic reticulum, mitochondrion, gloom apparatus, lysosomal vesicles, etc. Exemplary signal sequences or localisation sequences include, without being limited thereto, localisation sequences for the endoplasmic reticulum, such as KDEL (SEQ ID NO: 238), DDEL (SEQ ID NO: 239), DEEL (SEQ ID NO: 240), QEDL (SEQ ID NO: 241), RDEL (SEQ ID NO: 242), sequences for the localisation into the nucleus, such as PKKKRKV (SEQ ID NO: 243), PQKKIKS (SEQ ID NO: 244), QPKKP (SEQ ID NO: 245), RKKR (SEQ ID NO: 246), sequences for the localisation for the nuclear region, such as RKKRRQRRRAHQ (SEQ ID NO: 247), RQARRNRRRRWRERQR (SEQ ID NO: 248), MPLTRRRPAASQALAPPTP (SEQ ID NO: 249), sequences for the localisation into the endodomal compartiment, such as MDDQRDLISNNEQLP (SEQ ID NO: 250), etc.

Similarly, the at least one optional additional component (C), (D) and/or (E), etc. of the inventive transporter cargo conjugate molecule may be a signal sequence or localisation sequence, which efficiently directs the inventive transporter cargo conjugate molecule to a particular cell type, preferably without loss of the enhanced cell permeability properties of the inventive transporter cargo conjugate molecule.

The inventive transporter cargo conjugate molecule may furthermore comprise at least one modification, preferably at its termini, either at the C- or the N-terminus or both. The C-terminus may preferably be modified by an amide modification, whereas the N-terminus may be modified by any suitable NH₂-protection group, such as e.g. acylation, or any further modification as already indicated above for L-amino acids. Such modifications also includes introduction of labels as defined above for inventive transporter molecules according to general formula (I) above.

Finally, the components (B), (C), (D), and/or (E) of the inventive transporter cargo conjugate molecule as described above as well as linkers, which may be optionally used for interlinking these components, may comprise or consist of protein or peptide sequences. Such protein or peptide sequences may be composed of L-amino acids, D-amino acids, or a combination of both, preferably as described above for the inventive transporter construct as defined above according to generic formula (I) or according to any of subformulas (Ia), (Ib), (Ic), (Id), (Ie), or (If) above. Such D- and/or L-amino acids may be arranged in the components (B), (C), (D), and/or (E) in a blockwise, a non-blockwise or in an alternate manner. Alternatively, a pattern as described above for the inventive transporter construct as defined above according to generic formula (I) or according to any of subformulas (Ia), (Ib), (Ic), (Id), (Ie), or (If) above may be repeated fort these components. In other words, generic formula (I) (SEQ ID NO: 1) DₗLLLₓDₘ(LLL_{y}Dₙ)ₐ as defined above, may be applied, wherein the number of repeats as defined by a is not limited to a range of 0 - 3 but may be applied to the entire molecule, i.e. a will be 1 to 500, 1 to 250, 1 to 100, 1 to 50, 1 to 20, 1 to 10, 1 to 5 or 1, 2, 3, 4, 5, 6, 7, 8, 9, 10, etc., and is preferably determined by the length of the protein or peptide sequences, which is to be covered.

The components (A), (B) of the inventive transporter cargo conjugate molecule and, if present, further optional components (C), (D) and/or (E), etc. are typically coupled with each other, via covalent bonds or via electrostatic bonding (e.g. poly-lysine), preferably via covalent bonds. In this context the term "covalent bond" relates to a stable chemical link between two atoms produced by sharing one or more pairs of electrons. Preferably, all components (A) and (B) of the inventive transporter cargo conjugate molecule and, if present, further optional components (C), (D) and/or (E), etc. may be coupled as to form a linear molecule or a non-linear (branched) molecule, preferably a linear molecule. In a linear molecule, all the above components (A) and (B) and, if present, optional components (C), (D) and/or (E), etc. are linked to each other via their terminal ends of in a linear form without leading to branched transporter cargo conjugate molecule. In a non-linear (branched) molecule all the above components (A) and (B) and, if present, optional components (C), (D) and/or (E), etc. are linked to each other via their terminal ends of in a form which lieads to a branched transporter cargo conjugate molecule, e.g. having an Y-shaped form, etc.

As component (A) of the inventive transporter cargo conjugate molecule is per definition a peptide sequence consisting of D- and L- amino acids, the (covalent) attachment of further components (B) and, if present, of optional components (C), (D) and/or (E), may, of course, depend on the type and nature of the components to be attached, i.e. as to whether the single components are proteins or peptides, nucleic acids, (small) organic compounds, etc.

The order, in which component (B) of the inventive transporter cargo conjugate molecule and, if present, further optional components (C), (D) and/or (E), are linked with component (A) and each other to form a preferably linear molecule, typically may comprise any order. Accordingly, any of components (A), (B), and if present, (C), (D) and/or (E) may be attached with each other. However, component (A) is preferably attached at the terminal ends of the inventive transporter cargo conjugate molecule. If any of components (B), and if present, components (C), (D) and/or (E), is a protein or a peptide sequence, component (A) is preferably contained at the C-terminal end of the inventive transporter cargo conjugate molecule, e.g., at the C-terminal end of component (B) as defined above or, if present, of components (C), (D) and/or (E), when occurring as a peptide or a protein. Such a position of component (A) in the inventive transporter cargo conjugate molecule prevents the cargo peptide or protein sequence of components (B), (C), (D) and/or (E) to be degraded prior to its/their transport to the desired target site, e.g. the cell, the nucleus, etc., by a peptidase, particularly a carboxy peptidase such as carboxyterminal peptidase N. Alternatively, if there are aminoterminal peptidases in the cell systems used, the component (A) may be located at the aminoterminal end of the inventive transporter cargo conjugate molecule.

If the further component (B) and/or, if present, any of the optional component (C), (D) and/or (E), is a peptide or protein sequence, the link between these protein or peptide components of the inventive transporter cargo conjugate molecule is typically a peptide bond. Such a peptide bond may be formed using a chemical synthesis involving both components (an N-terminal end of one component and the C-terminal end of the other component) to be linked, or may be formed directly via a protein synthesis of the entire peptide sequence of both components, wherein both (protein or peptide) components are preferably synthesized in one step. Such protein synthesis methods include e.g., without being limited thereto, liquid phase peptide synthesis methods or solid peptide synthesis methods, e.g. solid peptide synthesis methods according to Merrifield, *t*-Boc solid-phase peptide synthesis, Fmoc solid-phase peptide synthesis, BOP (Benzotriazole-1-yl-oxy-tris-(dimethylamino)-phosphonium hexafluorophosphate) based solid-phase peptide synthesis, etc.

Furthermore, component (A) and component (B) of the inventive transporter cargo conjugate molecule can be coupled via a linker or directly (without linker) by e.g. an amide bridge, if the components to be linked have reactive amino or carboxy groups. Alternatively, ester or ether linkages are preferred.

If present, further components (C), (D) and/or (E), etc., as mentioned above, can be coupled in an analogous manner to component (A) and/or component (B) or, optionally, with each other to then be linked as one single moiety to either component (A) or component (B). Linker sequences can also be used to fuse the components of inventive transporter cargo conjugate molecule with at least one other component (see below). The mode of coupling further component(s) to the either component (A) or component (B) of the inventive transporter cargo conjugate molecule will depend on its chemical character. If additional components (C), (D), (E), etc., belong to the class of peptidic sequences, they will preferably linked to the inventive transporter cargo conjugate molecule to either terminus of component (A) or, alternatively, be linked via component (A)'s L- or D amino acid side chains, e.g. by a disulfide bridge. Further components of other chemical nature may be likewise attached to component (A) (terminal groups or chemically active side chain groups) or component (B). The linkage via a side chain will preferably be based on side chain amino, thiol or hydroxyl groups, e.g. via an amide or ester or ether linkage. It has to be noted that, according to the invention, all amino acids (of any of component (A), and, if built of amino acids, components (C), (D), (E) etc.,) are preferably D-enantiomeric amino acids, which reflect its eventually naturally occurring analogue by being linked in retro-inverso order. Nevertheless, components (C), (D), (E) etc., if composed of amino acids, may also be composed of L-amino acids (in their naturally occurring sequence order) or built of a combination of D and L amino acids.

If peptidic linker sequences are used to fuse component (A) and (B) or to fuse another component, e.g. (C) to component (A) and/or (B), the linker sequences preferably form a flexible sequence of 2 to 10 residues, more preferably 1 to 5 residues. In a preferred embodiment the linker sequence contains at least 20%, more preferably at least 40% and even more preferably at least 50% Gly or β-alanine residues, e.g. GlyGlyGlyGlyGly (SEQ ID NO: 255), GlyGlyGlyGly (SEQ ID NO: 256), GlyGlyGly, CysGlyGly or GlyGlyCys, etc. Appropriate linker sequences can be easily selected and prepared by a person skilled in the art. They may be composed of D and/or L amino acids.

Peptide linker sequences may also be introduced between a component (A) and a component (B), and/or further components (C), (D) and/or (E), of the inventive transporter cargo conjugate molecule, wherein an amino-terminal methionine is added to component (A) and/or prior to a protein or peptide sequence component (B), (C), (D) and/or (E).

Preferably, component (A) and component (B) are linked by chemical coupling in any suitable manner known in the art, such as cross-linking methods. However, attention is drawn to the fact that many known chemical cross-linking methods are non-specific, i.e., they do not direct the point of coupling to any particular site on the carrier moiety or cargo moiety. Thus, the use of non-specific cross-linking agents may attack functional sites or sterically block active sites, rendering the fused components of the inventive transporter cargo conjugate molecule biologically inactive. It is referred to the knowledge of the skilled artisan to block potentially reactice groups by using appropriate protecting groups. Alternatively, the use of the powerful and versatile oxime and hydrazone ligation techniques, which are chemo-selective entities that can be applied for the cross-linking of component (A) to component (B), may be employed. This linking technology is described e.g. by Rose et al. (1994), JACS 116, 30. If present, further components (C), (D), (E) etc., as mentioned above, can be chemically coupled in an analogous manner to one another or to component (A) and/or (B).

Coupling specificity can be increased by direct chemical coupling to a functional group found only once or a few times in component (A), which functional group is to be cross-linked to the organic molecule of component (B). As an example, the cystein thiol group may be used, if just one cystein residue is present on component (A) of the inventive transporter cargo conjugate molecule. Also, for example, if a conjugate molecule component (A) contains no lysine residues, a cross-linking reagent specific for primary amines will be selective for the amino terminus of component (A). Alternatively, cross-linking may also be carried out via the side chain of a glutamic acid residue placed at the N-terminus of the peptide such that a amide bond can be generated through its side-chain. Therefore, it may be advantageous to link a glutamic acid residue to the N-terminus of component (A) of the inventive transporter cargo conjugate molecule. However, if a cysteine residue is to be introduced into component (A), introduction at or near its N- or C-terminus is preferred. Conventional methods are available for such amino acid sequence alterations based on modifications of component (A) by either adding one or more additional amino acids, e.g. inter alia an cystein residue, to the translocation sequence or by substituting at least one residue of the translocation sequence(s) being comprised in component (A). In case a cystein side chain is used for coupling purposes, component (A) of the inventive transporter cargo conjugate molecule has preferably one cystein residue. Any second cystein residue should preferably be avoided and can, eventually, be replaced when they occur in component (A) of the inventive transporter cargo conjugate molecule. When a cysteine residue is replaced in the original translocation sequence to be used as or as part of component (A), it is typically desirable to minimize resulting changes in component (A) peptide folding. Changes in component (A) folding are minimized when the replacement is chemically and sterically similar to cysteine. Therefore, serine is preferred as a replacement for cystein.

Coupling of the two constituents of the inventive transporter cargo conjugate molecule can be accomplished via a coupling or conjugating agent including standard peptide synthesis coupling reagents such as HOBt, HBTU, DICI, TBTU. There are several intermolecular cross-linking reagents which can be utilized, see for example, Means and Feeney, Chemical Modification of Proteins, Holden-Day, 1974, pp. 39-43. Among these reagents are, for example, N-succinimidyl 3-(2-pyridyldithio)propionate (SPDP) or N,N'-(1,3-phenylene)bismaleimide; N,N'-ethylene-bis-(iodoacetamide) or other such reagent having 6 to 11 carbon methylene bridges; and 1,5-difluoro-2,4-dinitrobenzene. Other cross-linking reagents useful for this purpose include: p,p'-difluoro-m,m'-dinitrodiphenylsulfone; dimethyl adipimidate; phenol-1,4-disulfonylchloride; hexamethylenediisocyanate or diisothiocyanate, or azophenyl-p-diisocyanate; glutaraldehyde and disdiazobenzidine. Cross-linking reagents may be homobifunctional, i.e., having two functional groups that undergo the same reaction. A preferred homobifunctional cross-linking reagent is bismaleimidohexane (BMH). BMH contains two maleimide functional groups, which react specifically with sulfhydryl-containing compounds under mild conditions (pH 6.5-7.7). The two maleimide groups are connected by a hydrocarbon chain. Therefore, BMH is useful for irreversible cross-linking of proteins (or polypeptides) that contain cysteine residues. Cross-linking reagents may also be heterobifunctional. Heterobifunctional cross-linking agents have two different functional groups, for example an amine-reactive group and a thiol-reactive group, that will cross-link two proteins having free amines and thiols, respectively. Examples of heterobifunctional cross-linking agents are Succinimidyl-4-(N-maleimidomethyl)-cyclohexane-1-carboxylate (SMCC), m-maleimidobenzoyl-N-hydroxysuccinimide ester (MBS), and succinimide 4-(p-maleimidophenyl)butyrate (SMPB), an extended chain analog of MBS. The succinimidyl group of these cross-linkers reacts with a primary amine, and the thiol-reactive maleimide forms a covalent bond with the thiol of a cysteine residue. Because cross-linking reagents often have low solubility in water, a hydrophilic moiety, such as a sulfonate group, may be added to the cross-linking reagent to improve its water solubility. Sulfo-MBS and sulfo-SMCC are examples of cross-linking reagents modified for water solubility. Many cross-linking reagents yield a conjugate that is essentially non-cleavable under cellular conditions. Therefore, some cross-linking reagents contain a covalent bond, such as a disulfide, that is cleavable under cellular conditions. For example, Traut's reagent, dithiobis (succinimidylpropionate) (DSP), and N-succinimidyl 3-(2-pyridyldithio)propionate (SPDP) are well-known cleavable cross-linkers. The use of a cleavable cross-linking reagent permits the cargo moiety component (B), (C), (D) and/or (E) to separate from the transporter construct component (A) after delivery into the target cell. For this purpose, direct disulfide linkage may also be useful. Chemical cross-linking may also include the use of spacer arms. Spacer arms provide intramolecular flexibility or adjust intramolecular distances between conjugated moieties and thereby may help preserve biological activity. A spacer arm may be in the form of a protein (or polypeptide) moiety that includes spacer amino acids, e.g. proline. Alternatively, a spacer arm may be part of the cross-linking reagent, such as in "long-chain SPDP" (Pierce Chem. Co., Rockford, III., cat. No. 21651 H). Numerous cross-linking reagents, including the ones discussed above, are commercially available. Detailed instructions for their use are readily available from the commercial suppliers. A general reference on protein cross-linking and conjugate preparation is: Wong, Chemistry of Protein Conjugation and Cross-Linking, CRC Press (1991).

According to another preferred embodiment, an inventive transporter cargo conjugate molecule as defined above may comprise or consist of at least one variant and/or fragment of the above defined inventive transporter cargo conjugate molecules. Preferably, variants and/or fragments of the above defined inventive transporter cargo conjugate molecules retain biological activity of the inventive transporter cargo conjugate molecules as disclosed above. Functionality of such fragments or variants may be tested by various tests, e.g. transfection efficacy, correct expression of proteins encoded by cargo nucleic acids, or by biophysical methods, e.g. spectroscopy, computer modeling, structural analysis, etc. similar as described above for inventive transporter constructs as defined above according to generic formula (I)

Even more preferably, the above defined inventive transporter cargo conjugate molecules comprise or consist of at least one variant (and/or fragment), particularly if the single components are protein or peptide sequences. In the context of the invention such variants (and/or fragments) of the above defined inventive transporter cargo conjugate molecules may have a sequence identity to their native sequences, e.g. a fragment or a variant of the above defined inventive transporter cargo conjugate molecule to its native transporter cargo conjugate molecule sequence, of at least 70%, 80% or 85%, preferably at least 90%, more preferably at least 95% and most preferably at least 99% over the whole length of the native inventive transporter cargo conjugate molecule.

A "fragment" of the above defined inventive transporter cargo conjugate molecules, particularly if the single components are protein or peptide sequences, is preferably to be understood as a truncated sequence thereof, i.e. an amino acid sequence of the above defined inventive transporter cargo conjugate molecule, which is N-terminally, C-terminally and/or intrasequentially truncated compared to the amino acid sequence of the native inventive transporter cargo conjugate molecule as defined above.

A "variant" of the above defined inventive transporter cargo conjugate molecule preferably comprises a sequence wherein the amino acid sequence of the inventive transporter cargo conjugate molecule variant differs from the native sequence of the above defined inventive transporter cargo conjugate molecule in one or more mutation(s), such as one or more substituted, (or, if necessary, inserted and/or deleted) amino acid(s). Preferably, variants of the above defined inventive transporter cargo conjugate molecules have the same biological function or specific activity compared to the full-length native transporter cargo conjugate molecules as defined above. Preferably, a variant may comprise about 1 to 100, 1 to 50, 1 to 20, preferably 1 to 10 and more preferably 1 to 5, 4, 3, 2 or 1 amino acid alterations within the above meaning. Such alterations may comprise *inter alia* modifications of amino acids as defined above, introduction of labels into amino acids as defined above, substituting an amino acid with any of the (modified or labelled) amino acids mentioned herein, deletions or insertions of amino acids. Variants as defined herein furthermore preferably comprise conservative amino acid substitutions, preferably such as already defined above.

According to a third aspect, the present invention furthermore provides a pharmaceutical composition, the pharmaceutical composition preferably comprising the inventive transporter cargo conjugate molecule as defined above, and optionally a pharmaceutically acceptable carrier and/or vehicle, or any excipient, buffer, stabilizer or other materials well known to those skilled in the art.

As a first ingredient, the inventive pharmaceutical composition comprises the inventive transporter cargo conjugate molecule as defined above, i.e. an inventive transporter cargo conjugate molecule, comprising as a component (A) the inventive transporter construct as defined above according to generic formula (I) or according to any of subformulas (Ia), (Ib), (Ic), (Id), (Ie), or (If) above, and as a component (B) an effector molecule selected from therapeutically active proteins and peptides, protein kinase inhibitors, particularly inhibitors of the protein kinase c-Jun amino terminal kinase, antigens, antibodies, apoptotic factors, proteases implicated in pathological states, preferably peptidic protease inhibitors, BH3-domains, BH3-only proteins, nucleic acids encoding these proteins, siRNAs, antisense RNAs,cytotoxic agents, and small organic compounds. Optionally, the inventive transporter cargo conjugate molecule as defined above may furthermore contain additional components (C), (D), and/or (E), etc.

As a second ingredient, the inventive pharmaceutical composition may or may not comprise a pharmaceutically acceptable carrier and/or vehicle. In the context of the present invention, a pharmaceutically acceptable carrier typically includes the liquid or non-liquid basis of the inventive inventive pharmaceutical composition. If the inventive pharmaceutical composition is provided in liquid form, the carrier will typically be pyrogen-free water; isotonic saline or buffered (aqueous) solutions, e.g phosphate, citrate etc. buffered solutions. Particularly for injection of the inventive inventive pharmaceutical composition, water or preferably a buffer, more preferably an aqueous buffer, may be used, containing a sodium salt, preferably at least 50 mM of a sodium salt, a calcium salt, preferably at least 0.01 mM of a calcium salt, and optionally a potassium salt, preferably at least 3 mM of a potassium salt. According to a preferred embodiment, the sodium, calcium and, optionally, potassium salts may occur in the form of their halogenides, e.g. chlorides, iodides, or bromides, in the form of their hydroxides, carbonates, hydrogen carbonates, or sulfates, etc. Without being limited thereto, examples of sodium salts include e.g. NaCl, Nal, NaBr, Na₂CO₃, NaHCO₃, Na₂SO₄, examples of the optional potassium salts include e.g. KCI, Kl, KBr, K₂CO₃, KHCO₃, K₂SO₄, and examples of calcium salts include e.g. CaCl₂, CaI₂, CaBr₂, CaCO₃, CaSO₄, Ca(OH)₂. Furthermore, organic anions of the aforementioned cations may be contained in the buffer. According to a more preferred embodiment, the buffer suitable for injection purposes as defined above, may contain salts selected from sodium chloride (NaCl), calcium chloride (CaCl₂) and optionally potassium chloride (KCI), wherein further anions may be present additional to the chlorides. CaCl₂ can also be replaced by another salt like KCI. Typically, the salts in the injection buffer are present in a concentration of at least 50 mM sodium chloride (NaCl), at least 3 mM potassium chloride (KCI) and at least 0,01 mM calcium chloride (CaCl₂). The injection buffer may be hypertonic, isotonic or hypotonic with reference to the specific reference medium, i.e. the buffer may have a higher, identical or lower salt content with reference to the specific reference medium, wherein preferably such concentrations of the afore mentioned salts may be used, which do not lead to damage of cells due to osmosis or other concentration effects. Reference media are e.g. liquids occurring in *"in vivo"* methods, such as blood, lymph, cytosolic liquids, or other body liquids, or e.g. liquids, which may be used as reference media in *"in vitro"* methods, such as common buffers or liquids. Such common buffers or liquids are known to a skilled person. Ringer-Lactate solution is particularly preferred as a liquid basis.

However, one or more compatible solid or liquid fillers or diluents or encapsulating compounds may be used as well for the inventive pharmaceutical composition, which are suitable for administration to a patient to be treated. The term "compatible" as used here means that these constituents of the inventive pharmaceutical composition are capable of being mixed with the inventive transporter cargo conjugate molecule as defined above in such a manner that no interaction occurs which would substantially reduce the pharmaceutical effectiveness of the inventive pharmaceutical composition under typical use conditions. Pharmaceutically acceptable carriers, fillers and diluents must, of course, have sufficiently high purity and sufficiently low toxicity to make them suitable for administration to a person to be treated. Some examples of compounds which can be used as pharmaceutically acceptable carriers, fillers or constituents thereof are sugars, such as, for example, lactose, glucose and sucrose; starches, such as, for example, corn starch or potato starch; cellulose and its derivatives, such as, for example, sodium carboxymethylcellulose, ethylcellulose, cellulose acetate; powdered tragacanth; malt; gelatin; tallow; solid glidants, such as, for example, stearic acid, magnesium stearate; calcium sulfate; vegetable oils, such as, for example, groundnut oil, cottonseed oil, sesame oil, olive oil, corn oil and oil from theobroma; polyols, such as, for example, polypropylene glycol, glycerol, sorbitol, mannitol and polyethylene glycol; alginic acid.

The inventive pharmaceutical composition may be administered orally, parenterally, by inhalation spray, topically, rectally, nasally, buccally, vaginally or via an implanted reservoir. The term parenteral as used herein includes subcutaneous, intravenous, intramuscular, intra-articular, intra-synovial, intrasternal, intrathecal, intrahepatic, intralesional, intracranial, transdermal, intradermal, intrapulmonal, intraperitoneal, intracardial, intraarterial, and sublingual injection or infusion techniques.

Preferably, the inventive pharmaceutical composition may be administered by parenteral injection, more preferably by subcutaneous, intravenous, intramuscular, intra-articular, intra-synovial, intrasternal, intrathecal, intrahepatic, intralesional, intracranial, transdermal, intradermal, intrapulmonal, intraperitoneal, intracardial, intraarterial, and sublingual injection or via infusion techniques. Sterile injectable forms of the inventive pharmaceutical compositions may be aqueous or oleaginous suspension. These suspensions may be formulated according to techniques known in the art using suitable dispersing or wetting agents and suspending agents. The sterile injectable preparation may also be a sterile injectable solution or suspension in a non-toxic parenterally-acceptable diluent or solvent, for example as a solution in 1.3-butanediol. Among the acceptable vehicles and solvents that may be employed are water, Ringer's solution and isotonic sodium chloride solution. In addition, sterile, fixed oils are conventionally employed as a solvent or suspending medium. For this purpose, any bland fixed oil may be employed including synthetic mono- or diglycerides. Fatty acids, such as oleic acid and its glyceride derivatives are useful in the preparation of injectables, as are natural pharmaceutically-acceptable oils, such as olive oil or castor oil, especially in their polyoxyethylated versions. These oil solutions or suspensions may also contain a long-chain alcohol diluent or dispersant, such as carboxymethyl cellulose or similar dispersing agents that are commonly used in the formulation of pharmaceutically acceptable dosage forms including emulsions and suspensions. Other commonly used surfactants, such as Tweens, Spans and other emulsifying agents or bioavailability enhancers which are commonly used in the manufacture of pharmaceutically acceptable solid, liquid, or other dosage forms may also be used for the purposes of formulation of the inventive pharmaceutical composition.

For intravenous, cutaneous or subcutaneous injection, or injection at the site of affliction, the active ingredient will preferably be in the form of a parenterally acceptable aqueous solution which is pyrogen-free and has suitable pH, isotonicity and stability. Those of relevant skill in the art are well able to prepare suitable solutions using, for example, isotonic vehicles such as Sodium Chloride Injection, Ringer's Injection, Lactated Ringer's Injection. Preservatives, stabilizers, buffers, antioxidants and/or other additives may be included, as required. Whether it is a polypeptide, peptide, or nucleic acid molecule, other pharmaceutically useful compound according to the present invention that is to be given to an individual, administration is preferably in a "prophylactically effective amount" or a "therapeutically effective amount" (as the case may be), this being sufficient to show benefit to the individual. The actual amount administered, and rate and time-course of administration, will depend on the nature and severity of what is being treated.

The inventive pharmaceutical composition as defined above may also be administered orally in any orally acceptable dosage form including, but not limited to, capsules, tablets, aqueous suspensions or solutions. In the case of tablets for oral use, carriers commonly used include lactose and corn starch. Lubricating agents, such as magnesium stearate, are also typically added. For oral administration in a capsule form, useful diluents include lactose and dried cornstarch. When aqueous suspensions are required for oral use, the active ingredient, i.e. the inventive transporter cargo conjugate molecule as defined above, is combined with emulsifying and suspending agents. If desired, certain sweetening, flavoring or coloring agents may also be added.

The inventive pharmaceutical composition may also be administered topically, especially when the target of treatment includes areas or organs readily accessible by topical application, e.g. including diseases of the skin or of any other accessible epithelial tissue. Suitable topical formulations are readily prepared for each of these areas or organs. For topical applications, the inventive pharmaceutical composition may be formulated in a suitable ointment, containing the inventive immunostimulatory composition, particularly its components as defined above, suspended or dissolved in one or more carriers. Carriers for topical administration include, but are not limited to, mineral oil, liquid petrolatum, white petrolatum, propylene glycol, polyoxyethylene, polyoxypropylene compound, emulsifying wax and water. Alternatively, the inventive pharmaceutical composition can be formulated in a suitable lotion or cream. In the context of the present invention, suitable carriers include, but are not limited to, mineral oil, sorbitan monostearate, polysorbate 60, cetyl esters wax, cetearyl alcohol, 2-octyldodecanol, benzyl alcohol and water.

In this context, prescription of treatment, e.g. decisions on dosage etc. when using the above pharmaceutical composition is typically within the responsibility of general practitioners and other medical doctors, and typically takes account of the disorder to be treated, the condition of the individual patient, the site of delivery, the method of administration and other factors known to practitioners. Examples of the techniques and protocols mentioned above can be found in REMINGTON'S PHARMACEUTICAL SCIENCES, 16th edition, Osol, A. (ed), 1980. Accordingly, the inventive pharmaceutical composition typically comprises a "safe and effective amount" of the components of the inventive pharmaceutical composition, particularly of the inventive transporter cargo conjugate molecule as defined above. As used herein, a "safe and effective amount" means an amount of the inventive transporter cargo conjugate molecule as defined above that is sufficient to significantly induce a positive modification of a disease or disorder as defined herein. At the same time, however, a "safe and effective amount" is small enough to avoid serious side-effects, that is to say to permit a sensible relationship between advantage and risk. The determination of these limits typically lies within the scope of sensible medical judgment. A "safe and effective amount" of the components of the inventive pharmaceutical composition, particularly of the inventive transporter cargo conjugate molecule as defined above, will furthermore vary in connection with the particular condition to be treated and also with the age and physical condition of the patient to be treated, the body weight, general health, sex, diet, time of administration, rate of excretion, drug combination, the activity of the specific components (A), (B), (C), (D) and/or (E) of the inventive transporter cargo conjugate molecule as defined above, the severity of the condition, the duration of the treatment, the nature of the accompanying therapy, of the particular pharmaceutically acceptable carrier used, and similar factors, within the knowledge and experience of the accompanying doctor. The inventive pharmaceutical composition may be used for human and also for veterinary medical purposes, preferably for human medical purposes, as a pharmaceutical composition in general or as a vaccine.

According to a specific embodiment, the inventive pharmaceutical composition may be provided as a vaccine, e.g. if component (B) of the inventive transporter cargo conjugate molecule is a therpeutically active protein such as a(n) (protein or peptide) antigen or antigenic fragment or any molecule as described above, which is suitable to elicit an imune response. Such an inventive vaccine is typically composed like the inventive pharmaceutical composition and preferably supports an innate and/or an adaptive immune response of the immune system of a patient to be treated, depending on the nature of the components (B), (C), (D) and/or (E) of the inventive transporter cargo conjugate molecule as defined above. As an example, if any of these components provides or encodes a(n) (protein or peptide) antigen or antigenic fragment, the vaccine typically will lead to an adaptive immune reponse in the patient to be treated. Similarly, any of the further components (B), (C), (D) and/or (E) of the inventive transporter cargo conjugate molecule as defined above may lead to an innate and/or adaptive immune response.

The inventive vaccine may also comprise a pharmaceutically acceptable carrier, adjuvant, and/or vehicle as defined above for the inventive pharmaceutical composition. In the specific context of the inventive vaccine, the choice of a pharmaceutically acceptable carrier is determined in principle by the manner in which the inventive vaccine is administered. The inventive vaccine can be administered, for example, systemically or locally. Routes for systemic administration in general include, for example, transdermal, oral, parenteral routes, including subcutaneous, intravenous, intramuscular, intraarterial, intradermal and intraperitoneal injections and/or intranasal administration routes. Routes for local administration in general include, for example, topical administration routes but also intradermal, transdermal, subcutaneous, or intramuscular injections or intralesional, intracranial, intrapulmonal, intracardial, and sublingual injections. More preferably, vaccines may be administered by an intradermal, subcutaneous, or intramuscular route. Inventive vaccines are therefore preferably formulated in liquid (or sometimes in solid) form. The suitable amount of the inventive vaccine to be administered can be determined by routine experiments with animal models. Such models include, without implying any limitation, rabbit, sheep, mouse, rat, dog and non-human primate models. Preferred unit dose forms for injection include sterile solutions of water, physiological saline or mixtures thereof. The pH of such solutions should be adjusted to about 7.4. Suitable carriers for injection include hydrogels, devices for controlled or delayed release, polylactic acid and collagen matrices. Suitable pharmaceutically acceptable carriers for topical application include those which are suitable for use in lotions, creams, gels and the like. If the inventive vaccine is to be administered orally, tablets, capsules and the like are the preferred unit dose form. The pharmaceutically acceptable carriers for the preparation of unit dose forms which can be used for oral administration are well known in the prior art. The choice thereof will depend on secondary considerations such as taste, costs and storability, which are not critical for the purposes of the present invention, and can be made without difficulty by a person skilled in the art.

The inventive vaccine can additionally contain one or more auxiliary substances in order to further increase its immunogenicity. A synergistic action of the inventive transporter cargo conjugate molecule as defined above and of an auxiliary substance, which may be optionally contained in the inventive vaccine as described above, is preferably achieved thereby. Depending on the various types of auxiliary substances, various mechanisms can come into consideration in this respect. For example, compounds that permit the maturation of dendritic cells (DCs), for example lipopolysaccharides, TNF-alpha or CD40 ligand, form a first class of suitable auxiliary substances. In general, it is possible to use as auxiliary substance any agent that influences the immune system in the manner of a "danger signal" (LPS, GP96, etc.) or cytokines, such as GM-CFS, which allow an immune response produced by the immune-stimulating adjuvant according to the invention to be enhanced and/or influenced in a targeted manner. Particularly preferred auxiliary substances are cytokines, such as monokines, lymphokines, interleukins or chemokines, that further promote the innate immune response, such as IL-1, IL-2, IL-3, IL-4, IL-5, IL-6, IL-7, IL-8, IL-9, IL-10, IL-12, IL-13, IL-14, IL-15, IL-16, IL-17, IL-18, IL-19, IL-20, IL-21, IL-22, IL-23, IL-24, IL-25, IL-26, IL-27, IL-28, IL-29, IL-30, IL-31, IL-32, IL-33, INF-alpha, IFN-beta, INF-gamma, GM-CSF, G-CSF, M-CSF, LT-beta or TNF-alpha, growth factors, such as hGH.

Further additives which may be included in the inventive vaccine are emulsifiers, such as, for example, Tween^{®}; wetting agents, such as, for example, sodium lauryl sulfate; colouring agents; taste-imparting agents, pharmaceutical carriers; tablet-forming agents; stabilizers; antioxidants; preservatives.

The inventive vaccine can also additionally contain any further compound, which is known to be immune-stimulating due to its binding affinity (as ligands) to human Toll-like receptors TLR1, TLR2, TLR3, TLR4, TLR5, TLR6, TLR7, TLR8, TLR9, TLR10, or due to its binding affinity (as ligands) to murine Toll-like receptors TLR1, TLR2, TLR3, TLR4, TLR5, TLR6, TLR7, TLR8, TLR9, TLR10, TLR11, TLR12 or TLR13.

Another class of compounds, which may be added to an inventive vaccine in this context, may be CpG nucleic acids, in particular CpG-RNA or CpG-DNA. A CpG-RNA or CpG-DNA can be a single-stranded CpG-DNA (ss CpG-DNA), a double-stranded CpG-DNA (dsDNA), a single-stranded CpG-RNA (ss CpG-RNA) or a double-stranded CpG-RNA (ds CpG-RNA). The CpG nucleic acid is preferably in the form of CpG-RNA, more preferably in the form of single-stranded CpG-RNA (ss CpG-RNA). The CpG nucleic acid preferably contains at least one or more (mitogenic) cytosine/guanine dinucleotide sequence(s) (CpG motif(s)). According to a first preferred alternative, at least one CpG motif contained in these sequences, that is to say the C (cytosine) and the G (guanine) of the CpG motif, is unmethylated. All further cytosines or guanines optionally contained in these sequences can be either methylated or unmethylated. According to a further preferred alternative, however, the C (cytosine) and the G (guanine) of the CpG motif can also be present in methylated form.

According to a fourth aspect of the present invention, the inventive transporter cargo conjugate molecule as defined above may be used (for the preparation of a pharmaceutical composition or a vaccine, preferably both as defined herein,) for the prophylaxis, treatment and/or amelioration of cancer or tumor diseases, including diseases caused by defective apoptosis, inflammatory diseases, infectious diseases, viral (infectious) diseases, diseases strongly related to JNK signalling, autoimmune disorders or diseases, cardiovascular diseases, neuronal or neurodegenerative diseases, diseases of the liver, diseases of the spine, diseases of the uterus, major depressive disorders, non-chronic or chronic inflammatory digestive diseases and hearing loss or diseases of the inner ear. The inventive transporter cargo conjugate molecule may also be used (for the preparation of a pharmaceutical composition) for use in tissue transplantation either by treating the organs/tissue/cells to be transplanted or by treating the recipient of the organ/tissue/cells.

Prophylaxis, treatment and/or amelioration of a disease as defined herein typically include administration of a pharmaceutical composition as defined above. The term "prophylaxis" is typically directed to the prevention of a disease as defined herein in a patient, preferably prior to manifestation of the disease in the patient. The term "treatment" generally refers to any treatment of a disease as defined herein in a patient, wherein the disease may have already been diagnosed or shall be prevented, i.e. prior, parallel and subsequent to manifestation of the disease in the patient. The term "treatment", used for example in the term "treating a condition", furthermore preferably means at least the administration of a therapeutically effective amount of a therapeutic compound to elicit a therapeutic effect. It does not necessarily imply "curing", but rather having preferably at least some minimal physiological effect upon a condition upon administration to a living body having such a condition. For example, treatment could encompass administering an agent and the presence of that agent resulting in a change in the physiology of a recipient animal. Finally, the term "amelioration" preferably includes any modification of a disease as defined herein, preferably a positive modification f the disease as defined herein. The specific modification may be dependent on the disease to be treated.

According to one approach, an inventive pharmaceutical composition, a vaccine or an inventive transporter cargo conjugate molecule as defined above may be administered directly to a patient using the administration routes as described above for pharmaceutical compositions. Alternatively, a pharmaceutical composition, a vaccine or an inventive transporter cargo conjugate molecule as defined above may be administered to a patient using an *ex vivo* approach, e.g. by introducing the pharmaceutical composition, the vaccine or the inventive transporter cargo conjugate molecule as defined above into cells, preferably autologous cells, i.e. cells derived from the patient to be treated, and transplanting these cells into the site of the patient to be treated, optionally subsequent to storing and/or culturing these cells prior to treatment.

According to one preferred embodiment, the inventive transporter cargo conjugate molecule, the inventive pharmaceutical composition or the inventive vaccine as defined above, may be used for (the preparation of a medicament for) the prophylaxis, treatment and/or amelioration of e.g. cancer or tumor diseases, including diseases caused by defective apoptosis, preferably selected from acusticus neurinoma, anal carcinoma, astrocytoma, basalioma, Behcet's syndrome, bladder cancer, blastomas, bone cancer, brain metastases, brain tumors, brain cancer (glioblastomas), breast cancer (mamma carcinoma), Burkitt's lymphoma, carcinoids, cervical cancer, colon carcinoma, colorectal cancer, corpus carcinoma, craniopharyngeomas, CUP syndrome, endometrial carcinoma, gall bladder cancer, genital tumors, including cancers of the genitourinary tract, glioblastoma, gliomas, head/neck tumors, hepatomas, histocytic lymphoma, Hodgkin's syndromes or lymphomas and non-Hodgkin's lymphomas, hypophysis tumor, intestinal cancer, including tumors of the small intestine, and gastrointestinal tumors, Kaposi's sarcoma, kidney cancer, kidney carcinomas, laryngeal cancer or larynx cancer, leukemia, including acute myeloid leukaemia (AML), erythroleukemia, acute lymphoid leukaemia (ALL), chronic myeloid leukaemia (CML), and chronic lymphocytic leukaemia (CLL), lid tumor, liver cancer, liver metastases, lung carcinomas (= lung cancer = bronchial carcinoma), small cell lung carcinomas and non-small cell lung carcinomas, and lung adenocarcinoma, lymphomas, lymphatic cancer, malignant melanomas, mammary carcinomas (= breast cancer), medulloblastomas, melanomas, meningiomas, Mycosis fungoides, neoplastic diseases neurinoma, oesophageal cancer, oesophageal carcinoma (= oesophageal cancer), oligodendroglioma, ovarian cancer (= ovarian carcinoma), ovarian carcinoma, pancreatic carcinoma (= pancreatic cancer), penile cancer, penis cancer, pharyngeal cancer, pituitary tumour, plasmocytoma, prostate cancer (= prostate tumors), rectal carcinoma, rectal tumors, renal cancer, renal carcinomas, retinoblastoma, sarcomas, Schneeberger's disease, skin cancer, e.g. melanoma or non-melanoma skin cancer, including basal cell and squamous cell carcinomas as well as psoriasis, pemphigus vulgaris, soft tissue tumours, spinalioma, stomach cancer, testicular cancer, throat cancer, thymoma, thyroid carcinoma, tongue cancer, urethral cancer, uterine cancer, vaginal cancer, various virus-induced tumors such as, for example, papilloma virus-induced carcinomas (e.g. cervical carcinoma = cervical cancer), adenocarcinomas, herpes virus-induced tumors (e.g. Burkitt's lymphoma, EBV-induced B-cell lymphoma, cervix carcinoma), heptatitis B-induced tumors (hepatocell carcinomas), HTLV-1- and HTLV-2-induced lymphomas, vulval cancer, wart conditions or involvement, etc. In the present context, the terms "therapy" and "therapeutic" preferably mean to have at least some minimal physiological effect upon being administered to a living body. For example, a physiological effect upon administering a "therapeutic" anti-tumor compound may be the inhibition of tumor growth, or decrease in tumor size, or prevention reoccurrence of the tumor. Preferably, in the treatment of cancer or neoplastic disease, a compound which inhibits the growth of a tumor or decreased the size of the tumor or prevents the reoccurrence of the tumor would be considered therapeutically effective. The term "anti-tumor drug" therefore preferably means any therapeutic agent having therapeutic effect against a tumor, neoplastic disease or cancer.

According to an alternative preferred embodiment, the inventive transporter cargo conjugate molecule, the inventive pharmaceutical composition or the inventive vaccine as defined above, may be used for (the preparation of a medicament for) the prophylaxis, treatment, and/or amelioration of inflammatory diseases, such as inflammatory diseases of the lung or lung diseases, including Acute Respiratory Distress Syndrome (ARDS), or pulmonary fibrosis, inflammations of the tissue, including, without being limited thereto, formation of fibrous tissue, including cystic fibrosis, meningitis, and graft rejection or transplant rejection reactions, chronic illness involving the respiratory system, including Asthma, chronic obstructive pulmonary disease (COPD), pneumonia, and pulmonary fibrosis.

According to an alternative preferred embodiment, the inventive transporter cargo conjugate molecule, the inventive pharmaceutical composition or the inventive vaccine as defined above, may be used for (the preparation of a medicament for) the prophylaxis, treatment, and/or amelioration of e.g. infectious diseases, preferably viral, retroviral, bacterial or protozoological infectious diseases. Such infectious diseases are typically selected from AIDS, anthrax, Japanese encephalitis, bacterial infectious diseases such as miscarriage (prostate inflammation), anthrax, appendicitis, borreliosis, botulism, Camphylobacter, Chlamydia trachomatis (inflammation of the urethra, conjunctivitis), cholera, diphtheria, donavanosis, epiglottitis, typhus fever, gas gangrene, gonorrhoea, rabbit fever, Heliobacter pylori, whooping cough, climatic bubo, osteomyelitis, Legionnaire's disease, chicken-pox, condyloma acuminata, cytomegalic virus (CMV), dengue fever, early summer meningoencephalitis (ESME), Ebola virus, colds, fifth disease, foot-and-mouth disease, herpes simplex type I, herpes simplex type II, herpes zoster, HSV, infectious diseases caused by parasites, protozoa or fungi, such as amoebiasis, bilharziosis, Chagas disease, Echinococcus, fish tapeworm, fish poisoning (Ciguatera), fox tapeworm, athlete's foot, canine tapeworm, candidosis, yeast fungus spots, scabies, cutaneous Leishmaniosis, lambliasis (giardiasis), lice, malaria, microscopy, onchocercosis (river blindness), fungal diseases, bovine tapeworm, schistosomiasis, porcine tapeworm, toxoplasmosis, trichomoniasis, trypanosomiasis (sleeping sickness), visceral Leishmaniosis, nappy/diaper dermatitis or miniature tapeworm, infectious erythema, influenza, Kaposi's sarcoma, Lassa fever, Leishmaniasis, leprosy, listeriosis, Lyme borreliosis, malaria, Marburg virus infection, measles, meningitis, including bacterial meningitis, molluscum contagiosum, mononucleosis, mumps, Mycoplasma hominis, neonatal sepsis (Chorioamnionitis), noma, Norwalk virus infection, otitis media, paratyphus, Pfeiffer's glandular fever, plague, pneumonia, polio (poliomyelitis, childhood lameness), pseudo-croup, rabies, Reiter's syndrome, Rocky Mountain spotted fever, Salmonella paratyphus, Salmonella typhus, SARS, scarlet fever, shingles, hepatitis, smallpox, soft chancre, syphilis, tetanus,three-day fever, tripper, tsutsugamushi disease, tuberculosis, typhus, vaginitis (colpitis), viral diseases caused by cytomegalovirus (CMV), orthopox variola virus, orthopox alastrim virus, parapox ovis virus, molluscum contagiosum virus, herpes , simplex virus 1, herpes simplex virus 2, herpes B virus, varicella zoster virus, pseudorabies virus, human cytomegaly virus, human herpes virus 6, human herpes virus 7, Epstein-Barr virus, human herpes virus 8, hepatitis B virus, chikungunya virus, O'nyong'nyong virus, rubivirus, hepatitis C virus, GB virus C, West Nile virus, dengue virus, yellow fever virus, louping ill virus, St. Louis encephalitis virus, Japan B encephalitis virus, Powassan virus, FSME virus, SARS, SARS-associated corona virus, human corona virus 229E, human corona virus Oc43, Torovirus, human T cell lymphotropic virus type I, human T cell lymphotropic virus type II, HIV (AIDS), i.e. human immunodeficiency virus type 1 or human immunodeficiency virus type 2, influenza virus, Lassa virus, lymphocytic choriomeningitis virus, Tacaribe virus, Junin virus, Machupo virus, Borna disease virus, Bunyamwera virus, California encephalitis virus, Rift Valley fever virus, sand fly fever virus, Toscana virus, Crimean-Congo haemorrhagic fever virus, Hazara virus, Khasan virus, Hantaan virus, Seoul virus, Prospect Hill virus, Puumala virus, Dobrava Belgrade virus, Tula virus, sin nombre virus, Lake Victoria Marburg virus, Zaire Ebola virus, Sudan Ebola virus, Ivory Coast Ebola virus, influenza virus A, influenza virus B, influenza viruses C, parainfluenza virus, measles virus, mumps virus, respiratory syncytial virus, human metapneumovirus, vesicular stomatitis Indiana virus, rabies virus, Mokola virus, Duvenhage virus, European bat lyssavirus 1 + 2, Australian bat lyssavirus, adenoviruses A-F, human papilloma viruses, condyloma virus 6, condyloma virus 11, polyoma viruses, adeno-associated virus 2, rotaviruses, or orbiviruses, Varicella including Varizella zoster, and malaria virus, viral infectious diseases such as AIDS, infectious diseases caused by Condyloma acuminata, hollow warts, Dengue fever, three-day fever, Ebola virus, cold, early summer meningoencephalitis (FSME), flu, shingles, hepatitis, herpes simplex type I, herpes simplex type II, Herpes zoster, influenza, Japanese encephalitis, Lassa fever, Marburg virus, warts, West Nile fever, yellow fever, etc.

According to another preferred embodiment, the inventive transporter cargo conjugate molecule, the inventive pharmaceutical composition or the inventive vaccine, may be used for (the preparation of a medicament for) the prophylaxis, treatment, and/or amelioration of diseases strongly related to JNK signaling in a subject. Such diseases or disorders strongly related to JNK signaling in a subject, without being limited thereto, are preferably selected from autoimmune disorders, cardiovascular diseases, cancer or tumor diseases as defined above, diabetes, including diabetes type 1 or type 2, inflammatory.diseases as defined above, hair loss, including Alopecia areata, diseases of the lung, neuronal or neurodegenerative diseases, diseases of the liver, diseases of the spine, diseases of the uterus, (viral) infectious diseases and depressive disorders. In the case of diseases or disorders strongly related to JNK signaling the term "amelioration" may include the suppression of expression of JNK when it is over-expressed, and/or the suppression of phosphorylation of c-jun, ATF2 or NFAT4 in any of the above diseases, for example, by using at least one JNK inhibitor sequence as defined herein coupled to the inventive novel transporter molecule within the above definitions, as a competitive inhibitor of the natural c-jun, ATF2 and NFAT4 binding site in a cell. In this specific context, the term "modulate" also includes suppression of hetero- and homomeric complexes of transcription factors made up of, without being limited thereto, c-jun, ATF2, or NFAT4 and their related partners, such as for example the AP-1 complex that is made up of c-jun, AFT2 and c-fos. When a disease or disorder strongly related to JNK signaling as defined above is associated with JNK overexpression, such suppressive JNK inhibitor sequences can be introduced to a cell. In some instances, "modulate" in the context of diseases or disorders strongly related to JNK signaling may also include the increase of JNK expression, for example by use of an IB peptide-specific antibody that blocks the binding of an IB-peptide to JNK, thus preventing JNK inhibition by the IB-related peptide. Prevention and/or treatment of a subject with the pharmaceutical composition as disclosed above may be typically accomplished by administering *(in vivo*) an ("therapeutically effective") amount of said pharmaceutical composition to a subject, wherein the subject may be e.g. any mammal, e.g. a human, a primate, mouse, rat, dog, cat, cow, horse or pig. The term "therapeutically effective" means that the active component of the pharmaceutical composition is of sufficient quantity to ameliorate the disease or disorder strongly related to JNK signaling as defined above. Further example may be found for the other diseases mentioned herein.

Accordingly, the inventive transporter cargo conjugate molecule, the inventive pharmaceutical composition or the inventive vaccine, may be used for (the preparation of a medicament for) the prophylaxis, treatment, and/or amelioration of autoimmune disorders or diseases. Autoimmune disorders or diseases can be broadly divided into systemic and organ-specific or localised autoimmune disorders, depending on the principal clinico-pathologic features of each disease. Autoimmune diseases may be divided into the categories of systemic syndromes, including systemic lupus erythematosus (SLE), Sjögren's syndrome, Scleroderma, Rheumatoid Arthritis and polymyositis or local syndromes which may be endocrinologic (type I diabetes (Diabetes mellitus Type 1), Hashimoto's thyroiditis, Addison's disease etc.), dermatologic (pemphigus vulgaris), haematologic (autoimmune haemolytic anaemia), neural (multiple sclerosis) or can involve virtually any circumscribed mass of body tissue. The autoimmune diseases to be treated may be selected from the group consisting of type I autoimmune diseases or type II autoimmune diseases or type III autoimmune diseases or type IV autoimmune diseases, such as, for example, multiple sclerosis (MS), rheumatoid arthritis, diabetes, type I diabetes (Diabetes mellitus Type 1), chronic polyarthritis, Basedow's disease, autoimmune forms of chronic hepatitis, colitis ulcerosa, type I allergy diseases, type II allergy diseases, type III allergy diseases, type IV allergy diseases, fibromyalgia, hair loss, Bechterew's disease, Crohn's disease, Myasthenia gravis, neurodermitis, Polymyalgia rheumatica, progressive systemic sclerosis (PSS), Reiter's syndrome, rheumatic arthritis, psoriasis, vasculitis, etc, or type II diabetes. While the exact mode as to why the immune system induces an immune reaction against autoantigens has not been elucidated so far, there are several findings with regard to the etiology. Accordingly, the autoreaction may be due to a T-Cell bypass. A normal immune system requires the activation of B-cells by T-cells before the former can produce antibodies in large quantities. This requirement of a T-cell can be bypassed in rare instances, such as infection by organisms producing super-antigens, which are capable of initiating polyclonal activation of B-cells, or even of T-cells, by directly binding to the ß-subunit of T-cell receptors in a non-specific fashion. Another explanation deduces autoimmune diseases from a Molecular Mimicry. An exogenous antigen may share structural similarities with certain host antigens; thus, any antibody produced against this antigen (which mimics the self-antigens) can also, in theory, bind to the host antigens and amplify the immune response. The most striking form of molecular mimicry is observed in Group A beta-haemolytic streptococci, which shares antigens with human myocardium, and is responsible for the cardiac manifestations of rheumatic fever.

The inventive transporter cargo conjugate molecule, the inventive pharmaceutical composition or the inventive vaccine, may also be used for (the preparation of a medicament for) the prophylaxis, treatment, and/or amelioration of cardiovascular diseases, preferably selected from heart diseases and coronary heart diseases, arteriosclerosis, apoplexy, dilatation of the abdominal aorta, such as infrarenal aneurism hypertension, and myocardial infarction.

Additionally, the inventive transporter cargo conjugate molecule, the inventive pharmaceutical composition or the inventive vaccine, may be used for (the preparation of a medicament for) the prophylaxis, treatment, and/or amelioration of neuronal or neurodegenerative diseases selected from, without being limited thereto, Alzheimer's disease, Parkinson's disease, amyotrophic lateral sclerosis (ALS), dystonia, epilepsy, optic nerve disease, including glaucoma, eye infection, multiple sclerosis, meningitis, neuronal diseases caused by or disorders or diseases or disorders of the nervous system, including the "cutting" or disruption of axons, such as axotomy, pain, particularly neuropathic pain, stroke, including ischemic stroke, and viral encephalopathy.

The inventive transporter cargo conjugate molecule, the inventive pharmaceutical composition or the inventive vaccine, may also be used for (the preparation of a medicament for) the prophylaxis, treatment, and/or amelioration of diseases of the liver selected from, without being limited thereto, Hepatitis, and hepatotoxicity.

Additionally, the inventive transporter cargo conjugate molecule, the inventive pharmaceutical composition or the inventive vaccine, may be used for (the preparation of a medicament for) the prophylaxis, treatment, and/or amelioration of diseases of the spine, selected from, without being limited thereto, disc herniation.

According to one preferred embodiment, the inventive transporter cargo conjugate molecule, the inventive pharmaceutical composition or the inventive vaccine, may be used for (the preparation of a medicament for) the prophylaxis, treatment, and/or amelioration of diseases of the uterus selected from, without being limited thereto, endometriosis.

According to another preferred embodiment, the inventive transporter cargo conjugate molecule, the inventive pharmaceutical composition or the inventive vaccine, may be used for (the preparation of a medicament for) the prophylaxis, treatment, and/or amelioration of major depressive disorders, also known as major depression, unipolar depression, clinical depression, or simply depression.

According to a further preferred embodiment,, the inventive transporter cargo conjugate molecule, the inventive pharmaceutical composition or the inventive vaccine, may be used for (the preparation of a medicament for) the prophylaxis, treatment, and/or amelioration of non-chronic or chronic inflammatory digestive diseases in a subject. The term "non-chronic or chronic inflammatory digestive disease" as used herein typically denotes non-chronic or chronic inflammatory diseases that pertain to the gastrointestinal tract. This includes diseases of the esophagus, stomach, first, second, third and fourth part of the duodenum, jejunum, ileum, the ileo-cecal complex, large intestine, (ascending, transverse and descending colon) sigmoid colon and rectum. Preferably included in this respect are chronic inflammatory digestive diseases, which are characterized by an inflammation of the colon, such as colitis, including e.g. Colitis ulcerosa (ulcerative colitis), Morbus Crohn (Crohn's disease), diversion colitis, ischemic colitis, infectious colitis, fulminant colitis, chemical colitis, microscopic colitis, lymphocytic colitis, collageneous colitis, indeterminate colitis and atypical colitis, etc.

In the context of the above, the invention relates also to the use of the inventive transporter cargo conjugate molecule, the inventive pharmaceutical composition or the inventive vaccine, for the prophylaxis, treatment, and/or amelioration of diseases or disorders as mentioned herein. It also includes in particular the use of the inventive transporter cargo conjugate molecule, the inventive pharmaceutical composition or the inventive vaccine, for inoculation or the use of these components as an inoculant. A method for prophylaxis, treatment, and/or amelioration of the above-mentioned diseases or disorders, or an inoculation method for preventing the above-mentioned diseases, typically comprises administering the described inventive transporter cargo conjugate molecule, pharmaceutical composition or vaccine to a patient in need thereof (e.g. suffering from any of the above diseases or showing symptoms thereof), in particular to a human being, preferably in a "safe and effective amount" and in one of the above formulations as described above. The administration mode also may be as described above for inventive pharmaceutical compositions or vaccines.

According to a fifth aspect of the present invention, the inventive pharmaceutical composition, the inventive vaccine, the inventive transporter cargo conjugate molecule as defined above, may be utilized as a medicament. Such a medicament may be a pharmaceutical composition or a vaccine as shown above. It may be utilized in medical applications in general, preferably for any of the prophylaxis, treatment, and/or amelioration of diseases or disorders as mentioned herein.

According to a sixth aspect of the present invention, the inventive transporter cargo conjugate molecule as described above and preferably the inventive transporter construct as described above according to generic formula (I) or according to any of subformulas (Ia), (Ib), (Ic), (Id), (Ie), or (If) above may be utilized for the transport of any cargo molecule (preferably as defined herein) into cells or tissue of a patient to be treated. In this context, the cargo molecule is preferably suitable for a therapy as mentioned herein, particularly for the prophylaxis, treatment, and/or amelioration of diseases or disorders as mentioned herein and may be selected from any cargo molecule suitable therefore, more preferably from any cargo molecule as described above for any of components (B), (C), (D) and/or (E) of the inventive transporter cargo conjugate molecule. Thereby, the inventive transporter construct as defined above according to generic formula (I) or according to any of subformulas (Ia), (Ib), (Ic), (Id), (Ie), or (If) above may be coupled to such a cargo molecule using any of the coupling methods described for components (A), (B), (C), (D) and/or (E) above.

According to a particularly preferred embodiment of this aspect, the inventive transporter cargo conjugate molecule and more preferably the inventive transporter construct as defined above according to generic formula (I) or according to any of subformulas (Ia), (Ib), (Ic), (Id), (Ie), or (If) above, may be utilized for the transport of any cargo molecule as mentioned herein, e.g. as described above for any of components (B), (C), (D) and/or (E), into cells, preferably into blood cells, more preferably into white blood cells or preferably into neuronal cells. The transport may be effected in vitro, in vivo and/or ex vivo. Preferably, the transport is effected into the respective cells of a patient to be treated. Such a direction of the inventive transporter cargo conjugate molecule and more preferably of the inventive transporter construct as defined above according to generic formula (I) or according to any of subformulas (Ia), (Ib), (Ic), (Id), (Ie), or (If) conjugated to a cargo as mentioned above is particularly suitable in the treatment of inflammatory diseases as mentioned above. For this purpose, the inventive transporter cargo conjugate molecule and more preferably the inventive transporter construct as defined above according to generic formula (I) or according to any of subformulas (Ia), (Ib), (Ic), (Id), (Ie), or (If) above, may be administered as described above for an inventive pharmaceutical composition or an inventive vaccine. Furthermore, the inventive transporter cargo conjugate molecule and more preferably the inventive transporter construct as defined above according to generic formula (I) or according to any of subformulas (Ia), (Ib), (Ic), (Id), (Ie), or (If) above, may be formulated as an inventive pharmaceutical composition or an inventive vaccine as described above for the purpose of administration. Administration routes are as defined above for an inventive pharmaceutical composition or an inventive vaccine. Alternatively, the inventive transporter cargo conjugate molecule and more preferably the inventive transporter construct as defined above according to generic formula (I) or according to any of subformulas (Ia), (Ib), (Ic), (Id), (Ie), or (If) above, may be administered directly without any further formulation, i.e. "naked". Likewise, administration routes are preferably as described above for such a formulation.

According to a seventh aspect of the present invention, the inventive transporter construct as defined above according to generic formula (I) or according to any of subformulas (Ia), (Ib), (Ic), (Id), (Ie), or (If) above, the inventive transporter cargo conjugate molecule as defined above, the inventive pharmaceutical composition or the inventive vaccine may be utilized in diagnosis as a diagnostic tool, e.g. in (*in vivo* or *in vitro*) assays, e.g. in immunoassays, to detect, prognose, diagnose, or monitor various conditions and disease states of disorders or diseases mentioned.

As an example, immunoassay may be performed by a method comprising contacting a sample derived from a patient with an inventive transporter cargo conjugate molecule as defined above, wherein component (B) and/or any of components (C), (D) and/or (E) of the inventive transporter cargo conjugate molecule may be directed against a component or compound, e.g. a a (cell) specific component or compound, contained in the sample. Such a component (B) or any of components (C), (D) and/or (E) of the inventive transporter cargo conjugate molecule may be e.g. an antibody directed to a (cell) specific component or compound of the sample, wherein such (cell) specific component or compound of the sample may be e.g. a compound or component as described above for any of components (B), (C), (D) and/or (E) as defined above. Contacting of the sample is typically carried out under conditions that immunospecific-binding may occur, and subsequently detecting or measuring the amount of any immunospecific-binding by the antibody. In a specific embodiment, an antibody specific for a (cell) specific component or compound of the sample, e.g. component (B), (C), (D) and/or (E) as defined above, may be used to analyze a tissue or serum sample from a patient for the presence of such a component (B), (C), (D) and/or (E) as defined above or a disease associated therewith. Such diseases may include diseases or disorders as described herein. The immunoassays that may be utilized include, but are not limited to, competitive and non-competitive assay systems using techniques such as Western Blots, radioimmunoassays (RIA), enzyme linked immunosorbent assay (ELISA), "sandwich" immunoassays, immunoprecipitation assays, precipitin reactions, gel diffusion precipitin reactions, immunodiffusion assays, agglutination assays, fluorescent immunoassays, complement-fixation assays, immunoradiometric assays, and protein-A immunoassays, etc.

Alternatively, *(in vitro)* assays may be performed by delivering the inventive pharmaceutical composition, a vaccine or the inventive transporter cargo conjugate molecule as defined above or variants or fragments thereof within the above definitions to target cells typically selected from e.g. cultured animal cells, human cells or micro-organisms, and to monitor the cell response by biophysical methods typically known to a skilled person. The target cells typically used therein may be cultured cells (*in vitro*) or *in vivo cells,* i.e. cells composing the organs or tissues of living animals or humans, or microorganisms found in living animals or humans. Particularly preferable in this context are so called markes or labels, which may be contained as a component (B) or any of components (C), (D) and/or (E) of the inventive transporter cargo conjugate molecule, wherein such labels may be as defined in general above for the inventive transporter cargo conjugate molecule, e.g.

According to a final aspect of the present invention, the present invention also provides kits, particularly kits of parts, comprising as components alone or in combination, the inventive transporter construct as defined above according to generic formula (I) or according to any of subformulas (Ia), (lb), (Ic), (Id), (Ie), or (If) above, the inventive transporter cargo conjugate molecule, the inventive pharmaceutical composition and/or the inventive vaccine, and optionally technical instructions with information on the administration and dosage of these components. Such kits, preferably kits of parts, may be applied, e.g., for or in any of the above mentioned applications or uses. The present invention additionally particularly provides the use of kits for diagnostic or therapeutic purposes, particular for the treatment, prevention or monitoring of diseases or disorders as disclosed.

Unless otherwise defined, all technical and scientific terms used herein have the same meaning as commonly understood by one of ordinary skill in the art to which this invention belongs. Although methods and materials similar or equivalent to those described herein can be used in the practice or testing of the present invention, suitable methods and materials are described below. In the case of conflict, the present specification, including definitions, will control. In addition, the materials, methods, and examples are illustrative only and not intended to be limiting. Other features and advantages of the invention will be apparent from the following detailed description and claims.

### Figures

The following Figures are intended to illustrate the invention further. They are not intended to limit the subject matter of the invention thereto.

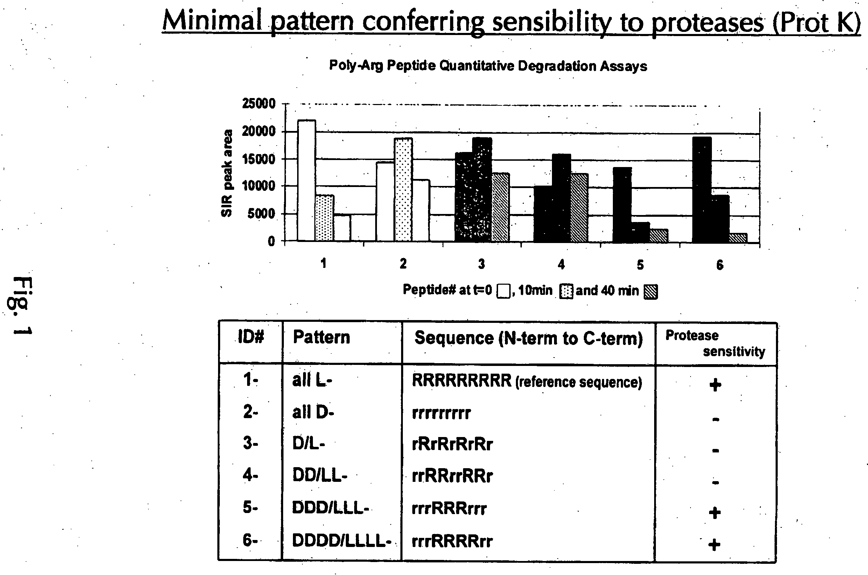

- **Figure 1:**: depicts the results of a quantitative degradation assay with Proteinase K involving several transporter constructs with poly-Arg sequences, each showing a different pattern of D- and L- amino acids (D-/L-pattern). The transporter constructs used in this assay were termed 1- to 6-. The different D-/L-pattern of the sequences is described using capitals and minor letters. The capitals in these sequences ("R"-amino acids) refer to L-enatiomeric arginine (L-Arg) and the minor letters in these sequences ("r"-amino acids) refer to D-enatiomeric arginine (L-Arg). The sensitivity to Proteinase K was measured at time intervals t = 0, 10 and 40 minutes. The results of the quantitative degradation assay with Proteinase K were then determined on the basis of samples taken at these specific time intervals. These samples were analyzed utilizing a mass spectrometry analysis using starting material ionintensities as reference values. As a conclusion, when a stretch of 3 or more L- Arg is present in the sequence the peptide shows sensitivity to Proteinase K and is degraded. Peptides with 2 or less L-Arg next to each other are not degraded and results are comparable to full-D sequence or D-TAT (SEO ID NO: 251), respectively D-JNKi.

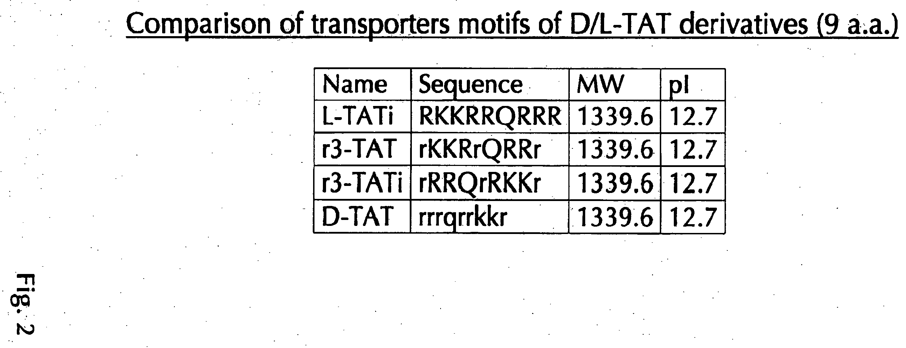

- **Figure 2:**: shows in a table a comparison of four different transporter constructs of D-/L-TAT derivatives (termed L-TAT (SEQ ID NO: 18), r3-TAT (also termed r3-L-Tat; SEQ ID NO: 20), r3-TATi (also termed r3-L-TATi: SEQ ID NO: 21), and D-TAT; SEQ ID NO: 251), each having a length of 9 amino acids but a different D-/L-pattern. The different D-/L-pattern of the sequences is described using capitals and minor letters. The capitals in the sequences ("R"-amino acids) refer to L-enatiomeric arginine (L-Arg) and the minor letters in these sequences ("r"-amino acids) refer to D-enatiomeric arginine (L-Arg). The table shown in Figure. 2 illustrates the amino acid sequences of these TAT derived transporter constructs with respect to their molecular weight (M_{w}) and pl-values.

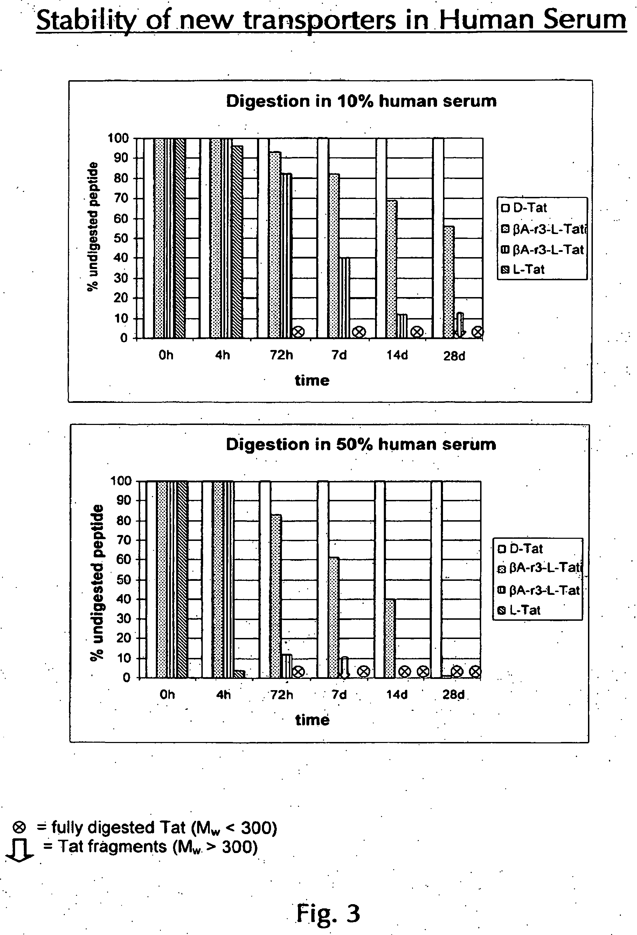

- Figure 3:: shows the results of a digestion of TAT derived transporter constructs in 10% and 50% human serum at 37°C until complete degradation of these TAT derived transporters. The TAT-derived transporter constructs are as described in Figure 2, wherein the transporter constructs r3-TAT (also termed r3-L-Tat; SEQ ID NO: 20), r3-TATi (also termed r3-L-TATi; SEQ ID NO: 21) were additionally protected N-terminally with a beta-Alanine. As can be seen in Figure 3, D-TAT transporter constructs are protease resistant, whereas L-TAT transporter constructs are degraded too early *in vivo* in order to effect an efficient transport into cells. Only transporter constructs r3-TAT (also termed r3-L-Tat; SEQ ID NO: 20), r3-TATi (also termed r3-L-TATi, SEQ ID NO: 21) show a degradation within a suitable time limit, allowing to limit the *in vivo* stability for therapeutic applications.

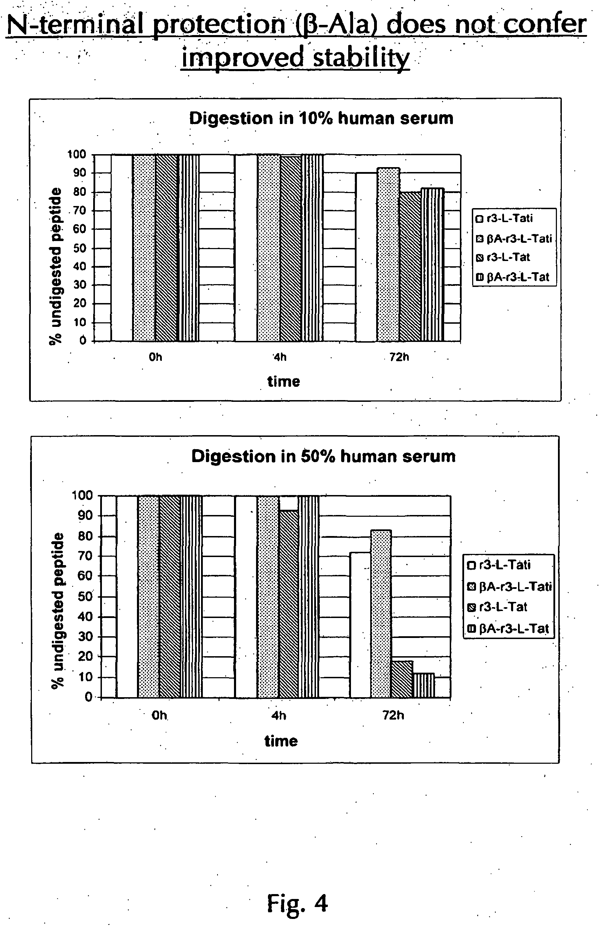

- Figure 4:: shows the results of a digestion of TAT derived transporter constructs in 10% and 50% human serum at 37°C until complete degradation of these TAT derived transporters under involvement of beta-Alanine (b-Ala) in protecting the peptides. Therefore, beta-Ala was added to the N-terminus of TAT derived transporter constructs L-TAT as L-TATi as already described in Figure 3. As can be seen in Figure 4 N-terminal protection of the transporter peptides with beta-Alanine does not lead to a significant effect, i.e. an improved stability.

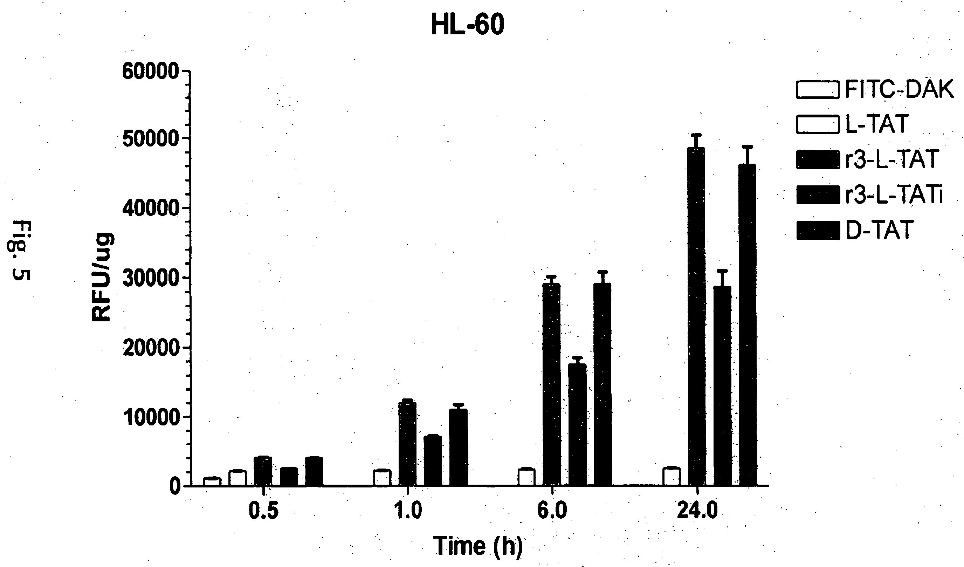

- Figure 5:: depicts the results of the time dependant internalization (uptake) of FITC-labeled TAT derived transporter constructs into cells of the HL-60 cell line. HL-60 cells were incubated 30 min, 1, 6 or 24 hours with 10µM of the TAT-derivative transporters. The cells were then washed twice with an acidic buffer (0.2 M Glycin, 0.15 M NaCl, pH 3.0) and twice with PBS. Cells were broken by the addition of RIPA lysis buffer. The relative amount of internalized peptide was then determined by reading the fluorescence intensity (Fusion Alpha plate reader; PerkinElmer) of each extract followed by background substraction and protein content normalization. The r3-L-TAT transporter construct (SEQ ID NO: 20) showed an internalization capability as effective as the D-TAT transporter construct. The r3-L-TATi transporter construct (SEQ ID NO: 21), which internalized in a time dependent manner, as both previous transporters, seems to be less efficient but still suitable, whereas L-TAT (SEQ ID NO: 18) doesn't accumulate over a period of 24 hours.

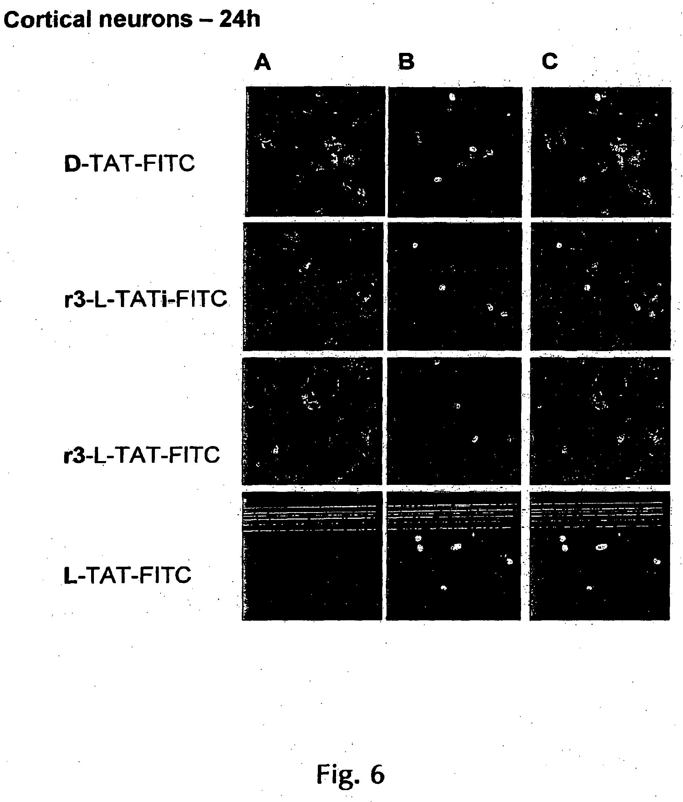

- **Figure 6:**: shows results of a confocal microscopy of of cells treated with fluorescently labeled TAT derivative transporters. The dissociated cortical primary neurons from P2 Sprague Dawley rats were cultured 12 days in neurobasal medium before exposure 24hours to 500nM of the FITC-labeled TAT derivative transporters. The cells were washed five times with PBS on ice and then mounted in fluorsave mounting medium without prior fixation. Acquisitions were performed on LSM510 metaconfocal microscope (Zeiss). Images were processed with LSM510 software and mounted using Adobe photoshop. Visualization by confocal microscopy of labeling with 500nM FITC-transporters (A: green). Nuclei were stained by Hoechst (B: blue). The r3-L-TAT (SEQ ID NO: 20) as well as the D-TAT (SEQ ID NO: 251) and the r3-L-TATi (SEQ ID NO: 21) transporter constructs were internalized into the cytoplasm of the non stressed neurons (C: Merge panel). However, after 24 hours incubation, the L-TAT transporter (SEQ ID NO: 18) was not present anymore.

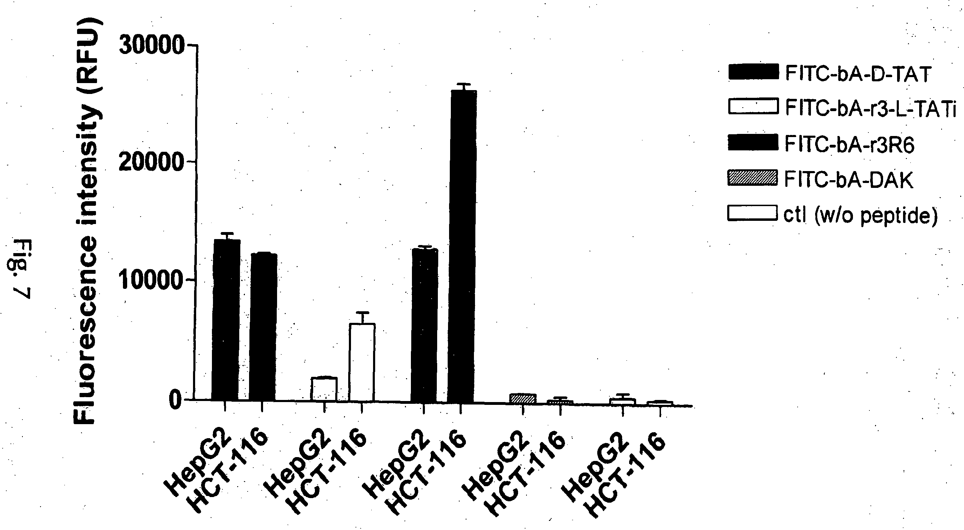

- **Figure 7:**: illustrates the uptake (internalization) of FITC-labeled TAT derived transporter constructs *in vitro* (10 µM, HepG2 hepatocarcinoma, HCT-116 tumoral colon, 24 h). The constructs used were different TAT derived transporter constructs termed D-TAT (SEQ ID NO: 251) and r3-TATi (also termed r3-L-TATi, SEQ ID NO: 21), each having a length of 9 amino acids but a different D-/L-pattern, and the test constructs r₆R₃ (SEQ ID NO: 260) and DAK, wherein the constructs additionally have been labeled with beta-Alanine at their N-terminus. As can be seen, uptake was most efficient for constructs D-TAT (SEQ ID NO: 251) and r₆R₃ (SEQ ID NO: 260), followed by r3-L-TATi (SEQ ID NO: 21).

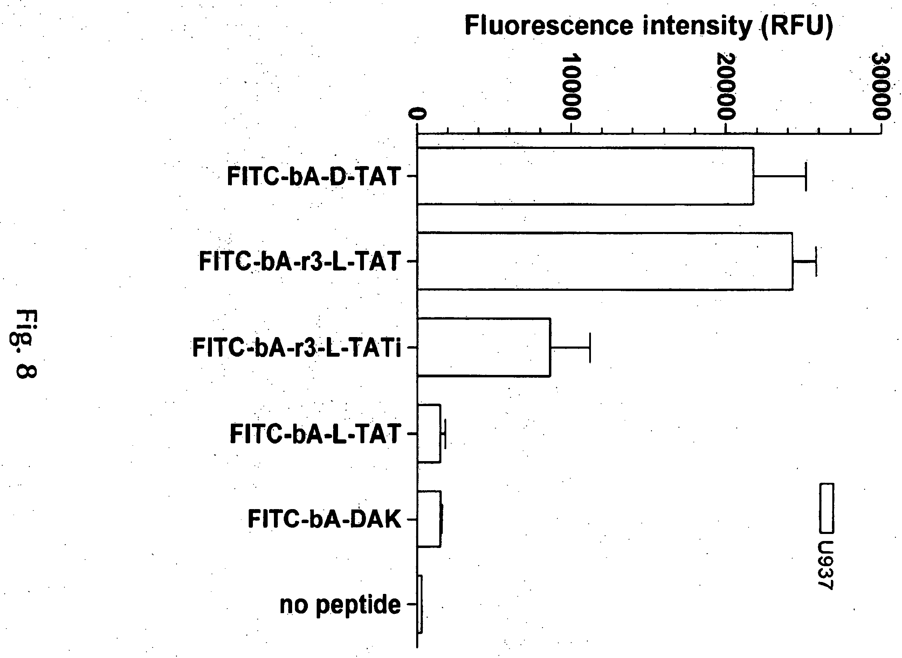

- **Figure 8:**: shows the uptake (internalization) of FITC-labeled TAT derived transporter constructs *in vitro* (10 µM, U937, Lymphoma, 24 h). The constructs used were four different TAT derived transporter constructs (termed L-TAT, SEQ ID NO: 18), r3-TAT (also termed r3-L-Tat, SEQ ID NO: 20), r3-TATi (also termed r3-L-TATi, SEQ ID NO: 21), and D-TAT, SEQ ID NO: 251), each having a length of 9 amino acids but a different D-/L-pattern. Additionally, the construct DAK was used for comparison and a control sample, containing only the amino acids D, A and K. As can be seen, the uptake of r3-TAT (SEQ ID NO: 20), r3-TATi (SEQ ID NO: 21) and D-TAT (SEQ ID NO: 251) transporter constructs into the cells was most efficient, wherein L-TAT (SEQ ID NO: 18) showed a signifivantly lower uptake into the cells.

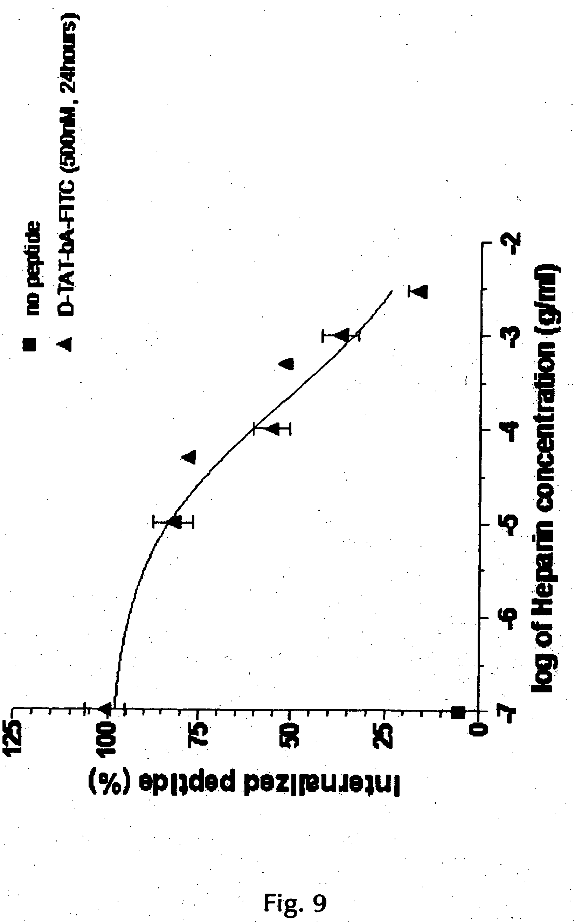

- **Figure 9:**: shows that the uptake (internalization) of the D-TAT transporter construct is HSPG-dependent at a concentration of 500 nM over 24 hours in U937 cells, Lymphoma. The construct used for the experiment was D-TAT (SEQ ID NO: 251), having a length of 9 amino acids and being labeled with FITC and at its N-terminus with beta-Alanine.

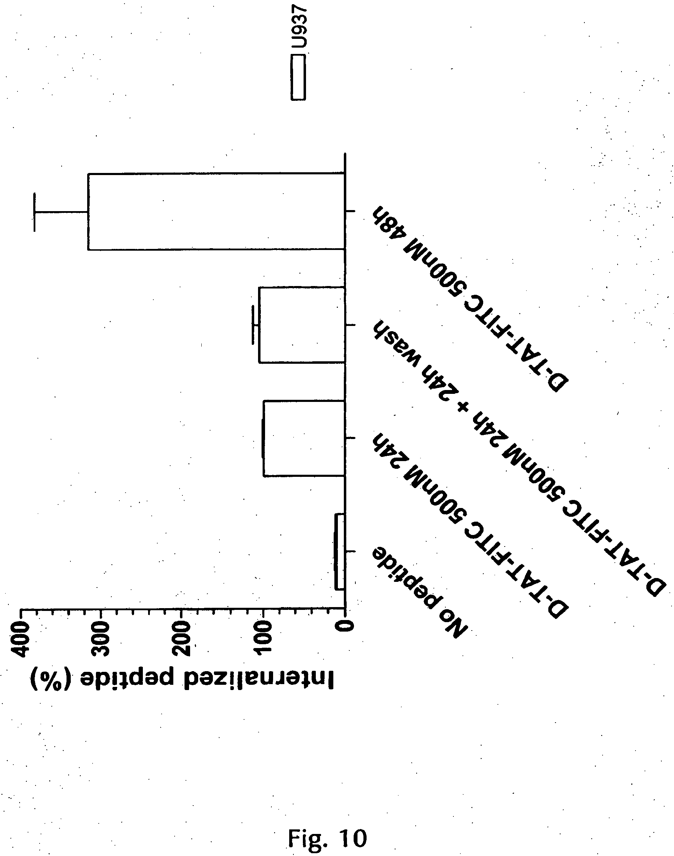

- **Figure 10:**: shows that an exit of the FITC-labeled TAT derived transporter constructs is not observed in U937 cells at 500 nM FITC-D-TAT. The construct used for the experiment was D-TAT (SEQ ID NO: 251), having a length of 9 amino acids and being labeled with FITC.

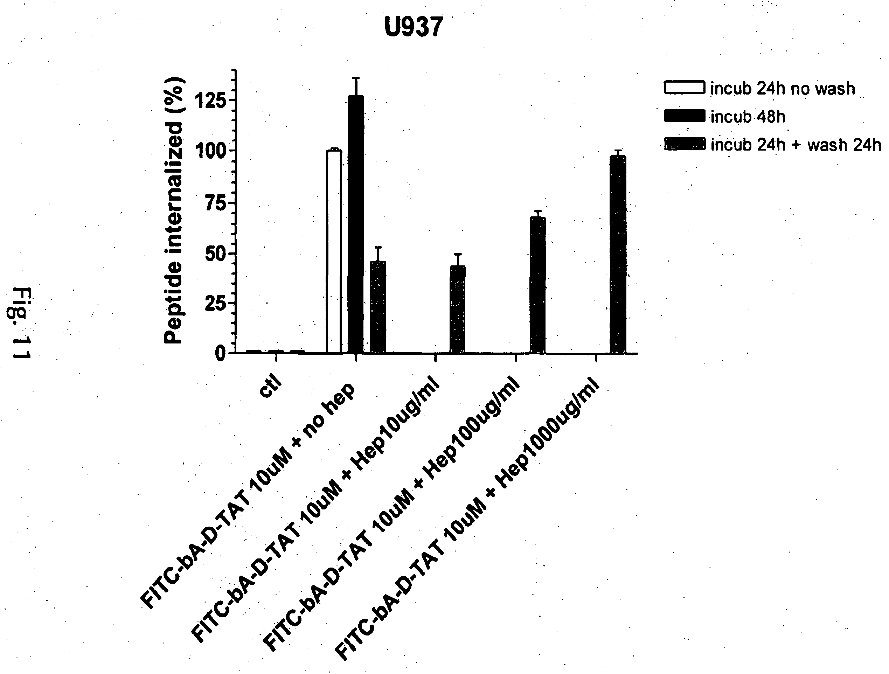

- **Figure 11:**: shows that an exit of the FITC-labeled TAT derived transporter constructs is observed at 10 µM FITC-D-TAT, and is HSPG-dependent (U937, lymphoma). The construct used for the experiment was D-TAT (SEQ ID NO: 251), having a length of 9 amino acids and being labeled with FITC and at its N-terminus with beta-Alanine.

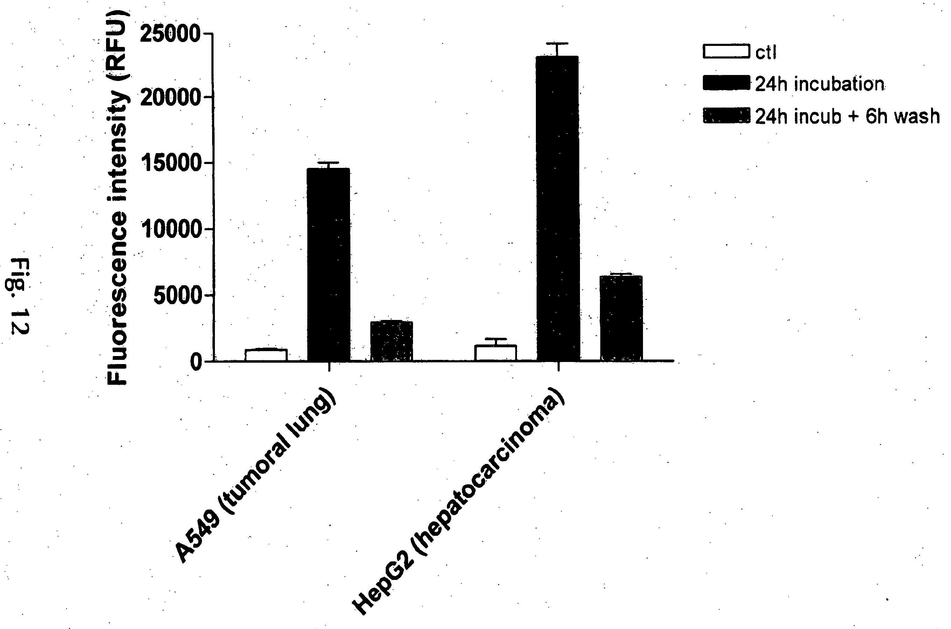

- **Figure 12:**: shows that an uptake (internalization) and an exit of the FITC-labeled TAT derived transporter constructs is observed at 10 µM FITC-D-TAT in non WBC-lines (white blood cells lines). The construct used for the experiment was D-TAT (SEQ ID NO: 251), having a length of 9 amino acids and being labeled with FITC.

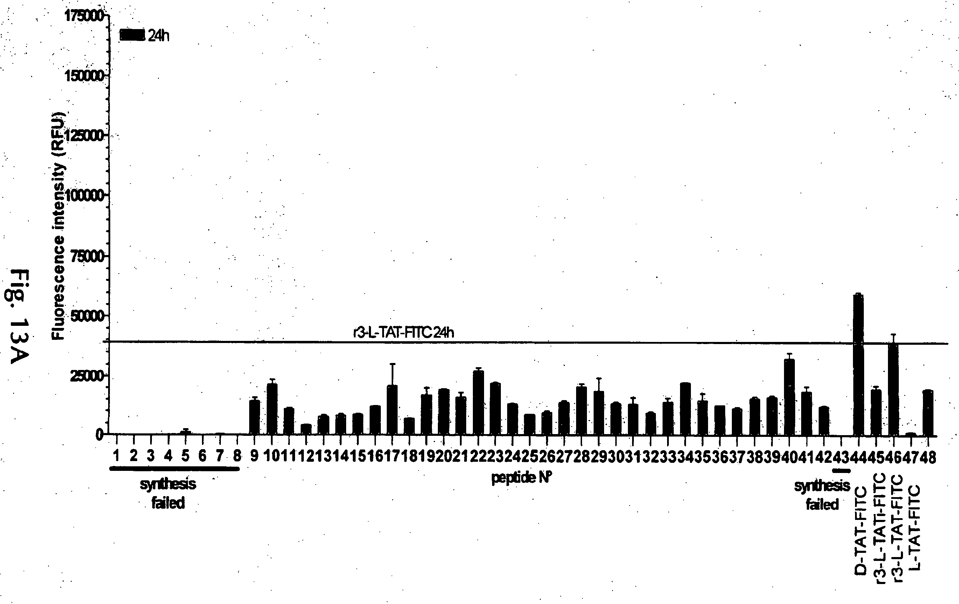

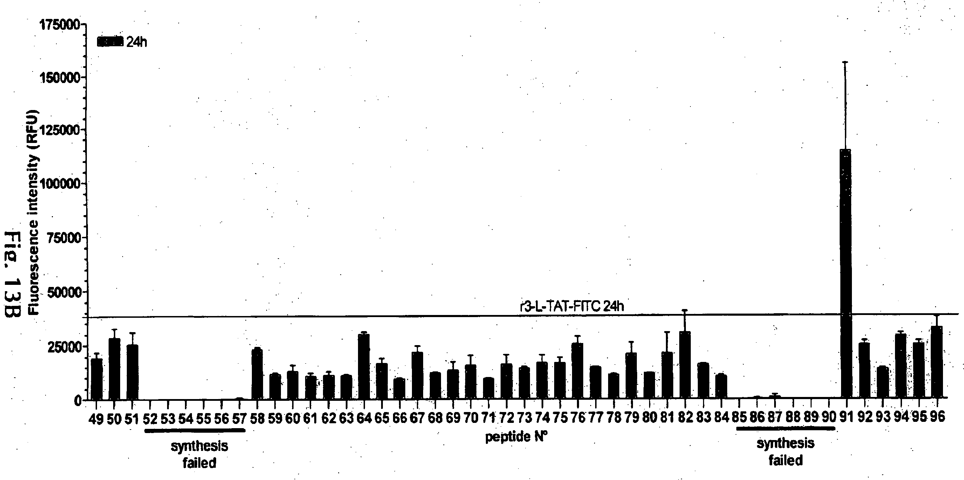

- **Figure 13:**: shows internalizations experiments using TAT derived transporter constructs of general formula (I). As can be seen in Figure 13, after 24 hours incubation, all transporters with the consensus sequence rXXXrXXXr (SEQ ID NO: 252; see above for a selection of potential sequences) showed a higher internalization capability than the L-TAT transporter (SEQ ID NO: 18). Hela cells were incubated 24 hours in 96well plate with 10mM of the r3-L-TAT-derived transporters. The cells were then washed twice with an acidic buffer (0.2M Glycin, 0.15M NaCl, pH 3.0) and twice with PBS. Cells were broken by the addition of RIPA lysis buffer. The relative amount of internalized peptide was then determined by reading the fluorescence intensity (Fusion Alpha plate reader; PerkinElmer) of each extract followed by background substraction.

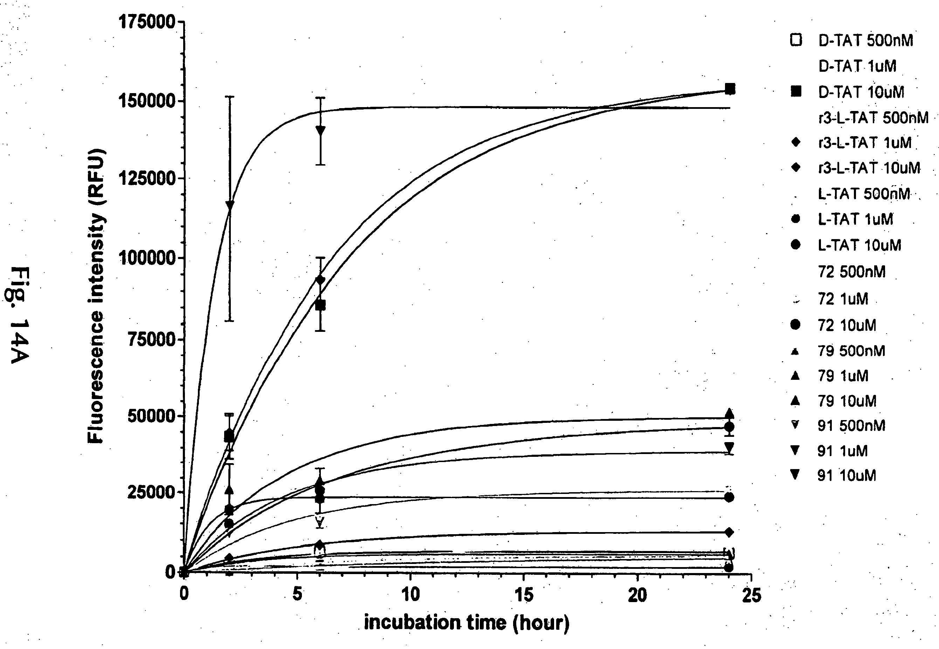

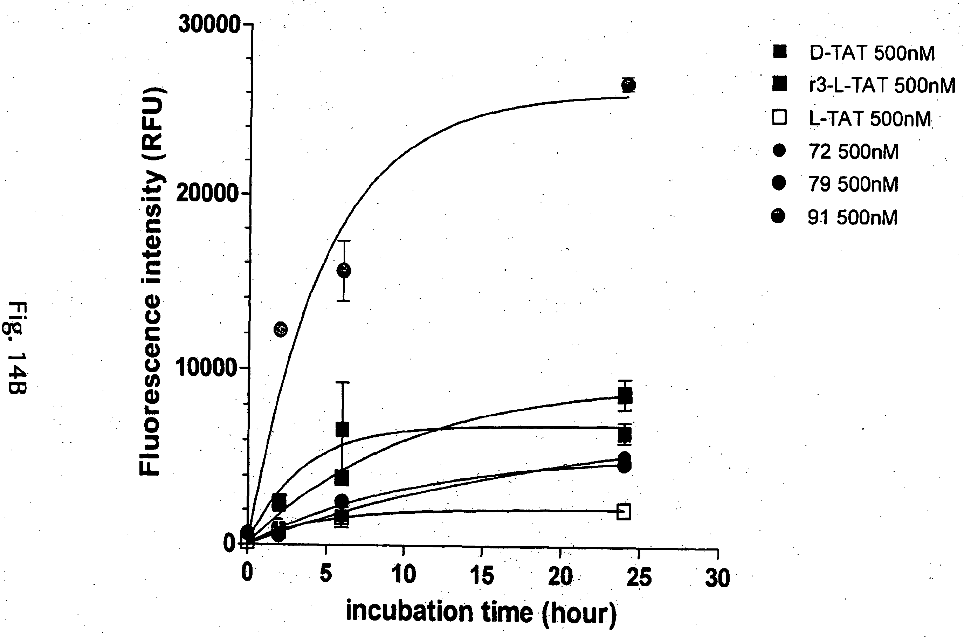

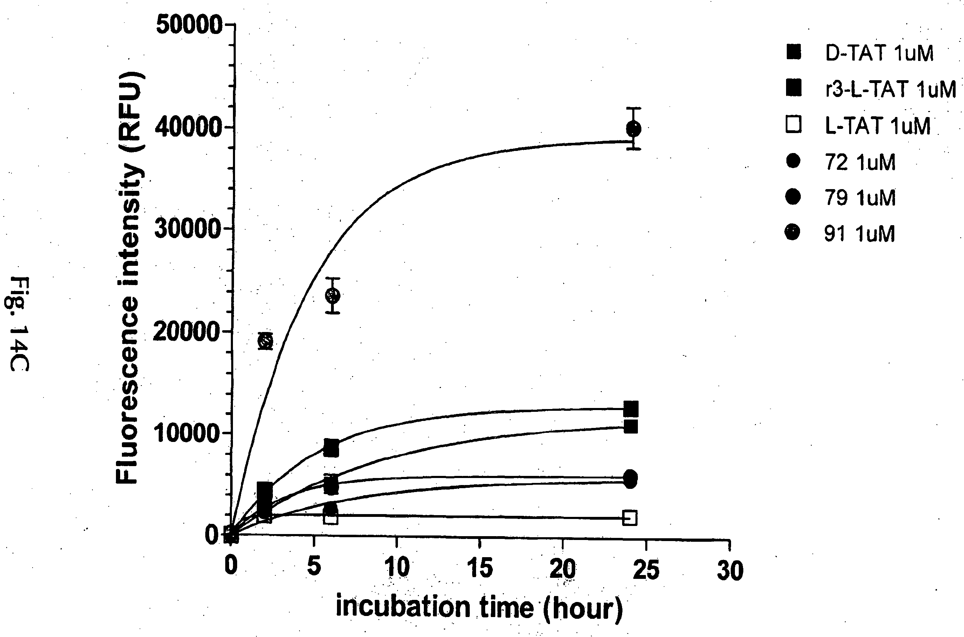

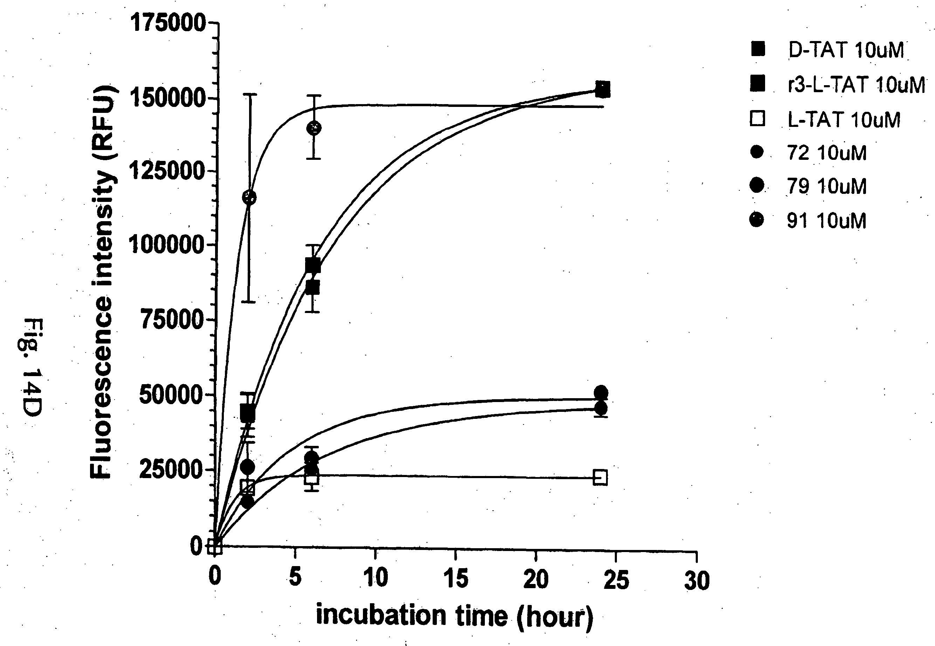

- **Figure 14:**: shows internalizations experiments using TAT derived transporter constructs of general formula (I). As can be seen in Figure 14, one position appears to be critical for highest transporter activity and for improved kinetics of transport activity: Y in position 2 (peptide N°91 corresponding to SEQ ID NO: 116). Briefly, Hela cells were incubated 2, 6 or 24 hours in 24well plate with increasing dose of the r3-L-TAT-derivative transporters (0, 500nM, 1mM or 10mM). The cells were then washed twice with an acidic buffer (0.2M Glycin, 0.15M NaCl, pH 3.0) and twice with PBS. Cells were broken by the addition of RIPA lysis buffer. The relative amount of internalized peptide was then determined by reading the fluorescence intensity (Fusion Alpha plate reader; PerkinElmer) of each extract followed by background substation.

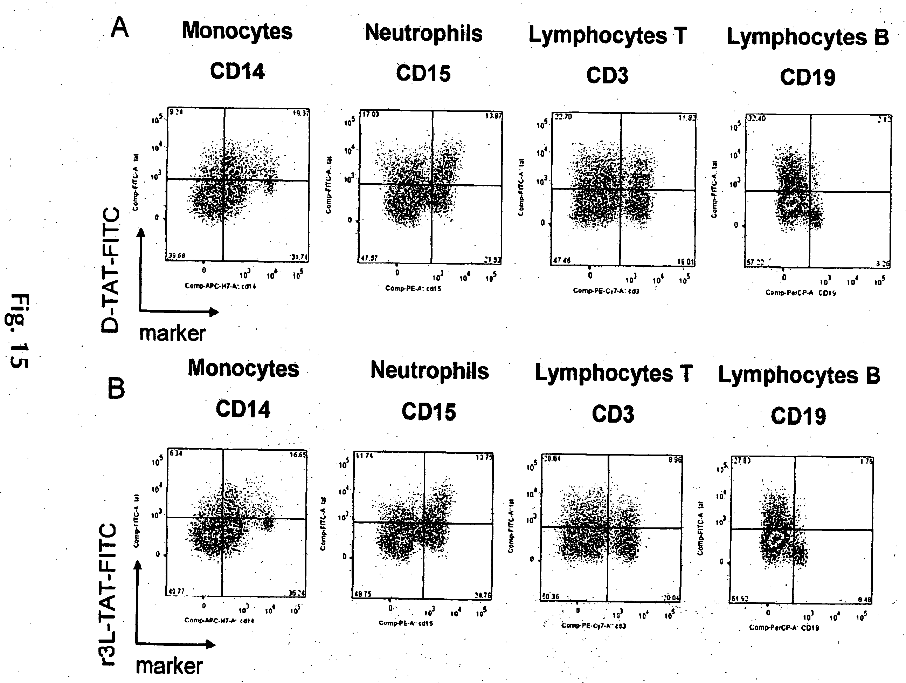

- **Figure 15:**: Fluorescent TAT derivative transporters D-TAT (SEQ ID NO: 251)-FITC or r3-L-TAT (SEQ ID NO: 20)-FITC target different human leukocyte populations.
A: D-TAT (SEQ ID NO: 251)-FITC. The percentage of cells gated in the respective quadrants is as follows (given clockwise beginning with upper left quadrant):
   Monocytes (CD14) 9,24; 19,37; 31,71; 39,68;
   Neutrophils (CD15) 17,03; 13,87; 21,53; 47,57;
   Lymphocytes T (CD3) 22,7; 11,82; 18,01; 47,46;
   Lymphocytes B (CD19) 32,40; 2,12; 8,26; 57,22.
B: r3- L-TAT (SEQ ID NO: 20)-FITC. The percentage of cells gated in the respective quadrants is as follows (given clockwise beginning with upper left quadrant):
   Monocytes (CD14) 6,34; 16,65; 36,24; 40,77;
   Neutrophils (CD15) 11,74; 13,75; 24,76; 49,75;
   Lymphocytes T (CD3) 20,64; 8,96; 20,04; 50,36;
   Lymphocytes B (CD19) 27,83; 1,76; 8,48; 61,92.

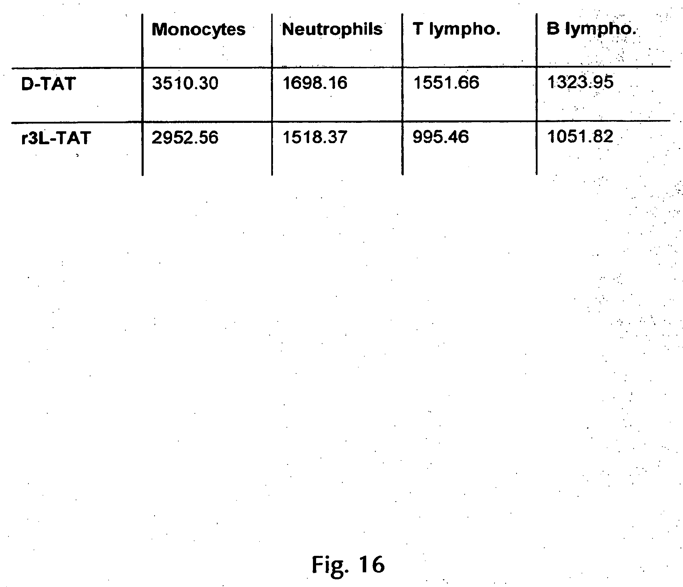

- **Figure 16:**: The table indicates the mean fluorescence values for fluorescent TAT derivative transporters D-TAT (SEQ ID NO: 251)-FITC or r3- L-TAT (SEQ ID NO: 20)-FITC in each cell type as shown in FIG. 15 (FITC channel).

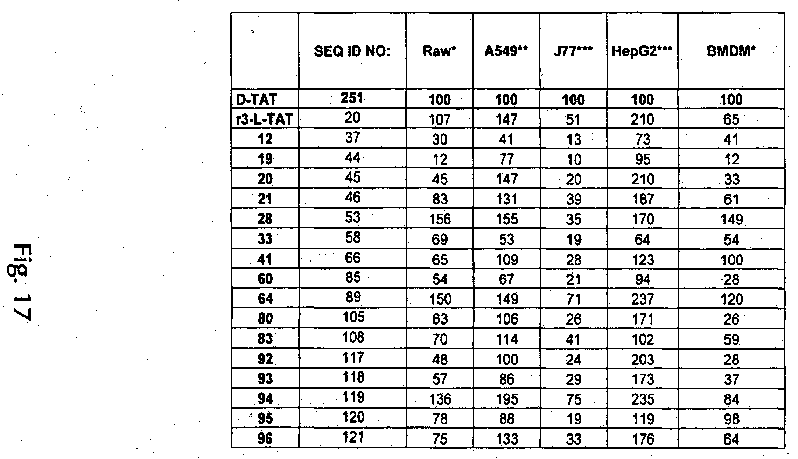

- **Figure 17:**: Uptake of selected transporter constructs according to the present invention by different cell types. Uptake is normalized versus D-TAT (SEQ ID NO: 251). HepG2: Hepatocarcinoma cells (human; non leucocyte cell line); A549: Lung epithelial cells (human; non leucocyte cell line); Raw: Macrophage cells (mouse; leucocyte cell line); J77: Macrophage cells (mouse; leucocyte cell line); BMDM: Bone Marrow-Derived Macrophages (mouse; purified primary leucocytes). *n=2 independent experiments (in duplicate) (except for peptide #64 n=1 in duplicate); **n=2 experiment (in duplicate) (except for peptide #64 n=2 in duplicate); ***n=1 experiment (in duplicate).

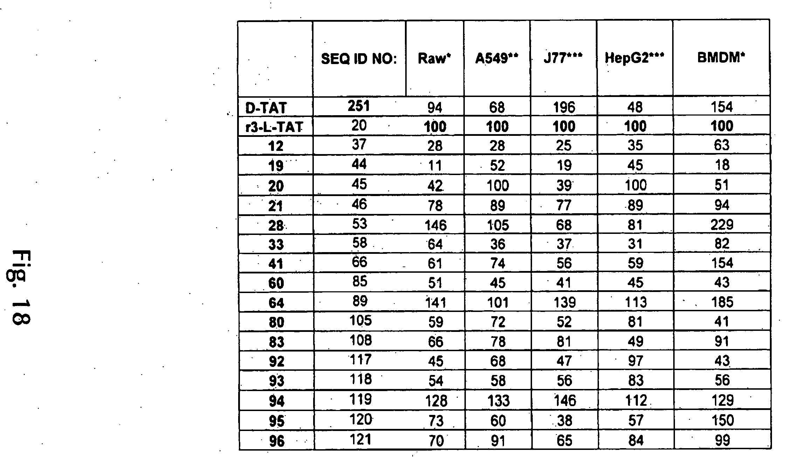

- **Figure 18:**: Uptake of selected transporter constructs according to the present invention by different cell types. Uptake is normalized versus r₃-L-TAT (SEQ ID NO: 20). HepG2: Hepatocarcinoma cells (human; non leucocyte cell line); A549: Lung epithelial cells (human; non leucocyte cell line); Raw: Macrophage cells (mouse; leucocyte cell line); J77: Macrophage cells (mouse; leucocyte cell line); BMDM: Bone Marrow-Derived Macrophages (mouse; purified primary leucocytes). *n=2 independent experiments (in duplicate) (except for peptide #64 n=1 in duplicate); **n=2 experiment (in duplicate) (except for peptide #64 n=2 in duplicate); ***n=1 experiment (in duplicate).

### Examples:

The following examples are intended to illustrate the invention further. They are not intended to limit the subject matter of the invention thereto.

### 1. Preparation of (peptidic) transporter constructs

The (peptidic) transporter constructs used in these examples were prepared using solid phase synthesis as described above. The constructs used for identification of the minimal D-/L-pattern of transporter constructs, which is sensitive to proteases were, inter alia, as follows:

| SEQ ID No. | D-/L-Pattern | Sequence (N-term to C-term) |
|---|---|---|
| 123 | all L | RRRRRRRRR (reference sequence) |
| 257 | all D | rrrrrrrrr |
| 258 | D/L | rRrRrRrRr |
| 259 | DD/LL | rrRRrrRRr |
| 260 | DDD/LLL (r₆R₃) | rrrRRRrrr |
| 261 | DDDD/LLLL | rrrRRRRrr |

The constructs used for uptake (internalization) experiments into various cells and cell lines were as follows:

| Name | Sequence (N-term to C-term) |
|---|---|
| FITC-βA-L-TAT | FITC-βA-RKKRQRRR |
| FITC-βA-D-TAT | FITC-βA-rrrqrrkkr |
| FITC-βA-r₃-L-TAT | FITC-βA-rKKRrQRRr |
| FITC-βA-r₃-L-TATi | FITC-βA-rRRQrRKKr |
| FITC-βA-DAK (ctl) | FITC-βA-DAK (no transporter sequence) |

Subsequent to synthesis the constructs were purified, stored as 10 mM solution in sterile water, and used as purified without any further treatment.

### 2. Identification of the minimal D-/L-pattern conferring sensibility to proteases (Proteinase K)

In order to identify the minimal D-/L-pattern of transporter constructs, which is sensitive to proteases but confers a sufficient long half life *in vivo,* a quantitative degradation assay was carried out with Proteinase K involving several transporter constructs with poly-Arg sequences, each showing a different pattern of D- and L-amino acids. The transporter constructs used in this assay were prepared as describend under example 1 above and termed 1 to 6.

| SEQ ID No. | Pattern | Sequence (N-term to C-term) | Protease sensitivity |
|---|---|---|---|
| 123 | all L | RRRRRRRRR (reference sequence) | + |
| 257 | all D | rrrrrrrrr | - |
| 258 | D/L | rRrRrRrRr | - |
| 259 | DD/LL | rrRRrrRRr | - |
| 260 | DDD/LLL (r₆R₃) | rrrRRRrrr | + |
| 261 | DDDD/LLLL | rrrRRRRrr | + |

The different pattern of the sequences is described using capitals and minor letters. The capitals in these sequences 1 to 6 ("R"-amino acids) refer to L-enatiomeric arginine (L-Arg) and the minor letters in these sequences ("r"-amino acids) refer to D-enatiomeric arginine (L-Arg). The sensitivity to Proteinase K was measured at time intervals t = 0, 10 and 40 minutes. The results of the quantitative degradation assay with Proteinase K were then determined on the basis of samples taken at these specific time intervals. These samples were analyzed utilizing a mass spectrometry analysis using starting material ion intensities as reference values. As a conclusion, when a stretch of 3 or more L- Arg is present in the sequence the peptide shows sensitivity to Proteinase K and is degraded. Peptides with 2 or less L-Arg next to each other are not degraded. These results are comparable to a sequence consisting entirely of D-enantiomeric amino acids, e.g. D-TAT (rrrqrrkkr; SEQ ID NO: 251) or D-JNKi, respectively (see also Figure 1).

A further comparison was carried out using four transporter constructs of D-/L-TAT derivatives termed L-TAT (SEQ ID NO: 18), r₃-TAT (also termed r₃-L-Tat; SEQ ID NO: 20) r₃-TATi (also termed r₃-L-TATi; SEQ ID NO: 21), and D-TAT (SEQ ID NO: 251), each having a length of 9 amino acids but a different D-/L-pattern. The different pattern of the sequences is described using capitals and minor letters. The capitals in the sequences ("R"-amino acids) refer to L-enatiomeric arginine (L-Arg) and the minor letters in these sequences ("r"-amino acids) refer to D-enatiomeric arginine (L-Arg). The table shown in Figure 2 illustrates the amino acid sequences of these TAT derived transporter constructs with respect to their molecular weight (Mw) and pl-values (see Figure 2).

The digestion was carried out in 10% and 50% human serum at 37°C until complete degradation of these TAT derived transporters construct. As can be seen in Figure 3, showing the results of the digestion of TAT derived transporter constructs, D-TAT (SEQ ID NO: 251) transporter constructs are protease resistant, whereas the L-TAT transporter construct (SEQ ID NO: 18) are degraded too early *in vivo* in order to effect an efficient transport into cells. Only transporter constructs r3-TAT (also termed r3-L-Tat, SEQ ID NO: 20), r3-TATi (also termed r3-L-TATi, SEQ ID NO: 21) show a degradation within a suitable time limit, allowing to limit the *in vivo* stability for therapeutic applications.

A mass spectrometry analysis of the degradation of the transporter construct r3-L-TAT in human serum was carried out. As a result, r3-L-TAT (SEQ ID NO: 20) is C-terminally degraded up to single amino acid in human serum.

Furthermore, a digestion of TAT derived transporter constructs in 10% and 50% human serum at 37° C was carried out until complete degradation of these TAT derived transporters under involvement of beta-Alanine (b-Ala) in protecting the peptides. Therefore, beta-Ala was added to the N-terminus of TAT derived transporter constructs L-TAT as L-TATi as already described in Figure 3. The results are shown in Figure 4. As can be seen in Figure 4 N-terminal protection of the transporter peptides with beta-Alanine does not lead to a significant effect, i.e. an improved stability.

Summarizing the above results with respect to their *in vivo* stability and functional half life the stability of the 4 TAT derived peptides tested in 10 and 50% HS is as follows: D-TAT (SEQ ID NO: 251) > r3-L-TATi (SEQ ID NO: 21) > r3-L-TAT (SEQ ID NO: 20) > L-TAT (SEQ ID NO: 18). The life time of both r3-L-TAT (SEQ ID NO: 20) and r3-L-TATi (SEQ ID NO: 21) is therefore increased compared to L-TAT (SEQ ID NO: 18) and decreased compared to D-TAT (SEQ ID NO: 251). As serum constitutes -50% of blood volume, values obtained from samples containing 50% HS are the most relevant. UPLC-MS results furthermore suggest that the degradation involves exoproteases that cleave at the Terminus of TAT. Carboxypeptidase N is assumed to be involved in the degradation as it is constitutively active and present at high concentration (30µg/mL) in the blood. Moreover, CPN cleaves specifically C-terminal Lys or Arg from peptides and proteins.

### 3. Uptake (internalization) of peptides into cells and measurement of peptide internalization into cells

In this experiment, the internalization (uptake) capacity of FITC-labeled TAT derived transporter constructs *in vitro* was evaluated with a fluorescence plate reader in cell lines HL-60 (Leukemia).

### 3.1. Test samples used in the experiments

The constructs used in this experiment were four different TAT derived transporter constructs (termed L-TAT (SEQ ID NO: 18), r3-TAT (also termed r3-L-Tat; SEQ ID NO: 20), r3-TATi (also termed r3-L-TATi; SEQ ID NO: 21), and D-TAT (SEQ ID NO: 251), each prepared as described above. These constructs have a length of 9 amino acids but a different D-/L-pattern. Furthermore, the construct DAK was used as a control, which comprised no transporter sequence. The constructs were N-terminally protected with beta-Alanine (βA) and labeled with FITC.

| | |
|---|---|
| FITC-βA-L-TAT | FITC-βA-RKKRQRRR |
| FITC-βA-D-TAT | FITC-βA-rrrqrrkkr |
| FITC-βA-r₃-L-TAT | FITC-βA-rKKRrQRRr |
| FITC-βA-r₃-L-TATi | FITC-βA-rRRQrRKKr |
| FITC-βA-DAK (ctl) | FITC-βA-DAK (no transporter sequence) |

The constructs were prepared as described above, purified, stored as 10mM solution in sterile water, and used as purified without any further treatment.

### 3.2. Further transporter contructs TAT(s2-1)- TAT(s2-96)

Further transporter contructs TAT(s2-1)- TAT(s2-96) were prepared as described above in general for inventive transporter constructs. Following sequences and protecting groups were used therefore during synthesis (bound to resin):

| | |
|---|---|
| TATs2-1: | |
| TATs2-2: | |
| TATs2-3: | |
| TATs2-4: | |
| TATs2-5: | |
| TATs2-6: | |
| TATs2-7: | |
| TATs2-8: | |
| TATs2-9: | |
| TATs2-10: | |
| TATs2-11: | |
| TATs2-12: | |
| TATs2-13: | |
| TATs2-14: | |
| TATs2-15: | |
| TATs2-16: | |
| TATs2-17: | |
| TATs2-18: | |
| TATs2-19: | |
| TATs2-20: | |
| TATs2-21: | |

| | |
|---|---|
| TATs2-22: | |
| TATs2-23: | |
| TATs2-24: | |
| TATs2-25: | |
| TATs2-26: | |
| TATs2-27: | |
| TATs2-28: | |
| TATs2-29: | |
| TATs2-30: | |
| TATs2-31: | |
| TATs2-32: | |
| TATs2-33: | |
| TATs2-34: | |
| TATs2-35: | |
| TATs2-36: | |
| TATs2-37: | |
| TATs2-38: | |
| TATs2-39: | |
| TATs2-40: | |
| TATs2-41: | |
| TATs2-42: | |
| TATs2-43: | |
| TATs2-44: | |
| TATs2-45: | |
| TATs2-46: | |

| | |
|---|---|
| TATs2-47: | |
| TATs2-48: | |
| TATs2-49: | |
| TATs2-50: | |
| TATs2-51: | |
| TATs2-52: | |
| TATs2-53: | |
| TATs2-54: | |
| TATs2-55: | |
| TATs2-56: | |
| TATs2-57: | |
| TATs2-58: | |
| TATs2-59: | |
| TATs2-60: | |
| TATs2-61: | |
| TATs2-62: | |
| TATs2-63: | |
| TATs2-64: | |
| TATs2-65: | |
| TATs2-66: | |
| TATs2-67: | |
| TATs2-68: | |
| TATs2-69: | |
| TATs2-70: | |
| TATs2-71: | |

| | |
|---|---|
| TATs2-72: | |
| TATs2-73: | |
| TATs2-74: | |
| TATs2-75: | |
| TATs2-76: | |
| TATs2-77: | |
| TATs2-78: | |
| TATs2-79: | |
| TATs2-80: | |
| TATs2-81: | |
| TATs2-82: | |
| TATs2-83: | |
| TATs2-84: | |
| TATs2-85: | |
| TATs2-86: | |
| TATs2-87: | |
| TATs2-88: | |
| TATs2-89: | |
| TATs2-90: | |
| TATs2-91: | |
| TATs2-92: | |
| TATs2-93: | |
| TATs2-94: | |
| TATs2-95: | |
| TATs2-96: | |

### 3.3 Materials and Methods for uptake experiments

### a) Cell line:

The cell line used for this experiment was HL-60 (Ref CCL-240, ATCC, Lot 116523)

### b) Culture medium and plates

RPMI (Ref 21875-091, Invitrogen, Lot 8296) or DMEM (Ref 41965, Invitrogen, Lot 13481) complemented on 05.05.2008 with:
   10% FBS (Ref A64906-0098, PAA, Lot A15-151): decomplemented at 56°C, 30 min, on 04.04.2008.
   1mM Sodium Pyruvate (Ref S8636, Sigma, Lot 56K2386)
   Penicillin (100 unit/ml)/Streptomycin (100µg/ml) (Ref P4333, Sigma, Lot 106K2321)
PBS 10X (Ref 70011, Invitrogen, Lot 8277): diluted to 1X with sterile H₂O
Trypsine-0.05% EDTA (Ref L-11660, PAA, Lot L66007-1194)
6 well culture plates (Ref 140675, Nunc, Lot 102613)
24 well culture plates (Ref 142475, Nunc, Lot 095849)
96 well culture plates (Ref 167008, Nunc, Lot 083310)
96 well plates for protein dosing (Ref 82.1581, Sarstedt)
96 well plates for fluorescence measurement (Ref 6005279, Perkin Elmer)

### c) Solutions

Poly-D-lysine coating solution (Sigma P9011 Lot 095K5104): 25µg/ml final diluted in PBS 1 x
Acidic wash buffer: 0.2M Glycin, 0.15M NaCl, pH 3.0
Ripa lysis buffer: 10mM NaH₂PO₄ pH 7.2, 150mM NaCl, 1% Triton X-100, 1mM EDTA pH 8.0, 200µM Na₃VO₂, 0.1% SDS, 1X protease inhibitor cocktail (Ref 11873580001, Roche, Lot 13732700)

### d) Microscopy and fluorescence plate reader

Cells were observed and counted using an inverted microscope (Axiovert 40 CFL; Zeiss; 20X).

The fluorescence was read with the Fusion Alpha Plate reader (Perkin Elmer).

### e) Method

FITC marked peptide internalization was studied on suspension cells. Cells were plated into poly-DL-lysine coated dishes at a concentration of 1 × 10⁶ cells/ml. Plates were then incubated for 24 h at 37 °C, 5 % CO₂ and 100% relative humidity prior to the addition of a known concentration of peptide. After peptide addition, the cells were incubated 30 min, 1, 6 or 24 h at 37 °C, 5 % CO₂ and 100 % relative humidity. Cells were then washed twice with an acidic buffer (Glycin 0.2 M, NaCl 0.15 M, pH 3.0) in order to remove the cell-surface adsorbed peptide (see Kameyama et al., (2007), Biopolymers, 88, 98-107). The acidic buffer was used as peptides rich in basic amino acids adsorb strongly on the cell surfaces, which often results in ovestimation of internalized peptide. The cell wash using an acidic buffer was thus employed to remove the cell-surface adsorbed peptides. The acid wash was carried out in determining cellular uptake of Fab/cell-permeating peptide conjugates, followed by two PBS washes. Cells were broken by the addition of the RIPA lysis buffer. The relative amount of internalized peptide was then determined by fluorescence after background substraction and protein content normalization.

The steps are thus:
1. Cell culture
2. Acidic wash and cellular extracts
3. Analysis of peptide internalization with a fluorescence plate reader

### f) Cell culture and peptide treatment

(1) The 6 well culture plates are coated with 3 ml of Poly-D-Lys (Sigma P9011; 25 µg/ml in PBS), the 24 well plates with 600 µl and the 96 well plates with 125 µl and incubated for 4 h at 37°C, CO₂ 5 % and 100 % relative humidity.
(2) After 4 hours the dishes were washed twice with 3.5ml PBS, 700 µl or 150 µl PBS for the 6, 24 or 96 well plates, respectively.
(3) The cells were plated into the dishes in 2.4 ml medium (RPMI) at plating densities of 1'000'000 cells/ml for suspension cells. After inoculation, the plates were incubated at 37°C, 5 % CO₂ and 100 % relative humidity for 24 hours prior to the addition of the peptide. Adherent cells should be at a density of 90-95% the day of treatment and were plated in DMEM :

| well | Surface of culture (cm²) | Medium | Nb adherent cells | Nb suspension cells |
|---|---|---|---|---|
| 96 well | 0.3 | 100 - 200 µl | 8'000 - 30'000 | 100'000 |
| 24 well | 2 | 500 - 1000 µl | 100'000 - 200'000 | 500'000-1'000'000 |
| 35mm (P35) / 6 well | 10 | 2,4 ml | 250'000 - 2'100'000 | 2'400'000 |
| 60mm (P60) | 20 | 3,5 ml | 15 * 10⁵ | 1'000'000/ml |
| 10cm (P100) | 60 | 10 ml | 15-60 * 10⁵ | |

(4) The cells were treated with the desired concentration of FITC labeled peptide (stock solution at a concentration of 10 mM in H₂O).
(5) Following peptide addition, the cells were incubated 0 to 24 hours (e.g. 30 min, 1, 6 or 24 hours) at 37 °C, CO₂ 5 % and 100 % relative humidity.

### Acidic wash and cellular extracts:

### (6) The extracts were cooled on ice.

### Suspension cells (or cells, which don attach well to the dish):

- Transfer the cells in « Falcon 15 ml ». To recover the maximum of cells, wash the dish with 1 ml of PBS.
- Harvest the cells 2 min at 2400 rpm max.
- Suspend the cells in 1 ml cold PBS.
- Transfer the cells into a coated "Eppendorf tube" (coated with 1 ml of poly D-Lys for 4hours and washed twice with 1 ml PBS).
- Wash three times with 1 ml of cold acidic wash buffer and centrifuge 2 min at 2400 rpm max. Beware of the spreading of the cells in the "eppendorf".
- Wash twice with 1 ml cold PBS to neutralize.
- Add 50 µl of lysis RIPA Buffer.
- Incubate 30 min-1 h on ice with agitation.

### Adherent cells:

- Wash three times with 3 ml, 1 ml or 200 µl (for 6, 24 or 96 well plates, respectively) of cold acidic wash buffer. Beware of the cells who detach from the dish.
- Wash twice with 1 ml cold PBS (for 6, 24 or 96 well plates, respectively) to neutralize.
- Add 50 µl of lysis RIPA buffer.
- Incubate 30 min-1 h on ice with agitation.
- Scrap the cells with a cold scrapper. The 24 and 96 well plates were directly centrifuged at 4000rpm at 4° for 15min to remove the cellular debris. Then the supernatants (100 or 50mi respectively for the 24 or 96 well plates) were directly transferred in a dark 96 well plated. The plates were read by a fluorescence plate reader (Fusion Alpha, Perkin Elmer).
- Transfer the lysate in a coated "eppendorf" (coated with 1 ml of poly D-Lys for 4hours and wash twice with 1 ml PBS).
- The lysed cells were then centrifuged 30 min at 10000 g at 4 °C to remove the cellular debris.
- Remove the supernatant and store it at -80 °C in a coated "Eppendorf tube" (coated with 1 ml of poly D-Lys for 4 hours and washed twice with 1 ml PBS).

### Analysis ofpeptide internalization with a fluorescence plate reader:

(7) The content of each protein extract was determined by a standard BCA assay (Kit N°23225, Pierce), following the instructions of the manufacturer.
(8) The relative fluorescence of each sample is determined after reading 10 µl of each sample in a fluorescence plate reader (Fusion Alpha, Perkin Elmer), background subtraction and normalization by protein concentration.

### 3.4. Internalization experiments and analysis

The time dependant internalization (uptake) of FITC-labeled TAT derived transporter constructs into cells of the HL-60 cell line was carried out with materials and methods as described above.

Briefly, HL-60 cells were incubated 30 min, 1, 6 or 24 hours with 10µM of the TAT-derivative transporters. The cells were then washed twice with an acidic buffer (0.2 M Glycin, 0.15 M NaCl, pH 3.0) and twice with PBS. Cells were broken by the addition of RIPA lysis buffer. The relative amount of internalized peptide was then determined by reading the fluorescence intensity (Fusion Alpha plate reader; PerkinElmer) of each extract followed by background substraction and protein content normalization. The r3-L-TAT transporter construct showed an internalization capability as effective as the D-TAT transporter construct. The r3-L-TATi transporter construct, which internalized in a time dependent manner, as both previous transporters, seems to be less efficient but still suitable, whereas L-TAT doesn't accumulate over a period of 24 hours (see Figure 5).

Furthermore, a confocal microscopy was carried out with cells treated with fluorescently labeled TAT derived transporter constructs as decribed above. The dissociated cortical primary neurons from P2 Sprague Dawley rats were cultured 12 days in neurobasal medium before exposure 24hours to 500nM of the FITC-labeled TAT derivative transporters. The cells were washed five times with PBS on ice and then mounted in fluorsave mounting medium without prior fixation. Acquisitions were performed on LSM510 metaconfocal microscope (Zeiss). Images were processed with LSM510 software and mounted using Adobe photoshop. Visualization by confocal microscopy of labeling with 500nM FITC-transporters (A: green). Nuclei were stained by Hoechst (B: blue). The r3-L-TAT as well as the D-TAT and the r3-L-TATi transporter constructs were internalized into the cytoplasm of the non stressed neurons (C: Merge panel). However, after 24 hours incubation, the L-TAT transporter was not present anymore (see Figure 6).

### 3.5. Further internalization experiments and analysis

The time dependant internalization (uptake) of FITC-labeled TAT derived transporter constructs into cells of the HL-60 cell line was furthermore carried out with materials and methods as described above using sequences of the 96-FITC-labeled D-TAT derivative transporters. These sequences are listed below in Table 6.

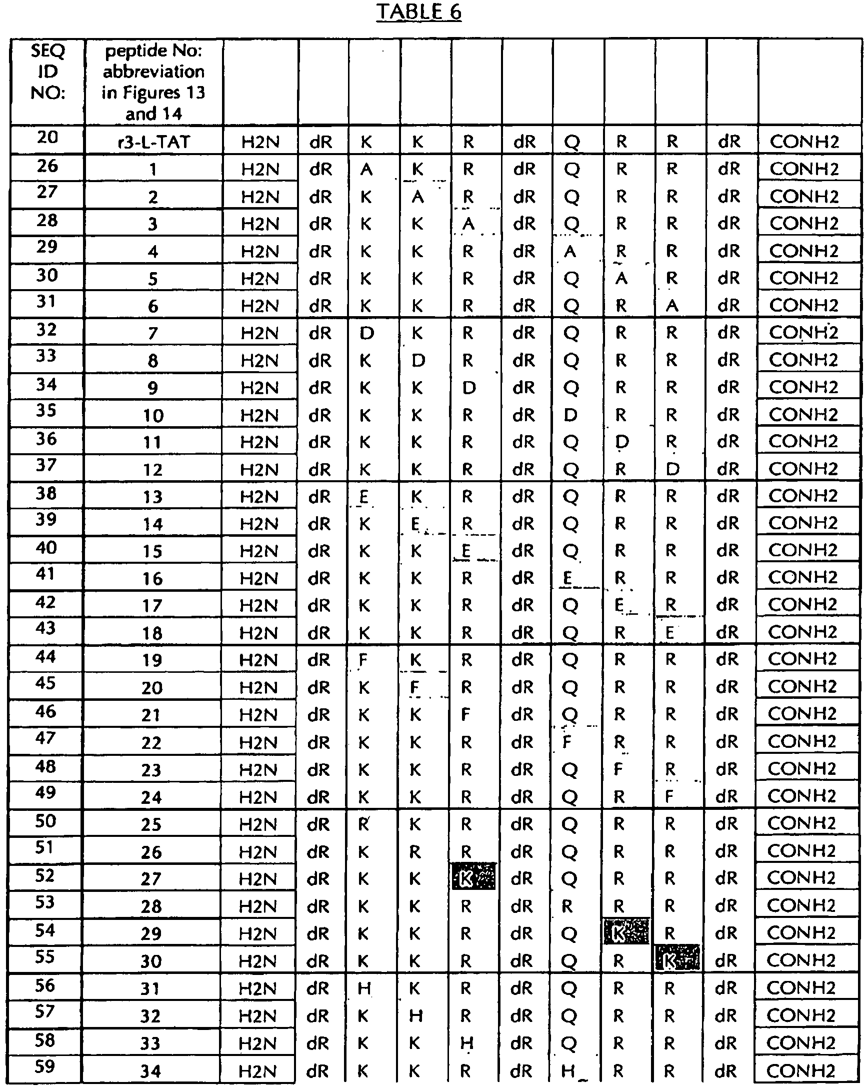

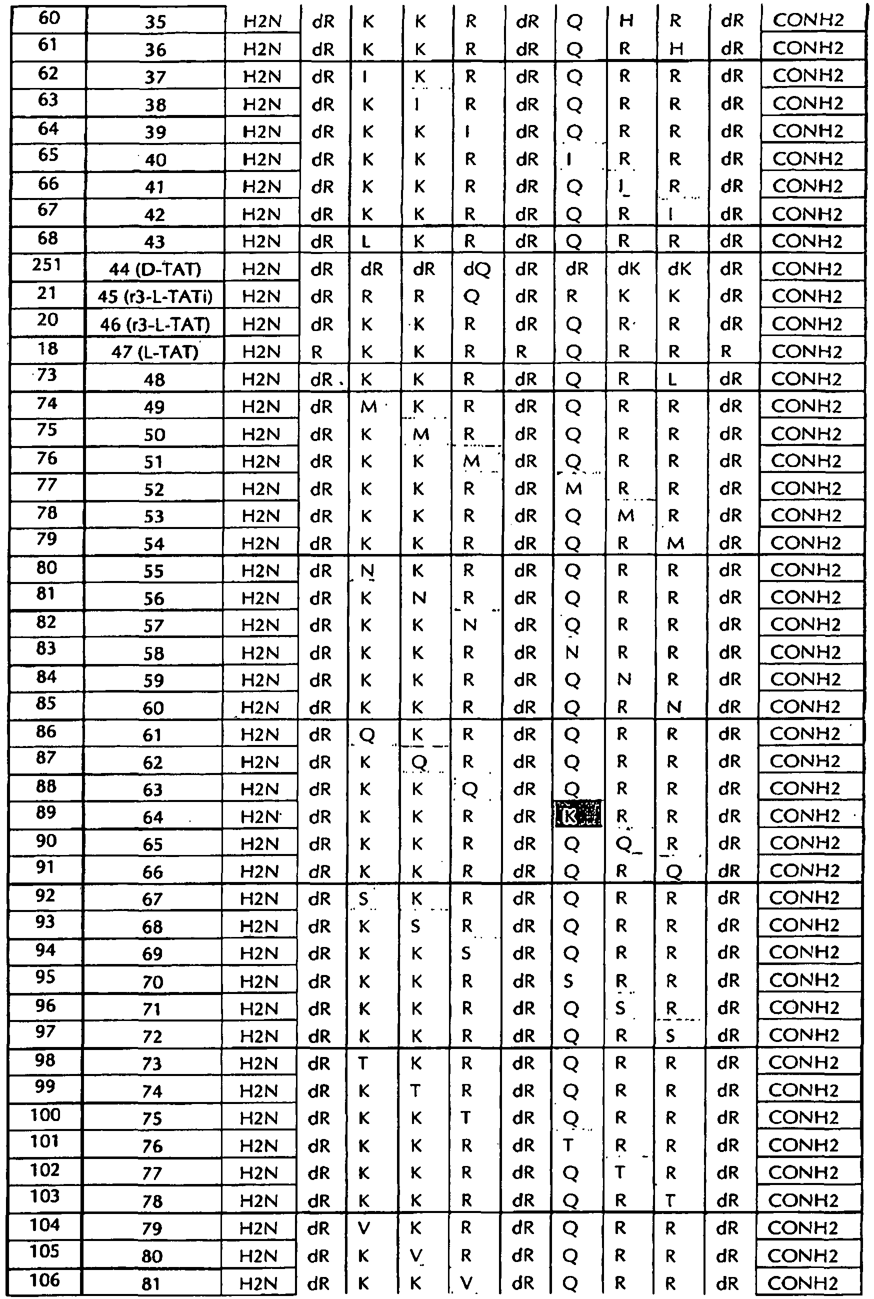

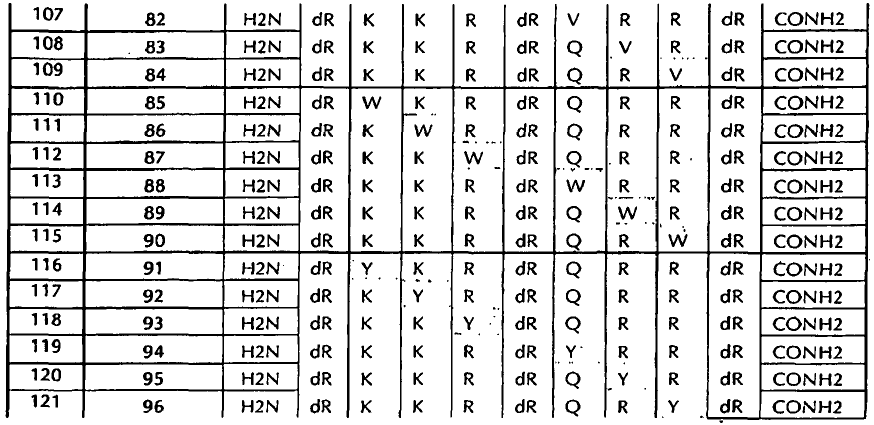

In the above table D amino acids are indicated by a small "d" prior to the respective amino acid residue (dR = D-Arg).

For a few sequences synthesis failed in the first approach unfortunately due to technical reasons. These sequences are abbreviated in Figure 13 as 1, 2, 3, 4, 5, 6, 7, 8, 43, 52, 53, 54, 55, 56, 57, 85, 86, 87, 88, 89, and 90. However, the remaining sequences were used in the internalization experiments.

The results are shown in Figures 13 and 14.

As can be seen in Figure 13, after 24 hours incubation, all transporters with the consensus sequence rXXXrXXXr (SEQ ID NO: 252) (see above for a selection of possible sequences) showed a higher internalization capability than the L-TAT transporter. Hela cells were incubated 24hours in 96well plate with 10mM of the r3-L-TAT-derived transporters. The cells were then washed twice with an acidic buffer (0.2M Glycin, 0.15M NaCl, pH 3.0) and twice with PBS. Cells were broken by the addition of RIPA lysis buffer. The relative amount of internalized peptide was then determined by reading the fluorescence intensity (Fusion Alpha plate reader; PerkinElmer) of each extract followed by background subtraction

As can be seen in Figure 14, one positions appears to be critical for highest transporter activity and for improved kinetics of transport activity: Y in position 2 (peptide N°91 corresponding to SEQ ID NO: 116). Briefly, Hela cells were incubated 2, 6 or 24 hours in 24well plate with increasing dose of the r3-L-TAT-derivative transporters (0, 500nM, 1mM or 10mM). The cells were then washed twice with an acidic buffer (0.2M Glycin, 0.15M NaCl, pH 3.0) and twice with PBS. Cells were broken by the addition of RIPA lysis buffer. The relative amount of internalized peptide was then determined by reading the fluorescence intensity (Fusion Alpha plate reader; PerkinElmer) of each extract followed by background substraction.

The conclusion of this experiment is as follows:
- After 24 hours incubation, all transporters with the consensus sequence rXXXrXXXr (SEQ ID NO: 252) (see Table 1 for a selection of possible sequences) showed a higher internalization capability than the L-TAT transporter (Figure 13). Those results fully validate the consensus sequence rXXXrXXXr (SEQ ID NO: 252).
- One position is critical for highest transporter activity and (Figure 13): Y in position 2 (sequence 91 corresponding to SEQ ID NO: 116).
- One position is critical for improved kinetics of transport activity (Figure 14): Y in position 2 (sequence 91 corresponding to SEQ ID NO: 116).

Accordingly, such TAT derived sequences as shown in Table 1 are preferred, which exhibit an Y in position 2, particularly when the sequence according to generic formula (I) exhibits 9 aa and has the consensus sequence rXXXrXXXr (SEQ ID NO: 252).

### 4. Determination of intracellular concentration of specific transporter constructs subsequent to uptake (internalization) of these peptides into U937 cells

According to a further experiment, the concentration of specific transporter constructs subsequent to uptake (internalization) of these peptides into U937 cells were determined. The experiments were carried out using the sequences RKKRRQRRR (L-TAT), rrrqrrkkr (D-TAT), rKKRrQRRr (r3-L-TAT) and rYKRrQRRr (XG-91), each in a concentration of 10 µM each.

| 10µM | 2h | 4h | 6h | 24h |
|---|---|---|---|---|
| RKKRRQRRR (L-TAT) (SEQ ID NO: 18) | 1.20 | 1.38 | 1.07 | 0.5 |
| rrrqrrkkr (D-TAT) (SEQ ID NO: 251) | 2.00 | 2.24 | 3.55 | 17.3 |
| rKKRrQRRr (r3-L-TAT) (SEQ ID NO: 20) | 2.34 | 3.16 | 3.56 | 11.2 |
| rYKRrQRRr (XG-91, sequence 91 corresponding to SEQ ID NO: 116) | 3.16 | 4.27 | 4.68 | 50 |

Surprisingly, the accumulation of rYKRrQRRr (XG-91, sequence 91 corresponding to SEQ ID NO: 116), shows an extremely accumulation in the cell, which is even significantly higher than the concentration of the transporter construct in the medium or the average concentration of about 20 µM which was expected for D-TAT construct. This underlines the importance of transporter constructs according to generic formula (I), particular of transporter constructs which comprise a TAT derived sequence as shown in Table 1, which exhibits an Y in position 2, and preferably has 9 aa and the consensus sequence rXXXrXXXr (SEQ ID NO: 252).

### 5. Uptake (internalization) of peptides into cells and measurement of peptide internalization in cell lines HepG2 (hepatocarcinoma), HCT-116 (tumoral colon), U937 (Lymphoma), in WBC cell lines (white blood cell lines) and non-WBC cell lines

In these experiments, the internalization (uptake) capacity of of FITC-labeled TAT derived transporter constructs *in vitro* was evaluated with a fluorescence plate reader in further cell lines HepG2 (hepatocarcinoma), HCT-116 (tumoral colon), U937 (Lymphoma), in WBC cell lines (white blood cell lines) and non-WBC cell lines.

### Test samples and conditions used in the experiments

The constructs and conditions used in this experiment were as described above for experiment 3 with following amendments and cell lines:

### a) Uptake (internalization) of FITC-labeled TAT derived transporter constructs in vitro (10 µM, HepG2 hepatocarcinoma, HCT-116 tumoral colon, 24 h)

The constructs used were different TAT derived transporter constructs termed D-TAT and r3-TATi (also termed r3-L-TATi), D-TAT, each having a length of 9 amino acids but a different D-/L-pattern, and the constructs r₆R₃ and DAK, wherein the constructs additionally have been labeled with beta-Alanine at their N-terminus. The results are shown in Figure 7. As can be seen in Figure 7, the uptake was most efficient for constructs D-TAT and r₆R₃, followed by r₃-L-TATi.

### b) Uptake (internalization) of FITC-labeled TAT derived transporter constructs in vitro (10 µM, U937, Lymphoma, 24 h).

The constructs used were four different TAT derived transporter constructs (termed L-TAT, r3-TAT (also termed r3-L-Tat), r3-TATi (also termed r3-L-TATi), arid D-TAT), each having a length of 9 amino acids but a different D-/L-pattern. Additionally, the construct DAK was used for comparison and a control sample, ontaining no peptide. The results are shown in Figure 8. As can be seen in Figure 8, the uptake of r3-TAT, r3-TATi and D-TAT transporter constructs into the cells was most efficient, wherein L-TAT showed a significantly lower uptake into the cells.

### c) HSPG dependency of uptake (internalization) of the D-TAT transporter construct

An experiment was carried out to see, whether the uptake (internalization) of the D-TAT transporter construct is HSPG-dependent. As found, the uptake (internalization) of the D-TAT transporter construct is HSPG-dependent at a concentration of 500 nm over 24 hours in U937 cells, Lymphoma (see also Figure 9). The construct used for the experiment was D-TAT, having a length of 9 amino acids and being labeled with FITC and at its N-terminus with beta-Alanine.

### d) Exit of the FITC-labeled TAT derived transporter constructs in U937 cells (lymphoma)

A further experiment was carried out to see, whether the FITC-labeled TAT derived transporter constructs exit U937 cells. As a result, an exit is not observed in U937 cells at 500 nM FITC-D-TAT (see Figure 10). The construct used for the experiment was D-TAT (SEQ ID NO: 251), having a length of 9 amino acids and being labeled with FITC and at its N-terminus with beta-Alanine.

Furthermore, it could be seen, that an exit of the FITC-labeled TAT derived transporter constructs is observed at 10 µM FITC-D-TAT, and is HSPG-dependent in U937 cells (lymphoma) (see Figure 11). The construct used for the experiment was again D-TAT (SEQ ID NO: 251) as above.

### e) Uptake (internalization) and an exit of the FITC-labeled TAT derived transporter constructs at 10 µM FITC-D-TAT in non WBC-lines (white blood cells lines)

In a further experiment an uptake (internalization) and an exit of the FITC-labeled TAT derived transporter constructs are observed at 10 µM FITC-D-TAT in non WBC-lines (white blood cells lines) (see Figure 12). The construct used for the experiment was D-TAT (SEQ ID NO: 251), having a length of 9 amino acids and being labeled with FITC and at its N-terminus with beta-Alanine.

### f) Conclusions

As a conclusion of the above uptake (internalization) experiments and as can be seen in the above Figures, uptake (internalization) of FITC-labeled TAT derived transporter constructs containing or exclusively composed of D-amino acids is linear over several hrs *in vitro.* Furthermore, at 24 hrs, the uptake (internalization) of these FITC-labeled TAT derived transporter constructs *in vitro* reaches 50-100 fold higher intracellular concentrations than L-TAT. Additionally, the uptake (internalization) of FITC-labeled TAT derived transporter constructs containing or exclusively composed of D-amino acids by WBC-lines (white blood cells lines) is 10-50 fold more efficient than by non-WBC-lines *in vitro.* For all these experiments, an exit was shown to be efficient at high intracellular concentration, but is not observed at low concentrations in WBCs

### 6. Synthesis of cytotoxic transporter cargo conjugate molecule D-Tat-cisplatin, r3-L-TAT-cisplatin and r3-L-TATi-cisplatin

### 6.1 Peptide synthesis

The peptide sequence of D-TAT (rrrqrrkkr; SEQ ID NO: 251), r3-L-TAT (rKKRrQRRr; SEQ ID NO: 20) and r3-L-TATi (rRRQrRKKr; SEQ ID NO: 21) including an additional methionine is synthesized manually on 0.4 mmol Fmoc-Amide-AM resin by using Fmoc chemistry. The peptide is then cleaved from the resin with TFA, filtered under a reduced pressure, precipitated with cold ether, and dried. The crude peptide is purified by Semi-preparative HPLC and characterized by ESI-MS.

### 6.2 Alkylation of peptide to Cisplatin

5.0 µmol of Cisplatin (1.5 mg in 3.0 ml Sodium Chloride buffer, pH 5.0) are dissolved in 2.0 ml of 10 mM Na₂HPO₄ buffer (pH 7.4), and pH value of the solution is 7.0. 5.0 µmol of D-TAT-Methionine peptide (or of r3-L-TAT-Methionine peptide or of r3-L-TATi-Methionine peptide) is prepared in 10 mM Na₂HPO₄ buffer (pH 7.4) and pH value of the solution is 6.0. Then the alkylation is started by mixing two solutions at room temperature in dark (pH value of the mixture is 7.0). After 0h, 1 h, 3h and 24h, the product is analysed by analytic RP-HPLC, and characterized by ESI-MS. The expected peak solution is finally purified by Semi-preparative RP-HPLC and lyophilized.

### 7. Synthesis of cytotoxic transporter cargo conjugate molecule D-Tat-Oxaliplatin, r3-L-TAT-Oxaliplatin and r3-L-TATi-Oxaliplatin

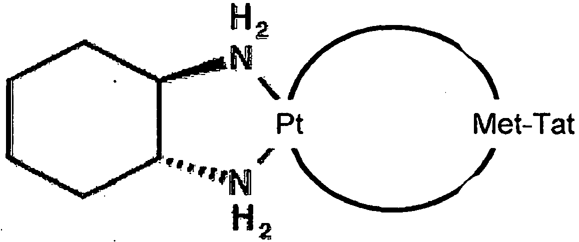

### 7.1. Peptide (D-Tat-Methionine, r3-L-TAT-Methionine and r3-L-TATi-Methionine) synthesis

The peptide sequence of D-TAT (rrrqrrkkr; SEQ ID NO: 251), r3-L-TAT (rKKRrQRRr; SEQ ID NO: 20) and r3-L-TATi (rRRQrRKKr; SEQ ID NO: 21) is synthesized manually on 0.23 mmol Fmoc-Rink Amide resin by using Fmoc chemistry. Thus, each amino acid from C-terminal Gly to N-terminal I-Met (L-Form) is sequentially attached to the resin with with a cycle of Fmoc-deprotection (20% piperidine in DMF) and amino acid coupling (HBTU/HOBt/DIEA in DMF activation). The peptide is cleaved from the resin with TFA (2h in the presence of 2.5% dH₂O, 0.5% EDT and 2.0% TIS), filtred at atmospheric pressure, volume reduced by N₂ bubbling, precipitated with cold ether and air-dried. The crude peptide is purified by semi-preparative RP-HPLC and characterized by ESI-MS.

### 7.2. Alkylation of peptide to oxaliplatin

10 µmol Oxaliplatin, formulated as Eloxatin^{®} (Oxaliplatinum 4.0 mg, lactosum monohydricum 36.0 mg) in 5.0 ml 10 mM Na₂HPO₄ buffer (pH 7.4). 10 µmol of D-Tat-Methionine peptide (or of r3-L-TAT-Methionine peptide or of r3-L-TATi-Methionine peptide) is prepared in dH₂O 5.0 ml. Alkylation is started by mixing the two solutions at room temperature. Reaction is then left at 37°C and monitored by analytical RP-HPLC at 214 and 280 nm over 24h, target peak is characterized by ESI-MS and purified by semi-preparative RP-HPLC followed by lyophilization.

### 7.3. Test conditions

Effects of a treatment with increasing concentrations of a conjugate molecule of the invention (D-Tat-oxaliplatin, r3-L-TAT-oxaliplatin, or r3-L-TATi-oxaliplatin) on the survival of MCF-7 (human breast adenocarcinoma cell line) and SiHa (human cervix squamous carcinoma cell line) are determined. The effects of D-Tat-oxaliplatin, r3-L-TAT-oxaliplatin, or r3-L-TATi-oxaliplatin is compared to the conjugate L-Tat-oxaliplatin and to two unconjugated anti-cancer drugs (Oxaliplatin and Cisplatin). Cells of each cell line (10'000 cells per well) are plated into 96 well plates (200 µl total volume of MEM supplemented with 10% FBS, 1% L-glutamine, 1 % Na-pyruvate, 1 % non-essential amino acids for MCF-7 and of MEM/Earle's supplemented with 10% FBS, 1% Na-pyruvate, 1% non-essential amino acids for SiHa cells). 6 to 10 different concentrations for each test substance are tested. The control cells are non-treated. Cells are incubated at 37°C for 24h before treatment with the test substance. Each experiment is carried in triplicate. Cell incubation after treatment is performed for 96 hours at 37°C. The effects of the test molecules on the survival of these cell lines (in vitro cytotoxic activity) is measured by the MTT assay. 20 µl of a 5 mg/ml 0.22 µm filtered Thiazolyl Blue Tetrazolium Bromide solution (MTT, Sigma, Ref. No. 88415) in Phosphate Buffered saline (PBS, CHUV) are added to each well and the plate is incubated for 4 hours at 37°C. The supernatant is removed and formazan crystals are dissolved with DMSO (200 µl per well). Absorbancy (OD) is measured in a microplate reader at 595 nm (Expert Plus Reader, Asys Hitech). The IC₅₀ (concentration of the drug inhibiting 50% of the cell growth) for the test substances is calculated using Prism software.

### 8. Synthesis of cytotoxic transporter cargo conjugate molecule D-Tat-Chlorambucil, r3-L-TAT-Chlorambucil and r3-L-TATi-Chlorambucil

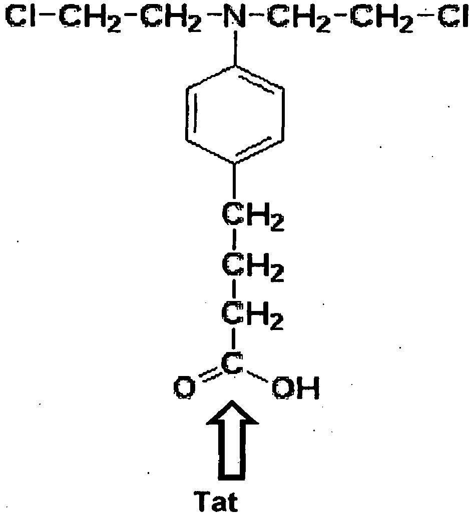

### 8.1 Conjugate molecule (D-Tat-Chlorambucil, r3-L-TAT-Chlorambucil and r3-L-TATi-Chlorambucil) synthesis

The D-TAT (rrrqrrkkr; SEQ ID NO: 251), r3-L-TAT (rKKRrQRRr; SEQ ID NO: 20) or r3-L-TATi (rRRQrRKKr; SEQ ID NO: 21) peptide sequence is synthesized manually on 0.23 mmol Fmoc-Rink Amide resin by using Fmoc chemistry. Thus, each amino acid from C-terminal Gly to N-terminal I- A (L-form) is sequentially attached to the resin with with a cycle of Fmoc-deprotection (20% piperidine in DMF) and amino acid coupling (HBTU/HOBt/DIEA in DMF activation).

Following Fmoc-deprotection (20% piperidine in DMF) of N-terminal I- A, coupling of chlorambucil is achieved using standard amino acid coupling conditions (HBTU/HOBt/DIEA in DMF activation). The conjugate molecule is cleaved from the resin with TFA (70min in the presence of 3% dH₂O and 3% TIS), filtred at atmospheric pressure, volume reduced by N₂ bubbling, precipitated with cold ether and air-dried. The crude conjugate molecule is purified by semi-preparative RP-HPLC, characterized by ESI-MS followed by lyophilization.

### 8.1 Comparative studies

Effects of a treatment with increasing concentrations of D-Tat-chlorambucil, r3-L-TAT-chlorambucil, or r3-L-TATi- chlorambucil on the survival of MCF-7 (human breast adenocarcinoma cell line) and SiHa (human cervix squamous carcinoma cell line) is determined. The effects of D-Tat-chlorambucil, r3-L-TAT- chlorambucil, or r3-L-TATi-chlorambucil is furthermore compared to the conjugate L-Tat-chlorambucil and to two u chlorambucil nconjugated anti-cancer drugs (Chlorambucil and Cisplatin). Cells of each cell line (10'000 cells per well) are plated into 96 well plates (200 µl total volume of MEM supplemented with 10% FBS, 1 % L-glutamine, 1 % Na-pyruvate, 1 % non-essential amino acids for MCF-7 and of MEM/Earle's supplemented with 10% FBS, 1% Na-pyruvate, 1% non-essential amino acids for SiHa cells). 6 to 10 different concentrations for each test substance are tested. The control cells are non-treated. Cells are incubated at 37°C for 24h before treatment with the test substance. Each experiment is carried in triplicate. Cell incubation after treatment is performed for 96 hours at 37°C. The effects of the test molecules on the survival of these cell lines (in vitro cytotoxic activity) is measured by the MTT assay. 20 µl of a 5 mg/ml 0.22 µm filtered Thiazolyl Blue Tetrazolium Bromide solution (MTT, Sigma, Ref. No. 88415) in Phosphate Buffered saline (PBS, CHUV) are added to each well and the plate is incubated for 4 hours at 37°C. The supernatant is removed and formazan crystals are dissolved with DMSO (200 µl per well). Absorbancy (OD) is measured in a microplate reader at 595 nm (Expert Plus Reader, Asys Hitech). The IC₅₀ (concentration of the drug inhibiting 50% of the cell growth) for the test substances is calculated using Prism software.

### 9. Synthesis of cytotoxic transporter cargo conjugates molecule D-Tat-Doxorubicine, r3-L-TAT-Doxorubicine and r3-L-TATi-Doxorubicine

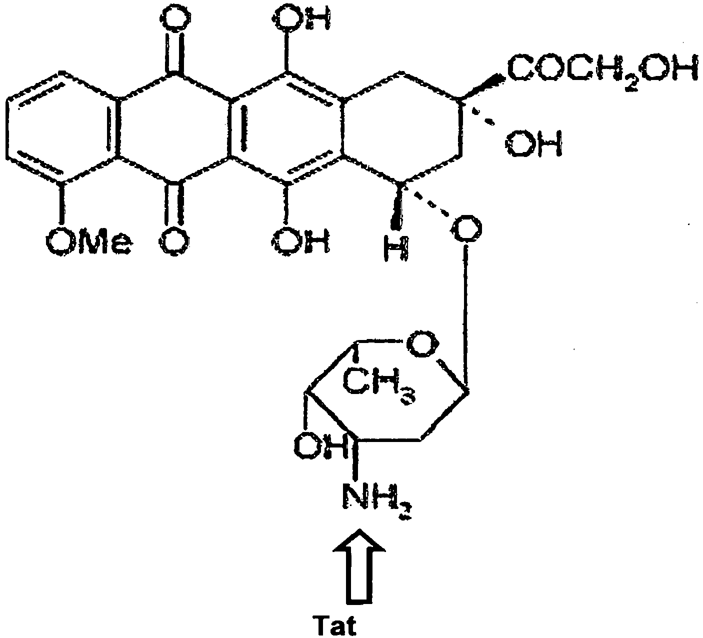

### 9.1. Conjugate molecule (D-Tat-doxorubicine, r3-L-TAT- doxorubicine and r3-L-TATi-doxorubicine) synthesis

The D-TAT (rrrqrrkkr; SEQ ID NO: 251), r3-L-TAT (rKKRrQRRr; SEQ ID NO: 20) and r3-L-TATi (rRRQrRKKr; SEQ ID NO: 21) peptide sequence is synthesized manually on 0.23 mmol Fmoc-Rink Amide resin by using Fmoc chemistry. Thus, each amino acid from C-terminal Gly to N-terminal I-E (L-form) is sequentially attached to the resin with with a cycle of Fmoc-deprotection (20% piperidine in DMF) and amino acid coupling (HBTU/HOBt/DIEA in DMF activation).

Following Fmoc-deprotection (20% piperidine in DMF) of N-terminal I-E, acetylation (acetic anhydride, DIEA in DMF activation) is done. Removal of the Odmab side-chain protecting group is performed using 2% hydrazine monohydrate in DMF. Coupling of chlorambucil formulated as Adriblastin® (Doxorubicinie.HCl 18%, NaCl 82% lyophilized) is achieved via OBt ester (DIPCDI/HOBt/DIEA in DCM/DMF activation).

The conjugate molecule is cleaved from the resin with TFA (2h in the presence of 1.7% dH₂O and 1.7% TIS), filtred at atmospheric pressure, volume reduced by N₂ bubbling, precipitated with cold ether and air-dried. The crude conjugate molecule is purified by semi-preparative RP-HPLC, characterized by ESI-MS followed by lyophilization.

### 10. Synthesis of cytotoxic transporter cargo conjugate molecule D-Tat-Saquinavir, r3-L-TAT-Saquinavir and r3-L-TATi-Saquinavir

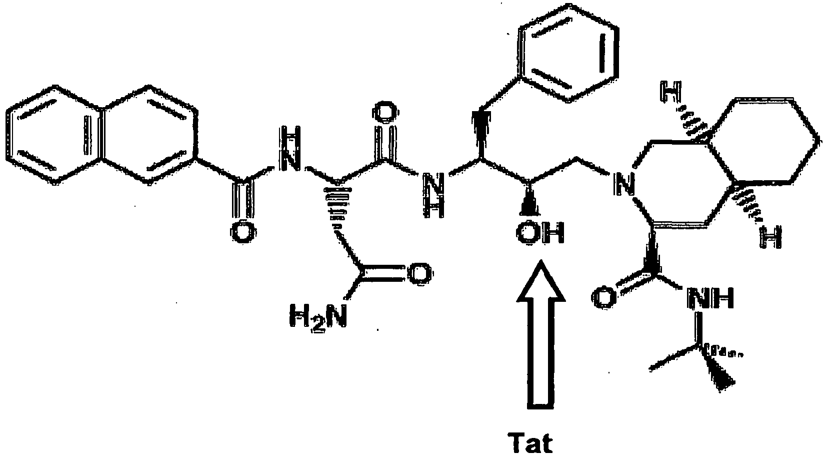

### 10.1. Peptide (D-TAT-D-Cysteine r3-L-TAT-D-Cysteine and r3-L-TATi-D-Cysteine) synthesis

The D-TAT (rrrqrrkkr; SEQ ID NO: 251), r3-L-TAT (rKKRrQRRr; SEQ ID NO: 20) and r3-L-TATi (rRRQrRKKr; SEQ ID NO: 21) peptide sequence is synthesized manually on 0.40 mmol Fmoc-Rink Amide resin by using Fmoc chemistry. Thus, each amino acid from C-terminal D-Arg to N-terminal D-Cys is sequentially attached to the resin with with a cycle of Fmoc-deprotection (20% piperidine in DMF) and amino acid coupling (TBTU/HOBt/DIEA in DMF activation). The peptide is cleaved from the resin with TFA, pre-incubated on ice (5h in the presence of 2.5% dH₂O, 2.5% EDT and 1.0% TIS), filtred at reduced pressure, precipitated with cold ether and vacuum dried. The crude peptide is purified by semi-preparative RP-HPLC and characterized by ESI-MS.

### 10.2. Preparation of Saquinavir active ester

375 µmol Boc-Gly-OH is dissolved in anhydrous DCM at room temperature, and to this is added 265 µmol DMAP, 375µmol DIPCI and 110 µmol Saquinavir, formulated as Invirase^{®} (lactose, excipiens pro compresso obducto) at 0°C. The reaction mixture is allowed to warm to room temperature and stirred overnight. The product is ished with 0.1 N HCl, dried over MgSO₄, and evaporated under reduced pressure to yield the solid product SQV-Gly(Boc). The Boc protecting group is removed by incubating SQV-Gly(Boc) ester for 3h in a mixture of CH₂Cl₂ and TFA (50 :50). The product is recristallized from cold ether and dried under vacuum overnight. 47 µmol SQV-Gly ester is dissolved in 3ml anhydrous DMSO at room temperature, and to this is added 94 µmol SPDP. The reaction mixture pH is adjusted to 8.0 under constant stirring at room temperature. The reaction is left for 3h under constant stirring. The crude product SQV-Gly-COCH2CH2-SS-pyridyl is purified by semi-preparative RP-HPLC and characterized by ESI-MS.

### 10.3. Conjugation of peptide D-TAT (rrrqrrkkr; SEQ ID NO: 251), r3-L-TAT (rKKRrQRRr;

SEQ ID NO: 20) or r3-L-TATi (rRRQrRKKr; SEQ ID NO: 21)-D-Cysteine to Saquinavir 27 µmol SQV-Gly-COCH2CH2-SS-pyridyl is dissolved in 0.5ml PBS buffer pH 7.5 at room temperature, and to this is added 54 µmol D-TAT (rrrqrrkkr; SEQ ID NO: 251), r3-L-TAT (rKKRrQRRr; SEQ ID NO: 20) or r3-L-TATi (rRRQrRKKr; SEQ ID NO: 21) - D-Cysteine in 0.5ml PBS buffer pH 7.5. The reaction is left at room temperature for 3h under constant stirring. The crude conjugate D-TAT (rrrqrrkkr; SEQ ID NO: 251) - Saquinavir, r3-L-TAT (rKKRrQRRr; SEQ ID NO: 20) -Saquinavir and r3-L-TATi (rRRQrRKKr; SEQ ID NO: 21) -Saquinavir is purified by semi-preparative RP-HPLC and characterized by ESI-MS.

### 11. Preparation of transporter cargo conjugate molecules compriring INK Inhibitor sequences as cargo moieties

### 11.1. Identification of JNK inhibitor sequences

Amino acid sequences important for efficient interaction with JNK are identified by sequence alignments between known JBDs, e.g. between the JBDs of IB1, IB2, c-Jun and ATF2 defined a weakly conserved 8 amino acid sequence. Using this alignment JNK inhibitor sequences could be identified leading to a number of suitable JNK inhibitor sequences defined herein as SEQ ID NOs: 137 to 220.

### 11.2. Preparation of transporter cargo conjugate molecules comprising JNK Inhibitor sequences

Transporter cargo conjugate molecules comprising the above JNK Inhibitor sequences are synthesized by covalently linking the C-terminal end of JNK inhibitor sequences as defined herein to an N-terminal transporter construct according to generic formula (I) as defined above. The transporter construct according to generic formula (I) as defined above is prepared using classical Fmock synthesis and further analysed by Mass Spectrometry. The components are finally purified by HPLC. Linkage may be carried out using a linker consisting of two proline residues. This linker can be used to allow for maximal flexibility and prevent unwanted secondary structural changes.

### 11.3 Inhibition of Cell Death

Effects of the on JNK biological activities are studied. The construct is linked N-terminally to the Green Fluorescent Protein and the effect of this construct on pancreatic β-cell apoptosis induced by IL1 is evaluated. This mode of apoptosis is previously shown to be blocked by transfection with JBD₁₋₂₈₀ whereas specific inhibitors of ERK1/2 or p38 did not protect.

Insulin producing TC-3 cells are cultured in RPMI 1640 medium supplemented with 10% Fetal Calf Serum, 100 µg/mL Streptomycin, 100 units/mL Penicillin and 2 mM Glutamine. Insulin producing TC-3 cells are transfected with the inventive tansporter cargo conjugate molecules and IL-1 (10 ng/mL) is added to the cell culture medium. The number of apoptotic cells is counted at 48 hours after the addition of IL-1 using an inverted fluorescence microscope. Apoptotic cells are discriminated from normal cells by the characteristic "blebbing out" of the cytoplasm and are counted after two days.

### 12. Cellular Import of inventive transporter cargo conjugate molecules comprising TAT derived transporter constructs according to SEQ ID NOs: 8 to 136 and INK1 or IB1 derived cargo peptides according to any of SEQ ID NOs: 137 to 220

The ability of the transporter cargo conjugate molecules comprising TAT derived transporter constructs according to any of SEQ ID NOs: 8 to 136 and JNK1 or IB1 derived cargo peptides according to any of SEQ ID NOs: 137 to 220 to enter cells is evaluated. Inventive transporter constructs and inventive transporter cargo conjugate molecules are labeled by N-terminal addition of a glycine residue conjugated to fluorescein. These labeled peptides (1 µM) are added to TC-3 cell cultures, which are maintained as described in Example 11. At predetermined times cells are fished with PBS and fixed for five minutes in ice-cold methanol-acetone (1:1) before being examined under a fluorescence microscope. Fluorescein-labeled BSA (1 µM, 12 moles/mole BSA) is used as a control.

Fluorescent signals from these transporter constructs and inventive transporter cargo conjugate molecules are determined.

### 13. In vitro Inhibition of c-JUN, ATF2 and Elk1 Phosphorylation bar inventive transporter cargo conjugate molecules comprising TAT derived transporter constructs according to any of SEQ ID NOs: 8 to 136 and INK1 or IB1 derived cargo peptides according to any of SEQ ID NOs: 137 to 220

The effects of the inventive transporter cargo conjugate molecules comprising TAT derived transporter constructs according to any of SEQ ID NOs: 8 to 136 and JNK1 or 1B1 derived cargo peptides according to any of SEQ ID NOs: 137 to 220 on JNKs-mediated phosphorylation of their target transcription factors are investigated *in vitro.* Recombinant and non activated JNK1, JNK2 and JNK3 are produced using a TRANSCRIPTION AND TRANSLATION rabbit reticulocyte lysate kit (Promega) and used in solid phase kinase assays with c-Jun, ATF2 and EIk1, either alone or fused to glutathione-S-transferase (GST), as substrates. Dose response studies are performed wherein inventive transporter cargo conjugate molecules comprising TAT derived transporter constructs according to any of SEQ ID NOs: 8 to 136 and JNK1 or IB1 derived cargo peptides according to any of SEQ ID NOs: 137 to 220 are mixed with the recombinant JNK1, JNK2, or JNK3 kinases in reaction buffer (20 mM Tris-acetate, 1mM EGTA, 10 mM p-nitrophenyl-phosphate (pNPP), 5 mM sodium pyrophosphate, 10 mM p-glycerophosphate,1 mM dithiothreitol) for 20 minutes. The kinase reactions are then initiated by the addition of 10 mM MgCl₂ and 5 pCi ³³P-dATP and 1 µg of either GST-Jun (aa 1-89), GST-AFT2 (aa 1-96) or GST-ELK1 (aa 307-428). GST-fusion proteins are purchased from Stratagene (La Jolla, CA).

Ten µL of glutathione-agarose beads are also added to the mixture. Reaction products are then separated by SDS-PAGE on a denaturing 10 % polyacrylamide gel. Gels are dried and subsequently exposed to X-ray films (Kodak) and inhibition of c-Jun, ATF2 and EIk1 phosphorylation by these inventive transporter cargo conjugate molecules comprising TAT derived transporter constructs according to any of SEQ ID NOs: 8 to 136 and JNK1 or IB1 derived cargo peptides according to any of SEQ ID NOs: 137 to 220 is determined.

### 14. Inhibition Of IL-1 Induced Pancreatic β-cell Death By inventive transporter cargo conjugate molecules comprising TAT derived transporter constructs according to any of SEQ ID NOs: 8 to 136 and INK1 or IB1 derived cargo peptides according to any of SEQ ID NOs: 137 to 220

The effects of the inventive transporter cargo conjugate molecules comprising TAT derived transporter constructs according to any of SEQ ID NOs: 8 to 136 and JNK1 or IB1 derived cargo peptides according to any of SEQ ID NOs: 137 to 220 on the promotion of -cell apoptosis elicited by IL-1 is determined. TC-3 cell cultures are incubated for 30 minutes with 1 µM of inventive L-TAT-IB1(s) peptides followed by 10 ng/ml of IL-1. A second addition of peptide (1 µM) is performed 24 hours later. Apoptotic cells are counted after two days of incubation with IL-1 β using propidium iodide (red stained cell are dead cells) and Hoechst 33342 (blue stained cell are cells with intact plasma membrane) nuclear staining. Addition of the inventive TAT-IB(s) peptides inhibited IL-1-induced apoptosis of TC-3 cells cultured in the presence of IL-1 β for two days.

Long term inhibition of IL-1 induced cells death is examined by treating TC-3 cells as described above, except that incubation of the cells with the inventive transporter cargo conjugate molecules comprising TAT derived transporter constructs according to any of SEQ ID NOs: 8 to 136 and JNK1 or IB1 derived cargo peptides according to any of SEQ ID NOs: 137 to 220 and IL-1 is sustained for 12 days. Additional peptides (1µM) are added each day and additional IL-1 (10 ng/mL) is added every 2 days.

### 15. Inhibition Of Irradiation Induced Pancreatic β-Cell Death by inventive transporter cargo conjugate molecules comprising TAT derived transporter constructs according to any of SEQ ID NOs: 8 to 136 and INK1 or IB1 derived cargo peptides according to any of SEQ ID NOs: 137 to 220

JNK is also activated by ionizing radiation. To determine whether inventive transporter cargo conjugate molecules comprising TAT derived transporter constructs according to any of SEQ ID NOs: 8 to 136 and JNK1 or IB1 derived cargo peptides according to any of SEQ ID NOs: 137 to 220 would provide protection against radiation-induced JNK damage, "WiDr" cells are irradiated (30 Gy) in presence or absence of D-TAT (SEQ ID NO: 251), L-TAT (SEQ ID NO: 18) and inventive transporter cargo conjugate molecules comprising TAT derived transporter constructs according to any of SEQ ID NOs: 8 to 136 and JNK1 or IB1 derived cargo peptides according to any of SEQ ID NOs: 137 to 220 (1 µM added 30 minutes before irradiation). Control cells (CTRL) are not irradiated. Cells are analyzed 48 hours later by means of PI and Hoechst 3342 staining, as described above. N = 3, SEM are indicated.

### 16. Radioprotection to Ionizing Radiation by inventive transporter cargo conjugate molecules comprising TAT derived transporter constructs according to any of SEQ ID NOs: 8 to 136 and INK1 or IB1 derived cargo peptides according to any of SEQ ID NOs: 137 to 220

To determine the radioprotective effects of the inventive transporter cargo conjugate molecules comprising TAT derived transporter constructs according to any of SEQ ID NOs: 8 to 136 and JNK1 or IB1 derived cargo peptides according to any of SEQ ID NOs: 137 to 220, C57B1/6 mice (2 to 3 months old) are irradiated with a Phillips RT 250 R-ray at a dose rate of 0.74 Gy/min (17 mA, 0.5 mm Cu filter). Thirty minutes prior to irradiation, the animals are injected i.p. with inventive transporter cargo conjugate molecules comprising TAT derived transporter constructs according to any of SEQ ID NOs: 8 to 136 and JNK1 or IB1 derived cargo peptides according to any of SEQ ID NOs: 137 to 220. Briefly, mice are irradiated as follows: mice are placed in small plastic boxes with the head lying outside the box. The animals are placed on their back under the irradiator, and their neck fixed in a small plastic tunnel to maintain their head in a correct position. The body is protected with lead. Prior to irradiation mice are maintained on standard pellet mouse chow, however post irradiation mice are fed with a semi-liquid food that is renewed each day. The reaction of the lip mucosa is then scored by 2 independent observers according to the scoring system developed by Parkins *et al.* (Parkins et al, Radiotherapy & Oncology, 1: 165-173, 1983), in which the erythema status as well as the presence of edema, desquamation and exudation is quoted. Additionally, animals are weighed before each recording of their erythema/edema status.

### 17. Suppression of INK Transcription Factors by inventive transporter cargo conjugate molecules comprising TAT derived transporter constructs according to any of SEQ ID NOs: 8 to 136 and INK1 or IB1 derived cargo peptides according to any of SEQ ID NOs: 137 to 220

Gel retardation assays are carried out with an AP-1 doubled labeled probe (5'-CGC TTG ATG ACT CAG CCG GAA-3' (SEQ ID NO: 262). HeLa cell nuclear extracts that are treated or not for one hour with 5 ng/mlTNF-α, as indicated. TAT and inventive transporter cargo conjugate molecules comprising TAT derived transporter constructs according to any of SEQ ID NOs: 8 to 136 and JNK1 or IB1 derived cargo peptides according to any of SEQ ID NOs: 137 to 220 are added 30 minutes before TNF-α.

### 18. Evaluation of the neuroprotection against focal cerebral ischemia, in a permanent MCAO model - Determination of the efficacity of the protection at different doses.

Focal cerebral ischemia is induced in 12-days-old rats. Pups are anesthetized in an induction chamber with 2% isoflurane and during the operation anaesthesia is maintained using a mask under 2% isoflurane. MCAO is induced by electrocoagulating a main branch of the middle cerebral artery (MCA). Rats are placed on the right side, and an oblique dermal incision is made between the ear and eye. After excision of the temporal muscle, the cranial bone is removed from the frontal suture to a level below the zygomatic arch. The left MCA, exposed just after its apparition over the rhinal fissure, is permanently electrocoagulated at the inferior cerebral vein level before the MCA bifurcated into frontal and parietal branches. The cranial skin incision is then closed. Rat pups are then placed in an incubator maintained at 37°C until they awoke, and are then transferred to their mother.

6 hours later inventive transporter cargo conjugate molecules comprising TAT derived transporter constructs according to any of SEQ ID NOs: 8 to 136 and JNK1 or IB1 derived cargo peptides according to any of SEQ ID NOs: 137 to 220 are injected intraperitoneally. 24 hours after the coagulation, the rats are anesthetized with chloral hydrate and perfused through the ascending aorta with 4% paraformaldehyde in PBS. Brains are then removed and kept for 2 hours in the same fixative solution, and placed in a gradient of 30% sucrose in PBS for about 15 hours at 4°C. Brains are frozen in isopentane (-40°C) and stored at -20°C. Coronal cryostat sections of 50 µm are collected on glass slides. The sections are stained with cresyl violet. Each tenth section is analyzed and the total volume of the lesion is calculated using the Neuroleucida programme.

### 19. Evaluation of neuroprotection by inventive chimeric peptides after iv administration against focal cerebral ischaemia, in a transient MCAO model

Transient ischemia in adult mice. Using male ICR-CD1 mice (6 weeks old; 18-37 g; Harlan), ischemia is provoked by introducing a filament from the common carotid artery into the internal carotid and advancing it into the arterial circle, thereby occluding the middle cerebral artery. The regional cerebral blood flow is measured by laser Doppler flowmetry, with a probe fixed on the skull throughout the ischemia until 10 min after reperfusion. Rectal temperature is measured and maintained at 37 °C. The mice are killed 48 h after reperfusion. Serial cryostat sections 20 µm thick are traced using a computer-microscope system equipped with the Neurolucida program (MicroBrightField) and the volumes of the ischemic area and of the whole brain are calculated (blinded) with the Neuroexplorer program. The infarct volume sizes (mm³) after bolus iv administration of placebo and inventive transporter cargo conjugate molecules comprising TAT derived transporter constructs according to any of SEQ ID NOs: 8 to 136 and JNK1 or IB1 derived cargo peptides according to any of SEQ ID NOs: 137 to 220, 1,3 mg/kg 6 hours after reperfusion (30 minutes clamp) in an adult mice model is determined.

### 20. Assay on neuronal cultures by measuring LDH release following NMDA stimulation

The neuroprotective effect of the inventive transporter cargo conjugate molecules comprising TAT derived transporter constructs according to any of SEQ ID NOs: 8 to 136 and JNK1 or IB1 derived cargo peptides according to any of SEQ ID NOs: 137 to 220 is evaluated in sister cultures pre-treated for 30 min with the indicated concentrations of peptides or MK-801 before continuous exposure to 100 µM NMDA. After 12 h of NMDA treatment, in cultures pretreated with 5 µM of inventive transporter cargo conjugate molecules comprising TAT derived transporter constructs according to any of SEQ ID NOs: 8 to 136 and JNK1 or IB1 derived cargo peptides according to any of SEQ ID NOs: 137 to 220 the degenerative changes due to NMDA exposure, the morphological appearance, number and distribution of the neurons are determined.

Cortical neuronal culture. Small pieces of cortex are dissected from the brains of two day old rat pups, incubated with 200 units of papain for 30 min at 34 °C, and then the neurons are plated at densities of approximately 1 x 10⁶ cells/plate on dishes pre-coated with 100 µg/ml poly-D-lysine. The plating medium consisted of B27/Neurobasal (Life Technologies, Gaithersburg, MD) supplemented with 0.5mM glutamine, 100 U/ml penicillin and 100 ug/ml streptomycin.

Lactate dehydrogenate (LDH) cytotoxicity assay. LDH released into the bathing medium 12, 24 and 48 h after NMDA administration is measured using the Cytotox 96 non-radioactive cytotoxicity assay kit (Promega, WI).

### 21. Inhibition of endogenous INK activity in HepG2 cells using an all-in one well approach.

HepG2 cells are seeded at 3'000 cells/well the day prior the experiment. Then, increasing concentrations of either interleukin-1 (IL-1β] or tumor necrosis factor α [TNFα] (a) are added to activate JNK for 30 minutes. Cells are lysed in 20mM Hepes, 0.5% Tween pH 7.4 and processed for AlphaScreen JNK. (b) Z' for the JNK activity induced by 10 ng/ml IL-1 and measured in 384 wells/plate (n=96). (c) Inhibition of endogenous IL-1 β-induced JNK activity with chemical JNK inhibitors [staurosporin and SP600125]. (d) Effect of inventive inventive transporter cargo conjugate molecules comprising a TAT derived transporter constructs according to SEQ ID NOs: 8 to 136 and peptidic inhibitors L-TAT-IB1(s) on IL-1α dependent JNK activity.
Methods: Alphascreen kinase assay
Principle: AlphaScreen is a non-radioactive bead-based technology used to study biomolecular interactions in a microplate format. The acronym ALPHA stands for Amplified Luminescence Proximity Homogenous Assay. It involves a biological interaction that brings a "donor" and an "acceptor" beads in close proximity, then a cascade of chemical reactions acts to produce an amplified signal. Upon laser excitation at 680 nm, a photosensitizer (phthalocyanine) in the "donor" bead converts ambient oxygen to an excited singlet state. Within its 4 µsec half-life, the singlet oxygen molecule can diffuse up to approximately 200 nm in solution and if an acceptor bead is within that proximity, the singlet oxygen reacts with a thioxene derivative in the "acceptor" bead, generating chemiluminescence at 370 nm that further activates fluorophores contained in the same "acceptor" bead. The excited fluorophores subsequently emit light at 520-620 nm. In the absence of an acceptor bead, singlet oxygen falls to ground state and no signal is produced.

Kinase reagents (B-GST-cJun, anti P-cJun antibody and active JNK3) are first diluted in kinase buffer (20 mM Tris-HCl pH 7.6, 10 mM MgCl₂, 1 mM DTT, 100 µM Na₃VO₄, 0.01% Tween-20) and added to wells (15 µl). Reactions are then incubated in presence of 10 µM of ATP for 1 h at 23°C. Detection is performed by an addition of 10 µl of beads mix (Protein A acceptor 20 µg/ml and Streptavidin donor 20 µg/ml), diluted in detection buffer (20 mM Tris-HCl pH 7.4, 20 mM NaCl, 80 mM EDTA, 0.3% BSA), followed by an another one-hour incubation at 23°C in the dark. For measurement of JNK endogenous activity, kinase assays are performed as described above except active JNK3 is replaced by cells lysates and reaction kinase components are added after the cells lysis. B-GST-cjun and P-cJun antibody are used at the same concentrations whereas ATP is used at 50 µM instead of 10 µM. AlphaScreen signal is analyzed directly on the Fusion or En Vision apparatus.

### 22. Treatment of noise-trauma

Inventive transporter cargo conjugate molecules comprising TAT derived transporteur constructs according to any of SEQ ID NOs: 8 to 136 and JNK1 or IB1 derived cargo peptides according to any of SEQ ID NOs: 137 to 220 are applied onto the round window membrane of the cochlea of 3 groups of guinea pigs (each group with 6 animals) in 2 microliters of a gel formulation of 2.6% buffered hyaluronic acid (Hylumed, Genzyme Corp.) at a concentration of 100 µM either 30 minutes before noise trauma (120 dB at 6 kHz during 30 minutes) or 30 minutes or 4 hours thereafter. Untreated ears served as control. Hearing threshold shifts are evaluated by auditory brainstem response measurements 20 minutes after noise trauma (temporary threshold shift, TTS) and 15 days following the trauma (permanent threshold shift, PTS). Administration of D-TAT-IB1(s) protected against permanent hearing loss even if applied after exposure to excessive noise compared to non-treated ears.

### 23. Evaluation of the therapeutical activity of inventive transporter cargo conjugate molecules comprising TAT derived transporter constructs according to any of SEQ ID NOs: 8 to 136 and JNK1 or IB1 derived cargo peptides according to any of SEQ ID NOs: 137 to 220 in the treatment of Colitis

### a) Test system:

i) Species/Strain: Mouse /BALB/c
ii) Source: Harlan Israel, Ltd.
iii) Gender: Female
iv) Total No. of Animals: n=150
v) Age: Young adults, 7 weeks of age at study initiation
vi) Body Weight: Weight variation of animals at the time of treatment initiation does not exceed ± 20% of the mean weight.
vii) Animals Health: The health status of the animals used in this study is examined on arrival, only animals in good health are acclimatized to laboratory conditions (at least seven days) and are used in the study.
viii) Randomization: Animals are randomly assigned to experimental groups according to a Table of Random Numbers.
ix) Termination: At the end of the study surviving animals are euthanized by cercical dislocation.

### b) Test Procedures

Colitis is induced by administration of TNBS dissolved in 50% Ethanol

All animals are then treated with doses of inventive transporter cargo conjugate molecules comprising TAT derived transporter constructs according to any of SEQ ID NOs: 8 to 136 and JNK1 or IB1 derived cargo peptides according to any of SEQ ID NOs: 137 to 220 in the range of 0.1 to 1000 µg/kg, either intraperitoneally or subcutaneously, as a single or repeated daily doses (see above).

### c) Observations and Examinations

### i)Clinical signs

Throughout the duration of the above experiment, careful clinical examinations are carried out and recorded. Observations included changes external appearance, e.g. of the skin, fur, eyes, mucous membranes, occurrence of secretions and excretions (e.g. diarrhea), and autonomic activity. Changes in gait, posture and response to handling, as well as the presence of bizarre behavior, tremors, convulsions, sleep and coma are also noted.

### ii) Body weights

Determination of individual body weight of animals is made on a daily basis.

### iii) Clinical assessment of colitis

Body weight, stool consistency and bleeding per rectum are all recorded daily and served as the parameters of disease severity score:

| Score | Weight loss (%) | Stool consistency | Presence of blood per rectum |
|---|---|---|---|
| 0 | None | Normal | Negative |
| 1 | 1-5 | Redness, swelling of the anus | Negative |
| 2 | 5-10 | Loose stool | Negative |
| 3 | 10-15 | Diarrhea | Negative |
| 4 | >15 | Diarrhea | Bleeding |
| 5 | Death | | |

### iv) Gross pathology of the colon

On the last day of the experiment, animals are euthanized and the colon is removed for gross pathology evaluation according to the following score:

| Grade | Signs |
|---|---|
| 0 | No abnormalities detected |
| 1 | Edema and redness on one location |
| 2 | Edema and redness on more than one location, or a very massive endema and redness capture more than 50% of the colon |
| 3 | One ulcer |
| 4 | More than one ulcer or a very long severe ulcer |

### 24. Determining the activity of inventive transporter cargo conjugate molecules comprising TAT derived transporter constructs according to any of SEQ ID NOs: 8 to 136 and INK1 or IB1 derived cargo peptides according to any of SEQ ID NOs: 137 to 220 in the treatment of viral infections - varicella-zoster virus (VZV)

Determination of the activity of inventive transporter cargo conjugate molecules comprising TAT derived transporter constructs according to any of SEQ ID NOs: 8 to 136 and JNK1 or IB1 derived cargo peptides according to any of SEQ ID NOs: 137 to 220 in cultured host cells (human foreskin fibroblasts (HFFs)). Viruses are obligate intracellular parasites that require a functional cell environment to complete their lifecycle; dying cells do not support virus replication. Additionally, inhibitors of cell functions may be toxic to cells, which could non-specifically prevent virus growth. Thus, sick or dying host cells could exhibit nonspecifically reduced virus titers. Since this may falsify the results, a cytotoxicity assay is carried out first, determining the tolerance of the cultured cells to the test compound. Subsequently, a plaque reduction assay is carried out and then activity of the inventive transporter cargo conjugate molecules comprising TAT derived transporter constructs according to any of SEQ ID NOs: 8 to 136 and JNK1 or IB1 derived cargo peptides according to any of SEQ ID NOs: 137 to 220 is tested with repect to Viral Zoster Virus (VZV) in infected cells.

### A) Determination of the cytotoxicity of inventive transporter cargo conjugate molecules comprising TAT derived transporter constructs according to any of SEQ ID NOs: 8 to 136 and JNK1 or IB1 derived cargo peptides according to any of SEQ ID NOs: 137 to 220:

For determination of toxicity, cultured cells (human foreskin fibroblasts (HFFs)) are seeded in 96-well tissue culture plates. Medium containing DMSO (same level as 5 µM inventive transporter cargo conjugate molecules comprising TAT derived transporter constructs according to any of SEQ ID NOs: 8 to 136 and JNK1 or IB1 derived cargo peptides according to any of SEQ ID NOs: 137 to 220) is added at several concentrations of (1, 2, and 5 µM) for 24 h. Subsequently, a Neutral Red assay is carried out. Neutral Red colorimetric assays for cytotoxicity assays (in sets of 6 replicates) are used to set the maximum dose for subsequent efficacy assays (as performed in Taylor et al, 2004, J. Virology, 78:2853-2862). Live cells absorb Neutral Red and, accordingly, the level of absorbance is a quantitative measure of cell viability and number. Neutral Red uptake is directly proportional to the number of cells and also reflects normal endocytosis. Therefore, a brief pulse of Neutral Red is added to the medium at 0 or 24 hours. After fixation and extraction, dye is added and the amount of dye in each sample is measured in an ELISA plate reader at 540nm.

### B) Plaque reduction assay to evaluate the antiviral effects of inventive transporter cargo conjugate molecules comprising TAT derived transporter constructs according to any of SEQ ID NOs: 8 to 136 and INK1 or IB1 derived cargos peptides according to any of SEQ ID NOs: 137 to 220 against varicella-zoster virus (VZV):

To determine whether inventive transporter cargo conjugate molecules comprising TAT derived transporter constructs according to any of SEQ ID NOs: 8 to 136 and JNK1 or IB1 derived cargo peptides according to any of SEQ ID NOs: 137 to 220 exhibit a dose-dependent antiviral effect, a range of concentrations surrounding the standard 1 µM dose are tested. In this plaque reduction assay, confluent human foreskin fibroblasts (HFFs) in 24-well plates are inoculated with VZV-infected HFFs at a ratio of 1:100 (multiplicity of infection MOI)=0.01) and adsorbed to the cells for 2 hours. The excess virus is ished out, and medium containing 0 (DMSO only), 0.5, 1, or 2 inventive transporter cargo conjugate molecules comprising TAT derived transporter constructs according to any of SEQ ID NOs: 8 to 136 and JNK1 or IB1 derived cargo peptides according to any of SEQ ID NOs: 137 to 220 is added. One sample is taken at this time to measure the initial level of infection; wherein each well contained ∼150 pfu. After 24 hours, duplicate wells are trypsinized, and then the cell suspensions are titered on MeWo cell monolayers in triplicate to determine the number of VZV-infected cells in each sample. During unrestricted growth, VZV usually increases by 10-fold over 1 day because it propagates by cell-cell spread. This is what is observed for the cultures treated with DMSO alone, which yielded 1200 ± 430 pfu. The cytotoxicity and efficacy data are determined. From these data, a preliminary Selective Index (Tox/ECso) of 5.0 µM / 0.3 µM is calculated.

### C) Measurement of varicella-zoster virus (VZV) replication in human foreskin fibroblasts (HFFs) with inventive transporter cargo conjugate molecules comprising TAT derived transporter constructs according to any of SEQ ID NOs: 8 to 136 and JNK1 or IB1 derived cargo peptides according to any of SEQ ID NOs: 137 to 220:

To determine the minimum effective dose of inventive transporter cargo conjugate molecules comprising TAT derived transporter constructs according to any of SEQ ID NOs: 8 to 136 and JNK1 or IB1 derived cargo peptides according to any of SEQ ID NOs: 137 to 220 that prevents varicella-zoster virus (VZV) replication in human foreskin fibroblasts (HFFs) confluent monolayers of HFFs are inoculated with VZV-BAC-Luc strain for 2h, then treated for 24h with inventive transporter cargo conjugate molecules comprising TAT derived transporter constructs according to any of SEQ ID NOs: 8 to 136 and JNK1 or IB1 derived cargo peptides according to any of SEQ ID NOs: 137 to 220 in concentrations of 0.25, 0.5, or 1.0 µM or with the a negative control (DAK, 1.0 µM). Virus yield is measured by luciferase assay. Samples are in triplicate and the average luminescence is shown; error bars represent the standard deviation of the mean.

### 25. Determining the activiy of inventive transporter cargo conjugate molecules comprising TAT derived transporter constructs according to any of SE ID NOs: 8 to 136 and INK1 or IB1 derived cargo peptides according to any of SEQ ID NOs: 137 to 220 in the treatment of Chronic Obstructive Pulmonary Disease (COPD)

In order to determine the activity of the exemplary inventive transporter cargo conjugate molecules comprising TAT derived transporter constructs according to any of SEQ ID NOs: 8 to 136 and JNK1 or IB1 derived cargo peptides according to any of SEQ ID NOs: 137 to 220 in the treatment of Chronic Obstructive Pulmonary Disease (COPD) these inventive transporter cargo conjugate molecules are used in an animal model of Bleomycin induced acute lung inflammation and fibrosis. The protocol of bleomycin induced inflammation and fibrosis has been described before in the literature. The aim of the Experiment is to investigate the effect of these inventive transporter cargo conjugate molecules by subcutaneous (s.c.) route on neutrophil recruitment in broncho alveolar lavage (BAL) and lung in bleomycin induced inflammation and fibrosis:
- at 1 day after a single bleomycin administration (10 mg/kg)
- and at day 10 with the development of fibrosis

### 1) Method and experimental approach

The test compounds selected from inventive transporter cargo conjugate molecules comprising TAT derived transporter constructs according to any of SEQ ID NOs: 8 to 136 and JNK1 or IB1 derived cargo peptides according to any of SEQ ID NOs: 137 to 220 at two doses and vehicle control are given s.c. with a single intranasal administration of bleomycin and mice are analyzed after 1 and 10 days. The animals used in the model are 10 C57BL/6 mice (8 weeks old) per group. The experimental groups include vehicle, 0.001 mg/kg of inventive transporter cargo conjugate molecules comprising TAT derived transporter constructs according to any of SEQ ID NOs: 8 to 136 and JNK1 or 1B1 derived cargo peptides according to any of SEQ ID NOs: 137 to 220 and 0.1 mg/kg of these inventive transporter cargo conjugate molecules, and the treatment consists of repeated sub-cutaneous administration of these inventive transporter cargo conjugate molecules prior to bleomycin administration every 3 days. Acute lung inflammation at 24h is monitored by BAL lavage, cytology, cell counts, and lung myeloperoxidase activity. The effect of the compound is compared with vehicle controls. Lung fibrosis is assessed histologically using hematoxylin and eosin staining at day 10 after the single dose of bleomycin.

### 1.1) Bleomycin administration

Bleomycin sulfate in saline (10 mg/kg body weight) from Bellon Laboratories (Montrouge, France) or saline are given through the airways by nasal instillation in a volume of 40 µL under light ketamine-xylasine anesthesia. The groups for Bleomycin administration for both bleomycin induced inflammation and fibrosis included: Vehicle, 0.001 mg/kg of the inventive transporter cargo conjugate molecules comprising TAT derived transporter constructs according to any of SEQ 1D NOs: 8 to 136 and JNK1 or IB1 derived cargo peptides according to any of SEQ ID NOs: 137 to 220 and 0.1 mg/kg of these inventive transporter cargo conjugate molecules. The route for bleomycin induced inflammation is subcutaneous (s.c.) route, and administration occurrs as a single dose. The route for bleomycin induced fibrosis is subcutaneous (s.c.) route, and administration occurred 3 times in 10 days.

### 1.2) Bronchoalveolar lavage fluid (BALF)

After incision of the trachea, a plastic cannula is inserted and airspaces are ished using 0.3ml of PBS solution, heated to 37°C. The samples collected are dispatched in 2 fractions: the first one (1 ml corresponding to the 2 first lavages) is used for mediator measurement and the second one for the cell determination (4ml). The first fraction is centrifuged (600g for 10 min) and supernatant is fractionated and kept at -80°C until mediator determination. The cell pellet is then resuspended in 0.4ml sterile NaCl, 0,9%, and pooled with the second fraction and is used for cell counts.

### 1.3) Lung homogenization

After BAL the whole lung is removed and placed inside a microtube (Lysing matrix D, Q Bio Gene, Illkrich, France) with 1 ml of PBS, total lung tissue extract is prepared using a Fastprep^{®} system (FP120, Q Bio Gene, Illkrich, France), the extract is then centrifuged and the supernatant stored at -80°C before mediator measurement and collagen assay with Sircol Collagen Assay (France Biochem Division, France).

### 1.4) Cell count and determination

Total cell count is determined in BAL fluid using a Malassez hemocytometer. Differential cell counts are performed on cytospin preparations (Cytospin 3, Thermo Shandon) after staining with MGG Diff-quick (Dade Behring AG). Differential cell counts are made on 200 cells using standard morphological criteria.

### 1.5) TNF measurement

TNF level in BALF is determined using ELISA assay kits (Mouse DuoSet, R&D system, Minneapolis, USA) according to manufacturer's instructions. Results are reported as pg/ml.

### 1.6) MPO-measurement

MPO-levels are measured upon administration of inventive transporter cargo conjugate molecules comprising TAT derived transporter constructs According to any of SEQ ID NOs: 8 to 136 and JNK1 or IB1 derived cargo peptides according to any of SEQ ID NOs: 137 to 220.

### 1.7) Histology

After BAL and lung perfusion, the large lobe is fixed in 4% buffered formaldehyde for standard microscopic analysis. 3-µm sections are stained with hematoxylin and eosin (H&E).

### 26. Determining the activity of inventive transporter cargo conjugate molecules comprising TAT derived transporter constructs according to any of SEQ ID NOs: 8 to 136 and INK1 or IB1 derived cargo peptides according to any of SEQ ID NOs: 137 to 220 in the treatment of Alzheimer's disease

In order to determine the activity of the exemplary inventive transporter cargo conjugate molecules comprising TAT derived transporter constructs according to any of SEQ ID NOs: 8 to 136 and JNK1 or IB1 derived cargo peptides according to any of SEQ ID NOs: 137 to 220 in Alzheimer's disease, these peptides are evaluated in the hAPP-transgenic mice model overexpressing APP751 with London and Swedish mutations using the behavioral Morris Water Maze test as well as immunohistological tests measuring plaque load and ELISA tests measuring ß-amyloid₁₋₄₀ and ß-amyloid₁₋₄₂ levels in the brain of mice.

### a) METHODS

### i) Introduction

The study is designed to evaluate the efficacy of the inventive transporter cargo conjugate molecules comprising TAT derived transporter constructs according to any of SEQ ID NOs: 8 to 136 and JNK1 or IB1 derived cargo peptides according to any of SEQ ID NOs: 137 to 220 on behavioral, biochemical and histological markers using 5 months (± 2 weeks) old female hAPP Tg mice. Therefore, mice are treated every two or three weeks up to 4 months and in the end of the treatment period behavior is evaluated in the Morris Water Maze. At sacrifice brain, CSF and blood are collected. Aß40and Aß42 levels are determined in four different brain homogenate fractions as well as in CSF of Tg mice. Plaque load is quantified in the cortex and the hippocampus of 8 Tg animals per treatment group.

### ii) Animals

Female Tg mice with a C57BL/6xDBA background and an age of 5 months (± 2 week) are randomly assigned to treatment groups 1 to 3 (n = 12). Animals are subjected to administration of vehicle or inventive transporter cargo conjugate molecules comprising TAT derived transporter constructs according to any of SEQ ID NOs: 8 to 136 and JNK1 or IB1 derived cargo peptides according to any of SEQ ID NOs: 137 to 220 in two different concentrations beginning at 5 months of age and continued for up to 4 months with subcutaneous (s.c.) applications every second or third week. All animals which are used for the present study had dark eyes and are likely to perceive the landmarks outside the MWM pool. However, it had to be excluded that seeing abilities of an animal are poor, which is controlled in the visible platform training, the so called pretest, before treatment start for all animals including reserves enclosed to the study. In case a seeing handicap for a specific animal would have been affirmed, the mouse would have been excluded from the study.

### iii) Animal Identification and Housing

Mice are individually identified by ear markings. They are housed in individual ventilated cages (IVCs) on standardized rodent bedding supplied by Rettenmaier^{®}. Each cage contained a maximum of five mice. Mice are kept according to the JSW Standard Operating Procedures (*SOP GEN011*) written on the basis of international standards. Each cage is identified by a colored card indicating the study number, sex, the individual registration numbers (IRN) of the animals, date of birth, as well as the screening date and the treatment group allocation. The temperature during the study is maintained at approximately 24°C and the relative humidity is maintained at approximately 40 - 70 %. Animals are housed under a constant light-cycle (12 hours light/dark). Normal tap water is available to the animals *ad libitum.*

### iv) Treatment

Forty female hAPP transgenic mice are treated with either 0.1 mg/kg b.w./every two weeks or 10 mg/kg b.w./every three weeks of inventive transporter cargo conjugate molecules comprising TAT derived transporter constructs according to any of SEQ ID NOs: 8 to 136 and JNK1 or IB1 derived cargo peptides according to any of SEQ ID NOs: 137 to 220 in two different dosages (n=12/group) or treated with the vehicle (n=12) s.c. once every three weeks over four months.

### v) Morris Water Maze (MWM)

The Morris Water Maze (MWM) task is conducted in a black circular pool of a diameter of 100 cm. Tap water is filled in with a temperature of 22±1°C and the pool is virtually divided into four sectors. A transparent platform (8 cm diameter) is placed about 0.5 cm beneath the water surface. During the whole test session, except the pretest, the platform is located in the southwest quadrant of the pool. One day before the 4 days lasting training session animals had to perform a so called "pre-test" (two 60 sec lasting trials) to ensure that the seeing abilities of each animal are normal. Only animals that fulfilled this task are enclosed to the MWM testing. In the MWM task each mouse had to perform three trials on four consecutive days. A single trial lasted for a maximum of maximum one minute. During this time, the mouse had the chance to find the hidden, diaphanous target. If the animal could not find a "way" out of the water, the investigator guided to or placed the mouse on the platform. After each trial mice are allowed to rest on the platform for 10-15 sec. During this time, the mice had the possibility to orientate in the surrounding. Investigations took place under dimmed light conditions, to prevent the tracking system from negative influences (Kaminski; PCS, Biomedical Research Systems). On the walls surrounding the pool, posters with black, bold geometric symbols (e.g. a circle and a square) are fixed which the mice could use the symbols as landmarks for their orientation. One swimming group per trial consists of five to six mice, so that an intertrial time of about five to ten minutes is ensured. For the quantification of escape latency (the time [second] - the mouse needs to find the hidden platform and therefore to escape from the water), of pathway (the length of the trajectory [meter] to reach the target) and of the abidance in the goal quadrant a computerized tracking system is used. The computer is connected to a camera placed above the centre of the pool. The camera detected the signal of the light emitting diode (LED), which is fixed with a little hairgrip on the mouse's tail. One hour after the last trial on day 4 the mice had to fulfill a so-called probe trial. At this time, the platform is removed from the pool and during the one-minute probe trial; the experimentator counts the number of crossings over the former target position. Additionally the abidance in this quadrant as well as the three other quadrants is calculated. Through out this trial a mouse could not get any, howsoever-natured, clue from the platform.

### vi) Tissue Sampling

At the end of the treatment period, and following all behavioral testing, all remaining mice (n = 28) are sacrificed. Therefore, all mice are sedated by standard inhalation anesthesia (Isofluran, Baxter) as described in *SOP MET030.* Cerebrospinal fluid (CSF) is obtained by blunt dissection and exposure of the *foramen magnum.* Upon exposure, a Pasteur pipette is inserted to the approximate depth of 0.3 - 1 mm into the *foramen magnum.* CSF is collected by suctioning and capillary action until flow fully ceases. Two aliquots of each sample are immediately frozen and kept at -80°C until ready for further analysis with ELISA technique. After CSF sampling, each mouse is placed in dorsal recumbence, thorax is opened and a 26-gauge needle attached to a 1 cc syringe is inserted into the right cardiac ventricular chamber. Light suction is applied to the needle and blood is collected into EDTA and consequently used to obtain plasma. To get plasma, blood samples from each mouse are spun at 1,750 rpm (700g) for 10 minutes in a centrifuge (GS - 6R Beckman) using a rotor with swing buckets (GH - 3.8 Beckman). Plasma is frozen and stored at -20°C until further analysis. After blood sampling transgenic mice are intracardially perfused with 0.9% sodium chloride. Brains are rapidly removed the cerebellum is cut off. The right hemispheres of all mice are immersion fixed in freshly produced 4% Paraformaldehyde/PBS (pH 7.4) for one hour at room temperature. Thereafter brains are transferred to a 15% sucrose PBS solution for 24 hours to ensure cryoprotection. On the next day brains are frozen in isopentane and stored at-80°C until used for histological investigations (SOP *MET042).* The left hemispheres are weighed and frozen in liquid nitrogen and stored at -80°C for biochemical analysis.

### vii) Determination of Aß₁₋₄₀ and Aß₁₋₄₂

In four different brain homogenate fractions of each Tg mouse as well as in CSF samples the Aß₁₋₄₀ and Aß₁₋₄₂ levels are evaluated with ELISA technique. Highly sensitive Aß₁₋₄₀ and Aß₁₋₄₂ ELISA test kits are purchased from *The Genetics Company™,* Switzerland *(SOP MET058).* CSF is prepared as described above. For the brain homogenates frozen hemispheres are homogenized in TRIS buffered saline (TBS) - buffer (5 ml) containing protease inhibitor cocktail. 1.25ml of this initial brain TBS homogenate is stored at -80°C, 1.25 ml have been further investigatated. The remaining brain homogenate (2.5 ml) is centrifuged and the resulting supernatant (= TBS fraction) is aliquoted and kept at -20°C until ELISA determination. The pellet is suspended in Triton X-100 (2.5 ml), centrifuged and the supernatant (= Triton X-100 fraction) is aliquoted and kept at -20°C. These steps are repeated with SDS (2.5 ml). The pellet out of the SDS fraction is suspended in 70 % formic acid (0.5ml) prior to subsequent centrifugation. The obtained supernatant is neutralized with 1 M TRIS (9.5 ml) aliquoted and kept at -20°C (= FA fraction). Samples of the four brain homogenate fraction (TBS, Triton X-100, SDS, and FA) are used for Aß₁₋₄₀ and Aß₁₋₄₂ determination with ELISA technique. ELISA test kits are purchased from *The Genetics Company*™, Switzerland *(SOP Met062).* It could be assumed that TBS and Triton X-100 solubilize monomeric to oligomeric structures. Polymers like protofibrils and water insoluble fibrils could be dissolved in SDS and FA. In this regard the investigation of all four fractions also provides insight in A polymerization status.

### viii) Evaluation of Brain Morphology

Brain tissues of all Tg animals investigated are handled in exactly the same way to avoid bias due to variation of this procedure. From brain halves of 24 Tg mice (8 of each group) 20 cryo-sections per layer (altogether 5 layers), each 10µm thick (Leica CM 3050S) are sagittally cut and 5 (one from each layer) are processed and evaluated for quantification of plaque load. The five sagittal layers corresponded with the Figures 104 to 105, 107 to 108, 111 to 112, 115 to 116 and 118 to 119 according to the morphology atlas "The Mouse Brain" from Paxinos and Franklin (2nd edition). The first layer is specified by the requirement to include the whole hippocampus with it's regions CA1, CA2, CA3, GDIb and GDmb. Immunoreactivity is quantitatively evaluated in the hippocampus and in the cortex using the monoclonal human Aß-specific antibody 6E10 (Signet) as well as ThioflavinS staining. Remaining brain hemispheres or tissue not used are saved and stored at JSW CNS until the end of the project.

### b) EVALUATION

### i) Behavior

In the Morris Water Maze trials length of swimming path, escape latencies, swimming speed and in the probe trial crossings over the former platform position and the time spent in each quadrant of the pool are measured for each Tg animal with a special computer software.

### ii) Biochemical Evaluation

From all Tg mice CSF samples as well as samples from the brain preparations are analyzed with commercially available AB₁₋₄₀ and Aß₁₋₄₂ ELISAs. Measurements of adequate standards are performed concurrently. Samples from brain preparations are analyzed in duplicates. Due to the small sample amount CSF samples are analyzed in a single measurement only.

### iii) Histology

### i1) Measurement of Amyloid Depositions and Plaque Load

For 6E10 immunohistochemistry the following evaluation procedure is used:
aa) Contrasting the image for visualization of slice borders without applying the contrast on the image.
bb) Interactive drawing of the cortical outlines and the following measurement of the cortical area (=region area).
cc) Interactive drawing of the area of interest (AOI), in which stained objects are detected over a certain intensity based threshold level (the same for each image) and above a size of 8 µm².
dd) Measurement of the area of each object, the sum of stained area in the AOI as well as the number of objects after a smooth contrasting to enhance signal/noise ratio (the same for each image).
ee)Repetition of aa)-dd) for the hippocampus.
ff) Calculation of the mean plaque size (= "sum area of plaques / number of plaques"), the relative plaque number and area (= "number of plaques / region area" and "sum area of plaques / region area * 100").
gg) Automated data export into an Excel spread sheet, including the parameters "image title, region area, number of plaques, sum of plaque area, relative plaque number, relative plaque area and mean plaque size. A field for remarks is used to record image quality and exclusion criteria, respectively. Exclusion criteria are missing parts of the slice, many wrinkles, dominant flaws or staining inconsistencies (e.g. due to bulges, which can impede the full reaction of the blocking reagent).
hh) Closing the image without saving (to keep raw data raw).

### 27. Determining the activity of inventive transporter cargo conjugate molecules comprising TAT derived transporter constructs according to any of SEQ ID NOs: 8 to 136 and INK1 or IB1 derived cargo peptides according to any of SEQ ID NOs: 137 to 220 in the treatment of Diabetes Type 2

This is designed to determine the activity of inventive transporter cargo conjugate molecules comprising TAT derived transporter constructs according to any of SEQ ID NOs: 8 to 136 and JNK1 or IB1 derived cargo peptides according to any of SEQ ID NOs: 137 to 220 in the treatment of Diabetes Type 2, particularly to determine the effect of chronic treatment with these inventive transporter cargo conjugate molecules in the db/db mice model of type 2 diabetes by evaluating fasting blood glucose levels every third day (28 days)

### a) Materials and methods

### i) Animals

A total of twenty (20) male db/db mice (8 weeks old) are obtained from Charles River (Germany). Upon arrival, animals are group housed (n = 6-7/group) and offered regular rodent chow (Altromin standard #1324 chow; C. Petersen, Ringsted, Denmark) and water *ad libitum* unless otherwise stated.

The mice are housed under a 12:12 L/D cycle (lights on at 4:00 and lights off at 16:00) and in temperature and humidity controlled rooms.

### ii) Groups and randomization

On day -4, mice are randomized according to blood glucose level (fasted; blood glucose measured on Biosen S line analyzer (EKF diagnostic, Germany) to participate in one of the following drug treatment groups (n=6):
1) Vehicle control, S.C. (physiological saline)
2) inventive transporter cargo conjugate molecules comprising TAT derived transporter constructs according to any of SEQ ID NOs: 8 to 136 and JNK1 or IB1 derived cargo peptides according to any of SEQ ID NOs: 137 to 220; 1 mg/kg; s.c.
3) inventive transporter cargo conjugate molecules comprising TAT derived transporter constructs according to any of SEQ ID NOs: 8 to 136 and JNK1 or IB1 derived cargo peptides according to any of SEQ ID NOs: 137 to 220; 10 mg/kg; s.c

All doses listed are calculated for the free-base. Drug purity: 95.28%, peptide content: 78.0%. All compounds are administered sub-cutaneously (s.c.) in a volume of 3 ml/kg. The formulation instructions for vehicle control and inventive transporter cargo conjugate molecules comprising TAT derived transporter constructs according to any of SEQ ID NOs: 8 to 136 and JNK1 or IB1 derived cargo peptides according to any of SEQ ID NOs: 137 to 220 are as follows:
First, inventive transporter cargo conjugate molecules comprising TAT derived transporter constructs according to any of SEQ ID NOs: 8 to 136 and JNK1 or IB1 derived cargo peptides according to any of SEQ ID NOs: 137 to 220 are dissolved in the vehicle. The formulations (concentrations of 0.33 and 3.3 mg/ml, corresponding to the doses of 1 and 10 mg/kg, respectively) are prepared according to the procedure detailed below. Concentrations are calculated and expressed taking into account test items purity and peptide content (multiplier coefficient is 1.346).

- Preparation of a stock solution: the freeze-dried inventive transporter cargo conjugates molecules comprising TAT derived transporter constructs according to any of SEQ ID NOs: 8 to 136 and JNK1 or IB1 derived cargo peptides according to any of SEQ ID NOs: 137 to 220 is thawed for one hour minimum and prepared as a stock solution in the vehicle at 1 mM. Aliquots are prepared for each treatment day and stored at approximately -80°C. Dilutions of this stock solution to the required concentrations are performed on each treatment day;
- Storage of the stock solution: at approximately -80°C;
- Storage of the diluted preparations: at room temperature for 24 hours maximum.

Prior to solubilisation, the powder is stored at -20°C. The stability of the stock solution is 3 months at approximately -80°C; the stability of the diluted formulations for animal dosing is 24 hours at room temperature. Unused diluted material could be stored for up to 7 days if kept at 4-8°C.

### c) Experimental procedure

Following 8 days of acclimatization the mice are treated daily at 08.00 AM for 21 days by SC dosing 8 hours prior to lights out at 04.00 PM according to the outline groups.

### i) Blood glucose

Blood glucose is measured from 7 hour fasted animals 6 hours post dosing by collection of 10 µl blood samples from the tail-vein in hematocrite tubes and subsequent analysis on a Biosen s-line analyzer (EKF-diagnostic; Germany).

### ii) Metabolic cages

Groups 1+3: Mice are placed in metabolic cages for the recording of 24-hour food and water intake as well as 24-hour urine and faeces production. Mice are stratified into two sub-teams of n = 6-7 and subsequently the metabolic characterisation is performed.

### iii) Adipokine panel

Groups 1+3: On three occasions blood is collected from the tail vein using EDTA coated hematocrite tubes (100µl). Following centrifugation of blood the plasma is collected and stored at-20°C until measurement. Then, the following panel of adipokines/cytokines is determined using Luminex based 7-plex: leptin, resistin, MCP-1, PAI-1, TNF , insulin and interieukin-6 (IL-6).

### iv) Termination

Groups 1+3 (day 111): The following organs are excised and weighed: inguinal subcutaneous fat, epididymal fat, retroperitoneal fat, brain, liver, kidney, spleen and heart. All organs described above are samples in 4% PFA for possible future histo-pathological examination. Also, pancreas (*en bloc*) is sampled for possible stereological and imunohistochemical analysis, and eyes are sampled for possible later analysis of retinopathy. Group 2 (day 28): No tissues or plasma are collected.

### 28. TAT derivatives target human leukocyte populations

Primary human white blood cells (WBC) were obtained from whole blood after red blood cell lysis. WBC were incubated with 1uM of D-TAT (SEQ ID NO: 251)-FITC or r3- L-TAT (SEQ ID NO: 20)-FITC for 30min at 37°C, washed in acid buffer and stained with fluorescent antibodies against cell type specific surface markers (CD14 for monocytes, CD15 for polymorphnuclears, CD3 for lymphocyte T, CD19 for lymphocyte B). Cells containing D-TAT-FITC and r3-L-TAT-FITC were finally analysed by flow cytometry to measure their respective transporter content. Both TAT derivatives target the human leukocyte populations. dTAT and r3LTAT binds to monocytes, neutrophils and lymphocyte T cells, and less efficiently to lymphocyte B cells. A minor difference between dTAT and r3-L-TAT specificity exists, D-TAT seeming to bind more efficiently to lymphocyte T than the r3-L-TAT.

### 29. Uptake of selected transporter constructs according to the present invention by different cell types

Cells were plated in Poly-D-lysine pre-coated 96-well-plates at subconfluent density (which can vary depending on the cell type used). Different FITC-coupled transporters were then incubated with the cells for 15h at 3µM. Following this time, cells were kept on ice for the rest of the procedure. To remove cell-surface bound peptides, cells were first washed 2 times with an acid wash to remove plasma membrane-bound molecules. Subsequently, cells were washed 2 times with PBS and lysed in a standard lysis buffer for 30min. Plates containing cell lysates were then centrifuged for 5min at 1500rpm at 4°c. Clear supernatant was then collected and transferred into a black 96-well-plate for the measure of intracellular FITC fluorescence. The following cells were used:

| | |
|---|---|
| Non leucocyte cell lines: | *HepG2:* Hepatocarcinoma cells (human) |
| | *A549:* Lung epithelial cells (human) |
| | |
| Leucocyte cell lines: | *Raw:* Macrophage cells (mouse) |
| | *J77:* Macrophage cells (mouse) |
| | |
| Primary purified leucocytes: | *BMDM:* Bone Marrow-Derived Macrophages (mouse) |

Results are expressed as percentage of D-TAT (SEQ ID NO: 251) (Fig. 17) or r3-L-TAT (SEQ ID NO: 20) (Fig. 18) uptake. All transporter constructs show uptake in the respective cells, albeit at different rates.

## Claims

1. Transporter construct comprising at least one D-/L-pattern of the generic formula (I):
DₗLLLₓDₘ(LLL_{y}Dₙ)ₐ
wherein:
D is a D-amino acid;
L is a L-amino acid;
a is 0-3;
I, m and n are independently from each other 1 or 2;
x and y are independently from each other 0, 1 or 2;
in particular wherein the transporter construct comprises at least one D-/L-pattern according to one of the following subformulas (Ia) to (If):
(Ia): DₗLLLₓDₘ ;
(Ib): DₗLLLₓDₘLLL_{y}Dₙ ;
(Ic): DₗLLLₓDₘLLL_{y}DₙLLL_{y}Dₙ ;
(Id): DₗLLLₓDₘLLL_{y}DₙLLL_{y}DₙLLL_{y}Dₙ,
(Ie): DLLLD(LLLD)ₐ,
or
(If): DLLLDLLLD,
and wherein the D-/L-pattern according to generic formula (I) or according to any of subformulas (Ia), (Ib), (Ic), (Id), (Ie), or (If), is applied to one of the following sequences:
| SEQUENCE/PEPTIDE NAME | SEQ ID NO | AA | SEQUENCE |
|---|---|---|---|
| TAT (1-86) | 8 | 86 | |
| TAT (37-72) | 9 | 36 | |
| TAT (37-58) | 10 | 22 | CFITKALGIS YGRKKRRQRR RP |
| TAT (38-58)GGC | 11 | 24 | FITKALGISY GRKKRRQRRR PGGC |
| TAT CGG(47-58) | 12 | 15 | CGGYGRKKRR QRRRP |
| TAT (47-58)GGC | 13 | 15 | YGRKKRRQRR RPGGC |
| TAT (1-72) Mut Cys/Ala 72 | 14 | 56 | |
| L-generic-TAT | 15 | 17 | XXXXRKKRRQRRRXXXX |
| | | | (NH₂- XXXXRKKRRQRRRXXXX -COOH) |
| L-generic-TAT (s) | 16 | 11 | NH₂-Xₙ^{b}-RKKRRQRRR-Xₙ^{b}-COOH |
| L-TAT (s1a) | 17 | 10 | GRKKRRQRRR |
| | | | (NH₂-GRKKRRQRRR-COOH) |
| L-TAT (s1b) | 18 | 9 | RKKRRQRRR |
| | | | (NH₂-GRKKRRQRRR-COOH) |
| L-TAT (s1c) | 19 | 11 | YDRKKRRQRRR |
| r₃-L-TAT | 20 | 9 | rKKRrQRRr |
| r₃-L-TATi | 21 | 9 | rRRQrRKKr |
| βA-r₃-L-TAT | 22 | 9 | βA-rKKRrQRRr |
| βA-r₃-L-TATi | 23 | 9 | βA-rRRQrRKKr |
| FITC-βA-r₃-L-TAT | 24 | 9 | FITC-βA-rKKRrQRRr |
| FITC-βA-r₃-L-TATi | 25 | 9 | FITC-βA-rRRQrRKKr |
| TAT(s2-1) | 26 | 9 | rAKRrQRRr |
| TAT(s2-2) | 27 | 9 | rKARrQRRr |
| TAT(s2-3) | 28 | 9 | rKKArQRRr |
| TAT(s2-4) | 29 | 9 | rKKRrARRr |
| TAT(s2-5) | 30 | 9 | rKKRrQARr |
| TAT(s2-6) | 31 | 9 | rKKRrQRAr |
| TAT(s2-7) | 32 | 9 | rDKRrQRRr |
| TAT(s2-8) | 33 | 9 | rKDRrQRRr |
| TAT(s2-9) | 34 | 9 | rKKDrQRRr |
| TAT(s2-10) | 35 | 9 | rKKRrDRRr |
| TAT(s2-11) | 36 | 9 | rKKRrQDRr |
| TAT(s2-12) | 37 | 9 | rKKRrQRDr |
| TAT(s2-13) | 38 | 9 | rEKRrQRRr |
| TAT(s2-14) | 39 | 9 | rKERrQRRr |
| TAT(s2-15) | 40 | 9 | rKKErQRRr |
| TAT(s2-16) | 41 | 9 | rKKRrERRr |
| TAT(s2-17) | 42 | 9 | rKKRrQERr |
| TAT(s2-18) | 43 | 9 | rKKRrQREr |
| TAT(s2-19) | 44 | 9 | rFKRrQRRr |
| TAT(s2-20) | 45 | 9 | rKFRrQRRr |
| TAT(s2-21) | 46 | 9 | rKKFrQRRr |
| TAT(s2-22) | 47 | 9 | rKKRrFRRr |
| TAT(s2-23) | 48 | 9 | rKKRrQFRr |
| TAT(s2-24) | 49 | 9 | rKKRrQRFr |
| TAT(s2-25) | 50 | 9 | rRKRrQRRr |
| TAT(s2-26) | 51 | 9 | rKRRrQRRr |
| TAT(s2-27) | 52 | 9 | rKKKrQRRr |
| TAT(s2-28) | 53 | 9 | rKKRrRRRr |
| TAT(s2-29) | 54 | 9 | rKKRrQKRr |
| TAT(s2-30) | 55 | 9 | rKKRrQRKr |
| TAT(s2-31) | 56 | 9 | rHKRrQRRr |
| TAT(s2-32) | 57 | 9 | rKHRrQRRr |
| TAT(s2-33) | 58 | 9 | rKKHrQRRr |
| TAT(s2-34) | 59 | 9 | rKKRrHRRr |
| TAT(s2-35) | 60 | 9 | rKKRrQHRr |
| TAT(s2-36) | 61 | 9 | rKKRrQRHr |
| TAT(s2-37) | 62 | 9 | rIKRrQRRr |
| TAT(s2-38) | 63 | 9 | rKIRrQRRr |
| TAT(s2-39) | 64 | 9 | rKKIrQRRr |
| TAT(s2-40) | 65 | 9 | rKKRrIRRr |
| TAT(s2-41) | 66 | 9 | rKKRrQIRr |
| TAT(s2-42) | 67 | 9 | rKKRrQRIr |
| TAT(s2-43) | 68 | 9 | rLKRrQRRr |
| TAT(s2-44) | 69 | 9 | rKLRrQRRr |
| TAT(s2-45) | 70 | 9 | rKKLrQRRr |
| TAT(s2-46) | 71 | 9 | rKKRrLRRr |
| TAT(s2-47) | 72 | 9 | rKKRrQLRr |
| TAT(s2-48) | 73 | 9 | rKKRrQRLr |
| TAT(s2-49) | 74 | 9 | rMKRrQRRr |
| TAT(s2-50) | 75 | 9 | rKMRrQRRr |
| TAT(s2-51) | 76 | 9 | rKKMrQRRr |
| TAT(s2-52) | 77 | 9 | rKKRrMRRr |
| TAT(s2-53) | 78 | 9 | rKKRrQMRr |
| TAT(s2-54) | 79 | 9 | rKKRrQRMr |
| TAT(s2-55) | 80 | 9 | rNKRrQRRr |
| TAT(s2-56) | 81 | 9 | rKNRrQRRr |
| TAT(s2-57) | 82 | 9 | rKKNrQRRr |
| TAT(s2-58) | 83 | 9 | rKKRrNRRr |
| TAT(s2-59) | 84 | 9 | rKKRrQNRr |
| TAT(s2-60) | 85 | 9 | rKKRrQRNr |
| TAT(s2-61) | 86 | 9 | rQKRrQRRr |
| TAT(s2-62) | 87 | 9 | rKQRrQRRr |
| TAT(s2-63) | 88 | 9 | rKKQrQRRr |
| TAT(s2-64) | 89 | 9 | rKKRrKRRr |
| TAT(s2-65) | 90 | 9 | rKKRrQQRr |
| TAT(s2-66) | 91 | 9 | rKKRrQRQr |
| TAT(s2-67) | 92 | 9 | rSKRrQRRr |
| TAT(s2-68) | 93 | 9 | rKSRrQRRr |
| TAT(s2-69) | 94 | 9 | rKKSrQRRr |
| TAT(s2-70) | 95 | 9 | rKKRrSRRr |
| TAT(s2-71) | 96 | 9 | rKKRrQSRr |
| TAT(s2-72) | 97 | 9 | rKKRrQRSr |
| TAT(s2-73) | 98 | 9 | rTKRrQRRr |
| TAT(s2-74) | 99 | 9 | rKTRrQRRr |
| TAT(s2-75) | 100 | 9 | rKKTrQRRr |
| TAT(s2-76) | 101 | 9 | rKKRrTRRr |
| TAT(s2-77) | 102 | 9 | rKKRrQTRr |
| TAT(s2-78) | 103 | 9 | rKKRrQRTr |
| TAT(s2-79) | 104 | 9 | rVKRrQRRr |
| TAT(s2-80) | 105 | 9 | rKVRrQRRr |
| TAT(s2-81) | 106 | 9 | rKKVrQRRr |
| TAT(s2-82) | 107 | 9 | rKKRrVRRr |
| TAT(s2-83) | 108 | 9 | rKKRrQVRr |
| TAT(s2-84) | 109 | 9 | rKKRrQRVr |
| TAT(s2-85) | 110 | 9 | rWKRrQRRr |
| TAT(s2-86) | 111 | 9 | rKWRrQRRr |
| TAT(s2-87) | 112 | 9 | rKKWrQRRr |
| TAT(s2-88) | 113 | 9 | rKKRrWRRr |
| TAT(s2-89) | 114 | 9 | rKKRrQWRr |
| TAT(s2-90) | 115 | 9 | rKKRrQRWr |
| TAT(s2-91) | 116 | 9 | rYKRrQRRr |
| TAT(s2-92) | 117 | 9 | rKYRrQRRr |
| TAT(s2-93) | 118 | 9 | rKKYrQRRr |
| TAT(s2-94) | 119 | 9 | rKKRrYRRr |
| TAT(s2-95) | 120 | 9 | rKKRrQYRr |
| TAT(s2-96) | 121 | 9 | rKKRrQRYr |
| r₃R₆ | 122 | 9 | rRRRrRRRr |
| L-R₉ | 123 | 9 | RRRRRRRRR |
| L-R₈ | 124 | 8 | RRRRRRRR |
| L-R₇ | 125 | 7 | RRRRRRR |
| L-R₆ | 126 | 6 | RRRRRR |
| L-R₅ | 127 | 5 | RRRRR |
| PTD-4 | 128 | 11 | YARAAARQARA |
| PTD-4 (variant 1) | 129 | 11 | WARAAARQARA |
| PTD-4 (variant 2) | 130 | 11 | WARAQRAAARA |
| L-P1 Penetratin | 131 | 16 | RQVKVWFQNRRMKWKK |
| D-P1 Penetratin | 132 | 16 | KKWKMRRNQFWVKVQR |
| JNKI, bestfit | 133 | 17 | WKRAAARKARAMSLNLF |
| JNKI, bestfit (variant 1) | 134 | 17 | WKRAAARAARAMSLNLF |
| MDCK transcytose sequence | 135 | 9 | RYRGDLGRR |
| YKGL | 136 | 4 | YKGL |
| r₃ (generic) | 252 | 9 | rXXXrXXXr |
or a reverse sequence thereof, or a variant or fragment of these sequences, wherein a variant or fragment of these sequences retains the function to provide for translocation across biological membranes and consists of a peptide sequence having at least 80% sequence identity over the whole length to the respective native sequence.

2. Transporter construct according to claim 1, wherein the transporter construct comprises at least one sequence according to rXXXrXXXr, wherein:
r represents an D-enantiomeric arginine;
X is any L-amino acid;
and wherein each X may be selected individually and independently of any other X within rXXXrXXXr, in particular, wherein at least 4 out of said 6 X L-amino acids are individually selected from the group of L amino acids consisting of K or R.

3. Transporter construct according to any of claims 1 to 2, wherein the transporter construct comprises a sequence selected from the group of sequences:
| SEQUENCE/PEPTIDE NAME | SEQ ID NO | AA | SEQUENCE |
|---|---|---|---|
| r₃-L-TAT | 20 | 9 | rKKRrQRRr |
| r₃-L-TATi | 21 | 9 | rRRQrRKKr |
| βA-r₃-L-TAT | 22 | 9 | βA-rKKRrQRRr |
| βA-r₃-L-TATi | 23 | 9 | βA-rRRQrRKKr |
| FITC-βA-r₃-L-TAT | 24 | 9 | FITC-βA-rKKRrQRRr |
| FITC-βA-r₃-L-TATi | 25 | 9 | FITC-βA-rRRQrRKKr |
| TAT(s2-1) | 26 | 9 | rAKRrQRRr |
| TAT(s2-2) | 27 | 9 | rKARrQRRr |
| TAT(s2-3) | 28 | 9 | rKKArQRRr |
| TAT(s2-4) | 29 | 9 | rKKRrARRr |
| TAT(s2-5) | 30 | 9 | rKKRrQARr |
| TAT(s2-6) | 31 | 9 | rKKRrQRAr |
| TAT(s2-7) | 32 | 9 | rDKRrQRRr |
| TAT(s2-8) | 33 | 9 | rKDRrQRRr |
| TAT(s2-9) | 34 | 9 | rKKDrQRRr |
| TAT(s2-10) | 35 | 9 | rKKRrDRRr |
| TAT(s2-11) | 36 | 9 | rKKRrQDRr |
| TAT(s2-12) | 37 | 9 | rKKRrQRDr |
| TAT(s2-13) | 38 | 9 | rEKRrQRRr |
| TAT(s2-14) | 39 | 9 | rKERrQRRr |
| TAT(s2-15) | 40 | 9 | rKKErQRRr |
| TAT(s2-16) | 41 | 9 | rKKRrERRr |
| TAT(s2-17) | 42 | 9 | rKKRrQERr |
| TAT(s2-18) | 43 | 9 | rKKRrQREr |
| TAT(s2-19) | 44 | 9 | rFKRrQRRr |
| TAT(s2-20) | 45 | 9 | rKFRrQRRr |
| TAT(s2-21) | 46 | 9 | rKKFrQRRr |
| TAT(s2-22) | 47 | 9 | rKKRrFRRr |
| TAT(s2-23) | 48 | 9 | rKKRrQFRr |
| TAT(s2-24) | 49 | 9 | rKKRrQRFr |
| TAT(s2-25) | 50 | 9 | rRKRrQRRr |
| TAT(s2-26) | 51 | 9 | rKRRrQRRr |
| TAT(s2-27) | 52 | 9 | rKKKrQRRr |
| TAT(s2-28) | 53 | 9 | rKKRrRRRr |
| TAT(s2-29) | 54 | 9 | rKKRrQKRr |
| TAT(s2-30) | 55 | 9 | rKKRrQRKr |
| TAT(s2-31) | 56 | 9 | rHKRrQRRr |
| TAT(s2-32) | 57 | 9 | rKHRrQRRr |
| TAT(s2-33) | 58 | 9 | rKKHrQRRr |
| TAT(s2-34) | 59 | 9 | rKKRrHRRr |
| TAT(s2-35) | 60 | 9 | rKKRrQHRr |
| TAT(s2-36) | 61 | 9 | rKKRrQRHr |
| TAT(s2-3 7) | 62 | 9 | rIKRrQRRr |
| TAT(s2-38) | 63 | 9 | rKIRrQRRr |
| TAT(s2-39) | 64 | 9 | rKKIrQRRr |
| TAT(s2-40) | 65 | 9 | rKKRrIRRr |
| TAT(s2-41) | 66 | 9 | rKKRrQIRr |
| TAT(s2-42) | 67 | 9 | rKKRrQRIr |
| TAT(s2-43) | 68 | 9 | rLKRrQRRr |
| TAT(s2-44) | 69 | 9 | rKLRrQRRr |
| TAT(s2-45) | 70 | 9 | rKKLrQRRr |
| TAT(s2-46) | 71 | 9 | rKKRrLRRr |
| TAT(s2-47) | 72 | 9 | rKKRrQLRr |
| TAT(s2-48) | 73 | 9 | rKKRrQRLr |
| TAT(s2-49) | 74 | 9 | rMKRrQRRr |
| TAT(s2-50) | 75 | 9 | rKMRrQRRr |
| TAT(s2-51) | 76 | 9 | rKKMrQRRr |
| TAT(s2-52) | 77 | 9 | rKKRrMRRr |
| TAT(s2-53) | 78 | 9 | rKKRrQMRr |
| TAT(s2-54) | 79 | 9 | rKKRrQRMr |
| TAT(s2-55) | 80 | 9 | rNKRrQRRr |
| TAT(s2-56) | 81 | 9 | rKNRrQRRr |
| TAT(s2-57) | 82 | 9 | rKKNrQRRr |
| TAT(s2-58) | 83 | 9 | rKKRrNRRr |
| TAT(s2-59) | 84 | 9 | rKKRrQNRr |
| TAT(s2-60) | 85 | 9 | rKKRrQRNr |
| TAT(s2-61) | 86 | 9 | rQKRrQRRr |
| TAT(s2-62) | 87 | 9 | rKQRrQRRr |
| TAT(s2-63) | 88 | 9 | rKKQrQRRr |
| TAT(s2-64) | 89 | 9 | rKKRrKRRr |
| TAT(s2-65) | 90 | 9 | rKKRrQQRr |
| TAT(s2-66) | 91 | 9 | rKKRrQRQr |
| TAT(s2-67) | 92 | 9 | rSKRrQRRr |
| TAT(s2-68) | 93 | 9 | rKSRrQRRr |
| TAT(s2-69) | 94 | 9 | rKKSrQRRr |
| TAT(s2-70) | 95 | 9 | rKKRrSRRr |
| TAT(s2-71) | 96 | 9 | rKKRrQSRr |
| TAT(s2-72) | 97 | 9 | rKKRrQRSr |
| TAT(s2-73) | 98 | 9 | rTKRrQRRr |
| TAT(s2-74) | 99 | 9 | rKTRrQRRr |
| TAT(s2-75) | 100 | 9 | rKKTrQRRr |
| TAT(s2-76) | 101 | 9 | rKKRrTRRr |
| TAT(s2-77) | 102 | 9 | rKKRrQTRr |
| TAT(s2-78) | 103 | 9 | rKKRrQRTr |
| TAT(s2-79) | 104 | 9 | rVKRrQRRr |
| TAT(s2-80) | 105 | 9 | rKVRrQRRr |
| TAT(s2-81) | 106 | 9 | rKKVrQRRr |
| TAT(s2-82) | 107 | 9 | rKKRrVRRr |
| TAT(s2-83) | 108 | 9 | rKKRrQVRr |
| TAT(s2-84) | 109 | 9 | rKKRrQRVr |
| TAT(s2-85) | 110 | 9 | rWKRrQRRr |
| TAT(s2-86) | 111 | 9 | rKWRrQRRr |
| TAT(s2-87) | 112 | 9 | rKKWrQRRr |
| TAT(s2-88) | 113 | 9 | rKKRrWRRr |
| TAT(s2-89) | 114 | 9 | rKKRrQWRr |
| TAT(s2-90) | 115 | 9 | rKKRrQRWr |
| TAT(s2-91) | 116 | 9 | rYKRrQRRr |
| TAT(s2-92) | 117 | 9 | rKYRrQRRr |
| TAT(s2-93) | 118 | 9 | rKKYrQRRr |
| TAT(s2-94) | 119 | 9 | rKKRrYRRr |
| TAT(s2-95) | 120 | 9 | rKKRrQYRr |
| TAT(s2-96) | 121 | 9 | rKKRrQRYr |
| r₃R₆ | 122 | 9 | rRRRrRRRr |
or a variant or fragment of these sequences, wherein a variant or fragment of these sequences retains the function to provide for translocation across biological membranes and consists of a peptide sequence having at least 80% sequence identity over the whole length to the respective native trafficking sequence.

4. Transporter construct according to any of claims 1 to 3, wherein the transporter construct comprises the subformula (If) DLLLDLLLD and wherein the subformula (If) is applied to a trafficking sequence derived from HIV-1 TAT protein having a Tyrosine (Y) at position 2 of the TAT derived sequence, the sequence preferably selected from sequence rYKRrQRRr (TAT(s2-91)).

5. Transporter construct according to claim 3, wherein the transporter construct comprises a sequence selected from the group consisting of rKKRrQRRr (r₃-L-TAT) and rRRQrRKKr (r₃-L-TATi).

6. Transporter construct according to any of claims 1 to 5, wherein the generic formula (I) or any of subformulas (Ia), (Ib), (Ic), (Id), (Ie), or (If), is applied to a variant or a fragment of any of the trafficking sequences listed in claims 1 to 4, having at least 80% or 85%, preferably at least 90%, more preferably at least 95% and most preferably at least 99% sequence identity over the whole length of any of the trafficking sequences listed in claims 1 to 5.

7. Transporter cargo conjugate molecule, comprising
a) a component (A), wherein component (A) comprises the transporter construct according to any of claims 1 to 6; and
b) a component (B), comprising an effector molecule, in particular wherein the effector molecule is selected from therapeutically active proteins and peptides, protein kinase inhibitors, inhibitors of the protein kinase c-Jun amino terminal kinase, antigens, antibodies, apoptotic factors, proteases implicated in pathological states, preferably peptidic protease inhibitors, BH3-domains or BH3-only proteins, nucleic acids encoding these proteins, siRNAs, antisense RNAs, cytotoxic agents, and small organic compounds including protease inhibitors.

8. Transporter cargo conjugate molecule according to claim 7, furthermore comprising at least one additional component (C), (D) and/or (E) different to component (B), wherein the at least one optional additional component (C), (D) and/or (E) is selected independently from each other from different effector molecules as defined for component (B).

9. Transporter cargo conjugate molecule according to any of claims 7 to 8, wherein:
a) components (A) and (B), and optional components (C), (D) and/or (E) are covalently linked with each other, and/or
b) wherein the at least one additional component (C), (D) and/or (E) is selected from a signal sequence or localisation sequence, which directs the transporter cargo conjugate molecule to a particular intracellular target localization or to a particular cell type, and/or
c) wherein component (B) and/or the at least one additional components (C), (D) and/or (E) is (are) a protein or peptide sequence and is (are) composed of L-amino acids, D-amino acids or a mixture of both, and/or
d) wherein component (A) is positioned at the C-terminal end of the transporter cargo conjugate molecule, provided that component (B) is a protein or peptide sequence.

10. Transporter cargo conjugate molecule according to any of claims 7 to 9 for use as a medicament.

11. Pharmaceutical composition comprising a transporter cargo conjugate molecule according to any of claims 7 to 10 and optionally a pharmaceutically acceptable carrier and/or vehicle.

12. Use of a transporter construct according to any of claims 1 to 6 for the preparation of a transporter cargo conjugate molecule, in particular wherein the transporter cargo conjugate molecule is as defined according to any of claims 7 to 9.

13. Use of a transporter construct according to any of claims 1 to 6 or a transporter cargo conjugate molecule as defined according to any of claims 7 to 9 for preparation of a medicament for the transport of a cargo molecule into cells or tissue of a patient to be treated, in particular wherein the cargo is as defined according to any of claims 7 to 9.

14. Use according to claim 13 for the transport of a cargo molecule into white blood cells and/or neuronal cells, in particular of a patient to be treated.

15. Use of a transporter cargo conjugate molecule according to any of claims 7 to 9 for the preparation of a medicament for the prophylaxis, treatment and/or amelioration of cancer or tumor diseases, including diseases caused by defective apoptosis, inflammatory diseases, infectious diseases, viral (infectious) diseases, diseases strongly related to JNK signalling, autoimmune disorders or diseases, cardiovascular diseases, neuronal or neurodegenerative diseases, diseases of the liver, diseases of the spine, diseases of the uterus, major depressive disorders, non-chronic or chronic inflammatory digestive diseases, hearing loss, diseases of the inner ear, or for use in tissue transplantation.

## Patentansprüche

1. Transporterkonstrukt, umfassend zumindest eine D-/L-Struktur der generischen Formel (I):
DₗLLLₓDₘ(LLL_{y}Dₙ)ₐ
wobei:
D eine D-Aminosäure ist;
L eine L-Aminosäure ist;
a 0 - 3 ist;
I, m und n unabhängig voneinander 1 oder 2 sind;
x und y unabhängig voneinander 0, 1 oder 2 sind;
insbesondere wobei das Transporterkonstrukt zumindest eine D-/L-Struktur gemäß einer der folgenden Unterformeln (la) bis (If) umfasst:
(Ia): DₗLLLₓDₘ ;
(Ib): DₗLLLₓDₘLLL_{y}Dₙ ;
(Ic): DₗLLLₓDₘLLL_{y}DₙLLL_{y}Dₙ ;
(Id): DₗLLLₓDₘLLL_{y}DₙLLL_{y}DₙLLL_{y}Dₙ,
(Ie): DLLLD(LLLD)ₐ,
oder
(If): DLLLDLLLD ,
und wobei die D-/L-Struktur gemäß der generischen Formel (I) oder gemäß irgendeiner der Unterformeln (la), (Ib), (Ic), (Id), (le), oder (If), auf eine der folgenden Sequenzen angewandt wird:
| SEQUENZ/ PEPTIDNAME | SEQ ID Nr. | AS | SEQUENZ |
|---|---|---|---|
| TAT (1-86) | 8 | 86 | |
| TAT (37-72) | 9 | 36 | |
| TAT (37-58) | 10 | 22 | CFITKALGIS YGRKKRRQRR RP |
| TAT (38-58)GGC | 11 | 24 | FITKALGISY GRKKRRQRRR PGGC |
| TAT CGG(47-58) | 12 | 15 | CGGYGRKKRR QRRRP |
| TAT (47-58)GGC | 13 | 15 | YGRKKRRQRR RPGGC |
| TAT (1-72) Mut Cys/Ala 72 | 14 | 56 | |
| L-generic-TAT | 15 | 17 | XXXXRKKRRQRRRXXXX |
| | | | (NH₂- XXXXRKKRRQRRRXXXX -COOH) |
| L-generic-TAT (s) | 16 | 11 | NH₂-Xₙ^{b}-RKKRRQRRR-Xₙ^{b}-COOH |
| L-TAT (s1a) | 17 | 10 | GRKKRRQRRR |
| | | | (NH₂-GRKKRRQRRR-COOH) |
| L-TAT (s1b) | 18 | 9 | RKKRRQRRR |
| | | | (NH₂-GRKKRRQRRR-COOH) |
| L-TAT (s1c) | 19 | 11 | YDRKKRRQRRR |
| r₃-L-TAT | 20 | 9 | rKKRrQRRr |
| r₃-L-TATi | 21 | 9 | rRRQrRKKr |
| βA-r₃-L-TAT | 22 | 9 | βA-rKKRrQRRr |
| βA-r₃-L-TATi | 23 | 9 | βA-rRRQrRKKr |
| FITC-βA-r₃-L-TAT | 24 | 9 | FITC-βA-rKKRrQRRr |
| FITC-βA-r₃-L-TATi | 25 | 9 | FITC-βA-rRRQrRKKr |
| TAT(s2-1) | 26 | 9 | rAKRrQRRr |
| TAT(s2-2) | 27 | 9 | rKARrQRRr |
| TAT(s2-3) | 28 | 9 | rKKArQRRr |
| TAT(s2-4) | 29 | 9 | rKKRrARRr |
| TAT(s2-5) | 30 | 9 | rKKRrQARr |
| TAT(s2-6) | 31 | 9 | rKKRrQRAr |
| TAT(s2-7) | 32 | 9 | rDKRrQRRr |
| TAT(s2-8) | 33 | 9 | rKDRrQRRr |
| TAT(s2-9) | 34 | 9 | rKKDrQRRr |
| TAT(s2-10) | 35 | 9 | rKKRrDRRr |
| TAT(s2-11) | 36 | 9 | rKKRrQDRr |
| TAT(s2-12) | 37 | 9 | rKKRrQRDr |
| TAT(s2-13) | 38 | 9 | rEKRrQRRr |
| TAT(s2-14) | 39 | 9 | rKERrQRRr |
| TAT(s2-15) | 40 | 9 | rKKErQRRr |
| TAT(s2-16) | 41 | 9 | rKKRrERRr |
| TAT(s2-1 7) | 42 | 9 | rKKRrQERr |
| TAT(s2-18) | 43 | 9 | rKKRrQREr |
| TAT(s2-19) | 44 | 9 | rFKRrQRRr |
| TAT(s2-20) | 45 | 9 | rKFRrQRRr |
| TAT(s2-21) | 46 | 9 | rKKFrQRRr |
| TAT(s2-22) | 47 | 9 | rKKRrFRRr |
| TAT(s2-23) | 48 | 9 | rKKRrQFRr |
| TAT(s2-24) | 49 | 9 | rKKRrQRFr |
| TAT(s2-25) | 50 | 9 | rRKRrQRRr |
| TAT(s2-26) | 51 | 9 | rKRRrQRRr |
| TAT(s2-27) | 52 | 9 | rKKKrQRRr |
| TAT(s2-28) | 53 | 9 | rKKRrRRRr |
| TAT(s2-29) | 54 | 9 | rKKRrQKRr |
| TAT(s2-30) | 55 | 9 | rKKRrQRKr |
| TAT(s2-31) | 56 | 9 | rHKRrQRRr |
| TAT(s2-32) | 57 | 9 | rKHRrQRRr |
| TAT(s2-33) | 58 | 9 | rKKHrQRRr |
| TAT(s2-34) | 59 | 9 | rKKRrHRRr |
| TAT(s2-35) | 60 | 9 | rKKRrQHRr |
| TAT(s2-36) | 61 | 9 | rKKRrQRHr |
| TAT(s2-37) | 62 | 9 | rIKRrQRRr |
| TAT(s2-38) | 63 | 9 | rKIRrQRRr |
| TAT(s2-39) | 64 | 9 | rKKIrQRRr |
| TAT(s2-40) | 65 | 9 | rKKRrIRRr |
| TAT(s2-41) | 66 | 9 | rKKRrQIRr |
| TAT(s2-42) | 67 | 9 | rKKRrQRIr |
| TAT(s2-43) | 68 | 9 | rLKRrQRRr |
| TAT(s2-44) | 69 | 9 | rKLRrQRRr |
| TAT(s2-45) | 70 | 9 | rKKLrQRRr |
| TAT(s2-46) | 71 | 9 | rKKRrLRRr |
| TAT(s2-47) | 72 | 9 | rKKRrQLRr |
| TAT(s2-48) | 73 | 9 | rKKRrQRLr |
| TAT(s2-49) | 74 | 9 | rMKRrQRRr |
| TAT(s2-50) | 75 | 9 | rKMRrQRRr |
| TAT(s2-51) | 76 | 9 | rKKMrQRRr |
| TAT(s2-52) | 77 | 9 | rKKRrMRRr |
| TAT(s2-53) | 78 | 9 | rKKRrQMRr |
| TAT(s2-54) | 79 | 9 | rKKRrQRMr |
| TAT(s2-55) | 80 | 9 | rNKRrQRRr |
| TAT(s2-56) | 81 | 9 | rKNRrQRRr |
| TAT(s2-57) | 82 | 9 | rKKNrQRRr |
| TAT(s2-58) | 83 | 9 | rKKRrNRRr |
| TAT(s2-59) | 84 | 9 | rKKRrQNRr |
| TAT(s2-60) | 85 | 9 | rKKRrQRNr |
| TAT(s2-61) | 86 | 9 | rQKRrQRRr |
| TAT(s2-62) | 87 | 9 | rKQRrQRRr |
| TAT(s2-63) | 88 | 9 | rKKQrQRRr |
| TAT(s2-64) | 89 | 9 | rKKRrKRRr |
| TAT(s2-65) | 90 | 9 | rKKRrQQRr |
| TAT(s2-66) | 91 | 9 | rKKRrQRQr |
| TAT(s2-67) | 92 | 9 | rSKRrQRRr |
| TAT(s2-68) | 93 | 9 | rKSRrQRRr |
| TAT(s2-69) | 94 | 9 | rKKSrQRRr |
| TAT(s2-70) | 95 | 9 | rKKRrSRRr |
| TAT(s2-71) | 96 | 9 | rKKRrQSRr |
| TAT(s2-72) | 97 | 9 | rKKRrQRSr |
| TAT(s2-73) | 98 | 9 | rTKRrQRRr |
| TAT(s2-74) | 99 | 9 | rKTRrQRRr |
| TAT(s2-75) | 100 | 9 | rKKTrQRRr |
| TAT(s2-76) | 101 | 9 | rKKRrTRRr |
| TAT(s2-77) | 102 | 9 | rKKRrQTRr |
| TAT(s2-78) | 103 | 9 | rKKRrQRTr |
| TAT(s2-79) | 104 | 9 | rVKRrQRRr |
| TAT(s2-80) | 105 | 9 | rKVRrQRRr |
| TAT(s2-81) | 106 | 9 | rKKVrQRRr |
| TAT(s2-82) | 107 | 9 | rKKRrVRRr |
| TAT(s2-83) | 108 | 9 | rKKRrQVRr |
| TAT(s2-84) | 109 | 9 | rKKRrQRVr |
| TAT(s2-85) | 110 | 9 | rWKRrQRRr |
| TAT(s2-86) | 111 | 9 | rKWRrQRRr |
| TAT(s2-87) | 112 | 9 | rKKWrQRRr |
| TAT(s2-88) | 113 | 9 | rKKRrWRRr |
| TAT(s2-89) | 114 | 9 | rKKRrQWRr |
| TAT(s2-90) | 115 | 9 | rKKRrQRWr |
| TAT(s2-91) | 116 | 9 | rYKRrQRRr |
| TAT(s2-92) | 117 | 9 | rKYRrQRRr |
| TAT(s2-93) | 118 | 9 | rKKYrQRRr |
| TAT(s2-94) | 119 | 9 | rKKRrYRRr |
| TAT(s2-95) | 120 | 9 | rKKRrQYRr |
| TAT(s2-96) | 121 | 9 | rKKRrQRYr |
| r₃R₆ | 122 | 9 | rRRRrRRRr |
| L-R₉ | 123 | 9 | RRRRRRRRR |
| L-R₈ | 124 | 8 | RRRRRRRR |
| L-R₇ | 125 | 7 | RRRRRRR |
| L-R₆ | 126 | 6 | RRRRRR |
| L-R₅ | 127 | 5 | RRRRR |
| PTD-4 | 128 | 11 | YARAAARQARA |
| PTD-4 (variant 1) | 129 | 11 | WARAAARQARA |
| PTD-4 (variant 2) | 130 | 11 | WARAQRAAARA |
| L-P1 Penetratin | 131 | 16 | RQVKVWFQNRRMKWKK |
| D-P1 Penetratin | 132 | 16 | KKWKMRRNQFWVKVQR |
| JNKI, bestfit | 133 | 17 | WKRAAARKARAMSLNLF |
| JNKI, bestfit (variant 1) | 134 | 17 | WKRAAARAARAMSLNLF |
| MDCK transcytose sequence | 135 | 9 | RYRGDLGRR |
| YKGL | 136 | 4 | YKGL |
| r₃ (generic) | 252 | 9 | rXXXrXXXr |
oder auf eine dazu reverse Sequenz oder auf eine Variante oder ein Fragment dieser Sequenzen, wobei eine Variante oder ein Fragment dieser Sequenzen die Funktion, Translokation über biologische Membranen zu ermöglichen, beibehält und aus einer Peptidsequenz besteht, die zumindest 80% Sequenzidentität über die gesamte Länge mit der entsprechenden natürlichen Sequenz aufweist.

2. Transporterkonstrukt nach Anspruch 1, wobei das Transporterkonstrukt zumindest eine Sequenz gemäß rXXXrXXXr umfasst, wobei:
r ein D-enantiomeres Arginin darstellt;
X irgendeine L-Aminosäure ist;
und wobei jedes X individuell und unabhängig von jedem anderen X in rXXXrXXXr gewählt sein kann, insbesondere wobei wenigstens 4 von den genannten 6 X L-Aminosäuren individuell aus der Gruppe der L-Aminosäuren, bestehend aus K oder R, ausgewählt sind.

3. Transporterkonstrukt nach einem der Ansprüche 1 bis 2, wobei das Transporterkonstrukt eine Sequenz umfasst, die ausgewählt ist aus der Gruppe von Sequenzen:
| SEQUENZ/ PEPTIDNAME | SEQ ID Nr. | AS | SEQUENZ |
|---|---|---|---|
| r₃-L-TAT | 20 | 9 | rKKRrQRRr |
| r₃-L-TATi | 21 | 9 | rRRQrRKKr |
| βA-r₃-L-TAT | 22 | 9 | βA-rKKRrQRRr |
| βA-r₃-L-TATi | 23 | 9 | βA-rRRQrRKKr |
| FITC-βA-r₃-L-TAT | 24 | 9 | FITC-βA-rKKRrQRRr |
| FITC-βA-r₃-L-TATi | 25 | 9 | FITC-βA-rRRQrRKKr |
| TAT(s2-1) | 26 | 9 | rAKRrQRRr |
| TAT(s2-2) | 27 | 9 | rKARrQRRr |
| TAT(s2-3) | 28 | 9 | rKKArQRRr |
| TAT(s2-4) | 29 | 9 | rKKRrARRr |
| TAT(s2-5) | 30 | 9 | rKKRrQARr |
| TAT(s2-6) | 31 | 9 | rKKRrQRAr |
| TAT(s2-7) | 32 | 9 | rDKRrQRRr |
| TAT(s2-8) | 33 | 9 | rKDRrQRRr |
| TAT(s2-9) | 34 | 9 | rKKDrQRRr |
| TAT(s2-10) | 35 | 9 | rKKRrDRRr |
| TAT(s2-11) | 36 | 9 | rKKRrQDRr |
| TAT(s2-12) | 37 | 9 | rKKRrQRDr |
| TAT(s2-13) | 38 | 9 | rEKRrQRRr |
| TAT(s2-14) | 39 | 9 | rKERrQRRr |
| TAT(s2-15) | 40 | 9 | rKKErQRRr |
| TAT(s2-16) | 41 | 9 | rKKRrERRr |
| TAT(s2-1 7) | 42 | 9 | rKKRrQERr |
| TAT(s2-18) | 43 | 9 | rKKRrQREr |
| TAT(s2-19) | 44 | 9 | rFKRrQRRr |
| TAT(s2-20) | 45 | 9 | rKFRrQRRr |
| TAT(s2-21) | 46 | 9 | rKKFrQRRr |
| TAT(s2-22) | 47 | 9 | rKKRrFRRr |
| TAT(s2-23) | 48 | 9 | rKKRrQFRr |
| TAT(s2-24) | 49 | 9 | rKKRrQRFr |
| TAT(s2-25) | 50 | 9 | rRKRrQRRr |
| TAT(s2-26) | 51 | 9 | rKRRrQRRr |
| TAT(s2-27) | 52 | 9 | rKKKrQRRr |
| TAT(s2-28) | 53 | 9 | rKKRrRRRr |
| TAT(s2-29) | 54 | 9 | rKKRrQKRr |
| TAT(s2-30) | 55 | 9 | rKKRrQRKr |
| TAT(s2-31) | 56 | 9 | rHKRrQRRr |
| TAT(s2-32) | 57 | 9 | rKHRrQRRr |
| TAT(s2-33) | 58 | 9 | rKKHrQRRr |
| TAT(s2-34) | 59 | 9 | rKKRrHRRr |
| TAT(s2-35) | 60 | 9 | rKKRrQHRr |
| TAT(s2-36) | 61 | 9 | rKKRrQRHr |
| TAT(s2-37) | 62 | 9 | rIKRrQRRr |
| TAT(s2-38) | 63 | 9 | rKIRrQRRr |
| TAT(s2-39) | 64 | 9 | rKKIrQRRr |
| TAT(s2-40) | 65 | 9 | rKKRrIRRr |
| TAT(s2-41) | 66 | 9 | rKKRrQIRr |
| TAT(s2-42) | 67 | 9 | rKKRrQRIr |
| TAT(s2-43) | 68 | 9 | rLKRrQRRr |
| TAT(s2-44) | 69 | 9 | rKLRrQRRr |
| TAT(s2-45) | 70 | 9 | rKKLrQRRr |
| TAT(s2-46) | 71 | 9 | rKKRrLRRr |
| TAT(s2-47) | 72 | 9 | rKKRrQLRr |
| TAT(s2-48) | 73 | 9 | rKKRrQRLr |
| TAT(s2-49) | 74 | 9 | rMKRrQRRr |
| TAT(s2-50) | 75 | 9 | rKMRrQRRr |
| TAT(s2-51) | 76 | 9 | rKKMrQRRr |
| TAT(s2-52) | 77 | 9 | rKKRrMRRr |
| TAT(s2-53) | 78 | 9 | rKKRrQMRr |
| TAT(s2-54) | 79 | 9 | rKKRrQRMr |
| TAT(s2-55) | 80 | 9 | rNKRrQRRr |
| TAT(s2-56) | 81 | 9 | rKNRrQRRr |
| TAT(s2-57) | 82 | 9 | rKKNrQRRr |
| TAT(s2-58) | 83 | 9 | rKKRrNRRr |
| TAT(s2-59) | 84 | 9 | rKKRrQNRr |
| TAT(s2-60) | 85 | 9 | rKKRrQRNr |
| TAT(s2-61) | 86 | 9 | rQKRrQRRr |
| TAT(s2-62) | 87 | 9 | rKQRrQRRr |
| TAT(s2-63) | 88 | 9 | rKKQrQRRr |
| TAT(s2-64) | 89 | 9 | rKKRrKRRr |
| TAT(s2-65) | 90 | 9 | rKKRrQQRr |
| TAT(s2-66) | 91 | 9 | rKKRrQRQr |
| TAT(s2-67) | 92 | 9 | rSKRrQRRr |
| TAT(s2-68) | 93 | 9 | rKSRrQRRr |
| TAT(s2-69) | 94 | 9 | rKKSrQRRr |
| TAT(s2-70) | 95 | 9 | rKKRrSRRr |
| TAT(s2-71) | 96 | 9 | rKKRrQSRr |
| TAT(s2-72) | 97 | 9 | rKKRrQRSr |
| TAT(s2-73) | 98 | 9 | rTKRrQRRr |
| TAT(s2-74) | 99 | 9 | rKTRrQRRr |
| TAT(s2-75) | 100 | 9 | rKKTrQRRr |
| TAT(s2-76) | 101 | 9 | rKKRrTRRr |
| TAT(s2-77) | 102 | 9 | rKKRrQTRr |
| TAT(s2-78) | 103 | 9 | rKKRrQRTr |
| TAT(s2-79) | 104 | 9 | rVKRrQRRr |
| TAT(s2-80) | 105 | 9 | rKVRrQRRr |
| TAT(s2-81) | 106 | 9 | rKKVrQRRr |
| TAT(s2-82) | 107 | 9 | rKKRrVRRr |
| TAT(s2-83) | 108 | 9 | rKKRrQVRr |
| TAT(s2-84) | 109 | 9 | rKKRrQRVr |
| TAT(s2-85) | 110 | 9 | rWKRrQRRr |
| TAT(s2-86) | 111 | 9 | rKWRrQRRr |
| TAT(s2-87) | 112 | 9 | rKKWrQRRr |
| TAT(s2-88) | 113 | 9 | rKKRrWRRr |
| TAT(s2-89) | 114 | 9 | rKKRrQWRr |
| TAT(s2-90) | 115 | 9 | rKKRrQRWr |
| TAT(s2-91) | 116 | 9 | rYKRrQRRr |
| TAT(s2-92) | 117 | 9 | rKYRrQRRr |
| TAT(s2-93) | 118 | 9 | rKKYrQRRr |
| TAT(s2-94) | 119 | 9 | rKKRrYRRr |
| TAT(s2-95) | 120 | 9 | rKKRrQYRr |
| TAT(s2-96) | 121 | 9 | rKKRrQRYr |
| r₃R₆ | 122 | 9 | rRRRrRRRr |
oder eine Variante oder ein Fragment dieser Sequenzen, wobei eine Variante oder ein Fragment dieser Sequenzen die Funktion, Translokation über biologische Membranen zu ermöglichen, beibehält und aus einer Peptidsequenz besteht, die zumindest 80% Sequenzidentität über die gesamte Länge mit der entsprechenden natürlichen Transportsequenz aufweist.

4. Transporterkonstrukt nach einem der Ansprüche 1 bis 3, wobei das Transporterkonstrukt die Unterformel (If) DLLLDLLLD umfasst und wobei die Unterformel (If) auf eine Transportsequenz angewendet ist, die vom HIV-1-TAT-Protein abstammt und ein Tyrosin (Y) an Position 2 der TAT-abgestammten Sequenz aufweist, die Sequenz ist vorzugsweise ausgewählt aus der Sequenz rYKRrQRRr (TAT(s2-91)).

5. Transporterkonstrukt nach Anspruch 3, wobei das Transporterkonstrukt eine Sequenz umfasst, die ausgewählt ist aus der Gruppe bestehend aus rKKRrQRRr (r₃-L-TAT) und rRRQrRKKr (r₃-L-TATi).

6. Transporterkonstrukt nach einem der Ansprüche 1 bis 5, wobei die generische Formel (I) oder irgendeine der Unterformeln (la), (Ib), (Ic), (Id), (le), oder (If) auf eine Variante oder ein Fragment irgendeiner der in den Ansprüchen 1 bis 4 aufgelisteten Transportsequenzen angewandt ist, die zumindest 80% oder 85%, vorzugsweise zumindest 90%, mehr bevorzugt zumindest 95% und am meisten bevorzugt zumindest 99% Sequenzidentität über die gesamte Länge mit irgendeiner der Transportsequenzen, die in den Ansprüchen 1 bis 5 aufgezählt sind, aufweisen.

7. Transporter-Fracht-Konjugat-Molekül, umfassend
a) eine Komponente (A), wobei die Komponente (A) comprises das Transporterkonstrukt nach einem der Ansprüche 1 bis 6 umfasst; und
b) eine Komponente (B), die ein Effektormolekül umfasst, insbesondere wobei das Effektormolekül ausgewählt ist aus therapeutisch aktiven Proteinen und Peptiden, Proteinkinaseinhibitoren, Inhibitoren der Proteinkinase c-Jun aminoterminale Kinase, Antigenen, Antikörpern, apoptotischen Faktoren, Proteasen, die in pathologischen Zuständen einbezogen sind, vorzugsweise peptidischen Proteaseinhibitoren, BH3-Domänen oder nur-BH3 Proteinen, Nukleinsäuren, die diese Proteine kodieren, siRNAs, antisense RNAs, zytotoxischen Agenzien, und kleinen organischen Stoffen einschließlich Proteaseinhibitoren.

8. Transporter-Fracht-Konjugat-Molekül nach Anspruch 7, weiterhin umfassend zumindest eine zusätzliche Komponente (C), (D) und/oder (E), die sich von Komponente (B) unterscheidet, wobei die zumindest eine optionale zusätzliche Komponente (C), (D) und/oder (E) unabhängig voneinander ausgewählt ist aus unterschiedlichen Effektormolekülen, wie für Komponente (B) definiert.

9. Transporter-Fracht-Konjugat-Molekül nach einem der Ansprüche 7 bis 8, wobei:
a) die Komponenten (A) und (B), und optional die Komponenten (C), (D) und/oder (E) kovalent miteinander verbunden sind, und/oder
b) wobei die zumindest eine zusätzliche Komponente (C), (D) und/oder (E) ausgewählt ist aus einer Signalsequenz oder einer Lokalisationssequenz, die das Transporter-Fracht-Konjugat-Molekül zu einer bestimmten intrazellulären Ziellokalisation oder zu einem bestimmten Zelltyp lenkt, und/oder
c) wobei die Komponente (B) und/oder die zumindest eine zusätzliche Komponente (C), (D) und/oder (E) eine Protein- oder Peptidsequenz ist (sind) und aus L-Aminosäuren, D-Aminosäuren oder einer Mischung von beidem besteht, und/oder
d) wobei die Komponente (A) am C-terminalen Ende des Transporter-Fracht-Konjugat-Moleküls angeordnet ist, wenn die Komponente (B) eine Protein- oder Peptidsequenz ist.

10. Transporter-Fracht-Konjugat-Molekül nach einem der Ansprüche 7 bis 9 zur Verwendung als Medikament.

11. Pharmazeutische Zusammensetzung umfassend ein Transporter-Fracht-Konjugat-Molekül nach einem der Ansprüche 7 bis 10 und optional einen pharmazeutisch akzeptablen Träger und/oder Vehikel.

12. Verwendung eines Transporterkonstrukts nach einem der Ansprüche 1 bis 6 zur Herstellung eines Transporter-Fracht-Konjugat-Moleküls, insbesondere wobei das Transporter-Fracht-Konjugat-Molekül wie in einem der Ansprüche 7 bis 9 definiert ist.

13. Verwendung eines Transporterkonstrukts nach einem der Ansprüche 1 bis 6 oder eines Transporter-Fracht-Konjugat-Molekül, wie in einem der Ansprüche 7 bis 9 definiert, zur Herstellung eines Arzneimittels zum Transport eines Frachtmoleküls in Zellen oder Gewebe eines zu behandelnden Patienten, insbesondere wobei die Fracht wie in einem der Ansprüche 7 bis 9 definiert ist.

14. Verwendung nach Anspruch 13 zum Transport eines Frachtmoleküls in weiße Blutzellen und/oder neuronale Zellen, insbesondere von einem zu behandelnden Patienten.

15. Verwendung eines Transporter-Fracht-Konjugat-Moleküls nach einem der Ansprüche 7 bis 9 zur Herstellung eines Medikaments zur Prophylaxe, Behandlung und/oder Verbesserung von Krebs- oder Tumorerkrankungen, einschließlich Krankheiten, die durch fehlerhafte Apoptose verursacht werden, Entzündungskrankheiten, infektiösen Erkrankungen, viralen (infektiösen) Erkrankungen, Erkrankungen mit Bezug zum JNK-Signalling, Autoimmunerkrankungen und -krankheiten, kardiovaskulären Erkrankungen, neuronalen oder neurodegenerativen Erkrankungen, Erkrankungen der Leber, Erkrankungen des Wirbelsäule, Erkrankungen des Uterus, depressiven Erkrankungen, nicht-chronischen oder chronischen entzündlichen Verdauungserkrankungen, Hörverlust, Krankheiten des Innenohrs, oder zur Verwendung bei Gewebetransplantation.

## Revendications

1. Construction de transporteur comprenant au moins un motif D-/L- ayant la formule générique (I) :
**DₗLLLₓDₘ(LLL_{y}Dₙ)ₐ**
dans laquelle :
D est un acide D-aminé ;
L est un acide L-aminé ;
a est 0-3 ;
1, m et n sont indépendamment les uns des autres 1 ou 2 ;
x et y sont indépendamment l'un de l'autre 0, 1 ou 2 ;
en particulier où la construction de transporteur comprend au moins un motif D-/L-selon l'une des sous-formules (la) à (If) suivantes :
(Ia): **DₗLLLₓDₘ ;**
(Ib) : **DₗLLLₓDₘLLL_{y}Dₙ ;**
(Ic) : **DₗLLLₓDₘLLL_{y}DₙLLL_{y}Dₙ ;**
(Id) : **DₗLLLₓDₘLLL_{y}DₙLLL_{y}DₙLLL_{y}Dₙ ;**
(Ie): **DLLLD(LLLD)ₐ,**
ou
(If) : **DLLLDLLLD,**
et où le motif D-/L- selon la formule générique (I) ou selon l'une quelconque des sous-formules (Ia), (Ib), (Ie), (Id), (Ie), ou (If), est appliqué à l'une des séquences suivantes :
| NOM DE LA SÉQUENCE/DU PEPTIDE | SEQ ID NO | AA | SÉQUENCE |
|---|---|---|---|
| TAT (1-86) | 8 | 86 | |
| TAT (37-72) | 9 | 36 | |
| TAT (37-58) | 10 | 22 | CFITKALGIS YGRKKRRQRR RP |
| TAT (38-58) GGC | 11 | 24 | FITKALGISY GRKKRRQRRR PGGC |
| TAT CGG (47-58) | 12 | 15 | CGGYGRKKRR QRRRP |
| TAT (47-58) GGC | 13 | 15 | YGRKKRRQRR RPGGC |
| TAT (1-72) Mut Cys/Ala 72 | 14 | 56 | |
| L-TAT-générique | 15 | 17 | XXXXRKKRRQRRRXXXX |
| | | | (NH₂- XXXXRKKRRQRRRXXXX -COOH) |
| L-TAT-générique (s) | 16 | 11 | NH₂-Xₙ^{b}-RKKRRQRRR-Xₙ^{b}-COOH |
| L-TAT (s1a) | 17 | 10 | GRKKRRQRRR |
| | | | (NH₂-GRKKRRQRRR-COOH) |
| L-TAT (s1b) | 18 | 9 | RKKRRQRRR |
| | | | (NH₂-GRKKRRQRRR-COOH) |
| L-TAT (s1c) | 19 | 11 | YDRKKRRQRRR |
| R₃-L-TAT | 20 | 9 | rKKRrQRRr |
| R₃-L-TATi | 21 | 9 | rRRQrRKKr |
| βA-r₃-L-TAT | 22 | 9 | βA-rKKRrQRRr |
| βA-r₃-L-TATi | 23 | 9 | βA-rRRQrRKKr |
| FITC-βA-r₃-L-TAT | 24 | 9 | FITC-βA-rKKRrQRRr |
| FITC-βA-r₃-L-TATi | 25 | 9 | FTTC-βA-rRRQrRKKr |
| TAT (s2-1) | 26 | 9 | rAKRrQRRr |
| TAT (s2-2) | 27 | 9 | rKARrQRRr |
| TAT (s2-3) | 28 | 9 | rKKArQRRr |
| TAT (s2-4) | 29 | 9 | rKKRrARRr |
| TAT (s2-5) | 30 | 9 | rKKRrQARr |
| TAT (s2-6) | 31 | 9 | rKKRrQRAr |
| TAT (s2-7) | 32 | 9 | rDKRrQRRr |
| TAT (s2-8) | 33 | 9 | rKDRrQRRr |
| TAT (s2-9) | 34 | 9 | rKKDrQRRr |
| TAT (s2-10) | 35 | 9 | rKKRrDRRr |
| TAT (s2-11) | 36 | 9 | rKKRrQDRr |
| TAT (s2-12) | 37 | 9 | rKKRrQRDr |
| TAT (s2-13) | 38 | 9 | rEKRrQRRr |
| TAT (s2-14) | 39 | 9 | rKERrQRRr |
| TAT (s2-15) | 40 | 9 | rKKErQRRr |
| TAT (s2-16) | 41 | 9 | rKKRrERRr |
| TAT (s2-17) | 42 | 9 | rKKRrQERr |
| TAT (s2-18) | 43 | 9 | rKKRrQREr |
| TAT (s2-19) | 44 | 9 | rFKRrQRRr |
| TAT (s2-20) | 45 | 9 | rKFRrQRRr |
| TAT (s2-21) | 46 | 9 | rKKFrQRRr |
| TAT (s2-22) | 47 | 9 | rKKRrFRRr |
| TAT (s2-23) | 48 | 9 | rKKRrQFRr |
| TAT (s2-24) | 49 | 9 | rKKRrQRFr |
| TAT (s2-25) | 50 | 9 | rRKRrQRRr |
| TAT (s2-26) | 51 | 9 | rKRRrQRRr |
| TAT (s2-27) | 52 | 9 | rKKKrQRRr |
| TAT (s2-28) | 53 | 9 | rKKRrRRRr |
| TAT (s2-29) | 54 | 9 | rKKRrQKRr |
| TAT (s2-30) | 55 | 9 | rKKRrQRKr |
| TAT (s2-31) | 56 | 9 | rHKRrQRRr |
| TAT (s2-32) | 57 | 9 | rKHRrQRRr |
| TAT (s2-33) | 58 | 9 | rKKHrQRRr |
| TAT (s2-34) | 59 | 9 | rKKRrHRRr |
| TAT (s2-35) | 60 | 9 | rKKRrQHRr |
| TAT (s2-36) | 61 | 9 | rKKRrQRHr |
| TAT (s2-37) | 62 | 9 | rIKRrQRRr |
| TAT (s2-38) | 63 | 9 | rKIRrQRRr |
| TAT (s2-39) | 64 | 9 | rKKIrQRRr |
| TAT (s2-40) | 65 | 9 | rKKRrIRRr |
| TAT (s2-41) | 66 | 9 | rKKRrQI Rr |
| TAT (s2-42) | 67 | 9 | rKKRrQRIr |
| TAT (s2-43) | 68 | 9 | rLKRrQRRr |
| TAT (s2-44) | 69 | 9 | rKLRrQRRr |
| TAT (s2-45) | 70 | 9 | rKKLrQRRr |
| TAT (s2-46) | 71 | 9 | rKKRrLRRr |
| TAT (s2-47) | 72 | 9 | rKKRrQLRr |
| TAT (s2-48) | 73 | 9 | rKKRrQRLr |
| TAT (s2-49) | 74 | 9 | rMKRrQRRr |
| TAT (s2-50) | 75 | 9 | rKMRrQRRr |
| TAT (s2-51) | 76 | 9 | rKKMrQRRr |
| TAT (s2-52) | 77 | 9 | rKKRrMRRr |
| TAT (s2-53) | 78 | 9 | rKKRrQMRr |
| TAT (s2-54) | 79 | 9 | rKKRrQRMr |
| TAT (s2-55) | 80 | 9 | rNKRrQRRr |
| TAT (s2-56) | 81 | 9 | rKNRrQRRr |
| TAT (s2-57) | 82 | 9 | rKKNrQRRr |
| TAT (s2-58) | 83 | 9 | rKKRrNRRr |
| TAT (s2-59) | 84 | 9 | rKKRrQNRr |
| TAT (s2-60) | 85 | 9 | rKKRrQRNr |
| TAT (s2-61) | 86 | 9 | rQKRrQRRr. |
| TAT (s2-62) | 87 | 9 | rKQRrQRRr |
| TAT (s2-63) | 88 | 9 | rKKQrQRRr |
| TAT (s2-64) | 89 | 9 | rKKRrKRRr |
| TAT (s2-65) | 90 | 9 | rKKRrQQRr |
| TAT (s2-66) | 91 | 9 | rKKRrQRQr |
| TAT (s2-67) | 92 | 9 | rSKRrQRRr |
| TAT (s2-68) | 93 | 9 | rKSRrQRRr |
| TAT (s2-69) | 94 | 9 | rKKSrQRRr |
| TAT (s2-70) | 95 | 9 | rKKRrSRRr |
| TAT (s2-71) | 96 | 9 | rKKRrQSRr |
| TAT (s2-72) | 97 | 9 | rKKRrQRSr |
| TAT (s2-73) | 98 | 9 | rTKRrQRRr |
| TAT (s2-74) | 99 | 9 | rKTRrQRRr |
| TAT (s2-75) | 100 | 9 | rKKTrQRRr |
| TAT (s2-76) | 101 | 9 | rKKRrTRRr |
| TAT (s2-77) | 102 | 9 | rKKRrQTRr |
| TAT (s2-78) | 103 | 9 | rKKRrQRTr |
| TAT (s2-79) | 104 | 9 | rVKRrQRRr |
| TAT (s2-80) | 105 | 9 | rKVRrQRRr |
| TAT (s2-81) | 106 | 9 | rKKVrQ.RRr |
| TAT (s2-82) | 107 | 9 | rKKRrVRRr |
| TAT (s2-83) | 108 | 9 | rKKRrQVRr |
| TAT (s2-84) | 109 | 9 | rKKRrQRVr |
| TAT (s2-85) | 110 | 9 | rWKRrQRRr |
| TAT (s2-86) | 111 | 9 | rKWRrQRRr |
| TAT (s2-87) | 112 | 9 | rKKWrQRRr |
| TAT (s2-88) | 113 | 9 | rKKRrWRRr |
| TAT (s2-89) | 114 | 9 | rKKRrQWRr |
| TAT (s2-90) | 115 | 9 | rKKRrQRWr |
| TAT (s2-91) | 116 | 9 | rYKRrQRRr |
| TAT (s2-92) | 117 | 9 | rKYRrQRRr |
| TAT (s2-93) | 118 | 9 | rKKYrQRRr |
| TAT (s2-94) | 119 | 9 | rKKRrYRRr |
| TAT (s2-95) | 120 | 9 | rKKRrQYRr |
| TAT (s2-96) | 121 | 9 | rKKRrQRYr |
| r₃R₆ | 122 | 9 | rRRRrRRRr |
| L-R₉ | 123 | 9 | RRRRRRRRR |
| L-R₈ | 124 | 8 | RRRRRRRR |
| L-R₇ | 125 | 7 | RRRRRRR |
| L-R₆ | 126 | 6 | RRRRRR |
| L-R₅ | 127 | 5 | RRRRR |
| PTD-4 | 128 | 11 | YARAAARQARA |
| PTD-4 (variant 1) | 129 | 11 | WARAAARQARA |
| PTD-4 (variant 2) | 130 | 11 | WARAQRAAARA |
| Pénétratine L-P1 | 131 | 16 | RQVKVWFQNRRMKWKK |
| Pénétratine D-P1 | 132 | 16 | KKWKMRRNQFWVKVQR |
| JNKI, bestfit | 133 | 17 | WKRAAARKARAMSLNLF |
| JNKI, bestfit (variant 1) | 134 | 17 | WKRAAARAARAMSLNLF |
| Séquence de transcytose de MDCK | 135 | 9 | RYRGDLGRR |
| YKGL | 136 | 4 | YKGL |
| r₃ (générique) | 252 | 9 | rXXXrXXXr |
ou une séquence inverse de celles-ci, ou un variant ou fragment de ces séquences, où un variant ou fragment de ces séquences conserve la fonction de permettre une translocation à travers des membranes biologiques et consiste en une séquence peptidique présentant au moins 80 % d'identité de séquence sur toute la longueur par rapport à la séquence native respective.

2. Construction de transporteur selon la revendication 1, où la construction de transporteur comprend au moins une séquence selon rXXXrXXXr, dans laquelle :
r représente un énantiomère D de l'arginine ;
X est un quelconque acide L-aminé ;
et dans laquelle chaque X peut être sélectionné individuellement et indépendamment d'un quelconque autre X dans rXXXrXXXr, en particulier, dans laquelle au moins 4 desdits 6 acides L-aminés X sont individuellement sélectionnés dans le groupe des acides L-aminés consistant en K ou R.

3. Construction de transporteur selon l'une quelconque des revendications 1 à 2, où la construction de transporteur comprend une séquence sélectionnée dans le groupe de séquences :
| NOM DE LA SÉQUENCE/DU PEPTIDE | SEQ ID NO | AA | SÉQUENCE |
|---|---|---|---|
| R₃-L-TAT | 20 | 9 | rKKRrQRRr |
| R₃-L-TATi | 21 | 9 | rRRQrRKKr |
| βA-r₃-L-TAT | 22 | 9 | βA-rKKRrQRFr |
| βA-r3-L-TATi | 23 | 9 | βA-rRRQrRKKr |
| FITC-βA-r₃-L-TAT | 24 | 9 | FITC-βA-rKKRrQRRr |
| FITC-βA-r₃-L-TATi | 25 | 9 | FITC-βA-rRRQrRKKr |
| TAT (s2-1) | 26 | 9 | rAKRrQRRr |
| TAT (s2-2) | 27 | 9 | rKARrQRRr |
| TAT (s2-3) | 28 | 9 | rKKArQRRr |
| TAT (s2-4) | 29 | 9 | rKKRrARRr |
| TAT (s2-5) | 30 | 9 | rKKRrQARr |
| TAT (s2-6) | 31 | 9 | rKKRrQRAr |
| TAT (s2-7) | 32 | 9 | rDKRrQRRr |
| TAT (s2-8) | 33 | 9 | rKDRrQRRr |
| TAT (s2-9) | 34 | 9 | rKKDrQRRr |
| TAT (s2-10) | 35 | 9 | rKKRrDRRr |
| TAT (s2-11) | 36 | 9 | rKKRrQDRr |
| TAT (s2-12) | 37 | 9 | rKKRrQRDr |
| TAT (s2-13) | 38 | 9 | rEKRrQRRr |
| TAT (s2-14) | 39 | 9 | rKERrQRRr |
| TAT (s2-15) | 40 | 9 | rKKErQRRr |
| TAT (s2-16) | 41 | 9 | rKKRrERRr |
| TAT (s2-17) | 42 | 9 | rKKRrQERr |
| TAT (s2-18) | 43 | 9 | rKKRrQREr |
| TAT (s2-19) | 44 | 9 | rFKRrQRRr |
| TAT (s2-20) | 45 | 9 | rKFRrQRRr |
| TAT (s2-21) | 46 | 9 | rKKFrQRRr |
| TAT (s2-22) | 47 | 9 | rKKRrFRRr |
| TAT (s2-23) | 48 | 9 | rKKRrQFRr |
| TAT (s2-24) | 49 | 9 | rKKRrQRFr |
| TAT (s2-25) | 50 | 9 | rRKRrQRRr |
| TAT (s2-26) | 51 | 9 | rKRRrQRRr |
| TAT (s2-27) | 52 | 9 | rKKKrQRRr |
| TAT (s2-28) | 53 | 9 | rKKRrRRRr |
| TAT (s2-29) | 54 | 9 | rKKRrQKRr |
| TAT (s2-30) | 55 | 9 | rKKRrQRKr |
| TAT (s2-31) | 56 | 9 | rHKRrQRRr |
| TAT (s2-32) | 57 | 9 | rKHRrQRRr |
| TAT (s2-33) | 58 | 9 | rKKHrQRRr |
| TAT (s2-34) | 59 | 9 | rKKRrHRRr |
| TAT (s2-35) | 60 | 9 | rKKRrQHRr |
| TAT (s2-36) | 61 | 9 | rKKRrQRHr. |
| TAT (s2-37) | 62 | 9 | rIKRrQRRr |
| TAT (s2-38) | 63 | 9 | rKIRrQRRr |
| TAT (s2-39) | 64 | 9 | rKKIrQRRr |
| TAT (s2-40) | 65 | 9 | rKKRrIRRr |
| TAT (s2-41) | 66 | 9 | rKKRrQIRr |
| TAT (s2-42) | 67 | 9 | rKKRrQRIr |
| TAT (s2-43) | 68 | 9 | rLKRrQRRr |
| TAT (s2-44) | 69 | 9 | rKLRrQRRr |
| TAT (s2-45) | 70 | 9 | rKKLrQRRr |
| TAT (s2-46) | 71 | 9 | rKKRrLRRr |
| TAT (s2-47) | 72 | 9 | rKKRrQLRr |
| TAT (s2-48) | 73 | 9 | rKKRrQRLr |
| TAT (s2-49) | 74 | 9 | rMKRrQRRr |
| TAT (s2-50) | 75 | 9 | rKMRrQRRr |
| TAT (s2-51) | 76 | 9 | rKKMrQRRr |
| TAT (s2-52) | 77 | 9 | rKKRrMRRr |
| TAT (s2-53) | 78 | 9 | rKKRrQMRr |
| TAT (s2-54) | 79 | 9 | rKKRrQRMr |
| TAT (s2-55) | 80 | 9 | rNKRrQRRr |
| TAT (s2-56) | 81 | 9 | rKNRrQRRr |
| TAT (s2-57) | 82 | 9 | rKKNrQRRr |
| TAT (s2-58) | 83 | 9 | rKKRrNRRr |
| TAT (s2-59) | 84 | 9 | rKKRrQNRr |
| TAT (s2-60) | 85 | 9 | rKKRrQRNr |
| TAT (s2-61) | 86 | 9 | rQKRrQRRr |
| TAT (s2-62) | 87 | 9 | rKQRrQRRr |
| TAT (s2-63) | 88 | 9 | rKKQrQRRr |
| TAT (s2-64) | 89 | 9 | rKKRrKRRr |
| TAT (s2-65) | 90 | 9 | rKKRrQQRr |
| TAT (s2-66) | 91 | 9 | rKKRrQRQr |
| TAT (s2-67) | 92 | 9 | rSKRrQRRr |
| TAT (s2-68) | 93 | 9 | rKSRrQRRr |
| TAT (s2-69) | 94 | 9 | rKKSrQRRr |
| TAT (s2-70) | 95 | 9 | rKKRrSRRr |
| TAT (s2-71) | 96 | 9 | rKKRrQSRr |
| TAT (s2-72) | 97 | 9 | rKKRrQRSr |
| TAT (s2-73) | 98 | 9 | rTKRrQRRr |
| TAT (s2-74) | 99 | 9 | rKTRrQRRr |
| TAT (s2-75) | 100 | 9 | rKKTrQRRr |
| TAT (s2-76) | 101 | 9 | rKKRrTRRr |
| TAT (s2-77) | 102 | 9 | rKKRrQTRr |
| TAT (s2-78) | 103 | 9 | rKKRrQRTr |
| TAT (s2-79) | 104 | 9 | rVKRrQRRr |
| TAT (s2-80) | 105 | 9 | rKVRrQRRr |
| TAT (s2-81) | 106 | 9 | rKKVrQRRr |
| TAT (s2-82) | 107 | 9 | rKKRrVRRr |
| TAT (s2-83) | 108 | 9 | rKKRrQVRr |
| TAT (s2-84) | 109 | 9 | rKKRrQRVr |
| TAT (s2-85) | 110 | 9 | rWKRrQRRr |
| TAT (s2-86) | 111 | 9 | rKWRrQRRr |
| TAT (s2-87) | 112 | 9 | rKKWrQRRr |
| TAT (s2-88) | 113 | 9 | rKKRrWRRr |
| TAT (s2-89) | 114 | 9 | rKKRrQWRr |
| TAT (s2-90) | 115 | 9 | rKKRrQRWr |
| TAT (s2-91) | 116 | 9 | rYKRrQRRr |
| TAT (s2-92) | 117 | 9 | rKYRrQRRr |
| TAT (s2-93) | 118 | 9 | rKKYrQRRr |
| TAT (s2-94) | 119 | 9 | rKKRrYRRr |
| TAT (s2-9 5) | 120 | 9 | rKKRrQYRr |
| TAT (s2-96) | 121 | 9 | rKKRrQRYr |
| r₃R₆ | 122 | 9 | rRRRrRRRr |
ou un variant ou fragment de ces séquences, où un variant ou fragment de ces séquences conserve la fonction de permettre une translocation à travers des membranes biologiques et consiste en une séquence peptidique présentant au moins 80 % d'identité de séquence sur toute la longueur par rapport à la séquence de trafic native respective.

4. Construction de transporteur selon l'une quelconque des revendications 1 à 3, où la construction de transporteur comprend la sous-formule (If) **DLLLDLLLD** et où la sous-formule (If) est appliquée à une séquence de trafic dérivée de la protéine TAT du VIH-1 comportant une tyrosine (Y) à la position 2 de la séquence dérivée de TAT, la séquence étant de préférence sélectionnée à partir de la séquence rYKRrQRRr (TAT(s2-91)).

5. Construction de transporteur selon la revendication 3, où la construction de transporteur comprend une séquence sélectionnée dans le groupe consistant en rKKRrQRRr (r₃-L-TAT) et rRRQrRKKr (r₃-L-TATi).

6. Construction de transporteur selon l'une quelconque des revendications 1 à 5, où la formule générique (I) ou l'une quelconque des sous-formules (Ia), (Ib), (Ic), (Id), (Ie), ou (If), est appliquée à un variant ou à un fragment de l'une quelconque des séquences de trafic listées dans les revendications 1 à 4, présentant au moins 80 % ou 85 %, de préférence au moins 90 %, de manière davantage préférée au moins 95 % et de la manière la plus préférée entre toutes au moins 99 % d'identité de séquence sur toute la longueur de l'une quelconque des séquences de trafic listées dans les revendications 1 à 5.

7. Molécule conjuguée cargo-transporteur, comprenant :
a) un composant (A), où le composant (A) comprend la construction de transporteur selon l'une quelconque des revendications 1 à 6 ; et
b) un composant (B), comprenant une molécule effectrice, en particulier où la molécule effectrice est sélectionnée parmi des protéines et des peptides thérapeutiquement actifs, des inhibiteurs de protéines kinases, des inhibiteurs de la protéine kinase c-Jun amino-terminale kinase, des antigènes, des anticorps, des facteurs apoptotiques, des protéases impliquées dans des états pathologiques, de préférence des inhibiteurs de protéases peptidiques, des domaines BH3 ou des protéines à BH3-seulement, des acides nucléiques codant pour ces protéines, des ARNsi, des ARN antisens, des agents cytotoxiques, et des petits composés organiques comprenant des inhibiteurs de protéases.

8. Molécule conjuguée cargo-transporteur selon la revendication 7, comprenant en outre au moins un composant (C), (D) et/ou (E) supplémentaire qui est différent du composant (B), où le au moins un composant (C), (D) et/ou (E) supplémentaire facultatif est sélectionné, indépendamment des autres, à partir de différentes molécules effectrices telles que définies pour le composant (B).

9. Molécule conjuguée cargo-transporteur selon l'une quelconque des revendications 7 à 8, dans laquelle :
a) les composants (A) et (B), et les composants (C), (D) et/ou (E) facultatifs sont liés les uns aux autres de façon covalente, et/ou
b) dans laquelle le au moins un composant (C), (D) et/ou (E) supplémentaire est sélectionné parmi une séquence signal ou une séquence de localisation, qui dirige la molécule conjuguée cargo-transporteur vers une localisation cible intracellulaire particulière ou vers un type cellulaire particulier, et/ou
c) dans laquelle le composant (B) et/ou le au moins un composant (C), (D) et/ou (E) supplémentaire est (sont) une séquence protéique ou peptidique et est (sont) composé(s) d'acides L-aminés, d'acides D-aminés ou d'un mélange des deux, et/ou
d) dans laquelle le composant (A) est positionné à l'extrémité C-terminale de la molécule conjuguée cargo-transporteur, à condition que le composant (B) soit une séquence protéique ou peptidique.

10. Molécule conjuguée cargo-transporteur selon l'une quelconque des revendications 7 à 9 destinée à être utilisée en tant que médicament.

11. Composition pharmaceutique comprenant une molécule conjuguée cargo-transporteur selon l'une quelconque des revendications 7 à 10 et facultativement un support et/ou véhicule pharmaceutiquement acceptable.

12. Utilisation d'une construction de transporteur selon l'une quelconque des revendications 1 à 6 pour la préparation d'une molécule conjuguée cargo-transporteur, en particulier où la molécule conjuguée cargo-transporteur est telle que définie selon l'une quelconque des revendications 7 à 9.

13. Utilisation d'une construction de transporteur selon l'une quelconque des revendications 1 à 6 ou d'une molécule conjuguée cargo-transporteur telle que définie selon l'une quelconque des revendications 7 à 9 pour la préparation d'un médicament destiné au transport d'une molécule cargo dans les cellules ou un tissu d'un patient devant être traité, en particulier où le cargo est tel que défini selon l'une quelconque des revendications 7 à 9.

14. Utilisation selon la revendication 13 pour le transport d'une molécule cargo dans des globules blancs et/ou des cellules neuronales, en particulier d'un patient devant être traité.

15. Utilisation d'une molécule conjuguée cargo-transporteur selon l'une quelconque des revendications 7 à 9 pour la préparation d'un médicament destiné à la prophylaxie, au traitement et/ou à l'amélioration du cancer ou de maladies tumorales, comprenant des maladies provoquées par une apoptose déficiente, des maladies inflammatoires, des maladies infectieuses, des maladies (infectieuses) virales, des maladies fortement associées à la signalisation de JNK, des troubles ou des maladies auto-immun(e)s, des maladies cardiovasculaires, des maladies neuronales ou neurodégénératives, des maladies du foie, des maladie du rachis, des maladies de l'utérus, des troubles dépressifs majeurs, des maladies digestives inflammatoires non chroniques ou chroniques, la perte d'audition, des maladies de l'oreille interne, ou pour une utilisation dans une transplantation de tissu.
